(19) Europäisches Patentamt
European Patent Office
Office européen des brevets

(11) **EP 3 269 385 A1**

(12) **EUROPEAN PATENT APPLICATION**


(43) Date of publication:
**17.01.2018 Bulletin 2018/03**

(51) Int Cl.:
***A61K 39/09*** *(2006.01)* ***A61K 31/7028*** *(2006.01)*

(21) Application number: **16179133.0**

(22) Date of filing: **12.07.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**



(71) Applicants:
- **Max-Planck-Gesellschaft zur Förderung der Wissenschaften e.V.**
**80539 München (DE)**
- **Vaxxilon AG**
**4153 Reinach (CH)**

(72) Inventor: **SEEBERGER, Peter H.**
**14532 Kleinmachnow (DE)**

(74) Representative: **Arth, Hans-Lothar**
**ABK Patent Attorneys**
**Jasminweg 9**
**14052 Berlin (DE)**


Remarks:
The references to the drawing(s) no.3 and 4 are deemed to be deleted.

(54) **PNEUMOCOCCAL POLYSACCHARIDE-PROTEIN CONJUGATE COMPOSITION**


(57) The present invention relates to immunogenic compositions comprising a conjugate of a saccharide from *Streptococcus pneumoniae* serotype 8 and a carrier protein, and a mixture consisting of capsular polysaccharides from *Streptococcus pneumoniae* serotypes 1, 3, 4, 5, 6A, 6B, 7F, 9V, 14, 18C, 19A, 19F and 23F individually conjugated to $CRM_{197}$ carrier protein, or a mixture consisting of capsular polysaccharides from *Streptococcus pneumoniae* serotypes 1, 4, 5, 6B, 7F, 9V, 14, 18C, 19F and 23F individually conjugated to a carrier protein, wherein the capsular polysaccharides from *Streptococcus pneumoniae* serotypes 1, 4, 5, 6B, 7F, 9V, 14, and 23F are individually conjugated to protein D, the capsular polysaccharide from *Streptococcus pneumoniae* serotype 18C is conjugated to tetanus toxoid and the capsular polysaccharide from *Streptococcus pneumoniae* serotype 19F is conjugated to diphtheria toxoid. Said compositions are useful for the prevention and/or treatment of diseases caused by *Streptococcus pneumoniae* serotype 8.


**Figure 2**

**B**

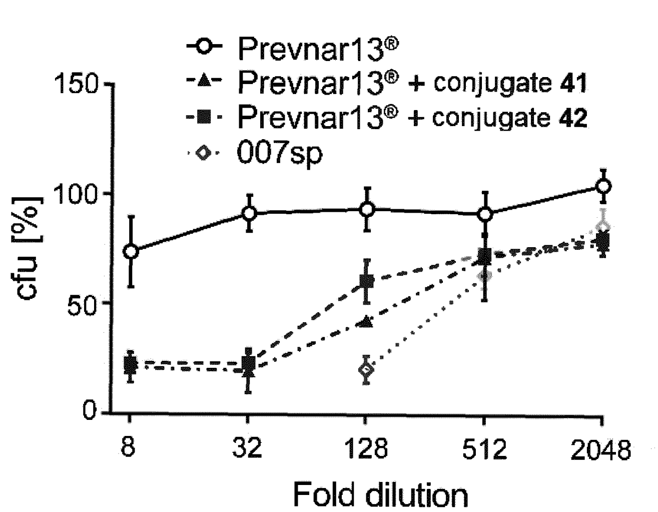


EP 3 269 385 A1

Printed by Jouve, 75001 PARIS (FR)

## Description

### Field of the invention

The present invention relates to immunogenic compositions comprising a conjugate of a saccharide from *Streptococcus pneumoniae* serotype 8 and a carrier protein, and a mixture consisting of capsular polysaccharides from *Streptococcus pneumoniae* serotypes 1, 3, 4, 5, 6A, 6B, 7F, 9V, 14, 18C, 19A, 19F and 23F individually conjugated to $CRM_{197}$ carrier protein, or a mixture consisting of capsular polysaccharides from *Streptococcus pneumoniae* serotypes 1, 4, 5, 6B, 7F, 9V, 14, 18C, 19F and 23F individually conjugated to a carrier protein, wherein the capsular polysaccharides from *Streptococcus pneumoniae* serotypes 1, 4, 5, 6B, 7F, 9V, 14, and 23F are individually conjugated to protein D, the capsular polysaccharide from *Streptococcus pneumoniae* serotype 18C is conjugated to tetanus toxoid and the capsular polysaccharide from *Streptococcus pneumoniae* serotype 19F is conjugated to diphtheria toxoid. Said compositions are useful for the prevention and/or treatment of diseases caused by *Streptococcus pneumoniae* serotype 8.

### Background of the invention

*Streptococcus pneumoniae (S. pneumoniae*) commonly known as pneumococci or diplococci is a human pathogenic Gram positive bacterium encapsulated with capsular polysaccharide. Based on the chemical nature of the polysaccharide capsule, pneumococci have been classified into more than 90 serotypes. Pneumococcus is a commensal bacterium that asymptomatically colonizes in the upper respiratory tract of human and is responsible for causing pneumonia, septicemia, meningitis and otitis media. Pneumococci is the most common cause of vaccine-preventable deaths in children aged <5 years and elderly peoples worldwide. Global estimates suggest that 18% of all deaths in children less than 5 years of age occur due to pneumonia.

Capsular polysaccharide is one of the major virulence factors responsible for pneumococcal pathogenesis. The spectrum of prevailing capsular types varies with age, time and geographical region, although common serotypes are consistently identified throughout the world. Globally, about 20 serotypes are associated with > 80% of invasive pneumococcal disease occurring in all age groups; the 13 most common serotypes cause at least 70-75% of invasive disease in children. Pneumococcal vaccines that are currently available are capsular polysaccharide based and designed to cover the serotypes most frequently associated with invasive pneumococcal disease.

The available 23-valent polysaccharide vaccine (23-PPV) is not effective in children less than 2 years of age, while the 7-valent conjugate vaccines (7-PCV) is effective in children, but has limited serotype coverage.

To increase the serotype coverage, 10-valent conjugate vaccine containing the conjugates of the capsular polysaccharides from *S. pneumoniae* type 1, 4, 5, 6B, 7F, 9V, 14, 18C, 19F and 23F and protein D (a non-typeable *Haemophilus influenzae* protein), tetanus toxoid and diphtheria toxoid protein, and 13-valent conjugate vaccine containing the conjugates of capsular polysaccharides from S. *pneumoniae* type 1, 3, 4, 5, 6A, 6B, 7F, 9V, 14, 18C, 19A, 19F, and 23F and diphtheria $CRM_{197}$ protein have been licensed for use.

It is the objective of the present invention to provide an improved immunogenic composition comprising a conjugate of a saccharide from *Streptococcus pneumoniae* serotype 8 and a carrier protein, such as a conjugate of general formula (**I**), and a mixture consisting of capsular polysaccharides from *Streptococcus pneumoniae* serotypes 1, 3, 4, 5, 6A, 6B, 7F, 9V, 14, 18C, 19A, 19F and 23F individually conjugated to $CRM_{197}$ carrier protein, or a mixture consisting of capsular polysaccharides from *Streptococcus pneumoniae* serotypes 1, 4, 5, 6B, 7F, 9V, 14, 18C, 19F and 23F individually conjugated to a carrier protein, wherein the capsular polysaccharides from *Streptococcus pneumoniae* serotypes 1, 4, 5, 6B, 7F, 9V, 14, and 23F are individually conjugated to protein D, the capsular polysaccharide from *Streptococcus pneumoniae* serotype 18C is conjugated to tetanus toxoid and the capsular polysaccharide from *Streptococcus pneumoniae* serotype 19F is conjugated to diphtheria toxoid. Immunization with the immunogenic compositions disclosed herein leads to elevated serum IgG titer and functional antibody activity. Therefore, the immunogenic compositions disclosed herein are useful for the treatment and prevention of diseases caused by *Streptococcus pneumoniae*, including *Streptococcus pneumoniae* serotype 8 in infants, children and adults.

The objective of the present invention is solved by the teaching of the independent claims. Further advantageous features, aspects and details of the invention are evident from the dependent claims, the description, the figures, and the examples of the present application.

### Description of the invention

Surprisingly, it was found that immunization with a composition comprising a conjugate of a saccharide from *S. pneumoniae* serotype 8 and a carrier protein and a mixture consisting of capsular polysaccharides from *S. pneumoniae* serotypes 1, 3, 4, 5, 6A, 6B, 7F, 9V, 14, 18C, 19A, 19F and 23F individually conjugated to $CRM_{197}$ carrier protein, induces a robust immune response against the capsular polysaccharide from *Streptococcus pneumoniae* serotype 8

(see for e.g. **Figure 1A** and **2A)** leading to opsonophagocytic killing of *Streptococcus pneumoniae* serotype 8 pneumococci (see for e.g. **Figure 2B),** without impairing the immune response against *Streptococcus pneumoniae* serotypes 1, 3, 4, 5, 6A, 6B, 7F, 9V, 14, 18C, 19A, 19F and 23F. The inventors have furthermore found that immunization with a composition comprising a conjugate of a saccharide from S. *pneumoniae* serotype 8 and a carrier protein, and a mixture consisting of capsular polysaccharides from *S. pneumoniae* serotypes 1, 4, 5, 6B, 7F, 9V, 14, 18C, 19F and 23F individually conjugated to a carrier protein, wherein the capsular polysaccharides from *S. pneumoniae* serotypes 1, 4, 5, 6B, 7F, 9V, 14, and 23F are individually conjugated to protein D, the capsular polysaccharide from *S. pneumoniae* serotype 18C is conjugated to tetanus toxoid and the capsular polysaccharide from *S. pneumoniae* serotype 19F is conjugated to diphtheria toxoid, induces also a robust immune response against the capsular polysaccharide from *Streptococcus pneumoniae* serotype 8 without impairing the immune response against *Streptococcus pneumoniae* serotypes 1, 4, 5, 6B, 7F, 9V, 14, 18C, 19F and 23F.

Thus, the present invention relates to an improved immunogenic composition comprising a conjugate of a saccharide from *S. pneumoniae* serotype 8 and a carrier protein and a mixture consisting of capsular polysaccharides from *S. pneumoniae* serotypes 1, 3, 4, 5, 6A, 6B, 7F, 9V, 14, 18C, 19A, 19F and 23F individually conjugated to $CRM_{197}$ carrier protein; or a mixture consisting of capsular polysaccharides from *S. pneumoniae* serotypes 1, 4, 5, 6B, 7F, 9V, 14, 18C, 19F and 23F individually conjugated to a carrier protein, wherein the capsular polysaccharides from *S. pneumoniae* serotypes 1, 4, 5, 6B, 7F, 9V, 14, and 23F are individually conjugated to protein D, the capsular polysaccharide from *S. pneumoniae* serotype 18C is conjugated to tetanus toxoid and the capsular polysaccharide from *S. pneumoniae* serotype 19F is conjugated to diphtheria toxoid.

As used herein the term **"a conjugate of a saccharide from *S. pneumoniae* serotype 8 and a carrier protein"** encompasses an isolated capsular polysaccharide from *S. pneumoniae* serotype 8 covalently coupled to a carrier protein, an isolated oligosaccharide from *S. pneumoniae* serotype 8 covalently coupled to a carrier protein and a conjugate of general (**I**).

The capsular polysaccharide from *S. pneumoniae* serotype 8 can be purchased from American Type Culture Collection (ATCC) or prepared by standard techniques known to those skilled in the art. For example, the capsular polysaccharide may be grown in a soy based medium and then purified through centrifugation, precipitation, ultra-filtration and column chromatography. Following activation, the capsular polysaccharide can be reacted with the carrier protein. In order to reduce viscosity of the polysaccharide or to improve filterability for the corresponding conjugate, the capsular polysaccharide may be sized to some degree by known methods (see for example EP497524 and EP497525) and preferably by microfluidization prior to its activation.

An isolated oligosaccharide from *S. pneumoniae* serotype 8 has typically a number of 3 - 10 repeating units and can be prepared by treatment of the capsular polysaccharide with appropriate chemicals or enzymes. These chemicals include, but are not limited to trifluoroacetic acid, acetic acid, fluorhydric acid, chlorhydric acid, sodium hydroxide and sodium acetate. Different time periods and temperatures may be used depending on the particular chemistry and concentration and on the resulting oligosaccharide desired. Commercially available enzymes, such as cellulase and ß-galactosidases, or isolated bacteriophage-associated endoglycans known in the art or prepared by known methods can also be used to prepare oligosaccharide from capsular polysaccharide.

The term **"carrier protein"** as used herein refers to a protein that is preferably non-toxic and non-reactogenic and obtainable in sufficient amount and purity. A carrier protein is amenable to standard conjugation procedures. Carrier proteins which may be used in the present invention include inactivated bacterial toxins such as tetanus toxoid (TT), pertussis toxoid (PT), cholera toxoid (CT), *E. coli* LT, *E. coli* ST, and exotoxin A from *Pseudomonas aeruginosa*. Bacterial outer membrane proteins such as outer membrane complex c (OMPC), porins, transferrin binding proteins, pneumolysin, pneumococcal surface protein A (PspA), pneumococcal adhesin protein (PsaA), C5a peptidase from Group A or Group B streptococcus, *Haemophilus influenzae* protein D (PD), or a member of the polyhistidine triad family (Pht) proteins, fragments or fusion proteins thereof can also be used. Other proteins, such as ovalbumin, keyhole limpet hemocyanin (KLH), bovine serum albumin (BSA) or purified protein derivative of tuberculin (PPD) can also be used as carrier proteins.

The term **"protein D"** as used herein refers to the *Haemophilus influenzae* protein D (PD). Protein D is surface lipoprotein having a molecular weight of about 42 kDa and which can be found in all *Haemophilus influenzae* (EP 0594610 B1). It is further potentially immunogenic. Protein D is commercially available or can be recombinantly prepared from bacterial expression systems such as genetically modified E. coli cells (EP 0594610 B1). Protein D may be used as a full length protein or as a fragment.

The term **"tetanus toxoid"** as used herein refers to the nontoxic toxoid of the tetanus toxin, which can be isolated from *Clostridium tetani*. The tetanus toxin is made nontoxic by treatment with formaldehyde. It has a molecular weight of 150 kDa and consists of two polypeptide chains. Tetanus toxoid is used as carrier in vaccines, especially in HiB conjugate vaccines.

The term **"diphtheria toxoid"** as used herein refers to the nontoxic toxoid of the diphtheria toxin, which is released extracellularly from *Cornyebacterium diphtheria.* The diphtheria toxin is also made nontoxic by treatment with formaldehyde. It consists of a single polypeptide chain of 62 kDa and may also be used as carrier protein.

The diphtheria toxoid **$CRM_{197}$** which is a non-toxic variant (i.e. toxoid) of diphteria toxin isolated from cultures of *Corynebacterium diphteriastrain* C7 (β197) grown casamino acids and yeast extract-based medium, may also be used as carrier protein. $CRM_{197}$ is purified through ultra-filtration, ammonium sulfate precipitation, and ion-exchange chromatography. Alternatively, $CRM_{197}$ is prepared recombinantly in accordance with U.S. Patent No. 5,614,382.

The capsular polysaccharides (CPS) from *S. pneumoniae* serotypes 1, 3, 4, 5, 6A, 6B, 7F, 9V, 14, 18C, 19A, 19F and 23F are either purchased from American Type Culture Collection (ATCC) or are isolated from bacteria and purified by known methods (see EP0072513A2) such as centrifugation, precipitation, ultra-filtration, and column chromatography. Further, they may be sized to some degree before forming conjugates with carrier proteins. Sizing of these full length capsular polysaccharides means that their size is reduced and it may be achieved by known methods (see for example EP497524 and EP497525), such as acid hydrolysis treatment, hydrogen peroxide treatment, sizing by emulsiflex® followed by a hydrogen peroxide treatment to generate oligosaccharide fragments or preferably by microfluidization. Sizing of polysaccharides is advantageous to filtering conjugated products due to the reduced viscosity of the sized polysaccharides. Sized polysaccharides have typically a molecular weight of 130 to 400 kDa depending on the serotype.

Each of the capsular polysaccharides from *S. pneumoniae* serotypes is conjugated to a carrier protein. Thus, each capsular polysaccharide from a *S. pneumoniae* serotype is covalently coupled or linked either directly or *via* a spacer to a carrier protein.

A spacer as used herein is preferably bifunctional. The spacer is optionally heterobifunctional or homobifunctional, having for example a reactive amino group and a reactive carboxylic acid group, 2 reactive amino groups or two reactive carboxylic acid groups. The spacer has for example between 4 and 20, 4 and 12, 5 and 10 carbon atoms. A possible spacer is adipic acid dihydrazide (ADH). Other spacers include B-propionamido, nitrophenyl-ethylamine, haloalkyl halides, glycosidic linkages, hexane diamine and 6-aminocaproic acid.

The capsular polysaccharide can be conjugated to a carrier protein by using any known coupling technique. The conjugation method may rely on activation of the saccharide with 1-cyano-4-dimethylamino pyridinium tetrafluoroborate (CDAP) to form a cyanate ester. The activated saccharide may thus be coupled directly or *via* a spacer group to an amino group on the carrier protein. For example, the spacer could be cystamine or cysteamine to give a thiolated polysaccharide, which could be coupled to the carrier *via* a thioether linkage obtained after reaction with a maleimide-activated carrier protein (for example using GMBS) or a haloacetylated carrier protein (for example using iodoacetimide [e.g. ethyl iodoacetimide HCl] or N-succinimidyl bromoacetate or SIAB, or SIA, or SBAP). Preferably, the cyanate ester (optionally made by CDAP chemistry) is coupled with hexane diamine or ADH and the amino-derivatised saccharide is conjugated to the carrier protein using carbodiimide (e.g. EDAC or EDC) chemistry via a carboxyl group on the protein carrier. Such conjugation methods are described in WO 93/15760, WO 95/08348 and WO 96/29094.

Other suitable conjugation techniques rely on the use of carbodiimides, hydrazides, active esters, norborane, p-nitrobenzoic acid, *N*-hydroxysuccinimide, S-NHS (N-hydroxysulfosuccinimide), EDC, TSTU (2-succinimide-1,1,3,3-tetramethyluronium tetrafluoroborate) (WO 98/42721). Conjugation may involve a carbonyl linker which may be formed by reaction of a free hydroxyl group of the saccharide with CDI (1,1'-carbonyldiimidazole) followed by reaction with a protein to form a carbamate linkage. This may involve reduction of the anomeric terminus to a primary hydroxyl group, optional protection/deprotection of the primary hydroxyl group, reaction of the primary hydroxyl group with CDI to form a CDI carbamate intermediate and coupling the CDI carbamate intermediate with an amino group on a protein.

The conjugates can also be prepared by direct reductive amination methods as described in US 4673574. Other preparation methods are described in EP0161188, EP208375 and EP0477508.

A further method involves the coupling of a cyanogen bromide (or CDAP) activated saccharide derivatised with adipic acid dihydrazide (ADH) to the protein carrier by carbodiimide condensation, for example using EDAC.

In an embodiment, a hydroxyl group (preferably an activated hydroxyl group for example a hydroxyl group activated to make a cyanate ester [e.g. with CDAP]) on a saccharide is linked to an amino or carboxylic group on a protein either directly or indirectly (through a spacer). Where a spacer is present, a hydroxyl group on a saccharide is preferably linked to an amino group on a linker, for example by using CDAP conjugation. A further amino group in the linker for example ADH) may be conjugated to a carboxylic acid group on a protein, for example by using carbodiimide chemistry, for example by using EDAC. In an embodiment, the pneumococcal capsular saccharide(s) is conjugated to the spacer first before the spacer is conjugated to the carrier protein. Alternatively, the spacer may be conjugated to the carrier before conjugation to the saccharide.

A combination of techniques may also be used, with some conjugates being prepared by CDAP, and some by reductive amination.

In general the following types of chemical groups on a protein carrier can be used for conjugation:

- Carboxyl (for instance *via* aspartic acid or glutamic acid). In one embodiment this group is linked to amino groups on saccharides directly or to an amino group on a spacer with carbodiimide chemistry e.g. with EDAC.
- Amino group (for instance *via* lysine). In one embodiment this group is linked to carboxyl groups on saccharides

directly or to a carboxyl group on a spacer with carbodiimide chemistry e.g. with EDAC. In another embodiment this group is linked to hydroxyl groups activated with CDAP or CNBr on saccharides directly or to such groups on a spacer; to saccharides or spacers having an aldehyde group; to saccharides or spacers having a succinimide ester group.

- Sulfhydryl (for instance *via* cysteine). In one embodiment this group is linked to a bromo or chloro acetylated saccharide or linker with maleimide chemistry. In one embodiment this group is activated/modified with bis diazobenzidine.
- Hydroxyl group (for instance *via* tyrosine). In one embodiment this group is activated/modified with bis diazobenzidine.
- Imidazolyl group (for instance *via* histidine). In one embodiment this group is activated/modified with bis diazobenzidine.
- Guanidyl group (for instance *via* arginine).
- Indolyl group (for instance *via* tryptophan).

On a saccharide, in general the following groups can be used for conjugation: OH, COOH or $NH_2$. Aldehyde groups can be generated after different treatments known in the art such as: periodate, acid hydrolysis, hydrogen peroxide, etc.

Examples of direct coupling approaches include, but are not limited to:

CPS-OH + CNBr or CDAP → CPS-cyanate ester + $NH_2$-carrier protein → conjugate;
CPS-aldehyde + $NH_2$-carrier protein → Schiff base + $NaCNBH_3$ → conjugate; CPS-COOH + $NH_2$-carrier protein + EDAC → conjugate;
CPS-$NH_2$ + carrier protein-COOH + EDAC → conjugate;

Indirect coupling *via* spacer approaches include, but are not limited to:

CPS-OH + CNBr or CDAP → CPS-cyanate ester + $NH_2$-$NH_2$ → CPS-$NH_2$ + COOH-carrier protein + EDAC → conjugate;
CPS-OH + CNBr or CDAP → CPS-cyanate ester + $NH_2$-SH → CPS-SH
+ SH-carrier protein (native protein with an exposed cysteine or obtained after modification of amino groups of the protein by SPDP for instance) → CPS-S-S-carrier protein;
CPS-OH + CNBr or CDAP → CPS-cyanate ester + $NH_2$-SH → CPS-SH + maleimide-carrier protein (modification of amino groups) → conjugate;
CPS-OH + CNBr or CDAP → CPS-cyanate ester + $NH_2$-SH → CPS-SH + haloacetylated-carrier protein → conjugate;
CPS-COOH + EDAC + $NH_2$-$NH_2$ → CPS-$NH_2$ + EDAC + COOH-carrier protein → conjugate;
CPS-COOH + EDAC+ $NH_2$-SH → CPS-SH + SH-carrier protein (native protein with an exposed cysteine or obtained after modification of amino groups of the protein by SPDP for instance) → saccharide-S-S-carrier protein;
CPS-COOH + EDAC+ $NH_2$-SH → CPS-SH + maleimide-carrier protein (modification of amino groups) → conjugate;
CPS-COOH + EDAC + $NH_2$-SH → CPS-SH + haloacetylated-carrier protein → conjugate;
CPS-aldehyde + $NH_2$-$NH_2$ → CPS-$NH_2$ + EDAC + carrier protein-COOH → conjugate.

In a preferred embodiment, CDAP (1-cyano-dimethylaminopyridinium tetrafluoroborate) cyanylating reagent is used for the synthesis of capsular polysaccharide-protein conjugates. This coupling method avoids hydrolysis of the alkaline sensitive polysaccharides and allows direct coupling to the carrier protein. The polysaccharide is dissolved in water or a saline solution. CDAP is dissolved in acetonitrile and added immediately to the polysaccharide solution. The CDAP reacts with the hydroxyl groups of the polysaccharide to form a cyanate ester in an activation step. Afterwards, the carrier protein is added. Amino groups of lysine react with the activated polysaccharide to form an isourea covalent linkage. After the conjugation reaction, a large excess of glycine is added to quench residual activated functions. The formed conjugate is then passed through a gel permeation to remove unreacted carrier protein and residual reagents.

Another preferred conjugation method is the reductive amination, wherein the capsular polysaccharide is oxidized with sodium periodate in an activation step and subsequently brought to reaction with the amino group of a carrier protein in the presence of sodium borohydride or sodium cyanoborohydride. The reductive amination can be performed in aqueous media or in DMSO. The crude conjugate is then passed through a HA or DEAE column and filtered sterile.

Preferably, the conjugate of a saccharide from *S. pneumoniae* serotype 8 and a carrier protein is an isolated capsular polysaccharide from *S. pneumoniae* type 8 covalently coupled to $CRM_{197}$ or an isolated oligosaccharide from *S. pneumoniae* serotype 8 covalently coupled to $CRM_{197}$.

Even more preferably, the conjugate of a saccharide from S. *pneumoniae* serotype 8 and a carrier protein has the following general formula (**I**):

$$[V^*\text{-}U_{x+3}\text{-}U_{x+2}\text{-}U_{x+1}\text{-}U_x\text{-}O\text{-}L^1\text{-}NH\text{-}W^1]_{m1}\text{-carrier-protein1} \quad (I)$$

wherein
x is an integer selected from 1, 2, 3 and 4;

$U_1 = U_5 =$ 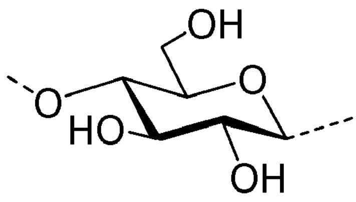 ; $U_2 = U_6 =$ 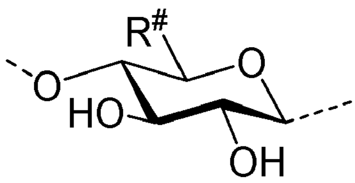 ;

$U_3 = U_7 =$ 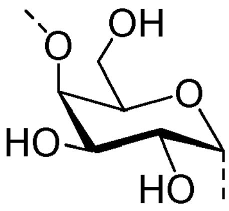 ; $U_4 =$ 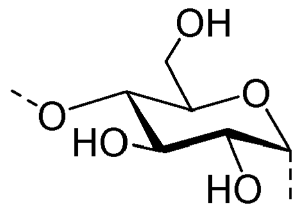 ;

V*- represents H-, H-$U_x$- or H-$U_{x+1}$-$U_x$-;
$R^\#$ represents -COOH or -$CH_2OH$;
$L^1$ represents a linker;
m1 is comprised between 2 and 18;
-$W^1$- is selected from:

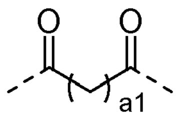 , 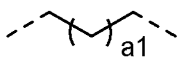 , and 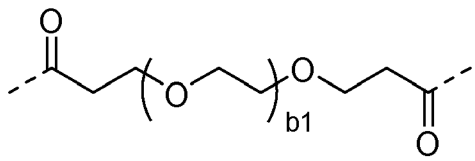 ;

a1 represents an integer from 1 to 10;
b1 represents an integer from 1 to 4; and
carrier-protein1 is selected from $CRM_{197}$, protein D, tetanus toxoid and diphtheria toxoid.

-$L^1$- is defined as a linker and is part of the fragment -O-$L^1$-NH-. Thus, the linker -$L^1$- is bound to an oxygen atom and to the nitrogen atom of the -NH-$W^1$-fragment. It is preferred that at least two carbon atoms of the linker are between the oxygen atom and the -NH-$W^1$- fragment, like -O-C-C-NH-$W^1$-. The linker -$L^1$- can be an aliphatic chain, wherein the aliphatic chain can optionally include an aromatic chain inserted in it, or a number of heteroatoms oscillating from 0 to 10. The linker -$L^1$- preferably contains between 2 and 40 carbon atoms (including the carbon atoms of optional side chains), more preferably between 2 and 30, more preferably between 2 and 20, more preferably between 2 and 14, more preferably between 2 and 12, and still more preferably between 2 and 10 carbon atoms. The shortest atom chain between the oxygen atom (i.e. the oxygen of -O-$L^1$-$NH_2$) and the -NH-$W^1$- fragment consists preferably of 2 to 14 atoms, more preferably of 2 to 12 atoms, more preferably of 2 to 10 atoms, more preferably of 2 to 8 atoms. In case the shortest chain (which is the shortest possible connection between the oxygen at the anomeric center and the -NH-$W^1$-fragment) consists of 2 to 6 atoms, these are preferably carbon atoms. In case the shortest chain consists of 4 to 8 atoms, the chain may contain 1, 2 or 3 heteroatoms selected from O, N and S. In case the shortest chain consists of 9 to 14 atoms, the chain may contain 1, 2, 3, 4, 5, or 6 heteroatoms selected from O, N and S.

It is also preferred that the linker -$L^1$- or the shortest chain is fully or partially fluorinated. The linker -$L^1$- may contain a 3-membered or a 4-membered or a 5-membered or a 6-membered saturated carbocycle or a 5-membered partly unsaturated (and not aromatic) carbocycle or a 4-membered or a 5-membered or a 6-membered saturated oxygen heterocycle or a 4-membered or a 5-membered or a 6-membered saturated nitrogen heterocycle or a 6-membered aromatic carbocycle.

The linker -$L^1$- may also contain amide (-NH-CO-, -CO-NH-) and/or urea (-NH-CO-NH-) residues and preferably only one amide or urea residue. The linker may also contain substituents and preferably two substituents, such as $R^{10}$

and $R^{11}$, or four substituents such as $R^{10}$, $R^{11}$, $R^{15}$ and $R^{14}$, which have the meanings as defined herein and which are preferably selected from: -F, -Cl, $-CH_3$, $-C_2H_5$, $-C_3H_7$, $-C_5H_9$, $-C_6H_{13}$, $-OCH_3$, $-OC_2H_5$, $-CH_2F$, $-CHF_2$, $-CF_3$, $-C(O)-NH_2$, $-SCH_3$, $-SC_2H_5$, $-NHC(O)CH_3$, $-N(CH_3)_2$, and $-N(C_2H_5)_2$.

In case the linker $-L^1-$ is fluorinated, more than two substituents -F are preferred.

Linker $-L^1-$ is preferably selected from: $-CH_2-$, $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-(CH_2)_9-$, $-(CH_2)_{10}-$ $-CF_2-$, $-(CF_2)_2-$, $-(CF_2)_3-$, $-(CF_2)_4-$, $-(CF_2)_5-$, $-(CF_2)_6-$, $-(CF_2)_7-$, $-(CF_2)_8-$, $-(CF_2)_9-$, $-(CF_2)_{10}-$ $-(CH_2)_2-O-(CH_2)_2-$, $-CH_2-O-(CH_2)_3-$, $-(CH_2)_3-O-CH_2--CH_2-O-(CH_2)_2-$, $-(CH_2)_2-O-CH_2-$, $-(CH_2)_3-O-(CH_2)_2-$, $-(CH_2)_2-O-(CH_2)_3-$, $-(CH_2)_4-O-CH_2-$, $-CH_2-O-(CH_2)_4-$, $-L^a-$, $-L^a-L^e-$, $-L^a-L^b-L^e-$, $-L^a-L^b-L^d-L^c-L^6-$, $-L^a-L^d-L^6-$; wherein $-L^a-$ is selected from: $-(CH_2)_o-$, $-(CF_2)_o-$, $-(CH_2-CH_2-O)_o-C_2H_4-$, $-(CH_2-CH_2-O)_o-CH_2-$, $-(CR^{10}R^{11})_o-$,

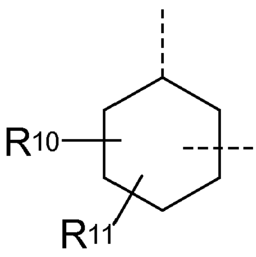
,
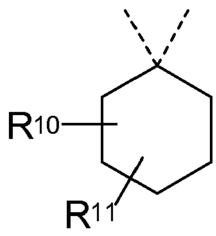
,
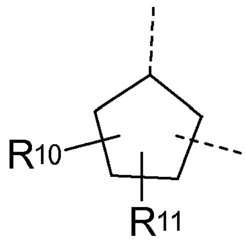
,
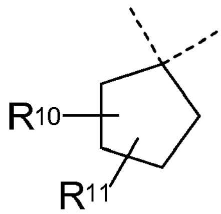
,
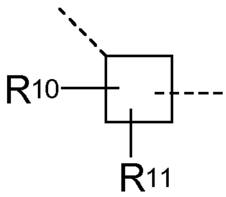
, and
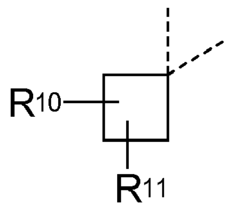
;

$-L^b-$ and $-L^c-$ are independently of each other selected from: -O-, -NH-C(O)-NH-, -NH-C(S)-NH-, -NH-C(O)-, -C(O)-NH-, -NH-C(O)-O-, $-NR^9-$, $-NR^{18}-$, $-SO_2-$,

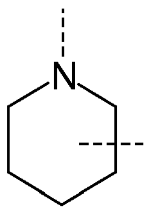
,
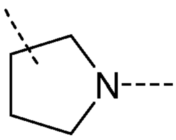
,
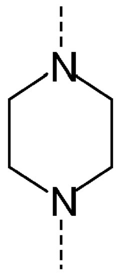
,
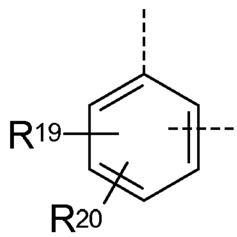
,
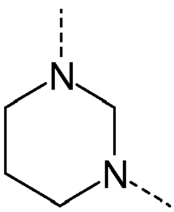
,
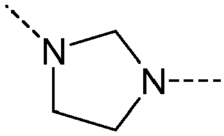
,
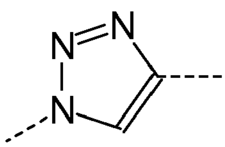
,
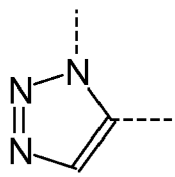
,

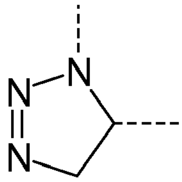, 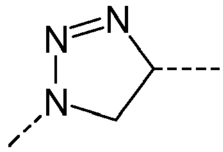, and 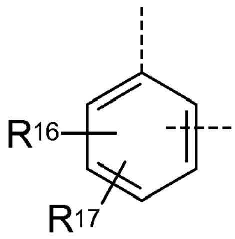;

-L$^d$- represents: $-(CH_2)_q-$, $-(CF_2)_q-$, $-(CR^{12}R^{13})_q$, $-(CH_2\text{-}CH_2\text{-}O)_q\text{-}C_2H_4-$, $-(CH_2\text{-}CH_2\text{-}O)_q\text{-}CH_2-$,

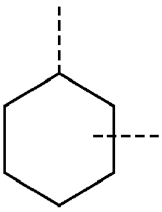, 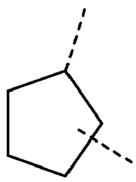, 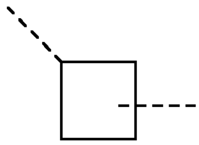, 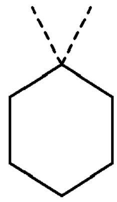,

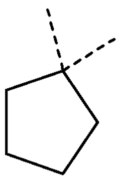, and 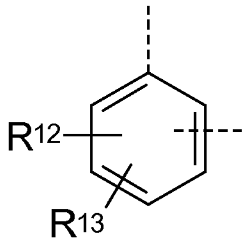;

-L$^e$- is selected from: $-(CH_2)_{p1}-$, $-(CF_2)_{p1}-$, $-C_2H_4\text{-}(O\text{-}CH_2\text{-}CH_2)_{p1}-$, $-CH_2\text{-}(O\text{-}CH_2\text{-}CH_2)_{p1}-$, $-(CH_2)_{p1}\text{-}O\text{-}(CH_2)_{p2}-$, $-(CR^{14}R^{15})_{p1}-$, $-(CR^{14}R^{15})_{p1}\text{-}O\text{-}(CR^{21}R^{22})_{p2}-$,

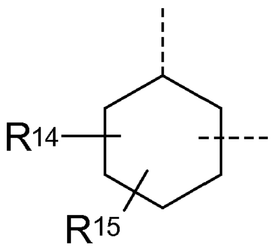, 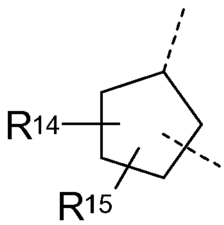, 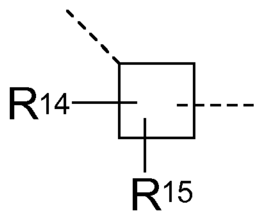, 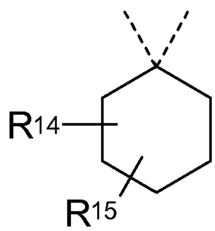,

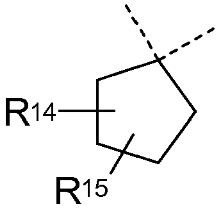, and 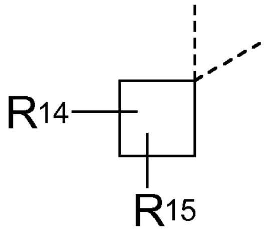;

$R^9$ and $R^{18}$ are independently of each other selected from: $-CH_3$, $-C_2H_5$, $-C_3H_7$, and $-C(O)CH_3$;
$R^{10}$, $R^{11}$, $R^{12}$, $R^{13}$, $R^{14}$, $R^{15}$, $R^{16}$, $R^{17}$, $R^{19}$, $R^{20}$, $R^{21}$ and $R^{22}$ are independently of each other selected from: -H, -F, -Cl, $-CH_3$, $-C_2H_5$, $-C_3H_7$, $-C_5H_9$, $-C_6H_{13}$, $-OCH_3$, $-OC_2H_5$, $-CH_2F$, $-CHF_2$, $-CF_3$, $-C(O)\text{-}NH_2$, $-SCH_3$, $-SC_2H_5$, $-NHC(O)CH_3$, $-N(CH_3)_2$ and $-N(C_2H_5)_2$;

o, q, p1 and p2 are independently of each other an integer selected from 1, 2, 3, 4, 5, 6, 7, 8, 9, and 10.

In general formula (**I**), preferably x represents 3. Hence, an immunogenic composition according to the present invention, wherein the conjugate of a saccharide from *S. pneumoniae* serotype 8 and a carrier protein is of the following general formula (**I-A**):

$$[V^*\text{-}U_6\text{-}U_5\text{-}U_4\text{-}U_3\text{-}O\text{-}L^1\text{-}NH\text{-}W^1]_{m1}\text{-carrier-protein1} \qquad (\textbf{I-A})$$

wherein

$U_5$ = 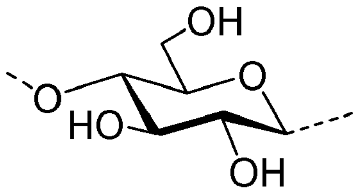 ; $U_6$ = 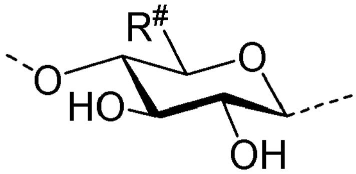 ;

$U_3$ = 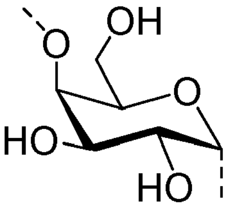 ; $U_4$ = 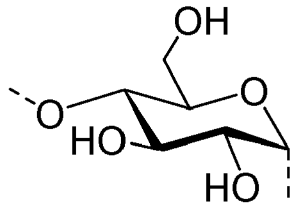 ;

V*- represents H-, H-$U_3$- or H-$U_4$-$U_3$-;
and $R^{\#}$, $L^1$, m1, -$W^1$-, a1, b1 and carrier-protein1 have the meanings defined herein, is especially preferred.

In a further preferred embodiment, the residue V*- in general formula (**I**) represents H-. Thus, an immunogenic composition comprising a *Streptococcus pneumoniae* serotype 8 conjugate of general formula (**I-B**):

$$[H\text{-}U_{x+3}\text{-}U_{x+2}\text{-}U_{x+1}U_x\text{-}O\text{-}L^1\text{-}NH\text{-}W^1]_{m1}\text{-carrier-protein1} \qquad (\textbf{I-B})$$

wherein
x is an integer selected from 1, 2, 3 and 4;

$U_1 = U_5$ = 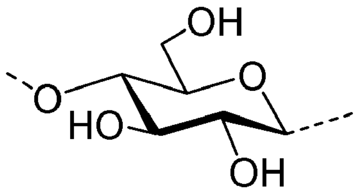 ; $U_2 = U_6$ = 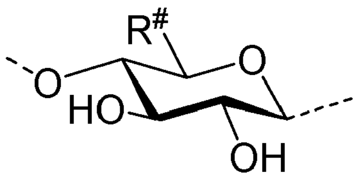 ;

$U_3 = U_7$ = 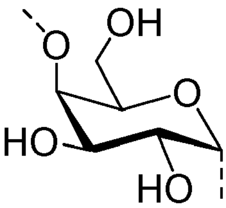 ; $U_4$ = 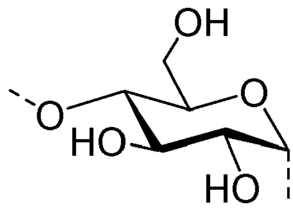 ;

and $R^{\#}$, $L^1$, m1, -$W^1$-, a1, b1 and carrier-protein1 have the meanings defined herein, and
a mixture consisting of capsular polysaccharides from *S. pneumoniae* serotypes 1, 3, 4, 5, 6A, 6B, 7F, 9V, 14, 18C, 19A, 19F and 23F individually conjugated to $CRM_{197}$ carrier protein;
or
a mixture consisting of capsular polysaccharides from *S. pneumoniae* serotypes 1, 4, 5, 6B, 7F, 9V, 14, 18C, 19F

and 23F individually conjugated to a carrier protein, wherein the capsular polysaccharides from *S. pneumoniae* serotypes 1, 4, 5, 6B, 7F, 9V, 14, and 23F are individually conjugated to protein D, the capsular polysaccharide from *S. pneumoniae* serotype 18C is conjugated to tetanus toxoid and the capsular polysaccharide from *S. pneumoniae* serotype 19F is conjugated to diphtheria toxoid; is also preferred.

Even more preferred is an immunogenic composition comprising a *S. pneumoniae* serotype 8 conjugate of general formula (**I-A**), wherein V*- represents H-, and a mixture consisting of capsular polysaccharides from *S. pneumoniae* serotypes 1, 3, 4, 5, 6A, 6B, 7F, 9V, 14, 18C, 19A, 19F and 23F individually conjugated to $CRM_{197}$ carrier protein; or a mixture consisting of capsular polysaccharides from *S. pneumoniae* serotypes 1, 4, 5, 6B, 7F, 9V, 14, 18C, 19F and 23F individually conjugated to a carrier protein, wherein the capsular polysaccharides from S. *pneumoniae* serotypes 1, 4, 5, 6B, 7F, 9V, 14, and 23F are individually conjugated to protein D, the capsular polysaccharide from *S. pneumoniae* serotype 18C is conjugated to tetanus toxoid and the capsular polysaccharide from *S. pneumoniae* serotype 19F is conjugated to diphtheria toxoid.

Preferably the linker $-L^1-$ is selected from: $-L^a-$, $-L^a-L^e-$, $-L^a-L^b-L^e-$, $-L^a-L^d-L^e-$; wherein
$-L^a-$ is selected from: $-(CH_2)_o-$, $-(CH_2-CH_2-O)_o-C_2H_4-$, $-(CH_2-CH_2-O)_o-CH_2-$;
$-L^b-$ represents -O-;
$-L^d-$ is selected from $-(CH_2)_q-$, $-(CF_2)_q-$, $-(CH_2-CH_2-O)_q-C_2H_4-$, and $-(CH_2-CH_2-O)_q-CH_2-$;
$-L^e-$ is selected from: $-(CH_2)_{p1}-$, $-(CF_2)_{p1}-$, $-C_2H_4-(O-CH_2-CH_2)_{p1}-$, $-CH_2-(O-CH_2-CH_2)_{p1}-$ and $-(CH_2)_{p1}-O-(CH_2)_{p2}-$; and o, q, p1 and p2 are independently of each other an integer selected from 1, 2, 3, 4, 5, and 6.

Therefore, the immunogenic composition of the present invention contains preferably a conjugate of a saccharide from *S. pneumoniae* serotype 8 and a carrier protein, wherein the conjugate has the general formula (**I**), (**I-A**) or (**I-B**), with -L- being selected from: $-L^a-$, $-L^a-L^e-$, $-L^a-L^b-L^e-$ and $-L^a-L^d-L^e-$; $-L^a-$ being selected from: $-(CH_2)_o-$, $-(CH_2-CH_2-O)_o-C_2H_4-$, $-(CH_2-CH_2-O)_o-CH_2-$;
$-L^b-$ representing -O-;
$-L^d-$ being selected from: $-(CH_2)_q-$, $-(CF_2)_q-$, $-(CH_2-CH_2-O)_q-C_2H_4-$, and $-(CH_2-CH_2-O)_q-CH_2-$;
$-L^e-$ being selected from: $-(CH_2)_{p1}-$, $-(CF_2)_{p1}-$, $-C_2H_4-(O-CH_2-CH_2)_{p1}-$, $-CH_2-(O-CH_2-CH_2)_{p1}-$ and $-(CH_2)_{p1}-O-(CH_2)_{p2}-$; and o, q, p1 and p2 being independently of each other an integer selected from 1, 2, 3, 4, 5, and 6.

Even more preferred is an immunogenic composition comprising a conjugate of a saccharide from *S. pneumoniae* serotype 8 and a carrier protein, wherein the conjugate has the general formula (**I**), (**I-A**) or (**I-B**), with $-L^1-$ representing $-(CH_2)_o-$ and o being an integer selected from 2, 3, 4, 5, 6, 7 and 8.

It is also preferred that $-W^1-$ represents

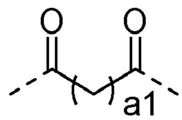

and a1 is an integer
selected from 2, 3, 4, 5 and 6.

Thus, an immunogenic composition according to the present invention comprising a conjugate of a saccharide from *S. pneumoniae* serotype 8 and a carrier protein, wherein said conjugate has the general formula (**I**), (**I-A**) or (**I-B**), with
$-L^1-$ being selected from: $-L^a-$, $-L^a-L^e-$, $-L^a-L^b-L^e-$ and $-L^a-L^d-L^e-$;
$-L^a-$ being selected from: $-(CH_2)_o-$, $-(CH_2-CH_2-O)_o-C_2H_4-$, $-(CH_2-CH_2-O)_o-CH_2-$;
$-L^b-$ representing -O-;
$-L^d-$ being selected from: $-(CH_2)_q-$, $-(CF_2)_q-$, $-(CH_2-CH_2-O)_q-C_2H_4-$, and $-(CH_2-CH_2-O)_q-CH_2-$;
$-L^e-$ being selected from: $-(CH_2)_{p1}-$, $-(CF_2)_{p1}-$, $-C_2H_4-(O-CH_2-CH_2)_{p1}-$, $-CH_2-(O-CH_2-CH_2)_{p1}-$ and $-(CH_2)_{p1}-O-(CH_2)_{p2}-$;
o, q, p1 and p2 being independently of each other an integer selected from 1, 2, 3, 4, 5, and 6, and
$-W^1-$ representing

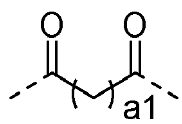

with a1 being an integer selected from 2,
3, 4, 5 and 6, is especially preferred.

Even more preferred is an immunogenic composition comprising a conjugate of general formula (**I**), (**I-A**) or (**I-B**), wherein $-L^1-$ represents $-(CH_2)_o-$, o is an integer selected from 2, 3, 4, 5, 6, 7 and 8, $-W^1-$ represents

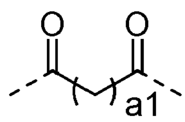,

and a1 is an integer selected from 2, 3, 4, 5 and 6.

Preferably, carrier-protein1 is $CRM_{197}$. Preferably m1 is comprised between 5 and 17, even more preferably between 7 and 16, and most preferably between 8 and 15.

The inventors have surprisingly found that an immunogenic composition comprising a conjugate of general formula (**I**), (**I-A**) or (**I-B**) as defined above and a mixture consisting of capsular polysaccharides from *S. pneumoniae* serotypes 1, 3, 4, 5, 6A, 6B, 7F, 9V, 14, 18C, 19A, 19F and 23F individually conjugated to $CRM_{197}$ carrier protein, or a mixture consisting of capsular polysaccharides from *S. pneumoniae* serotypes 1, 4, 5, 6B, 7F, 9V, 14, 18C, 19F and 23F individually conjugated to a carrier protein, wherein the capsular polysaccharides from *S. pneumoniae* serotypes 1, 4, 5, 6B, 7F, 9V, 14, and 23F are individually conjugated to protein D, the capsular polysaccharide from *S. pneumoniae* serotype 18C is conjugated to tetanus toxoid and the capsular polysaccharide from *S. pneumoniae* serotype 19F is conjugated to diphtheria toxoid induces in a host a robust immune response against the capsular polysaccharide from *Streptococcus pneumoniae* serotype 8, thereby leading to opsonophagocytic killing of *Streptococcus pneumoniae* serotype 8 pneumococci, without impairing the immune response against the capsular polysaccharides from the other *S. pneumoniae* serotypes, which are present in the immunogenic composition. Therefore, said immunogenic compositions are useful for the prevention and/or treatment of diseases caused by *Streptococcus pneumoniae* serotype 8.

Preferably, the immunogenic composition according to the present invention further comprises a *Streptococcus pneumoniae* serotype 2 conjugate of the following general formula (**II-C**):

$$[B^*\text{-}A_{y+3}\text{-}A_{y+2}\text{-}A_{y+1}\text{-}A_y\text{-O-}L^2\text{-NH-}W^2]_{m2}\text{-carrier-protein2} \qquad \textbf{(II-C)}$$

wherein

y is an integer selected from 1, 2, 3 and 4;

$A_1 = A_5 =$ 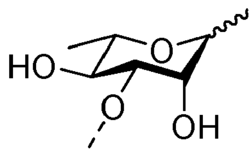 ; $A_2 = A_6 =$ 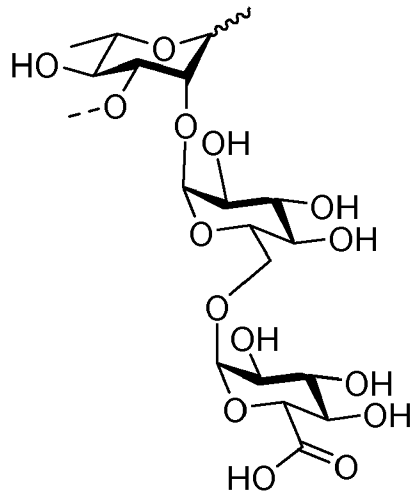 ,

$A_3 = A_7 =$ 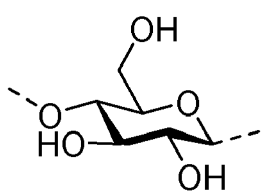 ; $A_4 =$ 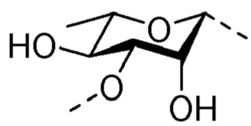 ;

B*- represents: H-, H-$A_y$-, H-$A_{y+1}$-$A_y$-, H-$A_{y+2}$-$A_{y+1}$-$A_y$- or H-$A_{y+3}$-$A_{y+2}$-$A_{y+1}$-$A_y$-;

$L^2$ represents a linker;

m2 is comprised between about 2 and about 18;
$-W^2-$ is selected from:

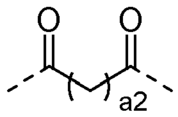, 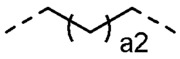, and 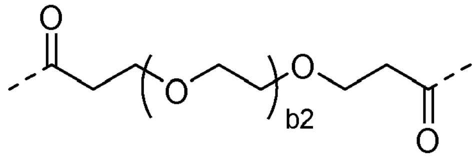;

a2 represents an integer from 1 to 10;
b2 represents an integer from 1 to 4; and
carrier-protein2 is selected from $CRM_{197}$, protein D, tetanus toxoid and diphtheria toxoid.

It is preferred that

$A_1 = A_5 =$ 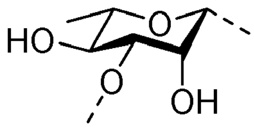; and $A_2 = A_6 =$ 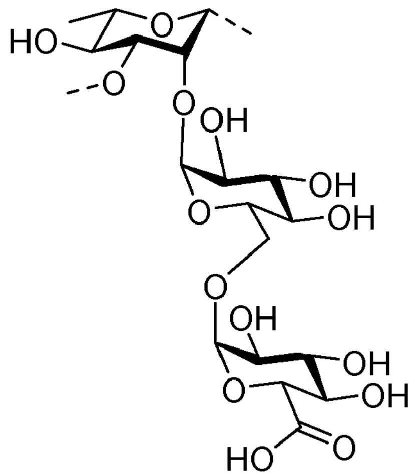

Thus, the *Streptococcus pneumoniae* serotype 2 conjugate has preferably the following general formula (**II**):

$$[B^*\text{-}A_{y+3}\text{-}A_{y+2}\text{-}A_{y+1}\text{-}A_y\text{-}O\text{-}L^2\text{-}NH\text{-}W^2]_{m2}\text{-carrier-protein2} \quad \textbf{(II)}$$

wherein
y is an integer selected from 1,2,3 and 4;

$A_1 = A_5 =$ 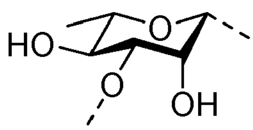; $A_2 = A_6 =$ 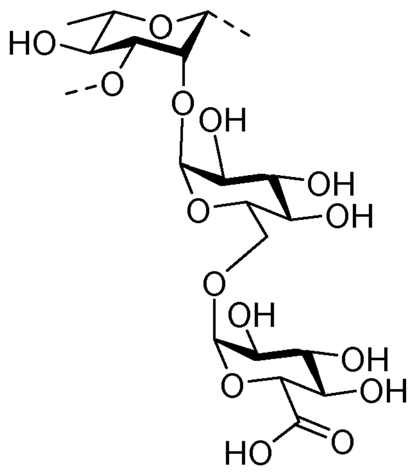,

$A_3 = A_7 =$ 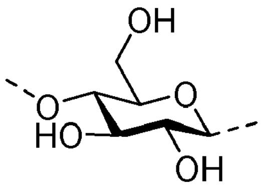 ; $A_4 =$ 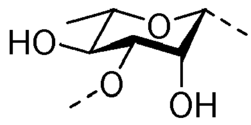 ;

B*- represents: H-, $H\text{-}A_y\text{-}$, $H\text{-}A_{y+1}\text{-}A_y\text{-}$, $H\text{-}A_{y+2}\text{-}A_{y+1}\text{-}A_y\text{-}$ or $H\text{-}A_{y+3}\text{-}A_{y+2}\text{-}A_{y+1}\text{-}A_y\text{-}$;
$L^2$ represents a linker;
m2 is comprised between about 2 and about 18;
$-W^2-$ is selected from:

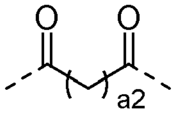 , 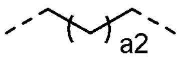 , and 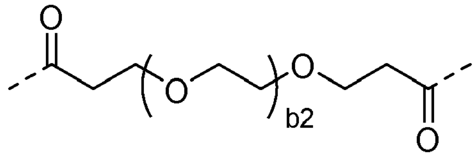 ;

a2 represents an integer from 1 to 10;
b2 represents an integer from 1 to 4; and
carrier-protein2 is selected from $CRM_{197}$, protein D, tetanus toxoid and diphtheria toxoid.

It is more preferred that:

$A_1 = A_5 =$ 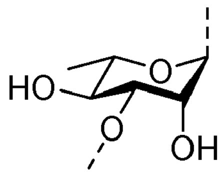 ; and $A_2 = A_6 =$ 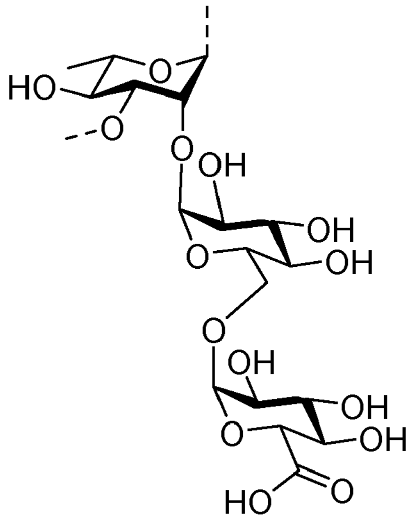

Hence more preferably, the *Streptococcus pneumoniae* serotype 2 conjugate has the following general formula (**II-D**):

$$[B^*\text{-}A_{y+3}\text{-}A_{y+2}\text{-}A_{y+1}\text{-}A_y\text{-}O\text{-}L^2\text{-}NH]_{m2}\text{-carrier-protein2} \qquad \textbf{(II-D)}$$

wherein
y is an integer selected from 1,2,3 and 4;

$A_1 = A_5 =$ 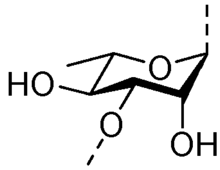 ; $A_2 = A_6 =$ 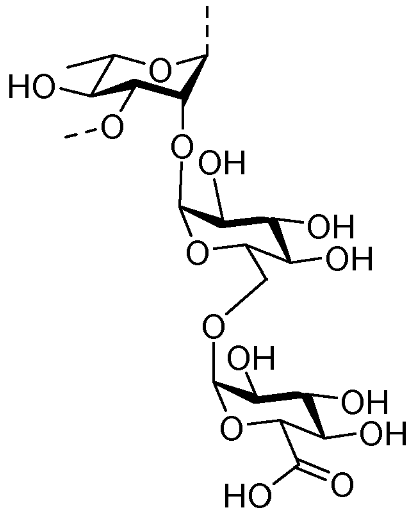 ,

$A_3 = A_7 =$ 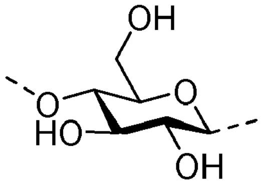 ; $A_4 =$ 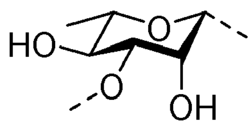 ;

B*- represents: H-, H-$A_y$-, H-$A_{y+1}$-$A_y$-, H-$A_{y+2}$-$A_{y+1}$-$A_y$- or H-$A_{y+3}$-$A_{y+2}$-$A_{y+1}$-$A_y$;
$L^2$ represents a linker;
m2 is comprised between about 2 and about 18;
-$W^2$- is selected from:

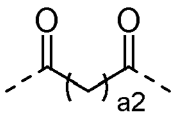 , 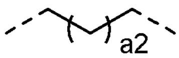 , and 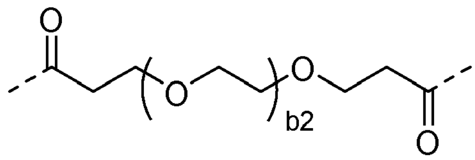 ;

a2 represents an integer from 1 to 10;
b2 represents an integer from 1 to 4; and
carrier-protein2 is selected from $CRM_{197}$, protein D, tetanus toxoid and diphtheria toxoid.

-$L^2$- is defined as a linker and is part of the fragment -O-$L^2$-NH-. Thus, the linker -$L^2$- is bound to an oxygen atom and to the nitrogen atom of the -NH-$W^2$-fragment. It is preferred that at least two carbon atoms of the linker are between the oxygen atom and the -NH-$W^2$- fragment, like -O-C-C-NH-$W^2$-. The linker -$L^2$- can be an aliphatic chain, wherein the aliphatic chain can optionally include an aromatic chain inserted in it, or a number of heteroatoms oscillating from 0 to 10.

The linker -$L^2$- preferably contains between 2 and 40 carbon atoms (including the carbon atoms of optional side chains), more preferably between 2 and 30, more preferably between 2 and 20, more preferably between 2 and 14, more preferably between 2 and 12, and still more preferably between 2 and 10 carbon atoms.

The shortest atom chain between the oxygen atom (i.e. the oxygen of -O-$L^2$-NH-) and the -NH-$W^2$- fragment consists preferably of 2 to 14 atoms, more preferably of 2 to 12 atoms, more preferably of 2 to 10 atoms, more preferably of 2 to 8 atoms. In case the shortest chain (which is the shortest possible connection between the oxygen at the anomeric center and the -NH-$W^2$-fragment) consists of 2 to 6 atoms, these are preferably carbon atoms. In case the shortest chain consists of 4 to 8 atoms, the chain may contain 1, 2 or 3 heteroatoms selected from O, N and S. In case the shortest chain consists of 9 to 14 atoms, the chain may contain 1, 2, 3, 4, 5, or 6 heteroatoms selected from O, N and S.

It is also preferred that the linker $-L^2-$, or the shortest chain is fully or partially fluorinated. The linker $-L^2-$ may contain a 3-membered or a 4-membered or a 5-membered or a 6-membered saturated carbocycle or a 5-membered partly unsaturated (and not aromatic) carbocycle or a 4-membered or a 5-membered or a 6-membered saturated oxygen heterocycle or a 4-membered or a 5-membered or a 6-membered saturated nitrogen heterocycle or a 6-membered aromatic carbocycle.

The linker $-L^2-$ may also contain amide (-NH-CO-, -CO-NH-) and/or urea (-NH-CO-NH-) residues and preferably only one amide or urea residue. The linker may also contain substituents and preferably two substituents, such as $R^{10*}$ and $R^{11*}$, or four substituents such as $R^{10*}$, $R^{11*}$, $R^{15*}$ and $R^{14*}$, which have the meanings as defined herein and which are preferably selected from: -F, -Cl, $-CH_3$, $-C_2H_5$, $-C_3H_7$, $-C_5H_9$, $-C_6H_{13}$, $-OCH_3$, $-OC_2H_5$, $-CH_2F$, $-CHF_2$, $-CF_3$, $-C(O)-NH_2$, $-SCH_3$, $-SC_2H_5$, $-NHC(O)CH_3$, $-N(CH_3)_2$, and $-N(C_2H_5)_2$.

In case the linker $-L^2-$ is fluorinated, more than two substituents -F are preferred.

Linker $-L^2-$ is preferably selected from: $-CH_2-$, $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-(CH_2)_9-$, $-(CH_2)_{10}-$, $-CF_2-$, $-(CF_2)_2-$, $-(CF_2)_3-$, $-(CF_2)_4-$, $-(CF_2)_5-$, $-(CF_2)_6-$, $-(CF_2)_7-$, $-(CF_2)_8-$, $-(CF_2)_9-$, $-(CF_2)_{10}-$, $-(CH_2)_2-O-(CH_2)_2-$, $-CH_2-O-(CH_2)_3-$, $-(CH_2)_3-O-CH_2--CH_2-O-(CH_2)_2-$, $-(CH_2)_2-O-CH_2-$, $-(CH_2)_3-O-(CH_2)_2-$, $-(CH_2)_2-O-(CH_2)_3-$, $-(CH_2)_4-O-CH_2-$, $-CH_2-O-(CH_2)_4-$, $-L^{a*}-$, $-L^{a*}-L^{e*}-$, $-L^{a*}-L^{b*}-L^{e*}-$, $-L^{a*}-L^{b*}-L^{d*}-L^{c*}-L^{e*}-$, $-L^a-L^d-L^e-$; wherein

$-L^{a*}-$ is selected from: $-(CH_2)_{o*}-$, $-(CF_2)_{o*}-$, $-(CH_2-CH_2-O)_{o*}-C_2H_4-$, $-(CH_2-CH_2-O)_{o*}-CH_2-$, $-(CR^{10*}R^{11*})_{o*}-$,

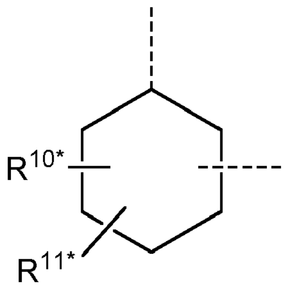,
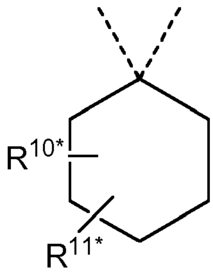,
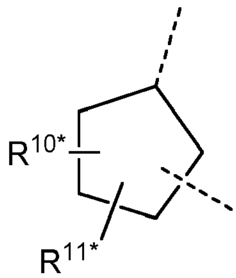,
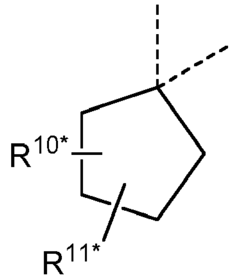,

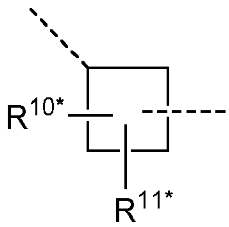,
and
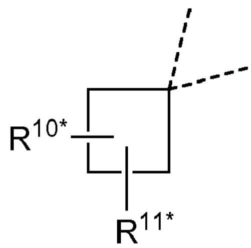;

$-L^{b*}-$ and $-L^{c*}-$ are independently of each other selected from: -O-, -NH-C(O)-NH-, -NH-C(S)-NH-, -NH-C(O)-, -C(O)-NH-, -NH-C(O)-O-, $-NR^{9*}-$, $-NR^{18*}-$, $-SO_2-$,

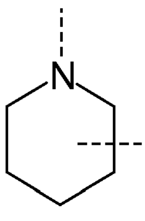,
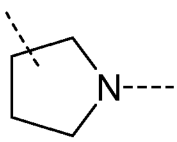,
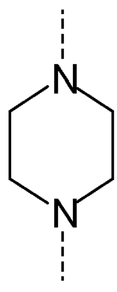,
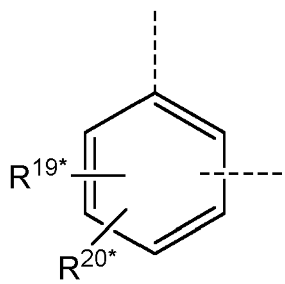,

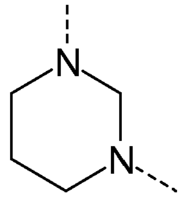, 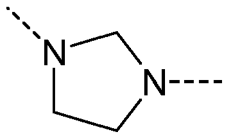, 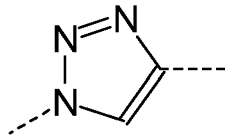, 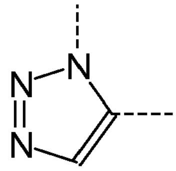,

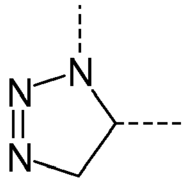, 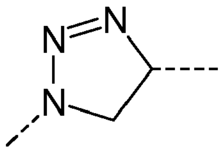, and 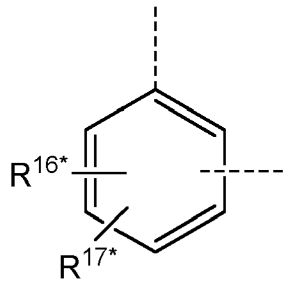;

$-L^{d*}-$ represents $-(CH_2)_{q*}-$, $-(CF_2)q_*-$, $-(CR^{12*}R^{13*})_{q*}-$, $-(CH_2\text{-}CH_2\text{-}O)_{q*}\text{-}C_2H_4-$, $-(CH_2\text{-}CH_2\text{-}O)_{q*}\text{-}CH_2-$,

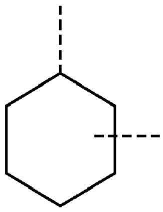, 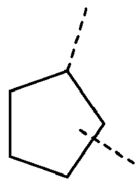, 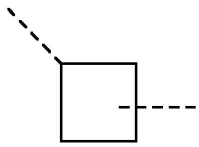, 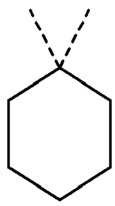,

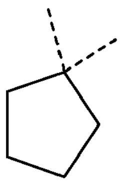, and 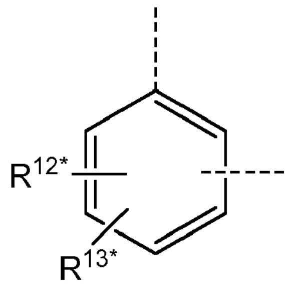;

$-L^{e*}-$ is selected from: $-(CH_2)_{p1*}-$, $-(CF_2)_{p1*}-$, $-C_2H_4\text{-}(O\text{-}CH_2\text{-}CH_2)_{p1*}-$, $-CH_2\text{-}(O\text{-}CH_2\text{-}CH_2)_{p1*}-$, $-(CH_2)_{p1*}\text{-}O\text{-}(CH_2)_{p2*}-$, $(CR^{14*}R^{15*})_{p1*}-$, $-(CR^{14*}R^{15*})_{p1*}\text{-}O\text{-}(CR^{21*}R^{22*})_{p2*}-$,

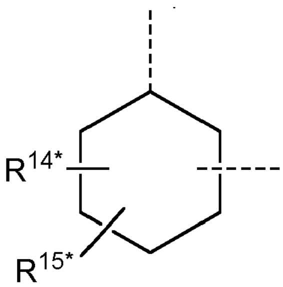, 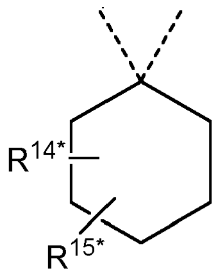, 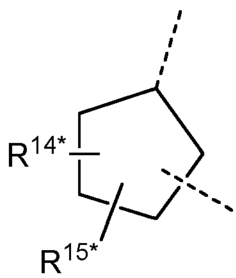, 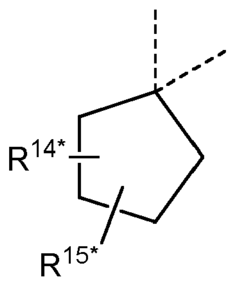,

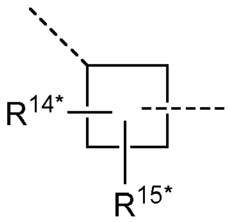

,

and

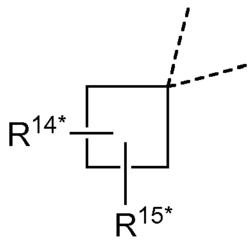

;

$R^{9*}$ and $R^{18*}$ are independently of each other selected from: $-CH_3$, $-C_2H_5$, $-C_3H_7$, and $-C(O)CH_3$;
$R^{10*}$, $R^{11*}$, $R^{12*}$, $R^{13*}$, $R^{14*}$, $R^{15*}$, $R^{16*}$, $R^{17*}$, $R^{19*}$, $R^{20*}$, $R^{21*}$ and $R^{22*}$ are independently of each other selected from: -H, -F, -Cl, $-CH_3$, $-C_2H_5$, $-C_3H_7$, $-C_5H_9$, $-C_6H_{13}$, $-OCH_3$, $-OC_2H_5$, $-CH_2F$, $-CHF_2$, $-CF_3$, $-C(O)-NH_2$, $-SCH_3$, $-SC_2H_5$, $-NHC(O)CH_3$, $-N(CH_3)_2$ and $-N(C_2H_5)_2$;
o*, q*, p1* and p2* are independently of each other an integer selected from 1, 2, 3, 4, 5, 6, 7, 8, 9, and 10.

Thus, another aspect of the present invention is directed to an immunogenic composition comprising a conjugate of a saccharide from *S. pneumoniae* serotype 8 and a carrier protein as defined above, a S. *pneumoniae* serotype 2 conjugate of general formula (**II**), (**II-C**) or (**II-D**) and a mixture consisting of capsular polysaccharides from *Streptococcus pneumoniae* serotypes 1, 3, 4, 5, 6A, 6B, 7F, 9V, 14, 18C, 19A, 19F and 23F individually conjugated to $CRM_{197}$ carrier protein; or
a mixture consisting of capsular polysaccharides from *Streptococcus pneumoniae* serotypes 1, 4, 5, 6B, 7F, 9V, 14, 18C, 19F and 23F individually conjugated to a carrier protein, wherein the capsular polysaccharides from *Streptococcus pneumoniae* serotypes 1, 4, 5, 6B, 7F, 9V, 14, and 23F are individually conjugated to protein D, the capsular polysaccharide from *Streptococcus pneumoniae* serotype 18C is conjugated to tetanus toxoid and the capsular polysaccharide from *Streptococcus pneumoniae* serotype 19F is conjugated to diphtheria toxoid.

In general formulae (**II**), (**II-C**) and (**II-D**), it is preferred that y represents 1. Thus, the immunogenic composition according to the present invention preferably contains besides the conjugate of a saccharide from *S. pneumoniae* serotype 8 and a carrier protein defined above, and a mixture consisting of capsular polysaccharides from *Streptococcus pneumoniae* serotypes 1, 3, 4, 5, 6A, 6B, 7F, 9V, 14, 18C, 19A, 19F and 23F individually conjugated to $CRM_{197}$ carrier protein; or
a mixture consisting of capsular polysaccharides from *Streptococcus pneumoniae* serotypes 1, 4, 5, 6B, 7F, 9V, 14, 18C, 19F and 23F individually conjugated to a carrier protein, wherein the capsular polysaccharides from *Streptococcus pneumoniae* serotypes 1, 4, 5, 6B, 7F, 9V, 14, and 23F are individually conjugated to protein D, the capsular polysaccharide from *Streptococcus pneumoniae* serotype 18C is conjugated to tetanus toxoid and the capsular polysaccharide from *Streptococcus pneumoniae* serotype 19F is conjugated to diphtheria toxoid, a conjugate of general formula (**II-E**):

$$[B^*\text{-}A_4\text{-}A_3\text{-}A_2\text{-}A_1\text{-}O\text{-}L^2\text{-}NH\text{-}W^2]_{m2}\text{-carrier-protein2} \qquad \textbf{(II-E)}$$

wherein

$A_1$ = 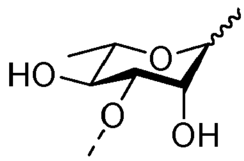 ;

$A_2$ = 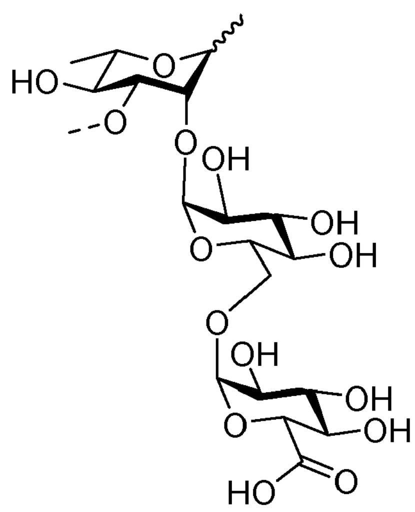 ,

$A_3$ = 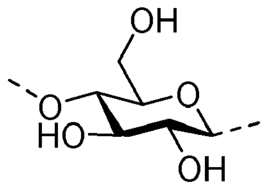 ; $A_4$ = 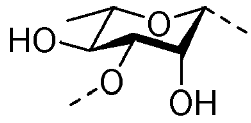 ;

B*- represents H-, H-$A_1$-, H-$A_2$-$A_1$-, H-$A_3$-$A_2$-$A_1$- or H-$A_4$-$A_3$-$A_2$-$A_1$-; and $L^2$, m2, -$W^2$-, a2, b2 and carrier-protein2 have the meanings defined herein.

A further preferred immunogenic composition according to the present invention contains besides the conjugate of a saccharide from *S. pneumoniae* serotype 8 and a carrier protein defined above, and a mixture consisting of capsular polysaccharides from *Streptococcus pneumoniae* serotypes 1, 3, 4, 5, 6A, 6B, 7F, 9V, 14, 18C, 19A, 19F and 23F individually conjugated to $CRM_{197}$ carrier protein; or

a mixture consisting of capsular polysaccharides from *Streptococcus pneumoniae* serotypes 1, 4, 5, 6B, 7F, 9V, 14, 18C, 19F and 23F individually conjugated to a carrier protein, wherein the capsular polysaccharides from *Streptococcus pneumoniae* serotypes 1, 4, 5, 6B, 7F, 9V, 14, and 23F are individually conjugated to protein D, the capsular polysaccharide from *Streptococcus pneumoniae* serotype 18C is conjugated to tetanus toxoid and the capsular polysaccharide from *Streptococcus pneumoniae* serotype 19F is conjugated to diphtheria toxoid, a conjugate of general formula (**II-A**):

$$[B^*\text{-}A_4\text{-}A_3\text{-}A_2\text{-}A_1\text{-}O\text{-}L^2\text{-}NH\text{-}W^2]_{m2}\text{-carrier-protein2} \qquad \text{(II-A)}$$

wherein

$A_1$ = 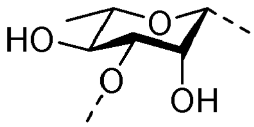 ; $A_2$ = 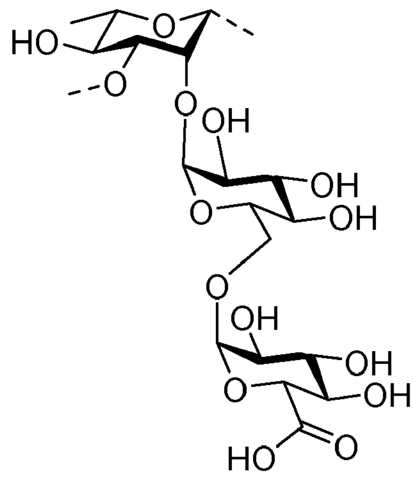 ,

$A_3$ = 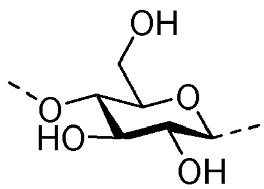 ; $A_4$ = 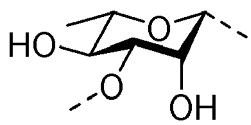 ;

B*- represents H-, H-$A_1$-, H-$A_2$-$A_1$-, H-$A_3$-$A_2$-$A_1$- or H-$A_4$-$A_3$-$A_2$-$A_1$-; and $L^2$, m2, -$W^2$- and carrier-protein2 have the meanings defined herein.

An especially preferred immunogenic composition according to the present invention contains besides the *S. pneumoniae* serotype 8 conjugate defined above, and a mixture consisting of capsular polysaccharides from *Streptococcus pneumoniae* serotypes 1, 3, 4, 5, 6A, 6B, 7F, 9V, 14, 18C, 19A, 19F and 23F individually conjugated to $CRM_{197}$ carrier protein; or

a mixture consisting of capsular polysaccharides from *Streptococcus pneumoniae* serotypes 1, 4, 5, 6B, 7F, 9V, 14, 18C, 19F and 23F individually conjugated to a carrier protein, wherein the capsular polysaccharides from *Streptococcus pneumoniae* serotypes 1, 4, 5, 6B, 7F, 9V, 14, and 23F are individually conjugated to protein D, the capsular polysaccharide from *Streptococcus pneumoniae* serotype 18C is conjugated to tetanus toxoid and the capsular polysaccharide from *Streptococcus pneumoniae* serotype 19F is conjugated to diphtheria toxoid, a S. *pneumoniae* serotype 2 conjugate of general formula (**II-F**):

$$[B^*\text{-}A_4\text{-}A_3\text{-}A_2\text{-}A_1\text{-}O\text{-}L^2\text{-}NH\text{-}W^2]_{m2}\text{-carrier-protein2} \qquad \text{(II-F)}$$

wherein

$A_1$ = 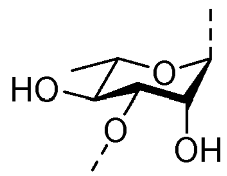 ; $A_2$ = 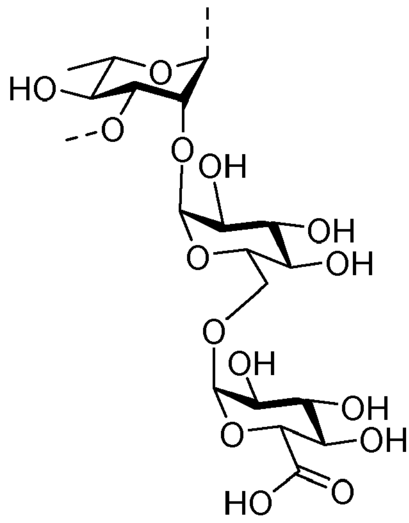 ,

$A_3$ = 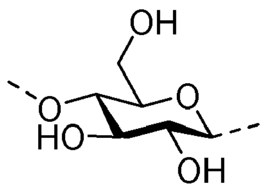 ; $A_4$ = 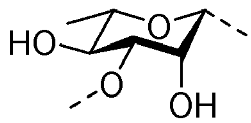 ;

B*- represents H-, H-$A_1$-, H-$A_2$-$A_1$-, H-$A_3$-$A_2$-$A_1$- or H-$A_4$-$A_3$-$A_2$-$A_1$-; and $L^2$, m2, -$W^2$- and carrier-protein2 have the meanings defined herein.

More preferably, the residue B*- in general formulae (**II**), (**II-A**), (**II-C**), (**II-E**) and (**II-F**) represents H-.

Hence, the immunogenic composition of the present invention preferably contains a *S. pneumoniae* serotype 2 conjugate of general formula (**II-G**):

$$[H\text{-}A_{y+3}\text{-}A_{y+2}\text{-}A_{y+1}\text{-}A_y\text{-}O\text{-}L^2\text{-}NH\text{-}W^2]_{m2}\text{-carrier-protein2} \qquad \text{(II-G)}$$

wherein

y is an integer selected from 1,2,3 and 4;

$A_1 = A_5 =$ 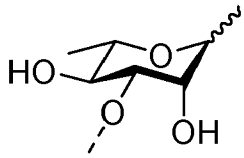 ; $A_2 = A_6 =$ 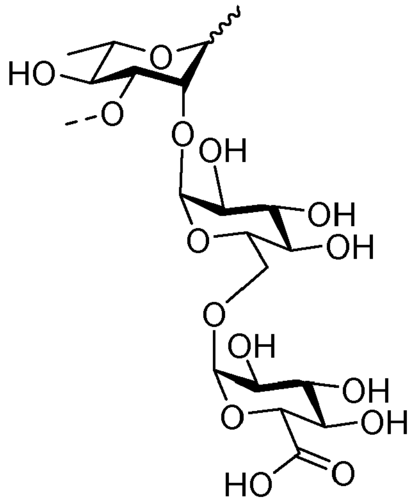 ,

$A_3 = A_7 =$ 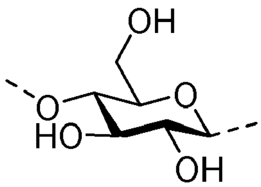 ; $A_4 =$ 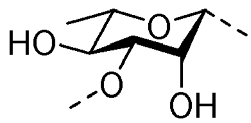 ;

and $L^2$, m2, -$W^2$- and carrier-protein2 have the meanings defined herein.

More preferably, the immunogenic composition of the present invention contains a *S. pneumoniae* serotype 2 conjugate of general formula (**II-B**):

$$\mathbf{[H\text{-}A_{y+3}\text{-}A_{y+2}\text{-}A_{y+1}\text{-}A_y\text{-}O\text{-}L^2\text{-}NH\text{-}W^2]_{m2}\text{-}carrier\text{-}protein2} \qquad (\mathbf{II\text{-}B})$$

wherein

y is an integer selected from 1,2,3 and 4;

$A_1 = A_5 =$ 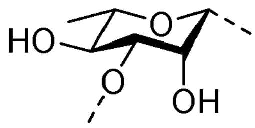 ; $A_2 = A_6 =$ 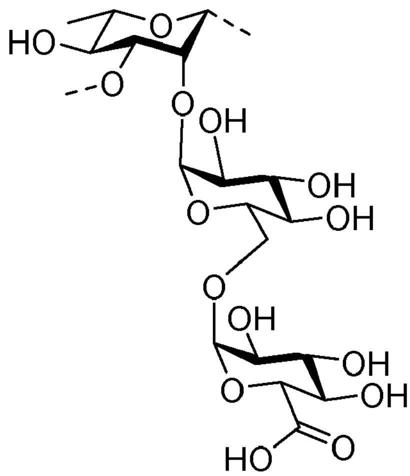 ,

$A_3 = A_7 =$ 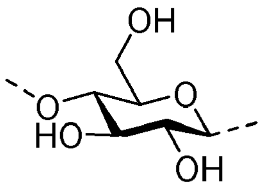 ; $A_4 =$ 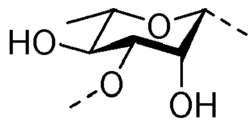 ;

and $L^2$, m2, $-W^2-$ and carrier-protein2 have the meanings defined herein.

Even more preferably, the immunogenic composition of the present invention contains a S. *pneumoniae* serotype 2 conjugate of general formula (**II-H**):

$$[H\text{-}A_{y+3}\text{-}A_{y+2}\text{-}A_{y+1}\text{-}A_y\text{-}O\text{-}L^2\text{-}NH\text{-}W^2]_{m2}\text{-carrier-protein2} \qquad (\mathbf{II\text{-}H})$$

wherein

y is an integer selected from 1,2,3 and 4;

$A_1 = A_5 =$ 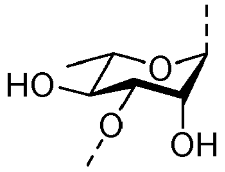 ; $A_2 = A_6 =$ 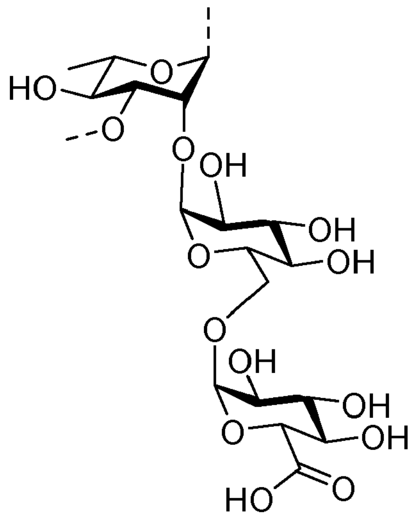 ,

$A_3 = A_7 =$ 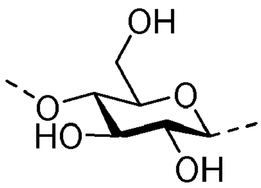 ; $A_4 =$ 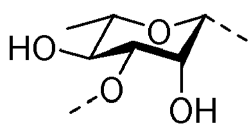 ;

and $L^2$, m2, $-W^2-$and carrier-protein2 have the meanings defined herein.

Even more preferred is an immunogenic composition comprising a conjugate of a saccharide from *S. pneumoniae* serotype 8 and a carrier protein as defined above, a conjugate of general formula (**II-A**) wherein B*- represents H-, and a mixture consisting of capsular polysaccharides from *Streptococcus pneumoniae* serotypes 1, 3, 4, 5, 6A, 6B, 7F, 9V, 14, 18C, 19A, 19F and 23F individually conjugated to $CRM_{197}$ carrier protein; or

a mixture consisting of capsular polysaccharides from *Streptococcus pneumoniae* serotypes 1, 4, 5, 6B, 7F, 9V, 14, 18C, 19F and 23F individually conjugated to a carrier protein, wherein the capsular polysaccharides from *Streptococcus pneumoniae* serotypes 1, 4, 5, 6B, 7F, 9V, 14, and 23F are individually conjugated to protein D, the capsular polysaccharide from *Streptococcus pneumoniae* serotype 18C is conjugated to tetanus toxoid and the capsular polysaccharide from *Streptococcus pneumoniae* serotype 19F is conjugated to diphtheria toxoid.

Most preferably, the immunogenic composition of the present invention contains a *S. pneumoniae* serotype 2 conjugate of general formula (**II-I**)

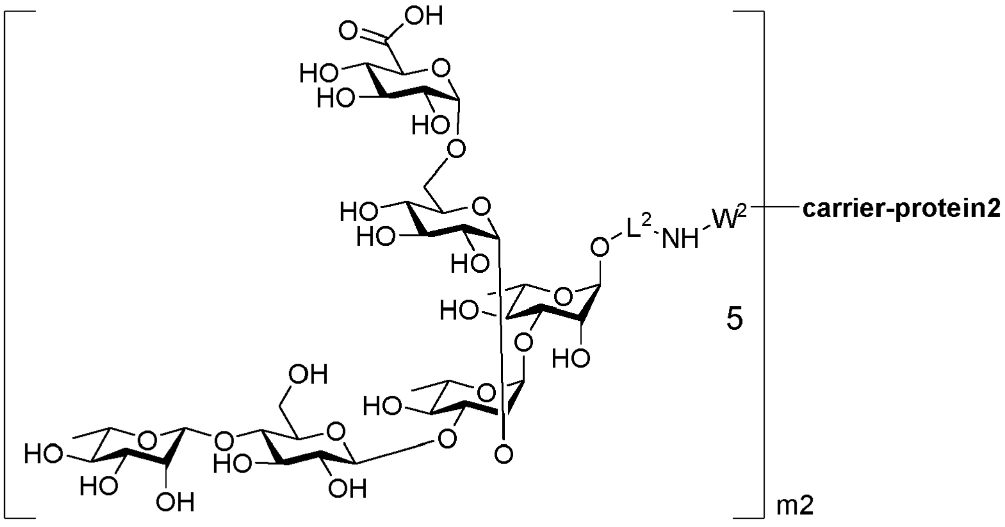

(II-I)

wherein $L^2$, m2, $-W^2-$ and carrier-protein2 have the meanings defined herein.

Preferably the linker $-L^2-$ is selected from: $-L^{a*}-$, $-L^{a*}-L^{e*}-$, $-L^{a*}-L^{b*}-L^{e*}-$, $-L^{a*}-L^{d*}-L^{e*}-$; wherein

$-L^{a*}-$ is selected from: $-(CH_2)_{o*}-$, $-(CH_2-CH_2-O)_{o*}-C_2H_4-$, $-(CH_2-CH_2-O)_{o*}-CH_2-$;

$-L^{b*}-$ represents -O-;

$-L^{d*}-$ is selected from: $-(CH_2)_{q*}-$, $-(CF_2)q_*-$, $-(CH_2-CH_2-O)_{q*}-C_2H_4-$, and $-(CH_2-CH_2-O)_{q*}-CH_2-$;

$-L^{e*}-$ is selected from: $-(CH_2)_{p1*}-$, $-(CF_2)_{p1*}-$, $-C_2H_4-(O-CH_2-CH_2)_{p1*}-$, $-CH_2-(O-CH_2-CH_2)_{p1*}-$ and $-(CH_2)_{p1*}-O-(CH_2)_{p2*}-$;

and o*, q*, p1* and p2* are independently of each other an integer selected from 1, 2, 3, 4, 5, and 6.

Hence, the inventive immunogenic composition provided herein preferably contains a conjugate of general formula (**II**), (**II-A**), (**II-B**), (**II-C**), (**II-D**), (**II-E**), (**II-F**). (**II-H**), (**II-I**) or (**II-A**) with B*- being H-, wherein $-L^2-$ is selected from: $-L^{a*}-$, $-L^{a*}-L^{e*}-$, $-L^{a*}-L^{b*}-L^{e*}-$, $-L^{a*}-L^{d*}-L^{e*}-$; wherein $-L^{a*}-$ is selected from: $-(CH_2)_{o*}-$, $-(CH_2-CH_2-O)_{o*}-C_2H_4-$, $-(CH_2-CH_2-O)_{o*}-CH_2-$;

$-L^{b*}-$ represents -O-;

$-L^{d*}-$ is selected from: $-(CH_2)_{q*}-$, $-(CF_2)_{q*}-$, $-(CH_2-CH_2-O)_{q*}-C_2H_4-$, and $-(CH_2-CH_2-O)_{q*}-CH_2-$;

$-L^{e*}-$ is selected from: $-(CH_2)_{p1*}-$, $-(CF_2)_{p1*}-$, $-C_2H_4-(O-CH_2-CH_2)_{p1*}-$, $-CH_2-(O-CH_2-CH_2)_{p1*}-$ and $-(CH_2)_{p1*}-O-(CH_2)_{p2*}-$;

and o*, q*, p1* and p2* are independently of each other an integer selected from 1, 2, 3, 4, 5, and 6.

In a more preferred embodiment, the linker $-L^2-$ in general formulae (**II**), (**II-A**), (**II-B**), (**II-C**), (**II-D**), (**II-E**), (**II-F**). (**II-H**), (**II-I**) and (**II-A**) with B*- being H-, represents $-(CH_2)_{o*}-$ and o* is an integer selected from 2, 3, 4, 5, 6, 7 and 8.

It is also preferred that $-W^2-$ represents

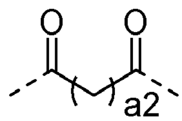

and a2 is an integer selected from 2, 3, 4, 5 and 6.

It is further preferred that the carrier-protein2 is $CRM_{197}$ and/or m2 is comprised between 4 and 15, and more preferably between 6 and 12.

Immunization with an immunogenic composition comprising a conjugate of a saccharide from *S. pneumoniae* serotype 8 and a carrier protein, such as a conjugate of general formula (**I**), (**I-A**) or (**I-B**) defined above, a conjugate of general formula (**II**), (**II-A**), (**II-B**), (**II-C**), (**II-D**), (**II-E**), (**II-F**), (**II-H**) or (**II-I**) as defined above, and a mixture consisting of capsular polysaccharides from *S. pneumoniae* serotypes 1, 3, 4, 5, 6A, 6B, 7F, 9V, 14, 18C, 19A, 19F and 23F individually conjugated to $CRM_{197}$ carrier protein, or a mixture consisting of capsular polysaccharides from *S. pneumoniae* serotypes 1, 4, 5, 6B, 7F, 9V, 14, 18C, 19F and 23F individually conjugated to a carrier protein, wherein the capsular polysaccharides from *S. pneumoniae* serotypes 1, 4, 5, 6B, 7F, 9V, 14, and 23F are individually conjugated to protein D, the capsular polysaccharide from S. *pneumoniae* serotype 18C is conjugated to tetanus toxoid and the capsular polysaccharide from *S. pneumoniae* serotype 19F is conjugated to diphtheria toxoid induces a robust immune response against the capsular polysaccharide from *Streptococcus pneumoniae* serotypes 8 and 2, thereby leading to opsonophagocytic killing of

Streptococcus pneumoniae serotype 8 and 2 pneumococci, without impairing the immune response against the capsular polysaccharides from the other *S. pneumoniae* serotypes, which are present in the immunogenic composition. Therefore, said immunogenic compositions are useful for the prevention and/or treatment of diseases caused by *Streptococcus pneumoniae* serotype 8 and serotype 2.

A further aspect of the present invention is directed to an improved immunogenic composition comprising a conjugate of general formula (**II**), (**II-A**), (**II-B**), (**II-C**), (**II-D**), (**II-E**), (**II-F**), (**II-H**), or (**II-I**) as defined above and a mixture consisting of capsular polysaccharides from *S. pneumoniae* serotypes 1, 3, 4, 5, 6A, 6B, 7F, 9V, 14, 18C, 19A, 19F and 23F individually conjugated to $CRM_{197}$ carrier protein; or a mixture consisting of capsular polysaccharides from *S. pneumoniae* serotypes 1, 4, 5, 6B, 7F, 9V, 14, 18C, 19F and 23F individually conjugated to a carrier protein, wherein the capsular polysaccharides from *S. pneumoniae* serotypes 1, 4, 5, 6B, 7F, 9V, 14 and 23F are individually conjugated to protein D, the capsular polysaccharide from *S. pneumoniae* serotype 18C is conjugated to tetanus toxoid and the capsular polysaccharide from *S. pneumoniae* serotype 19F is conjugated to diphtheria toxoid. Immunization with such immunogenic composition induces a robust immune response against the capsular polysaccharide from *Streptococcus pneumoniae* serotypes 2, thereby leading to opsonophagocytic killing of *Streptococcus pneumoniae* serotype 2 pneumococci, without impairing the immune response against the capsular polysaccharides from the other *S. pneumoniae* serotypes, which are present in the immunogenic composition. Therefore, said immunogenic composition is useful for the prevention and/or treatment of diseases caused by *Streptococcus pneumoniae* serotype 2.

The immunogenic compositions comprising the above-defined conjugate of a saccharide from *S. pneumoniae* serotype 8 and a carrier protein, the mixture consisting of capsular polysaccharides from *S. pneumoniae* serotypes 1,4,5, 6B, 7F, 9V, 14, 18C, 19F and 23F individually conjugated to a carrier protein, wherein the capsular polysaccharides from *S. pneumoniae* serotypes 1, 4, 5, 6B, 7F, 9V, 14 and 23F are individually conjugated to protein D, the capsular polysaccharide from *S. pneumoniae* serotype 18C is conjugated to tetanus toxoid and the capsular polysaccharide from *S. pneumoniae* serotype 19F is conjugated to diphtheria toxoid, and optionally a *S. pneumoniae* serotype 2 conjugate of general formula (**II**), (**II-A**), (**II-B**), (**II-C**), (**II-D**), (**II-E**), (**II-F**), (**II-H**) or (**II-I**) as defined above, may further contain a S. *pneumoniae* serotype 3 conjugate of general formula (**III**):

$$\mathbf{[H\text{-}(C)_{c3}\text{-}(D\text{-}C)_{c2}\text{-}(D)_{c1}\text{-}O\text{-}L^3\text{-}NH\text{-}W^3]_{m3}\text{-}carrier\text{-}protein3} \qquad \mathbf{(III)}$$

wherein

C represents:

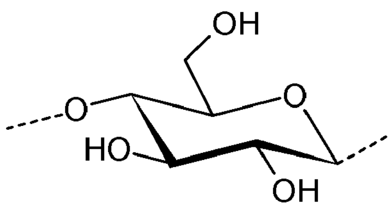

D represents

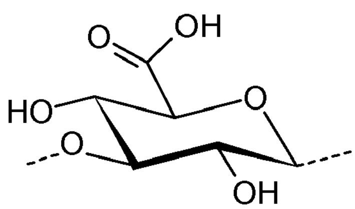

c2 is an integer selected from 1, 2, 3, 4, 5, 6, 7, 8, 9 and 10;
c1 and c3 represent independently of each other an integer selected from 0 and 1;
$L^3$ represents a linker;
m3 is comprised between about 2 and about 18;
$-W^3-$ is selected from:

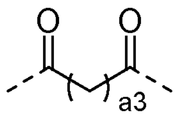 , 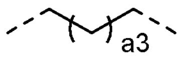 , and 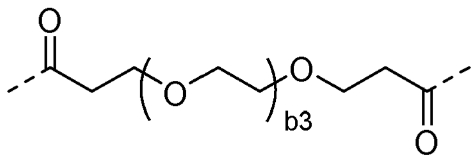 ;

a3 represents an integer from 1 to 10;
b3 represents an integer from 1 to 4; and
carrier-protein3 is selected from $CRM_{197}$, protein D, tetanus toxoid and diphtheria toxoid.

$-L^3-$ is defined as a linker and is part of the fragment $-O-L^3-NH-$. Thus, the linker $-L^3-$ is bound to an oxygen atom and to the nitrogen atom of the $-NH-W^3$-fragment. It is preferred that at least two carbon atoms of the linker are between the oxygen atom and the $-NH-W^3-$ fragment, like $-O-C-C-NH-W^3-$. The linker $-L^3-$ can be an aliphatic chain, wherein the aliphatic chain can optionally include an aromatic chain inserted in it, or a number of heteroatoms oscillating from 0 to 10. The linker $-L^3-$ preferably contains between 2 and 40 carbon atoms (including the carbon atoms of optional side chains), more preferably between 2 and 30, more preferably between 2 and 20, more preferably between 2 and 14, more preferably between 2 and 12, and still more preferably between 2 and 10 carbon atoms. The shortest atom chain between the oxygen atom (i.e. the oxygen of $-O-L^3-NH-$) and the $-NH-W^3-$ fragment consists preferably of 2 to 14 atoms, more preferably of 2 to 12 atoms, more preferably of 2 to 10 atoms, more preferably of 2 to 8 atoms. In case the shortest chain (which is the shortest possible connection between the oxygen at the anomeric center and the $-NH-W^3$-fragment) consists of 2 to 6 atoms, these are preferably carbon atoms. In case the shortest chain consists of 4 to 8 atoms, the chain may contain 1, 2 or 3 heteroatoms selected from O, N and S. In case the shortest chain consists of 9 to 14 atoms, the chain may contain 1, 2, 3, 4, 5, or 6 heteroatoms selected from O, N and S.

It is also preferred that the linker $-L^3-$, or the shortest chain is fully or partially fluorinated. The linker $-L^3-$ may contain a 3-membered or a 4-membered or a 5-membered or a 6-membered saturated carbocycle or a 5-membered partly unsaturated (and not aromatic) carbocycle or a 4-membered or a 5-membered or a 6-membered saturated oxygen heterocycle or a 4-membered or a 5-membered or a 6-membered saturated nitrogen heterocycle or a 6-membered aromatic carbocycle.

The linker $-L^3-$ may also contain amide (-NH-CO-, -CO-NH-) and/or urea (-NH-CO-NH-) residues and preferably only one amide or urea residue. The linker may also contain substituents and preferably two substituents, such as $R^{10**}$ and $R^{11**}$, or four substituents such as $R^{10**}$, $R^{11**}$, $R^{15**}$ and $R^{14**}$, which have the meanings as defined herein and which are preferably selected from: -F, -Cl, $-CH_3$, $-C_2H_5$, $-C_3H_7$, $-C_5H_9$, $-C_6H_{13}$, $-OCH_3$, $-OC_2H_5$, $-CH_2F$, $-CHF_2$, $-CF_3$, $-C(O)-NH_2$, $-SCH_3$, $-SC_2H_5$, $-NHC(O)CH_3$, $-N(CH_3)_2$, and $-N(C_2H_5)_2$;
In case the linker $-L^3-$ is fluorinated, more than two substituents -F are preferred.

Linker $-L^3-$ is preferably selected from: $-CH_2-$, $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-(CH_2)_9-$, $-(CH_2)_{10}-$, $-CF_2-$, $-(CF_2)_2-$, $-(CF_2)_3-$, $-(CF_2)_4-$, $-(CF_2)_5-$, $-(CF_2)_6-$, $-(CF_2)_7-$, $-(CF_2)_8-$, $-(CF_2)_9-$, $-(CF_2)_{10}-$, $-(CH_2)_2-O-(CH_2)_2-$, $-CH_2-O-(CH_2)_3-$, $-(CH_2)_3-O-CH_2--CH_2-O-(CH_2)_2-$, $-(CH_2)_2-O-CH_2-$, $-(CH_2)_3-O-(CH_2)_2-$, $-(CH_2)_2-O-(CH_2)_3-$, $-(CH_2)_4-O-CH_2-$, $-CH_2-O-(CH_2)_4-$, $-L^{a**}-$, $-L^{a**}-L^{e**}-$, $-L^{a**}-L^{b**}-L^{e**}-$, $-L^{a**}-L^{b**}-L^{d**}-L^{c**}-L^{e**}-$, $-L^{a*}-L^{d**}-L^{e**}-$;
wherein
$-L^{a**}-$ is selected from: $-(CH_2)_{o**}-$, $-(CF_2)_{o**}-$, $-(CH_2-CH_2-O)_{o**}-C_2H_4-$, $-(CH_2-CH_2-O)_{o**}-CH_2-$, $-(CR^{10*}R^{11*})_{o**}-$,

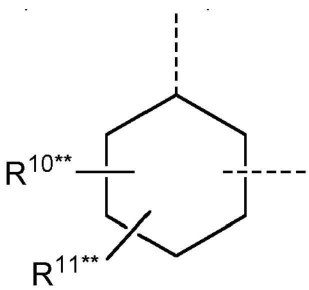 , 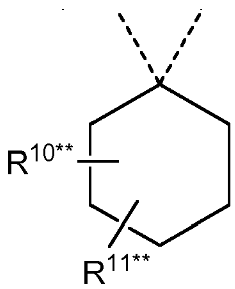 , 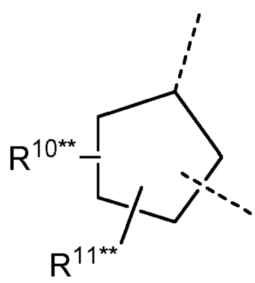 , 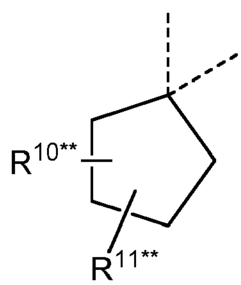 ,

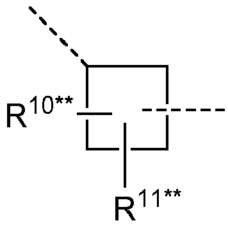,

and

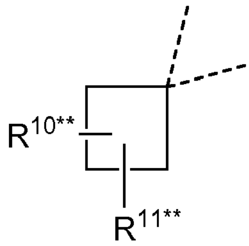;

$-L^{b**}-$ and $-L^{c**}-$ are independently of each other selected from: -O-, -NH-C(O)-NH-, -NH-C(S)-NH-, -NH-C(O)-, -C(O)-NH-, -NH-C(O)-O-, $-NR^{9**}-$, -NR18**-, $-SO_2-$,

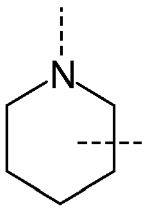, 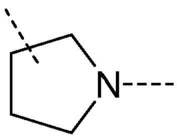, 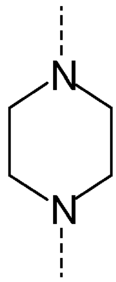, 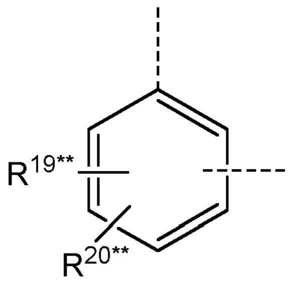,

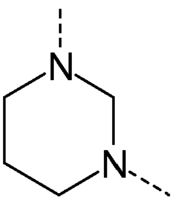, 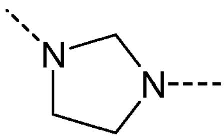, 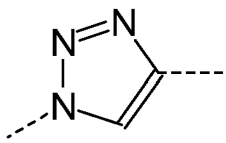, 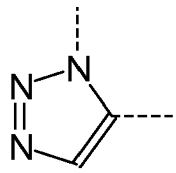,

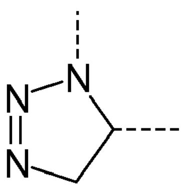, 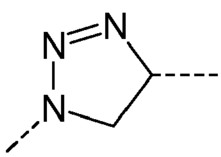, and 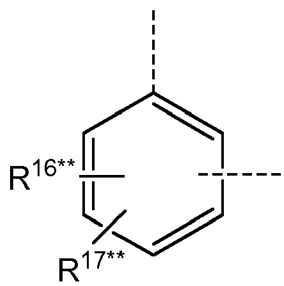;

$-L^{d**}-$ represents: $-(CH_2)_{q**}-$, $-(CF_2)_{q**}-$, $-(CR^{12**}R^{13**})_{q**}-$, $-(CH_2-CH_2-O)_{q**}-C_2H_4-$, $-(CH_2-CH_2-O)_{q**}-CH_2-$,

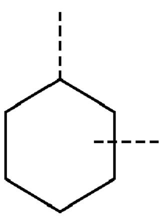, 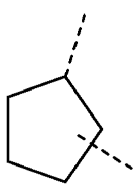, 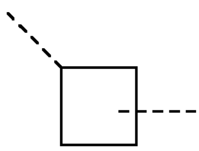, 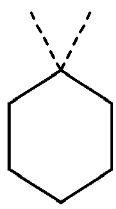,

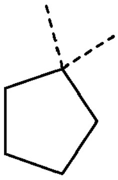

,

and

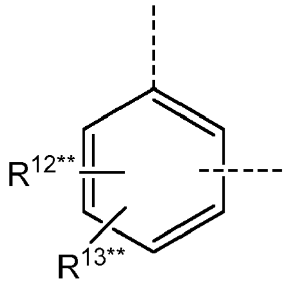

;

-$L^{e**}$- is selected from: -$(CH_2)_{p1**}$-, -$(CF_2)_{p1**}$-, -$C_2H_4$-$(O-CH_2-CH_2)_{p1**}$-, -$CH_2$-$(O-CH_2-CH_2)_{p1**}$-, -$(CH_2)_{p1**}$-O-$(CH_2)_{p2**}$-, -$(CR^{14**}R^{15**})_{p1**}$-, -$(CR^{14**}R^{15**})_{p1**}$-O-$(CR^{21**}R^{22**})_{p2**}$-,

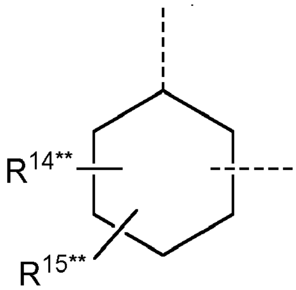

,

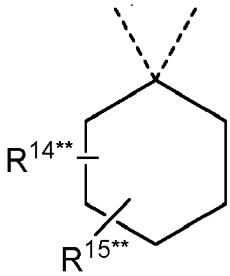

,

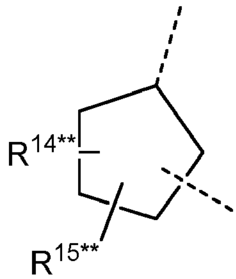

,

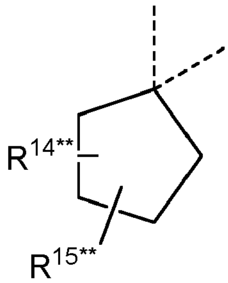

,

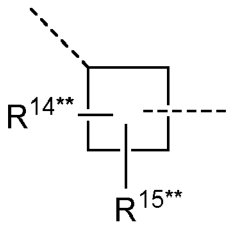

,

and

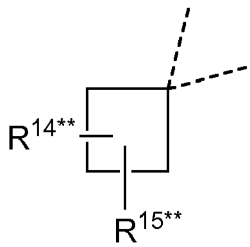

;

$R^{9**}$ and $R^{18**}$ are independently of each other selected from: -$CH_3$, -$C_2H_5$, -$C_3H_7$, and -C(O)$CH_3$;

$R^{10**}$, $R^{11**}$, $R^{12**}$, $R^{13**}$, $R^{14**}$, $R^{15**}$, $R^{16**}$, $R^{17**}$, $R^{19**}$, $R^{20**}$, $R^{21**}$ and $R^{22**}$ are independently of each other selected from: -H, -F, -Cl, -$CH_3$, -$C_2H_5$, -$C_3H_7$, -$C_5H_9$, -$C_6H_{13}$, -$OCH_3$, -$OC_2H_5$, -$CH_2F$, -$CHF_2$, -$CF_3$, -C(O)-$NH_2$, -$SCH_3$, -$SC_2H_5$, -NHC(O)$CH_3$, -$N(CH_3)_2$ and -$N(C_2H_5)_2$;

o**, q**, p1** and p2** are independently of each other an integer selected from 1, 2, 3, 4, 5, 6, 7, 8, 9, and 10.

In general formula (**III**), preferably c1 and c3 represent 0. Thus, an immunogenic composition comprising the above-defined conjugate of a saccharide from *S. pneumoniae* serotype 8 and a carrier protein, the mixture consisting of capsular polysaccharides from *S. pneumoniae* serotypes 1, 4, 5, 6B, 7F, 9V, 14, 18C, 19F and 23F individually conjugated to a carrier protein, wherein the capsular polysaccharides from *S. pneumoniae* serotypes 1, 4, 5, 6B, 7F, 9V, 14, and 23F are individually conjugated to protein D, the capsular polysaccharide from *S. pneumoniae* serotype 18C is conjugated to tetanus toxoid and the capsular polysaccharide from *S. pneumoniae* serotype 19F is conjugated to diphtheria toxoid, optionally a *S. pneumoniae* serotype 2 conjugate of general formula (**II**), (**II-A**), (**II-B**), (**II-C**), (**II-D**), (**II-E**), (**II-F**), (**II-H**) or (**II-I**) as defined above, and a *S. pneumoniae* serotype 3 conjugate of general formula (**III-A**):

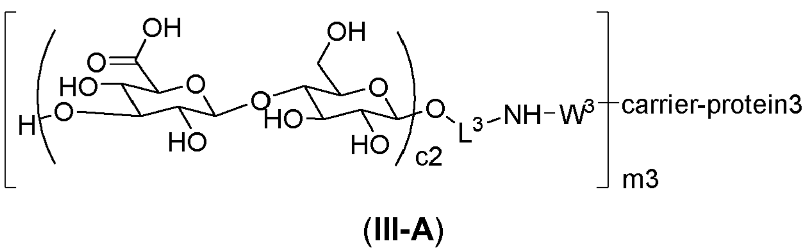

**(III-A)**

wherein c2, $L^3$, $-W^3-$, m3 and carrier-protein3 have the meanings defined herein, is especially preferred.

Preferably the linker $-L^3-$ is selected from: $-L^{a**}-$, $-L^{a**}-L^{e**}-$, $-L^{a**}-L^{b**}-L^{e**}-$, $-L^{a**}-L^{d**}-L^{e**}-$; wherein
$-L^{a**}-$ is selected from: $-(CH_2)_{o**}-$, $-(CH_2-CH_2-O)_{o**}-C_2H_4-$,
$-(CH_2-CH_2-O)_{o**}-CH_2-$;
$-L^{b**}-$ represents -O-;
$-L^{d**}-$ is selected from: $-(CH_2)_{q**}-$, $-(CF_2)_{q**}-$, $-(CH_2-CH_2-O)_{q**}-C_2H_4-$, and $-(CH_2-CH_2-O)_{q**}-CH_2-$;
$-L^{e**}-$ is selected from: $-(CH_2)_{p1**}-$, $-(CF_2)_{p1**}-$, $-C_2H_4-(O-CH_2-CH_2)_{p1**}-$,
$-CH_2-(O-CH_2-CH_2)_{p1**}-$ and $-(CH_2)_{p1*}-O-(CH_2)_{p2**}-$;
and o**, q**, p1** and p2** are independently of each other an integer selected from 1, 2, 3, 4, 5, and 6.

More preferably, the linker $-L^3-$ represents $-(CH_2)_{o**}-$ and o** is an integer selected from 2, 3, 4, 5, 6, 7 and 8.

It is also preferred that $-W^3-$ represents

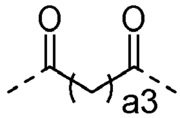

and a3 is an integer selected from 2, 3, 4, 5 and 6.

It is further preferred that the carrier-protein3 is $CRM_{197}$ and/or m3 is comprised between 3 and 14, and more preferably between 4 and 10. Moreover, it is preferred that c2 is an integer selected from 2, 3, 4 and 5, and even more preferred that c2 represents 2.

Surprisingly, it was found that immunization with an immunogenic composition comprising a conjugate of a saccharide from *S. pneumoniae* serotype 8 and a carrier protein, such as a conjugate of general formula (**I**), (**I-A**) or (**I-B**) defined above, a conjugate of general formula (**III**) or (**III-A**) as defined above, and a mixture consisting of capsular polysaccharides from *S. pneumoniae* serotypes 1, 4, 5, 6B, 7F, 9V, 14, 18C, 19F and 23F individually conjugated to a carrier protein, wherein the capsular polysaccharides from *S. pneumoniae* serotypes 1, 4, 5, 6B, 7F, 9V, 14 and 23F are individually conjugated to protein D, the capsular polysaccharide from *S. pneumoniae* serotype 18C is conjugated to tetanus toxoid and the capsular polysaccharide from *S. pneumoniae* serotype 19F is conjugated to diphtheria toxoid induces a robust immune response against the capsular polysaccharide from *Streptococcus pneumoniae* serotypes 8 and 3, thereby leading to opsonophagocytic killing of *Streptococcus pneumoniae* serotype 8 and 3 pneumococci, without impairing the immune response against the capsular polysaccharides from the other *S. pneumoniae* serotypes, which are present in the immunogenic composition. Therefore, said immunogenic compositions are useful for the prevention and/or treatment of diseases caused by *Streptococcus pneumoniae* serotype 8 and serotype 3.

Furthermore, immunization with an immunogenic composition comprising a conjugate of a saccharide from *S. pneumoniae* serotype 8 and a carrier protein, such as a conjugate of general formula (**I**), (**I-A**) or (**I-B**) defined above, a *S. pneumoniae* serotype 2 conjugate of general formula (**II**), (**II-A**), (**II-B**), (**II-C**), (**II-D**), (**II-E**), (**II-F**). (**II-H**), or (**II-I**) as defined above, a *S. pneumoniae* serotype 3 conjugate of general formula (**III**) or (**III-A**) as defined above, and a mixture consisting of capsular polysaccharides from *S. pneumoniae* serotypes 1,4,5, 6B, 7F, 9V, 14, 18C, 19F and 23F individually conjugated to a carrier protein, wherein the capsular polysaccharides from *S. pneumoniae* serotypes 1, 4, 5, 6B, 7F, 9V, 14 and 23F are individually conjugated to protein D, the capsular polysaccharide from *S. pneumoniae* serotype 18C is conjugated to tetanus toxoid and the capsular polysaccharide from *S. pneumoniae* serotype 19F is conjugated to diphtheria toxoid induces a robust immune response against the capsular polysaccharide from *Streptococcus pneumoniae* serotypes 8, 2 and 3, thereby leading to opsonophagocytic killing of *Streptococcus pneumoniae* serotype 8, 2 and 3 pneumococci, without impairing the immune response against the capsular polysaccharides from the other *S. pneumoniae* serotypes, which are present in the immunogenic composition. Therefore, said immunogenic compositions are useful for the prevention and/or treatment of diseases caused by *Streptococcus pneumoniae* serotypes 8, 2 and 3.

A further aspect of the present invention is directed to a vaccine that comprises the inventive immunogenic composition disclosed herein, a physiologically acceptable vehicle and an adjuvant. Thus, a vaccine can be obtained by formulating the inventive immunogenic composition with a physiologically acceptable vehicle and an adjuvant by methods known in the art.

A physiologically acceptable vehicle may include non-toxic levels of alcohols and salts, 5% dextrose or other sugars, saline, and other pharmaceutically acceptable excipients, and any combination of any of these solvents. Such excipients are well known and described. Preferably used physiologically acceptable vehicles are water, buffered saline, succinic acid, polysorbate 80, polyols (e.g., glycerol, propylene glycol, liquid polyethylene glycol) and dextrose solutions.

The term **"adjuvant"** as used herein refers to an immunological adjuvant i.e. a material used in a vaccine composition that modifies or augments the effects of said vaccine by enhancing the immune response to a given antigen contained in the vaccine without being antigenically related to it. For the persons skilled in the art, classically recognized examples of immunological adjuvants include, but are not restricted to

(1) aluminum salts (alum), such as aluminum hydroxide, aluminum phosphate, aluminum sulfate, etc.;
(2) oil-in-water emulsion formulations (with or without other specific immunostimulating agents such as muramyl peptides (defined below) or bacterial cell wall components), such as, for example,

(a) MF59 (WO 90/14837), containing 5% Squalene, 0.5% Tween 80, and 0.5% Span 85 (optionally containing various amounts of MTP-PE (see below, although not required)) formulated into submicron particles using a microfluidizer such as Model 110Y microfluidizer (Microfluidics, Newton, MA),
(b) SAF, containing 10% Squalene, 0.4% Tween 80, 5% pluronic-blocked polymer L121 , and thr-MDP (see below) either microfluidized into a submicron emulsion or vortexed to generate a larger particle size emulsion, and
(c) Ribi(TM) adjuvant system (RAS), (Corixa, Hamilton, MT) containing 2% Squalene, 0.2% Tween 80, and one or more bacterial cell wall components from the group consisting of 3-O-deaylated monophosphorylipid A (MPL(TM)) described in U.S. Patent No. 4,912,094 (Corixa), trehalose dimycolate (TDM), and cell wall skeleton (CWS), preferably MPL + CWS (Detox™);

(3) saponin adjuvants, such as Quil A or STIMULON(TM) QS-21 (Antigenics, Framingham, MA) (U.S. Patent No. 5,057,540) may be used or particles generated therefrom such as ISCOMs (immunostimulating complexes);
(4) bacterial lipopolysaccharides, synthetic lipid A analogs such as aminoalkyl glucosamine phosphate compounds (AGP), or derivatives or analogs thereof, which are available from Corixa, and which are described in U.S. Patent No. 6,113,918; one such AGP is 2-[(R)-3-tetradecanoyl-oxytetradecanoylamino]ethyl 2-deoxy-4-O-phosphono-3-O-[(R)-3-tetradecanoyloxytetradecanoyl]-2-[(R)-3-tetradecanoyloxytetradecanoylamino-β-D-glucopyranoside, which is also known as 529 (formerly known as RC529), which is formulated as an aqueous form or as a stable emulsion, synthetic polynucleotides such as oligonucleotides containing CpG motif(s) (U.S. Patent No. 6,207,646);
(5) cytokines, such as interleukins (e.g., IL-1 , IL-2, IL-4, IL-5, IL-6, IL-7, IL-12, IL-15, IL-18, etc.), interferons (e.g., gamma interferon), granulocyte macrophage colony stimulating factor (GM-CSF), macrophage colony stimulating factor (M-CSF), tumor necrosis factor (TNF), costimulatory molecules B7-1 and B7-2, etc.;
(6) detoxified mutants of a bacterial ADP-ribosylating toxin such as a cholera toxin (CT) either in a wild-type or mutant form, for example, where the glutamic acid at amino acid position 29 is replaced by another amino acid, preferably a histidine, in accordance with WO 00/18434 (see also WO 02/098368 and WO 02/098369), a pertussis toxin (PT), or an E. coli heat-labile toxin (LT), particularly LT-K63, LT-R72, CT-S109, PT-K9/G129 (see, e.g., WO 93/13302 and WO 92/19265); and
(7) other substances that act as immunostimulating agents to enhance the effectiveness of the composition. Muramyl peptides include, but are not limited to, N-acetyl-muramyl-L-threonyl-D-isoglutamine (thr-MDP), N-acetyl-normuramyl-L-alanine-2-(1'-2'dipalmitoyl-sn-glycero-3-hydroxyphosphoryloxy)-ethylamine (MTP-PE)1 etc.

Vaccines are preferably in aqueous form, particularly at the point of administration, but they can also be presented in non-aqueous liquid forms or in dried forms e.g. as gelatin capsules, or as lyophilisates, etc. Vaccines may include one or more preservatives, such as thiomersal or 2-phenoxyethanol. Mercury-free compositions are preferred, and preservative-free vaccines can be prepared.

Vaccines may include a physiological salt, such as a sodium salt e.g. to control tonicity. Sodium chloride (NaCl) is typical and may be present at between 1 and 20 mg/ml. Other salts that may be present include potassium chloride, potassium dihydrogen phosphate, disodium phosphate dehydrate, magnesium chloride, calcium chloride, etc.

Vaccines can have an osmolality of between 200 mOsm/kg and 400 mOsm/kg.

Vaccines may include compounds (with or without an insoluble metal salt) in plain water (e.g. w.f.i.), but will usually include one or more buffers. Typical buffers include: a phosphate buffer; a Tris buffer; a borate buffer; a succinate buffer; a histidine buffer (particularly with an aluminium hydroxide adjuvant); or a citrate buffer. Buffer salts will typically be included in the 5-20 mM range. Vaccines typically have a pH between 5.0 and 9.5 e.g. between 6.0 and 8.0. Vaccines are preferably sterile and gluten free.

Vaccines are suitable for administration to animal (and, in particular, human) patients, and thus, include both human and veterinary uses. They may be used in a method of raising an immune response in a patient, comprising the step of administering the composition to the patient. The vaccines of the present invention may be administered before a subject is exposed to a *Streptococcus pneumoniae* type 8 and/or after a subject is exposed to a *Streptococcus pneumoniae* type 8.

Vaccines may be prepared in unit dose form. In some embodiments a unit dose may have a volume of between 0.1 - 1.0 mL e.g. about 0.5 mL. The invention also provides a delivery device (e.g. syringe, nebulizer, sprayer, inhaler, dermal patch, etc.) containing a vaccine of the invention e.g. containing a unit dose. This device can be used to administer the composition to a vertebrate subject.

The invention also provides a sterile container (e.g. a vial) containing a vaccine of the invention e.g. containing a unit dose.

The invention also provides a unit dose of a vaccine of the invention.

The invention also provides a hermetically sealed container containing a vaccine of the invention. Suitable containers include e.g. a vial.

Vaccines of the invention may be prepared in various forms. For example, the vaccines may be prepared as injectables, either as liquid solutions or suspensions. Solid forms suitable for solution in, or suspension in, liquid vehicles prior to injection can also be prepared (e.g. a lyophilized composition or a spray-freeze dried composition). The vaccine may be prepared for topical administration e.g. as an ointment, cream or powder. The vaccine may be prepared for oral administration e.g. as a tablet or capsule, as a spray, or as a syrup (optionally flavored). The vaccine may be prepared for pulmonary administration e.g. by an inhaler, using a fine powder or a spray. The vaccine may be prepared as a suppository. The vaccine may be prepared for nasal, aural or ocular administration e.g. as a spray or drops. Injectables for intramuscular administration are typical.

The inventive vaccine comprises an effective amount of an adjuvant i.e. an amount which, when administered to an individual, either in a single dose or as part of a series, is effective for enhancing the immune response to a co-administered *S. pneumoniae* type 8 antigen. This amount can vary depending upon the health and physical condition of the individual to be treated, age, the taxonomic group of individual to be treated (e.g. non-human primate, primate, etc.), the capacity of the individual's immune system to synthesize antibodies, the degree of protection desired, the formulation of the vaccine, the treating doctor's assessment of the medical situation, and other relevant factors. The amount will fall in a relatively broad range that can be determined through routine trials.

Techniques for the formulation and administration of the vaccine of the present invention may be found in "Remington's Pharmaceutical Sciences" Mack Publishing Co., Easton PA.

The amount of conjugate in each vaccine dose is selected as an amount that induces an immunoprotective response without significant, adverse effects. Such amount can vary depending upon the *S. pneumoniae* serotype. Preferably, each dose will comprise 0.1 to 100 μg of each capsular polysaccharide or saccharide, more preferably 0.1 to 10 μg, and most preferably 1 to 5 μg.

In one embodiment of the present invention the vaccine comprises the herein disclosed immunogenic composition, wherein each conjugate is adsorbed on aluminum phosphate.

Preferably, the vaccine comprises the *S. pneumoniae* serotype 8 conjugate defined above and a mixture consisting of capsular polysaccharides from *S. pneumoniae* serotypes 1, 3, 4, 5, 6A, 6B, 7F, 9V, 14, 18C, 19A, 19F and 23F individually conjugated to $CRM_{197}$ carrier protein, wherein each conjugate is adsorbed on aluminum phosphate. More preferably, the vaccine comprises the *S. pneumoniae* serotype 8 conjugate and the *S. pneumoniae* serotype 2 conjugate defined above and a mixture consisting of capsular polysaccharides from *S. pneumoniae* serotypes 1, 3, 4, 5, 6A, 6B, 7F, 9V, 14, 18C, 19A, 19F and 23F individually conjugated to $CRM_{197}$ carrier protein, wherein each conjugate is adsorbed on aluminum phosphate.

In another embodiment of the present invention the vaccine comprises the *S. pneumoniae* serotype 8 conjugate described herein, as well as a mixture consisting of 2.2 μg of each capsular polysaccharides from *S. pneumoniae* serotypes 1, 3, 4, 5, 6A, 7F, 9V, 14, 18C, 19A, 19F and 23F individually conjugated to $CRM_{197}$ carrier protein and 4.4 μg of capsular polysaccharide from *S. pneumoniae* serotype 6B conjugated to $CRM_{197}$ carrier protein, wherein each conjugate is adsorbed on aluminum phosphate and wherein the total amount of aluminum phosphate is 500 μg. In a further preferred embodiment, the vaccine comprises the *S. pneumoniae* serotype 8 conjugate and the *S. pneumoniae* serotype 2 conjugate described herein, as well as a mixture consisting of 2.2 μg of each capsular polysaccharides from *S. pneumoniae* serotypes 1, 3, 4, 5, 6A, 7F, 9V, 14, 18C, 19A, 19F and 23F individually conjugated to $CRM_{197}$ carrier protein and 4.4 μg of capsular polysaccharide from *S. pneumoniae* serotype 6B conjugated to $CRM_{197}$ carrier protein, wherein each conjugate is adsorbed on aluminum phosphate and wherein the total amount of aluminum phosphate is 500 μg. Preferably, the amount of *S. pneumoniae* serotype 8 saccharide contained in the above-disclosed vaccines is approximately 2.2 μg. Even more preferably, the amount of *S. pneumoniae* serotype 2 saccharide contained in the above-disclosed vaccines is approximately 2.2 μg.

In another embodiment of the present invention the vaccine comprises the *S. pneumoniae* serotype 8 conjugate described herein, as well as a mixture consisting of capsular polysaccharides from *S. pneumoniae* serotypes 1, 4, 5, 6B, 7F, 9V, 14, 18C, 19F and 23F individually conjugated to a carrier protein, wherein the capsular polysaccharides from *S. pneumoniae* serotypes 1, 4, 5, 6B, 7F, 9V, 14, and 23F are individually conjugated to protein D, the capsular polysaccharide from *S. pneumoniae* serotype 18C is conjugated to tetanus toxoid and the capsular polysaccharide from *S. pneumoniae* serotype 19F is conjugated to diphtheria toxoid and wherein each conjugate is adsorbed on aluminum phosphate. In a more preferred embodiment of the present invention the vaccine comprises the *S. pneumoniae* serotype 8 conjugate and the *S. pneumoniae* serotype 2 conjugate described herein, as well as a mixture consisting of capsular polysaccharides from *S. pneumoniae* serotypes 1, 4, 5, 6B, 7F, 9V, 14, 18C, 19F and 23F individually conjugated to a carrier protein, wherein the capsular polysaccharides from *S. pneumoniae* serotypes 1, 4, 5, 6B, 7F, 9V, 14, and 23F are individually conjugated to protein D, the capsular polysaccharide from *S. pneumoniae* serotype 18C is conjugated to tetanus toxoid and the capsular polysaccharide from *S. pneumoniae* serotype 19F is conjugated to diphtheria toxoid

and wherein each conjugate is adsorbed on aluminum phosphate. In a further preferred embodiment of the present invention the vaccine comprises the *S. pneumoniae* serotype 8 conjugate, the *S. pneumoniae* serotype 2 conjugate and the *S. pneumoniae* serotype 3 conjugate described herein, as well as a mixture consisting of capsular polysaccharides from *S. pneumoniae* serotypes 1, 4, 5, 6B, 7F, 9V, 14, 18C, 19F and 23F individually conjugated to a carrier protein, wherein the capsular polysaccharides from *S. pneumoniae* serotypes 1, 4, 5, 6B, 7F, 9V, 14, and 23F are individually conjugated to protein D, the capsular polysaccharide from *S. pneumoniae* serotype 18C is conjugated to tetanus toxoid and the capsular polysaccharide from *S. pneumoniae* serotype 19F is conjugated to diphtheria toxoid and wherein each conjugate is adsorbed on aluminum phosphate.

Preferably, the vaccine comprises the *S. pneumoniae* serotype 8 conjugate disclosed herein, as well as a mixture consisting of 1 μg of capsular polysaccharides from *S. pneumoniae* serotypes 1, 5, 6B, 7F, 9V, 14 and 23F individually conjugated to protein D, 3 μg of capsular polysaccharide from *S. pneumoniae* serotype 4 conjugated to protein D, 3 μg of capsular polysaccharide from *S. pneumoniae* serotype 18C conjugated to tetanus toxoid and 3 μg of capsular polysaccharide from *S. pneumoniae* serotype 19F conjugated to diphtheria toxoid and, wherein each conjugate is adsorbed on aluminum phosphate and wherein the total amount of aluminum phosphate is 500 μg. More preferably, the vaccine comprises the *S. pneumoniae* serotype 8 conjugate and the *S. pneumoniae* serotype 2 conjugate disclosed herein, as well as a mixture consisting of 1 μg of capsular polysaccharides from *S. pneumoniae* serotypes 1, 5, 6B, 7F, 9V, 14, and 23F individually conjugated to protein D, 3 μg of capsular polysaccharide from *S. pneumoniae* serotype 4 conjugated to protein D, 3 μg of capsular polysaccharide from *S. pneumoniae* serotype 18C conjugated to tetanus toxoid and 3 μg of capsular polysaccharide from *S. pneumoniae* serotype 19F conjugated to diphtheria toxoid and, wherein each conjugate is adsorbed on aluminum phosphate and wherein the total amount of aluminum phosphate is 500 μg. Even more preferably, the vaccine comprises the *S. pneumoniae* serotype 8 conjugate, the *S. pneumoniae* serotype 2 conjugate and *S. pneumoniae* serotype 3 conjugate disclosed herein, as well as a mixture consisting of 1 μg of capsular polysaccharides from *S. pneumoniae* serotypes 1, 5, 6B, 7F, 9V, 14 and 23F individually conjugated to protein D, 3 μg of capsular polysaccharide from *S. pneumoniae* serotype 4 conjugated to protein D, 3 μg of capsular polysaccharide from *S. pneumoniae* serotype 18C conjugated to tetanus toxoid and 3 μg of capsular polysaccharide from *S. pneumoniae* serotype 19F conjugated to diphtheria toxoid and, wherein each conjugate is adsorbed on aluminum phosphate and wherein the total amount of aluminum phosphate is 500 μg.

**Synthesis of *S. pneumoniae* serotype 8 conjugates of general formula (I)**

The conjugates of general formula **I**

$$[V^*\text{-}U_{x+3}\text{-}U_{x+2}\text{-}U_{x+1}\text{-}U_x\text{-O-}L^1\text{-NH-}W^1]_{m1}\text{-carrier-protein1} \qquad \text{(I)}$$

wherein V*-, x, $U_{x+3}$, $U_{x+2}$, $U_{x+1}$, $U_x$, $L^1$, $-W^1-$, m1 and carrier-protein1 have the meanings defined herein, can be obtained starting from a saccharide of general formula **1*** and carrier-protein1 using coupling methods well known to the skilled person (see **Scheme 1**).

**I**

$$V^*\text{-}U_{x+3}\text{-}U_{x+2}\text{-}U_{x+1}\text{-}U_x\text{-O-}L^1\text{-}NH_2 \quad + \quad \text{carrier-protein1}$$

**1***

**Scheme 1**: Retrosynthetic analysis of the *S. pneumoniae* serotype 8 conjugates of general formula (**I**).

Such coupling methods include, for example, treatment of the saccharide **1*** with a commercially available bifunctional spacer (Thermo Fischer, Sigma Aldrich) such as, disuccinimidyl adipate (DSA), bis-(4-nitrophenyl) adipate, bis-(4-nitrophenyl)succinate, disuccinimidyl glutarate (DSG), bis(sulfosuccinimidyl)disuccinimidyl glutarate bis(sulfosuccinimidyl)disuccinimidyl suberate (DSS), bis(sulfosuccinimidyl)suberate ($BS^3$), sebacic acid bis(N-succinimidyl) ester bis(succinimidyl)penta(ethyleneglycol), bis(succinimidyl) tetra(ethyleneglycol), in presence of a base, such as triethylamine, pyridine, followed by reaction of the resulting construct with carrier-protein1. Alternatively, saccharide **1*** can be

reacted with a suitable dialdehyde, such as glutaraldehyde, under reductive amination condition, and the resulting construct can be subsequently treated with the carrier-protein1 under reductive amination conditions.

A saccharide of general formula **1*** can be synthesized *via* several synthetic routes.

For example, saccharide **1*** can be assembled starting from thioglycoside building blocks **BB2** (see **Example C.5**, precursor for the sugar fragment $U_1$, $U_5$, $U_2$ and $U_6$), **BB3** (Angew. Chem. Int. Ed. 2013, 52, 5858; precursor for the sugar fragment $U_2$ and $U_6$), **BB4** (see **Example C.5**, precursor for the sugar fragment $U_3$ and $U_7$) and **BB5** (see **Example C.5**, precursor for the sugar fragment $U_4$) and functionalized solid support **BB1** (Angew. Chem. Int. Ed. 2013, 52, 5858.) (see **Scheme 2)** by automated solid phase synthesis.

The synthetic process, which is summarized in **Scheme 2** involves:

- assembly of the desired oligosaccharide, which includes glycosylation with the appropriate thioglycoside **(BB2, BB3, BB4** or **BB5)** by activation with NIS/TfOH; followed by removal of the temporary protecting group Fmoc by treatment with $Et_3N$;
- cleavage from the solid support; and
- removal of the permanent protecting groups.

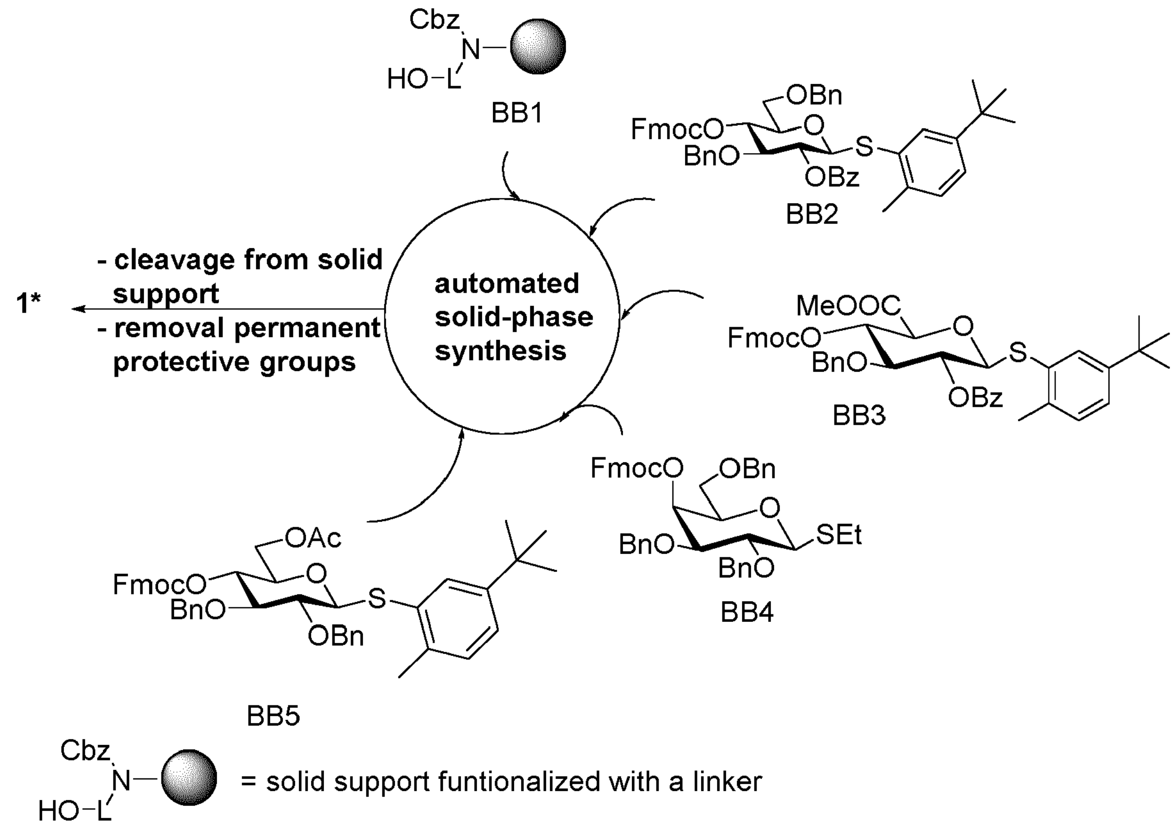

BB1
BB2
BB3
BB4
BB5

**Scheme 2**: Automated solid phase synthesis of saccharides of general formula **1***.

<u>**Synthesis of *S. pneumoniae* serotype 2 conjugates of general formula (II-C)**</u>

The conjugates of general formula **II-C**

$$[B^*\text{-}A_{y+3}\text{-}A_{y+2}\text{-}A_{y+1}\text{-}A_y\text{-}O\text{-}L^2\text{-}NH_{m2}\text{-carrier-protein2} \qquad \textbf{(II-C)}$$

wherein

y is an integer selected from 1,2,3 and 4;

$A_1 = A_5 =$ 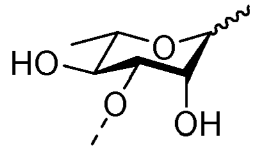 ; $A_2 = A_6 =$ 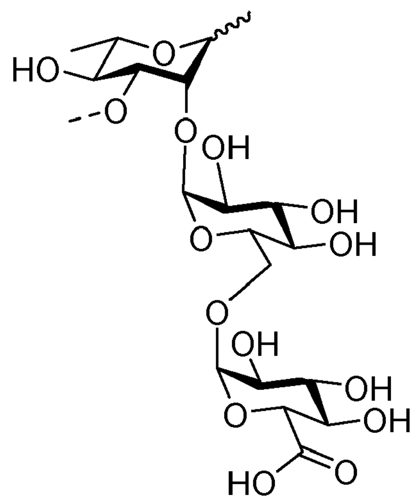 ,

$A_3 = A_7 =$ 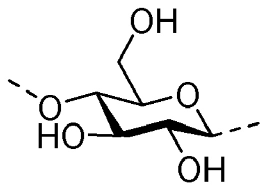 ; $A_4 =$ 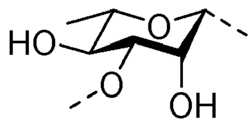 ;

B*- represents: H-, H-$A_y$, H-$A_{y+1}$-$A_y$, H-$A_{y+2}$-$A_{y+1}$-$A_y$- or H-$A_{y+3}$-$A_{y+2}$-$A_{y+1}$-$A_y$-;
$L^2$ represents a linker and is defined as above;
m2 is comprised between about 2 and about 18;
-$W^2$- is selected from:

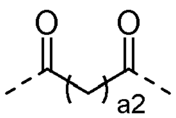 , 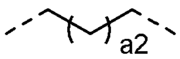 , and 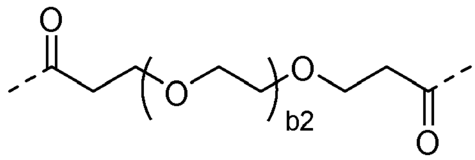 ;

a2 represents an integer from 1 to 10;
b2 represents an integer from 1 to 4; and
carrier-protein2 is selected from $CRM_{197}$, protein D, tetanus toxoid and diphtheria toxoid, can be obtained starting from a saccharide of general formula **2*** and carrier-protein2 using coupling methods well known to the skilled person (see **Scheme 3).**

**II-C**

**B*-$A_{y+3}$-$A_{y+2}$-$A_{y+1}$-$A_y$-O-$L^2$-$NH_2$** + **carrier-protein2**

**2***

**Scheme 3**: Retrosynthetic analysis of the *S. pneumoniae* serotype 2 conjugates of general formula (**II-C**).

Such coupling methods include, for example, treatment of the saccharide **2*** with a commercially available bifunctional spacer (Thermo Fischer, Sigma Aldrich) such as, disuccinimidyl adipate (DSA), bis-(4-nitrophenyl) adipate, bis-(4-nitrophenyl)succinate, disuccinimidyl glutarate (DSG), bis(sulfosuccinimidyl)disuccinimidyl glutarate bis(sulfosuccinimidyl)disuccinimidyl suberate (DSS), bis(sulfosuccinimidyl)suberate ($BS^3$), sebacic acid bis(N-succinimidyl) ester bis(succinimidyl)penta(ethyleneglycol), bis(succinimidyl) tetra(ethyleneglycol), in presence of a base, such as triethylamine, pyridine, followed by reaction of the resulting construct with carrier-protein2. Alternatively, saccharide **2*** can be reacted with a suitable dialdehyde, such as glutaraldehyde, under reductive amination conditions, and the resulting construct can be subsequently treated with the carrier-protein2 under reductive amination conditions.

A saccharide of general formula **2*** can be efficiently assembled starting from building blocks **3*** (see **Example A-1), 4*** (see **Example A-4), 5*** (see **Example A-1), 6***, **7*** (see **Example A-2),** as well as amino-alcohol linker **8*** as starting material **(Scheme 4)** and using glycosylation and deprotection method well known to the skilled person in the art. Building block **6*** can be prepared from known building block **9*** (Bundle D. R. et al. ACS Chem. Biol. 2012, 7, 1754). Conversion of building block **9*** to building block **6*** involves installing the Fmoc protecting group at 4th position of the glucoside by reaction with FmocCl and pyridine in dichloromethane, followed by treatment with CAN in a mixture of acetonitrile and water, and finally installation of the trichloroacetimidate leaving group at the anomeric position by treatment with $CCl_3CN$ and DBU in dichloromethane at room temperature.

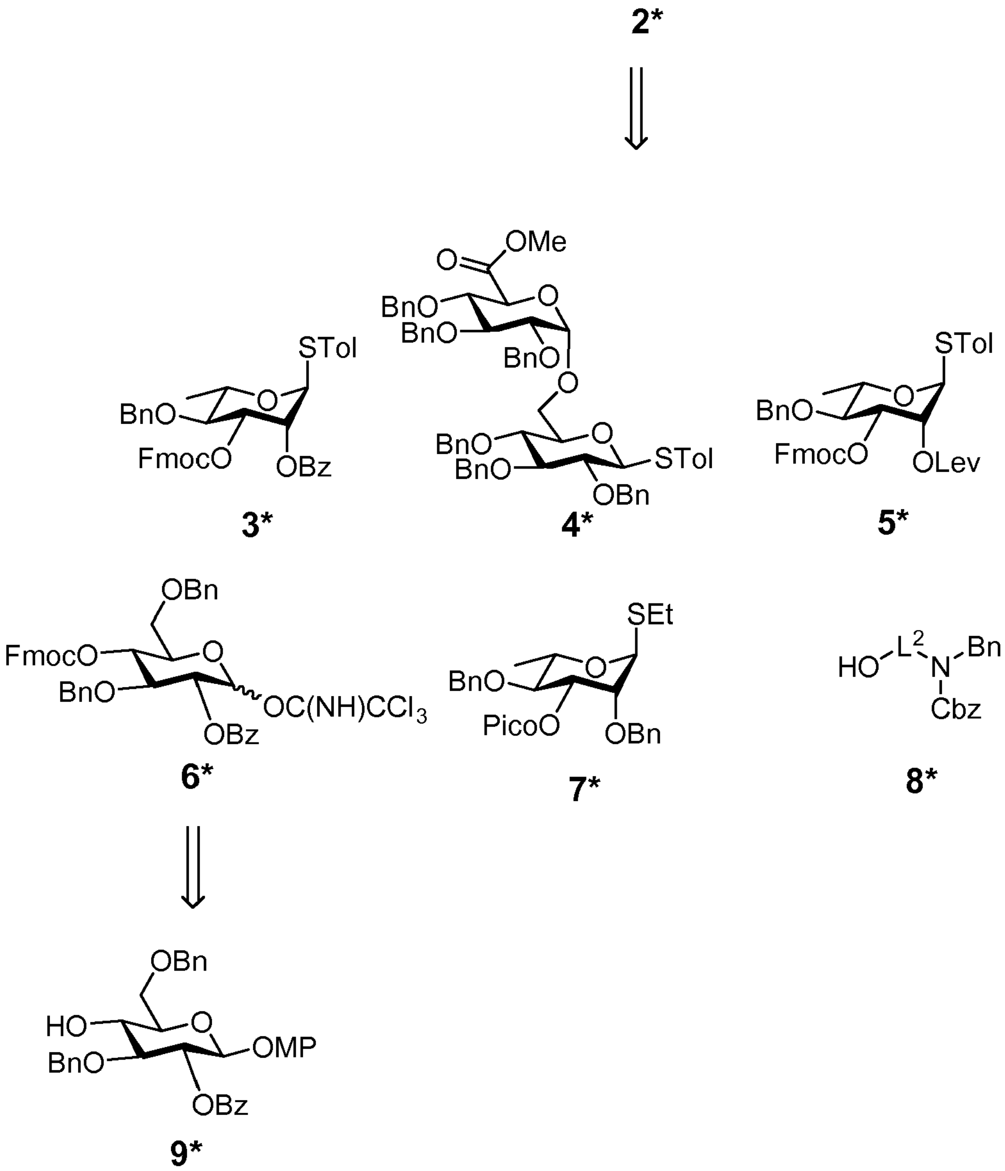

**2***

**3*** **4*** **5***

**6*** **7*** **8***

**9***

**Scheme 4**: Retrosynthetic analysis of a saccharide of general formula **2***.

**Synthesis of *S. pneumoniae* serotype 3 conjugates of general formula (III)**

The conjugates of general formula **III**

$$[H\text{-}(C)_{c3}\text{-}(D\text{-}C)_{c2}\text{-}(D\text{-}C)_{c1}\text{-}O\text{-}L^3\text{-}NH\text{-}W^3]_{m3}\text{-carrier-protein3} \qquad \textbf{(III)}$$

wherein C, D, c1, c2, c3, L3, m3, $-W^3-$ and carrier-protein3 have the meanings defined herein, can be generated starting from saccharide **10*** and carrier-protein3 using coupling methods well known to the skilled person.

**III**

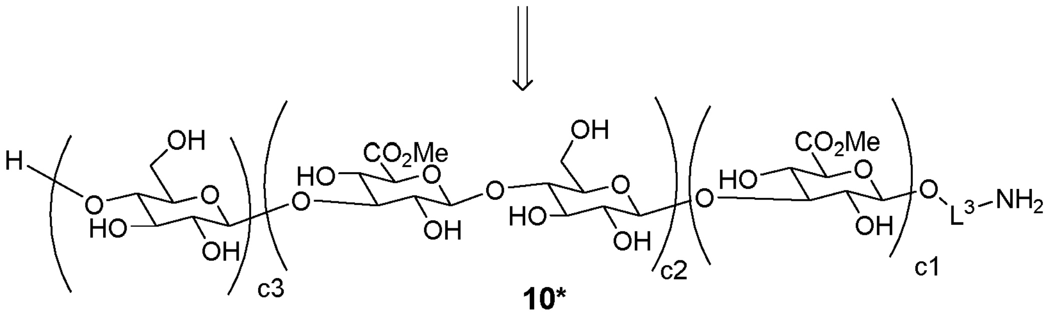

**10***

+

**carrier-protein3**

**Scheme 1**: Retrosynthetic analysis of the *S. pneumoniae* serotype 3 conjugates of general formula (**III**).

Such coupling methods include, for example, treatment of the saccharide **10*** with a commercially available bifunctional spacer (Thermo Fischer, Sigma Aldrich) such as, disuccinimidyl adipate (DSA), bis-(4-nitrophenyl) adipate, bis-(4-nitrophenyl)succinate, disuccinimidyl glutarate (DSG), bis(sulfosuccinimidyl) disuccinimidyl glutarate bis(sulfosuccinimidyl)disuccinimidyl suberate (DSS), bis(sulfosuccinimidyl)suberate ($BS^3$), sebacic acid bis(N-succinimidyl) ester, bis(succinimidyl)penta(ethyleneglycol), bis(succinimidyl)tetra(ethyleneglycol), in presence of a base, such as trimethylamine or pyridine, followed by reaction of the resulting construct with carrier-protein3. Alternatively, saccharide **10*** can be reacted with a suitable dialdehyde, such as glutaraldehyde, under reductive amination condition, and the resulting construct can be subsequently treated with the carrier-protein3 under reductive amination conditions.

Saccharides **10*** can be prepared following synthetic methods disclosed in the international patent application WO 2015/040140A1.

**Description of the figures:**

**Figure 1** shows the effect of coformulation of the conjugates **41** and **42** with Prevnar13® on the immune response against several capsular polysaccharides of *S. pneumoniae*, as assessed by polysaccharide ELISA. Sera were pre-adsorbed to pneumococcal cell wall polysaccharides (CWPs) and *S. pneumoniae* serotype 22F CPS before application. Bars depict mean ± SD of polysaccharide binding of three rabbits per group. **A** binding to *S. pneumoniae* serotype 8-CPS; **B** binding to *S. pneumoniae* serotype 1-CPS; **C:** binding to *S. pneumoniae* serotype 4-CPS; **D:** binding to *S. pneumoniae* serotype 5-CPS; **E**: binding to *S. pneumoniae* serotype 7F-CPS; **F**: binding to *S. pneumoniae* serotype 9V-CPS; **G**: binding to *S. pneumoniae* serotype 19A-CPS.

**Figure 2** (**A**) shows the evaluation of polysaccharide binding by rabbit sera at day 35 (1:200 dilution). Statistical analysis (two-tailed, unpaired t test) was performed and asterisk indicates P value: * $P < 0.05$. Bars depict mean ± SD of three rabbits per group. (**B**) shows a comparison of opsonophagocytic killing of *S. pneumoniae* serotype 8 pneumococci of pooled rabbit sera or human serum standard 007sp at different dilutions. Bars depict mean ± SD of triplicate wells of one out of three independent experiments.

**Figure 3** shows the characterization of *S. pneumoniae* serotype 2 conjugate **1** (**$CRM_{197}$- *S. pneumoniae* serotype 2 hexasaccharide 22). (A)** The protein amount was estimated using the standard curve plotted with known concentration of BSA. **(B)** The conjugate **1** (**$CRM_{197}$- *S. pneumoniae* serotype 2 hexasaccharide 22)** was resolved on 10% SDS-PAGE along with $CRM_{197}$ and stained with Coomassie brilliant blue R250. **(C)** *Matrix-assisted laser desorption/ionization (MALDI)* analysis was carried out to measure the average molecular size of the conjugate. $CRM_{197}$ was used as standard.

**Figure 4** shows the characterization of *S. pneumoniae* serotype 3 conjugate **2** (**$CRM_{197}$ - *S. pneumoniae* serotype 3 tetrasaccharide 25)** by MALDI-TOF. The mass shift (3625 Da) compared to unmodified $CRM_{197}$ (peak at m/z 61741) for this conjugate batch indicates an average loading of 4 - 5 oligosaccharides per $CRM_{197}$ molecule. An increase in molecular weight is also visible as band shift in the SDS-PAGE.

**Figure 5** shows the characterization of the absorption of the *S. pneumoniae* serotype 8 conjugates **41** and **42** to Prevnar®13: bars depict mean ± SD of the median fluorescence intensity of three independent experiments. Gates were set by omitting primary antibodies. Pooled murine sera against Prevnar13® were used as a positive control for mouse antibody binding.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

## EXAMPLES

### Abbreviations

| | |
|---|---|
| d | day(s) |
| TLC | thin layer chromatography |
| TEMPO | 2,2,6,6-tetramethyl-1-piperidinyloxy, free radical |
| Ac | Acetyl |
| AcOH | Acetic acid |
| $Ac_2O$ | Acetic anhydride |
| BAIB | Bisacetyliodobenzene |
| Bn | Benzyl |
| $^t$BuOH | *t*-Butanol |
| Bz | Benzoyl |
| CAN | Cericammonium nitrate |
| Cbz | Benzyloxycarbonyl |
| $Cu(OAc)_2$ | Copper(II) acetate |
| DBU | 1,8-Diazabicyclo[5.4.0]undec-7-ene |
| DCC | *N*,*N'*-Dicyclohexylcarbodiimide |
| DMAP | *N*,*N*-Dimethylaminopyridine |
| DMF | *N*,*N'*-Dimethylformamide |
| ESI | Electrosprayionization |
| $Et_3N$ | Triethylamine |
| Et | Ethyl |
| EtOAc | Ethyl acetate |
| FmocCl | 9-Fluorenylmethylchloroformate |
| g | Grams |
| h | Hours |

| | |
|---|---|
| HRMS | High resolution mass spectrometry |
| Lev | Levulinyl |
| min | Minute |
| mL | Millilitre |
| Me | Methyl |
| MeI | Methyl iodide |
| MeOH | Methanol |
| MP | *p*-Methoxy phenyl |
| MS | Molecular sieves |
| $NaHCO_3$ | Sodium bicarbonate |
| NaOH | Sodium hydroxide |
| NaOMe | Sodium methoxide |
| NIS | *N*-Iodo succinimide |

## General information

Commercial grade solvents were used unless stated otherwise. Dry solvents were obtained from a Waters Dry Solvent System. Solvents for chromatography were distilled prior to use. Sensitive reactions were carried out in heat-dried glassware and under an argon atmosphere. Analytical thin layer chromatography (TLC) was performed on silica gel 60 F254 glass plates precoated with a 0.25 mm thickness of silica gel. Spots were visualized by staining with vanillin solution (6% (w/v) vanillin and 10% (v/v) sulfuric acid in 95% EtOH) or Hanessian's stain (5% (w/v) ammonium molybdate, 1% (w/v) cerium(II) sulfate and 10% (v/v) sulfuric acid in water). Silica column chromatography was performed on Fluka silica gel 60 (230-400 mesh).
$^1H$, $^{13}C$ and two-dimensional NMR spectra were measured with a Varian 400-MR, 600-MR and Bruker Avance 700 spectrometer at 296 K. Chemical shifts (δ) are reported in parts per million (ppm) relative to the respective residual solvent peaks ($CDCl_3$: δ 7.27 in $^1H$ and 77.16 in $^{13}C$ NMR; $CD_3OD$: δ 3.31 in $^1H$ and 49.00 in $^{13}C$ NMR; $D_2O$: δ 4.80 in $^1H$ NMR; acetone-$d_6$: δ 2.05 in $^1H$ and 29.92 in $^{13}C$ NMR). The following abbreviations are used to indicate peak multiplicities: s singlet; d doublet; *dd* doublet of doublets; *t triplet; dt* doublet of triplets; *q* quartet; *m* multiplet. Coupling constants (*J*) are reported in Hertz (Hz). Optical rotation (OR) measurements were carried out with a Schmidt & Haensch UniPol L1000 polarimeter at λ = 589 nm and a concentration (c) expressed in g/100 mL in the solvent noted in parentheses. High resolution mass spectrometry (HRMS) was performed at the Free University Berlin, Mass Spectrometry Core Facility, with an Agilent 6210 TOF mass spectrometer. Infrared (IR) spectra were measured with a Perkin Elmer 100 FTIR spectrometer.

### Example A. Synthesis of *S. pneumoniae* serotype 2 conjugate 1 ($CRM_{197}$ - *S. pneumoniae* serotype 8 hexasaccharide 22)

### Example A.1: Synthesis of disaccharide acceptor 2

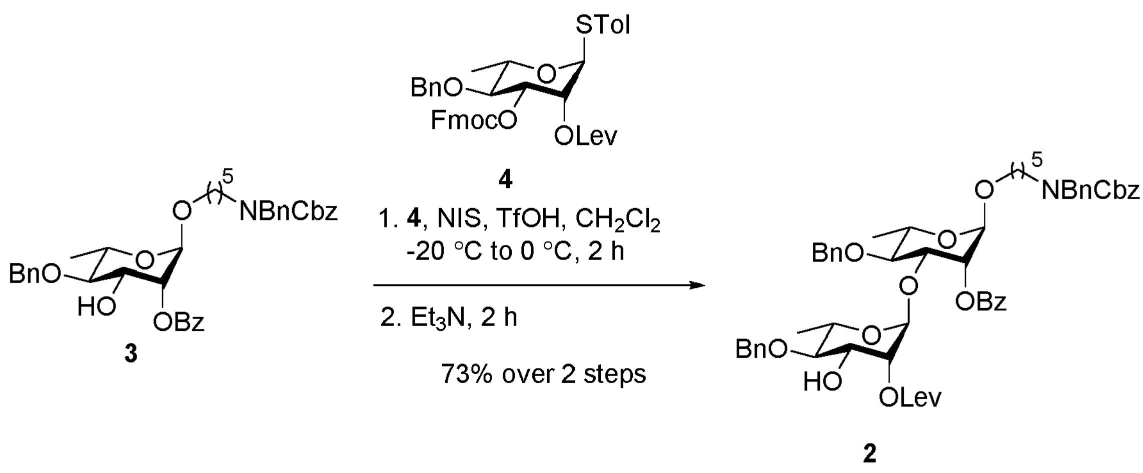


4

3

2

**Synthesis of building block 15**

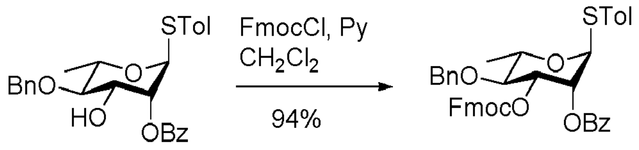


**6** **5**

To a clear solution of **6** (Dhénin S. G. Y. et al., Org. Biomol. Chem. 2009, 7, 5184) (6.7 g, 14.4 mmol) in $CH_2Cl_2$ (80 mL) were added FmocCl (5.6 g, 21.62 mmol), and pyridine (2.4 mL, 28.8 mmol) and stirred at room temperature for 12 h. After complete consumption of starting material, the reaction mixture was diluted with $CH_2Cl_2$ (80 mL) and washed successively with 1 M HCl (60 mL), water (60 mL) and aq. sat. $NaHCO_3$ (60 mL). The organic layer was dried over $Na_2SO_4$, filtered and concentrated. The crude was purified by flash chromatography using hexanes and ethyl acetate as eluent (9:1) to afford the desired product **5** as white foam (9.3 g, 94%).

H NMR (400 MHz, $CDCl_3$) δ 8.06 (d, *J* = 7.7 Hz, 2H), 7.78-7.67 (m, 2H), 7.63 (t, *J* = 7.5 Hz, 1 H), 7.58-7.52 (m, 2H), 7.49 (t, *J* = 7.6 Hz, 2H), 7.44-7.05 (m, 13H), 5.89 (s, 1 H), 5.49 (s, 1 H), 5.29 (dd, *J* = 9.5, 3.2 Hz, 1 H), 4.87 (d, *J* = 11.1 Hz, 1 H), 4.71 (d, *J* = 11.1 Hz, 1 H), 4.61-4.48 (m, 1H), 4.41 (dq, *J* = 12.2, 6.5 Hz, 1H), 4.28 (d, *J* = 6.0 Hz, 2H), 3.76 (t, *J* = 9.6 Hz, 1 H), 2.32 (s, 3H), 1.42 (d, *J* = 6.1 Hz, 3H); $^{13}C$ NMR (101 MHz, $CDCl_3$) δ 165.6, 154.3, 143.7, 143.2, 141.4, 141.3, 138.2, 137.9, 133.6, 132.7, 130.1, 130.0, 129.7, 129.6, 128.6, 128.5, 128.1, 128.0 (2C), 127.9, 127.3, 127.2, 125.5, 125.2, 120.1 (2C), 86.2, 78.8, 76.8, 75.4, 72.1, 70.4, 69.1, 46.8, 21.3, 18.1;
HRMS (ESI): Calcd for $C_{42}H_{38}O_7S$ $[M+Na]^+$ 709.2236, found: 709.2238.

**Synthesis of building block 3**

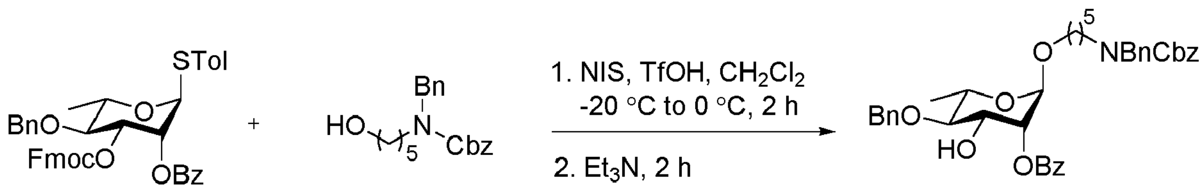


82% over 2 steps

**5** **7** **3**

A solution of compound **5** (0.17 g, 0.25 mmol), aminopentyl linker **15** (0.16 g, 0.5 mmol) and 4 Å acid washed molecular sieves (AWMS) (0.3 g) in $CH_2Cl_2$ (5 mL) were stirred at room temperature for 30 min. The solution was cooled to -20 °C and NIS (62 mg, 0.28 mmol), and TfOH (2.5 μL, 0.028 mmol) were added. The reaction mixture was gradually brought to room temperature over 2 h. After complete consumption of starting material, $Et_3N$ (2 mL) was added and the reaction mixture was stirred at room temperature for another 2 h. Reaction mixture was diluted with $CH_2Cl_2$ (25 mL) and washed with aq. sat. $Na_2S_2O_3$ (10 mL). Separated organic layer was dried over $Na_2SO_4$, filtered and concentrated. The crude was purified by flash chromatography using hexanes and ethyl acetate as eluent (4:1) to obtain the desired product **3** as colorless oil (0.135 g, 82%).
$^1H$ NMR (400 MHz, $CDCl_3$) δ 8.06 (d, *J* = 7.7 Hz, 2H), 7.59 (q, *J* = 9.3, 8.4 Hz, 1 H), 7.47 (t, *J* = 7.6 Hz, 2H), 7.42-7.13 (m, 15H), 5.32 (s, 1 H), 5.18 (d, *J* = 12.1 Hz, 2H), 4.86 (d, *J* = 11.1 Hz, 1 H), 4.83-4.79 (m, 1 H), 4.75 (d, *J* = 11.1 Hz, 1 H), 4.51 (d, *J* = 6.4 Hz, 2H), 4.21 (s, 1 H), 3.78 (d, *J* = 8.1 Hz, 1 H), 3.62 (d, *J* = 16.6 Hz, 1 H), 3.46 (t, *J* = 9.4 Hz, 1H), 3.42-3.13 (m, 2H), 2.16 (s, 1H), 1.70-1.42 (m, 6H), 1.39 (d, *J* = 6.2 Hz, 3H);
$^{13}C$ NMR (101 MHz, $CDCl_3$) δ 174.0, 166.4, 133.5, 130.0, 129.8, 128.7, 128.6, 128.2, 128.1, 128.0, 127.4, 125.3, 110.1, 97.4, 81.9, 75.4, 73.5, 70.7, 67.6, 67.3, 50.4, 29.2, 18.3;
HRMS (ESI): Calcd for $C_{40}H_{45}O_8N$ $[M+K]^+$ 706.2782, found: 706.2705.

**Synthesis of building block 4**

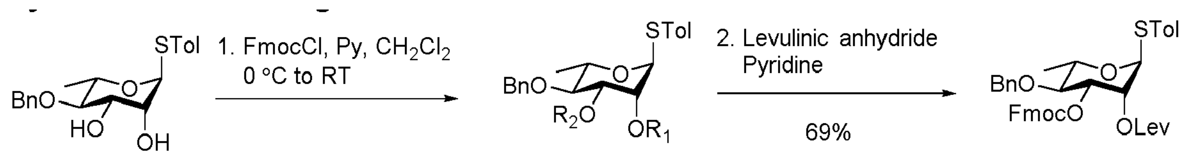


**9a**, $R_1$= Fmoc, $R_2$= H, 24%
**9b**, $R_1$= H, $R_2$= Fmoc, 34%

**8** **4**

Pyridine (0.8 mL, 10.0 mmol) was added dropwise at 0 °C to a stirred solution of **8** (Rajput V. K. J. Org. Chem. 2008, 73, 6924) (2.4 g, 6.6 mmol) and FmocCl (1.8 g, 7.0 mmol) in $CH_2Cl_2$ (50 mL). The mixture was gradually heated to room temperature over 2 h, and diluted $CH_2Cl_2$ (100 mL), washed successively with 1 M HCl (50 mL) and water (50 mL). The organic layer was dried over $Na_2SO_4$, filtered and concentrated. The crude was purified by flash chromatography using hexanes and ethyl acetate as eluent (10:1 to 4:1) to obtain **9a** (0.92 g, 24%) and **9b** (1.3 g, 34%; 20% of **8** was recovered).

$^1H$ NMR (400 MHz, $CDCl_3$) δ 7.78 (d, *J* = 7.5 Hz, 2H), 7.71 (t, *J* = 8.4 Hz, 2H), 7.42 (t, *J* = 8.3 Hz, 4H), 7.38-7.28 (m, 7H), 7.13 (d, *J* = 7.8 Hz, 2H), 5.39 (d, *J* = 3.5 Hz, 1 H), 4.86-4.71 (m, 3H), 4.56-4.42 (m, 2H), 4.36 (t, *J* = 7.6 Hz, 1 H), 3.88 (dt, *J* = 8.2, 3.6 Hz, 1 H), 3.49 (t, *J* = 9.2 Hz, 1 H), 3.41 (dd, *J* = 9.5, 5.8 Hz, 1 H), 2.34 (s, 3H), 1.46 (d, *J* = 6.0 Hz, 3H); $^{13}C$ NMR (101 MHz, $CDCl_3$) δ 155.8, 143.5, 141.5, 138.3, 138.2, 132.5, 130.2, 130.0, 128.8, 128.2 (2C), 128.0, 127.4, 127.3, 125.6, 125.5, 120.2, 120.1, 85.7, 80.9, 77.9, 76.2, 75.6, 74.3, 70.7, 46.9, 21.3, 18.4; HRMS (ESI): Calcd for $C_{35}H_{34}O_6S$ $[M+Na]^+$ 605.1974, found: 609.1993.

Levulinic anhydride (1.4 g, 6.69 mmol) and pyridine (0.54 mL, 6.69 mmol) were added to a stirred solution **of** **9b** (1.3 g, 2.23 mmol) in $CH_2Cl_2$ (20 mL). After stirring at room temperature for 2 days, the reaction mixture was diluted with $CH_2Cl_2$ (50 mL) and washed successively with 1 M HCl (50 mL) and aq. sat. $NaHCO_3$ (50 mL). The organic layer was dried over $Na_2SO_4$, filtered and concentrated. The crude was purified by flash chromatography using hexanes and ethyl acetate as eluent (4:1) to obtain **4** as viscous oil (1.04 g, 69%).

$^1H$ NMR (400 MHz, $CDCl_3$) δ 7.77 (d, *J* = 7.6 Hz, 2H), 7.61 (dd, *J* = 15.4, 7.5 Hz, 2H), 7.45-7.27 (m, 11 H), 7.12 (d, *J* = 7.8 Hz, 2H), 5.62 (dd, *J* = 3.2, 1.6 Hz, 1 H), 5.33 (d, *J* = 1.6 Hz, 1H), 5.16 (dd, *J* = 9.7, 3.3 Hz, 1H), 4.83 (d, *J* = 11.0 Hz, 1 H), 4.67 (d, *J* = 11.0 Hz, 1 H), 4.51 (dd, *J* = 10.3, 6.7 Hz, 1 H), 4.42-4.24 (m, 3H), 3.62 (t, *J* = 9.5 Hz, 1 H), 2.80-2.65 (m, 4H), 2.33 (s, 3H), 2.15 (s, 3H), 1.36 (d, *J* = 6.2 Hz, 3H);

$^{13}C$ NMR (101 MHz, $CDCl_3$) δ 206.1, 171.8, 154.1, 143.6, 143.1, 141.3, 141.2, 138.1, 137.8, 132.6, 129.9, 129.5, 128.4, 127.9, 127.8 (2C), 127.2, 127.1, 125.2, 125.1, 120.1, 120.0, 85.8, 78.6, 76.3, 75.3, 71.7, 70.1, 68.9, 46.7, 37.9, 29.8, 28.0, 21.1, 17.8;

HRMS (ESI): Calcd for $C_{40}H_{40}O_8S$ $[M+Na]^+$ 703.2342, found: 703.2359.

**Synthesis of disaccharide acceptor 2**

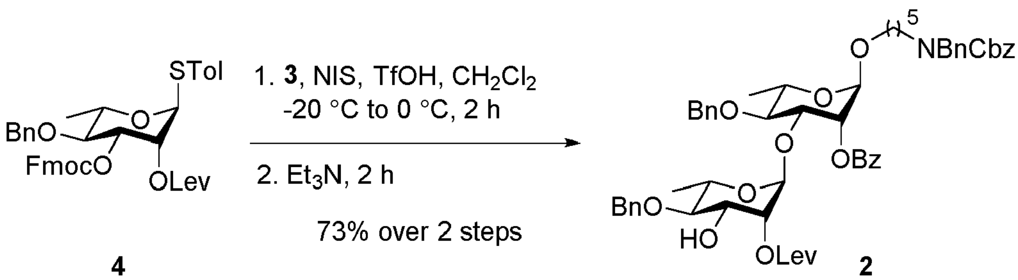


**4** **2**

A solution of donor **4** (0.25 g, 0.37 mmol), **acceptor 3** (0.165 g, 0.25 mmol) and 4 Å acid washed molecular sieves (AWMS) (0.3 g) in $CH_2Cl_2$ (5 mL) were stirred at room temperature for 30 min. The solution was cooled to -20°C and NIS (83 mg, 0.37 mmol), TfOH (3.3 μL, 0.037 mmol) were added. The reaction mixture was gradually brought to room temperature over 2 h. After complete consumption of starting material, $Et_3N$ (2 mL) was added and the reaction mixture was stirred at room temperature for another 2 h. Reaction mixture was diluted with $CH_2Cl_2$ and washed with aq. sat. $Na_2S_2O_3$. The organic layer was dried over $Na_2SO_4$, filtered and concentrated. The crude was purified by flash chromatography using hexanes and ethyl acetate as eluent (3:1) to obtain the desired product **2** as colorless oil (0.18

g, 73%).

$^1$H NMR (400 MHz, $CDCl_3$) δ 8.05 (d, *J* = 7.7 Hz, 2H), 7.60 (t, *J* = 7.4 Hz, 1 H), 7.48 (t, *J* = 7.6 Hz, 2H), 7.39-7.18 (m, 20H), 5.32 (s, 1 H), 5.24-5.12 (m, 3H), 4.87 (d, *J* = 10.8 Hz, 1 H), 4.83-4.76 (m, 2H), 4.76-4.55 (m, 3H), 4.50 (d, *J* = 4.8 Hz, 2H), 4.26-4.15 (m, 1 H), 3.97 (dd, *J* = 9.5, 3.4 Hz, 1 H), 3.77 (dd, *J* = 9.7, 6.0 Hz, 2H), 3.57 (t, *J* = 9.4 Hz, 2H), 3.24 (dd, *J* = 19.8, 10.2 Hz, 4H), 2.75 (q, *J* = 6.4, 5.8 Hz, 2H), 2.60 (qd, *J* = 16.7, 8.1 Hz, 2H), 2.18 (s, 3H), 1.64-1.43 (m, 6H), 1.31 (d, *J* = 6.2 Hz, 3H), 1.18 (d, *J* = 6.2 Hz, 3H);

$^{13}$C NMR (101 MHz, $CDCl_3$) δ 207.1, 172.2, 166.0, 138.4, 138.0, 133.4, 129.9, 129.8, 128.6 (2C), 128.5 (2C), 128.51, 128.4, 128.3, 128.0, 127.7 (2C), 127.3, 99.5, 97.0, 81.4, 80.5, 77.9, 77.4, 75.7, 74.1, 73.0, 69.9, 68.3, 67.8, 67.3, 50.6, 50.3, 47.2, 46.3, 38.3, 29.9, 29.2, 28.3, 23.5, 18.2, 17.9;

HRMS (ESI): Calcd for $C_{58}H_{67}O_{14}N$ $[M+Na]^+$ 1024.4459, found: 1024.4321.

**Example A.2: Synthesis of disaccharide donor 10**

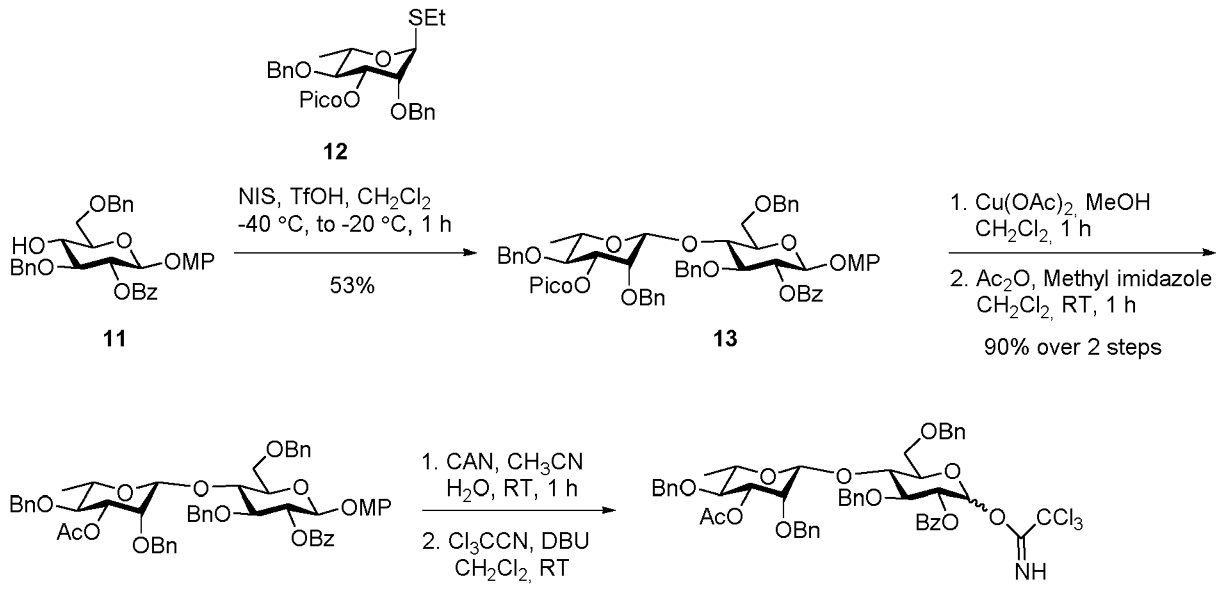


12

11 13

14 10

**Synthesis of building block 12**

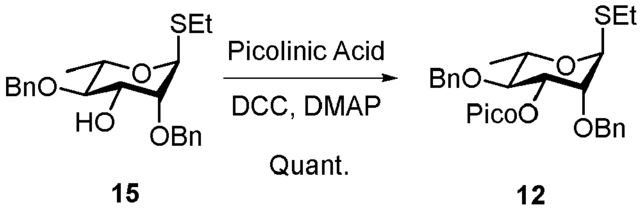


15 12

To a stirred solution of compound 15 (Bourke J. Org. Biomol. Chem. 2014, 12, 1114) (0.25 g, 0.55 mmol) in $CH_2Cl_2$ (2.5 mL) were added picolinic acid (93 mg, 0.75 mmol), DCC (0.17 g, 0.8 mmol) and DMAP (13.5 mg, 0.11 mmol). After stirring at room temperature for 2.5 h, the reaction mixture was diluted with $CH_2Cl_2$ (25 mL) and washed successively with cold water (10 mL) and aq. sat. $NaHCO_3$ (10 mL). The organic layer was dried over $Na_2SO_4$ filtered and concentrated. The crude was purified by flash chromatography using hexanes and ethyl acetate as eluent (3:1) to give the desired product **12** as pale yellowish oil (0.307 g, quantitative).

$^1$H NMR (400 MHz, $CDCl_3$) δ 8.80 (ddd, *J* = 4.7, 1.8, 0.9 Hz, 1 H), 8.01 (dt, *J* = 7.9, 1.1 Hz, 1 H), 7.81 (td, *J* = 7.7, 1.8 Hz, 1 H), 7.49 (ddd, *J* = 7.6, 4.7, 1.2 Hz, 1 H), 7.32 - 7.27 (m, 2H), 7.25 - 7.17 (m, 4H), 7.17 - 7.09 (m, 4H), 5.43 (dd, *J* = 9.5, 3.4 Hz, 1 H), 5.31 (d, *J* = 1.7 Hz, 1 H), 4.85 (d, *J* = 11.1 Hz, 1 H), 4.74 - 4.65 (m, 2H), 4.57 (d, *J* = 12.3 Hz, 1 H), 4.27 - 4.14 (m, 1 H), 4.11 (dd, *J* = 3.4, 1.7 Hz, 1 H), 3.90 (t, *J* = 9.5 Hz, 1 H), 2.74 - 2.51 (m, 2H), 1.38 (d, *J* = 6.2 Hz,

3H), 1.27 (t, *J* = 7.4 Hz, 3H).

**Synthesis of disaccharide 13**

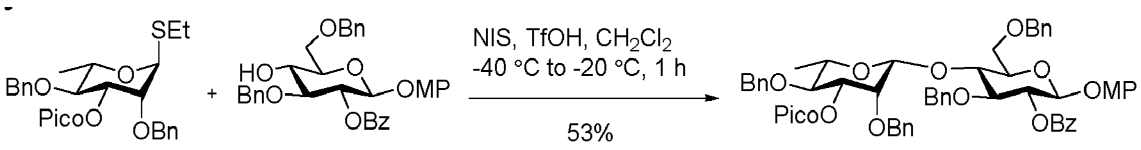


**12** **11** **13**

NIS (0.15 g, 0.65 mmol) and TfOH (6.0 μL, 0.065 mmol) were added to a cooled solution of donor **12** (0.32 g, 0.64 mmol), acceptor **11** (Bundle D. R. et al. ACS Chem. Biol. 2012, 7, 1754) (0.25 g, 0.43 mmol) and 4 Å acid washed molecular sieves (AWMS) (2.0 g) in $CH_2Cl_2$ (20 mL) at -40 °C. Reaction mixture was gradually warmed to -20 °C over 1 h, diluted with $CH_2Cl_2$ (30 mL) and washed with aq. sat. $Na_2S_2O_3$ (15 mL). The organic layer was dried over $Na_2SO_4$ filtered and concentrated. The crude was purified by flash chromatography using hexanes and ethyl acetate as eluent (4:1 to 3:1) to obtain the desired product **13** as pale yellowish oil (0.23 g, 53%).
$^1H$ NMR (400 MHz, $CDCl_3$) δ 8.88 (d, *J* = 4.7 Hz, 1 H), 8.15 (d, *J* = 7.8 Hz, 2H), 8.05 (d, *J* = 8.0 Hz, 1 H), 7.87 (t, *J* = 7.9 Hz, 1 H), 7.64 (t, *J* = 7.4 Hz, 1 H), 7.59-7.44 (m, 3H), 7.42-7.17 (m, 20H), 7.04 (d, *J* = 8.6 Hz, 2H), 6.77 (d, *J* = 8.8 Hz, 2H), 5.59 (t, *J* = 8.3 Hz, 1 H), 5.09 (d, *J* = 7.9 Hz, 1 H), 5.03 (dd, *J* = 9.8, 3.2 Hz, 1 H), 4.88-4.82 (m, 3H), 4.76-4.58 (m, 6H), 4.54 (d, *J* = 10.4 Hz, 1 H), 4.27 (d, *J* = 10.4 Hz, 1 H),4.11 (d, *J* = 3.2 Hz, 1 H), 4.00-3.79 (m, 4H), 3.77 (s, 3H), 3.39 (dq, *J* = 11.9, 6.2 Hz, 1 H), 1.40 (d, *J* = 6.0 Hz, 3H);
$^{13}C$ NMR (101 MHz, $CDCl_3$) δ 165.3, 164.0, 155.4, 151.5, 150.2, 147.7, 138.7, 138.4, 138.0, 137.0, 133.4, 129.8 (2C), 128.9, 128.6, 128.4, 128.3 (3C), 128.1, 128.0, 127.8, 127.6, 127.5 (2C), 127.0, 125.2, 118.6, 114.5, 100.8, 100.5, 83.2, 78.6, 75.8, 75.7, 75.3, 74.8, 74.0, 73.5, 71.7, 70.0, 55.6, 17.9;
HRMS (ESI): Calcd for $C_{60}H_{59}O_{13}N$ $[M+Na]^+$ 1024.3884, found: 1024.3896.

**Synthesis of disaccharide building block 14**

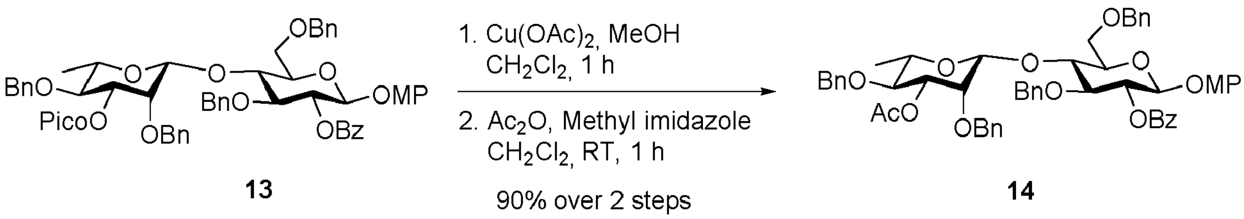


**13** **14**

$Cu(OAc)_2 \cdot H_2O$ (70 mg, 0.347 mmol) was added to a solution of **13** (0.23 g, 0.23 mmol) in $CH_2Cl_2$ (6 mL) and MeOH (3 mL). After stirring at room temperature for 1 h, reaction mixture was filtered through celite pad and the filtrate was concentrated. The crude product was dissolved in $CH_2Cl_2$ (5 mL) and to this $Ac_2O$ (1 mL), and methyl imidazole (0.2 mL) was added. After 1 h, the reaction mixture was evaporated and purified by flash chromatography using hexanes and ethyl acetate as eluent (6:1 to 5:1) to obtain the desired product **14** as colorless oil (0.193 g, 90%).
$^1H$ NMR (400 MHz, $CDCl_3$) δ 8.01 (d, *J* = 7.7 Hz, 2H), 7.51 (t, *J* = 7.4 Hz, 1 H), 7.39 (t, *J* = 7.6 Hz, 2H), 7.33-7.01 (m, 20H), 6.89 (d, *J* = 8.9 Hz, 2H), 6.64 (d, *J* = 8.9 Hz, 2H), 5.42 (t, *J* = 8.3 Hz, 1 H), 4.93 (d, *J* = 7.9 Hz, 1 H), 4.71 (t, *J* = 6.2 Hz, 2H), 4.65-4.43 (m, 7H), 4.39 (d, *J* = 10.5 Hz, 1 H), 4.15-4.07 (m, 1 H), 3.83-3.67 (m, 4H), 3.65 (s, 3H), 3.63-3.41 (m, 2H), 3.19 (dq, *J* = 12.1, 6.2 Hz, 1 H), 1.87 (s, 3H), 1.23 (d, *J* = 6.1 Hz, 3H);
$^{13}C$ NMR (101 MHz, $CDCl_3$) δ 170.2, 165.3, 155.4, 151.6, 138.7, 138.6, 138.2, 137.1, 133.4, 129.9, 129.8, 128.8, 128.7, 128.6, 128.5, 128.4, 128.3 (2C), 128.0, 127.9, 127.8, 127.6, 127.5, 118.7, 114.5, 100.8, 100.6, 83.1, 78.7, 77.4, 76.0, 75.79, 75.76, 75.4 (2C), 75.3, 74.9, 74.0, 73.5, 71.8, 70.1, 55.7, 29.8, 21.1, 17.9;
HRMS (ESI): Calcd for $C_{56}H_{58}O_{13}$ $[M+Na]^+$ 961.3775, found: 961.3841.

**Synthesis of imidate donor 10**

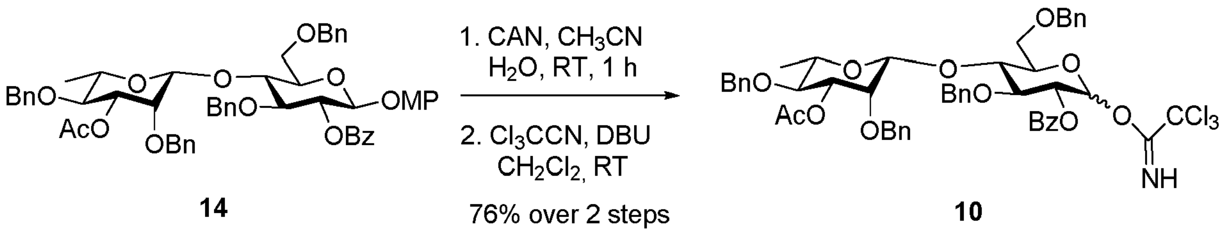


14 10

Ceric ammonium nitrate (0.46 g, 0.85 mmol) was added to a solution of **14** (0.16 g, 0.17 mmol) in acetonitrile (5 mL) and $H_2O$ (1 mL). After stirring at room temperature for 1 h, $Na_2SO_4$ was added to the reaction mixture and filtered through celite pad. The filtrate was concentrated and purified by flash chromatography using hexanes and ethyl acetate as eluent (4:1) to obtain the desired hemiacetal as pale yellowish oil.
The obtained hemiacetal was dissolved in $CH_2Cl_2$ (5 mL) and to this $Cl_3CCN$ (0.17 mL, 0.17 mmol), DBU (5.2 μL) were added. After 30 min, hexanes ( 5 mL) was added to the reaction mixture and purified by flash chromatography using hexanes and ethyl acetate as eluent (5:1) to afford the desired product **10** as colorless oil (0.126 g, 76%, α/β=9:1).
$^1H$ NMR (400 MHz, $CDCl_3$) δ 8.47 (s, 1 H), 7.95 (d, *J* = 7.8 Hz, 2H), 7.50 (t, *J* = 7.5 Hz, 1 H), 7.36 (t, *J* = 7.7 Hz, 2H), 7.32-7.01 (m, 20H), 6.56 (d, *J* = 3.5 Hz, 1 H), 5.29 (dd, *J* = 9.9, 3.5 Hz, 1 H), 4.76 (s, 1 H), 4.70-4.49 (m, 7H), 4.43 (dd, *J* = 23.8, 11.2 Hz, 2H), 4.12 (t, *J* = 9.3 Hz, 1 H), 4.05-3.90 (m, 2H), 3.90-3.76 (m, 2H), 3.73 (dd, *J* = 11.2, 4.8 Hz, 1H), 3.51 (t, *J* = 9.5 Hz, 1 H), 3.19 (dt, *J* = 12.1, 6.2 Hz, 1 H), 1.90 (s, 3H), 1.19 (d, *J* = 4.9 Hz, 3H);
$^{13}C$ NMR (101 MHz, $CDCl_3$) δ 170.2, 165.5, 160.6, 138.5 (2C), 138.2, 137.2, 133.6, 129.9, 129.3, 128.8, 128.7, 128.6, 128.5, 128.4, 128.3 (2C), 127.9, 127.8 (2C), 127.6, 127.5, 101.2, 94.1, 91.3, 79.6, 78.7, 76.0, 75.9, 75.9, 75.3, 75.0, 74.8, 73.4, 73.3, 72.9, 71.8, 68.6, 29.8, 21.2, 17.9.

**Example A.3: Synthesis of tetrasaccharide acceptor 16**

**Synthesis of tetrasaccharide 17**

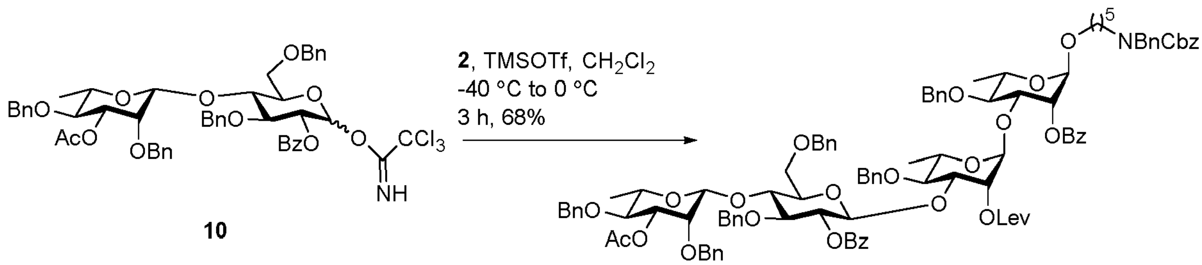


10 17

To a solution of donor **10** (60 mg, 0.06 mmol), acceptor **2** (40 mg, 0.04 mmol) and 4 Å acid washed molecular sieves (AWMS) (100 mg) in $CH_2Cl_2$ (2 mL) at -40 °C was added TMSOTf (1.5 μL, 8 μmol). The reaction mixture was gradually warmed to 0 °C over 3 h. After complete consumption of donor, a drop of $Et_3N$ was added and the solvents were removed under vacuum. The crude was purified by flash chromatography using hexanes and ethyl acetate as eluent (3:1 to 2:1) to afford the desired product **17** as pale yellowish oil (49 mg, 68%).
$^1H$ NMR (400 MHz, $CDCl_3$) δ 8.02 (d, *J* = 7.6 Hz, 2H), 7.82 (d, *J* = 7.7 Hz, 2H), 7.54-7.33 (m, 6H), 7.31-7.14 (m, 36H), 6.98-6.90 (m, 2H), 6.79-6.70 (m, 2H), 5.30 (s, 1 H), 5.21 (s, 1 H), 5.19-5.05 (m, 3H), 4.95 (s, 1 H), 4.80 (d, *J* = 10.5 Hz, 1 H), 4.76-4.62 (m, 3H), 4.61-4.45 (m, 5H), 4.43-4.37 (m, 4H), 4.35-4.21 (m, 4H), 4.12-4.09 (m, 2H), 3.80-3.69 (m, 2H), 3.67-3.57 (m, 3H), 3.55-3.37 (m, 5H), 3.30-3.03 (m, 7H), 2.63 (t, *J* = 7.0 Hz, 2H), 2.55-2.45 (m, 2H), 2.06 (s, 3H), 1.85 (s, 3H), 1.62-1.31 (m, 6H), 1.19 (s, 3H), 1.10 (d, *J* = 6.1 Hz, 3H), 0.91 (d, *J* = 6.1 Hz, 3H);
$^{13}C$ NMR (101 MHz, $CDCl_3$) δ 206.9, 171.7, 170.1, 166.1, 165.0, 139.1, 138.6, 138.5, 138.2, 138.1, 138.0, 136.7, 133.3, 130.1, 129.9, 129.7, 129.6, 128.7, 128.6 (2C), 128.5 (2C), 128.4, 128.3 (2C), 128.2 (2C), 128.0, 127.9 (2C), 127.8,

127.7, 127.3, 127.2, 127.1, 126.7, 101.1, 100.9, 99.29, 97.0, 83.1, 80.4, 80.1, 78.6, 78.0, 77.4, 76.0, 75.8, 75.6, 75.3, 75.2, 74.8, 74.7, 74.1, 74.0, 73.1, 73.0, 72.4, 71.6, 69.3, 68.3, 67.8, 67.5, 67.2, 62.4, 60.5, 50.6, 50.3, 47.2, 46.2, 38.3, 29.8, 29.2, 28.3, 28.0, 27.6, 25.0, 23.4, 22.8, 22.3, 21.2, 21.1, 18.1, 17.7, 17.6;
HRMS (ESI): Calcd for $C_{107}H_{117}O_{25}N$ $[M+Na]^+$ 1839.7846, found: 1839.7621.

**Synthesis of tetrasaccharide acceptor 16**

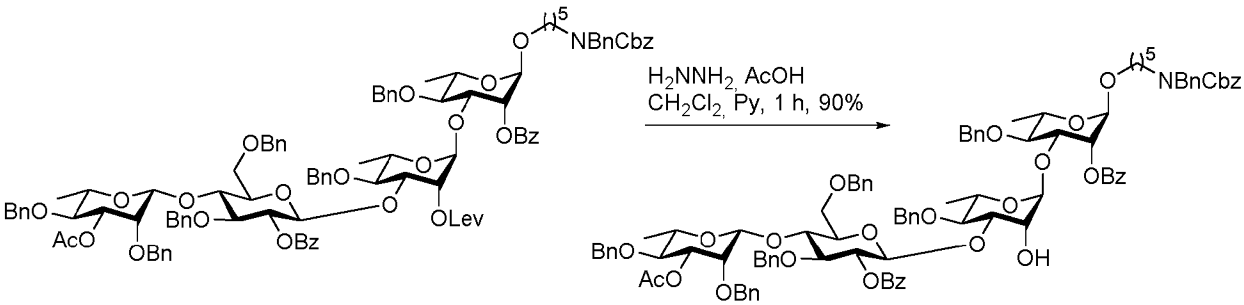


**17**

**16**

Hydrazine solution [310 μL, a premixed solution of $H_2NNH_2 \cdot H_2O$ (50 μL), pyridine (0.6 mL), AcOH (0.4 mL)] was added to a stirred solution of compound **17** (57 mg, 0.03 mmol) in $CH_2Cl_2$ (2.0 mL) and pyridine (2 mL) at 0 °C. After stirring at 0 °C for 1 h, the reaction mixture was diluted with $CH_2Cl_2$ (10 mL) and washed successively with 1 M HCl (5 mL) and aq. sat. $NaHCO_3$ (5 mL). The organic layer was dried over $Na_2SO_4$ filtered and concentrated. The crude was purified by flash chromatography using hexanes and ethyl acetate as eluent (3:1 to 2.5:1) to give the desired product **16** as colorless oil (49 mg, 90%).
$^1H$ NMR (400 MHz, $CDCl_3$) δ 7.98 (d, *J* = 7.7 Hz, 2H), 7.81 (d, *J* = 7.8 Hz, 2H), 7.52-7.32 (m, 6H), 7.31-7.13 (m, 28H), 7.12-6.94 (m, 10H), 6.77 (d, *J* = 7.3 Hz, 2H), 5.24 (dd, *J* = 14.9, 6.1 Hz, 2H), 5.09 (d, *J* = 9.9 Hz, 2H), 4.90 (s, 1 H), 4.81-4.67 (m, 3H), 4.65-4.38 (m, 10H), 4.36-4.21 (m, 3H), 4.20-3.96 (m, 4H), 3.85-3.63 (m, 5H), 3.60-3.39 (m, 6H), 3.35-3.07 (m, 7H), 1.86 (s, 3H), 1.56-1.38 (m, 6H), 1.23 (d, *J* = 6.2 Hz, 3H), 1.19 (d, *J* = 6.2 Hz, 3H), 0.85 (d, *J* = 6.4 Hz, 3H);
$^{13}C$ NMR (101 MHz, $CDCl_3$) δ 170.2, 165.9, 165.2, 138.6, 138.5, 138.4, 138.3, 138.1, 138.0, 136.9, 133.3, 133.2, 130.2, 129.9, 129.8, 129.5, 128.8, 128.7, 128.6, 128.5 (3C) 128.4 (3C), 128.3, 128.1, 127.9 (3C), 127.8 (2C), 127.6, 127.4, 127.2, 127.1, 101.2, 100.9, 100.5, 97.1, 83.0, 82.1, 80.2, 79.2, 78.6, 78.3, 77.4, 76.0, 75.7, 75.6, 75.5, 75.3, 74.8, 74.4, 74.3, 73.6, 73.5, 72.9, 71.7, 70.1, 69.5, 68.1, 68.0, 67.6, 67.3, 50.7, 50.3, 47.2, 46.3, 21.1, 18.2, 17.9, 17.7;
HRMS (ESI): Calcd for $C_{102}H_{111}O_{23}N$ $[M+Na]^+$ 1741.7478, found: 1741.7240.

**Example A.4: Synthesis of disaccharide donor 18**

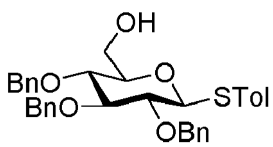

**20**

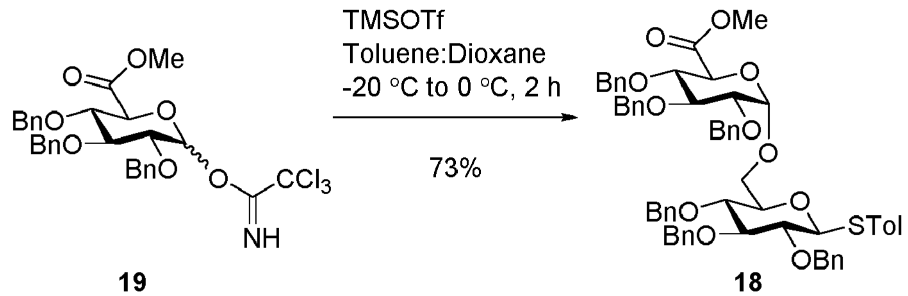


19 18

**Synthesis of Glucuronic acid building block 21**

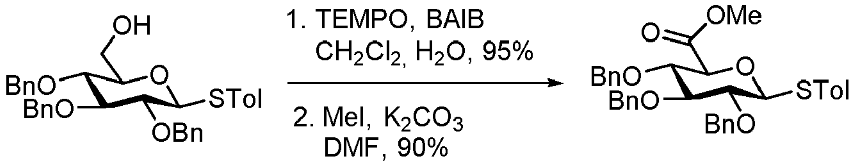


20 21

BAIB (4.34 g, 13.47 mmol) and TEMPO (0.17 g, 1.08 mmol) were added to a solution of **20** (Z. Guan et al. J. Org. Chem. 2012, 77, 8888) (3 g, 5.39 mmol) in $CH_2Cl_2$
(15 mL) and $H_2O$ (7.5 mL). The reaction mixture was stirred at room temperature for 2 h and quenched using aq. sat. $Na_2S_2O_3$ solution (150 mL). The aqueous phase was extracted with EtOAc (3x100 mL) and dried over $Na_2SO_4$. After concentration, the residue was purified by flash chromatography using cyclohexane and ethyl acetate as eluent (7:1 and 0.5% formic acid in eluent) to afford the acid as a white solid (2.92 g, 95%).
To a stirred solution of the acid (2.92 g, 5.12 mmol) in DMF (25 mL) was added MeI (1.45 g, 10.23 mmol) and $K_2CO_3$ (1.7 g, 12.3 mmol). The solution was stirred at room temperature for 10 h and quenched by the addition of MeOH (20 mL). The reaction mixture was diluted with EtOAc (80 mL) and washed with $H_2O$ (50 mL). The organic layer was dried over $Na_2SO_4$ and concentrated. The crude was purified by flash chromatography using hexanes and ethyl acetate as eluent (9:1 to 6:1) to give the desired product **21** as a white solid (2.7 g, 90%).
$^1H$ NMR (400 MHz, $CDCl_3$) δ 7.49-7.42 (m, 2H), 7.40-7.27 (m, 13H), 7.22 (m, 2H), 7.15-7.09 (m, 2H), 4.91-4.67 (m, 5H), 4.61 (m, 2H), 3.90 (m, 1 H), 3.81 (t, *J* = 9.3 Hz, 1 H), 3.73 (s, 3H), 3.70 (t, *J* = 8.8 Hz, 1 H), 3.49 (dd, *J* = 9.7, 8.7 Hz, 1 H), 2.34 (s, 3H); $^{13}C$ NMR (101 MHz, $CDCl_3$) δ 168.9, 138.2 (2C), 138.0, 137.8, 133.0, 129.9, 129.4, 128.6, 128.5 (3C), 128.3, 128.1, 128.0 (2C), 127.9 (2C), 127.7, 88.7, 86.0, 80.4, 79.4, 78.1, 76.0, 75.6, 75.2, 52.6, 21.3;
HRMS (ESI): Calcd for $C_{35}H_{36}O_6S$ $[M+Na]^+$ 607.2130, found: 607.2140.

**Synthesis of imidate donor 19**

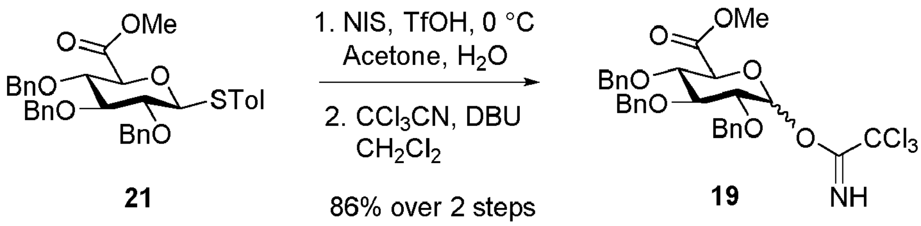


21 19

NIS (92 mg, 0.41 mmol) and TfOH (3 μL, 0.34 mmol) were added at 0 °C to a solution of **21** (0.2 g, 0.34 mmol) in acetone (2 mL) and water (1 mL). After stirring at 0 °C for 4 h, the reaction mixture was quenched with $Et_3N$ (0.5 mL). Diluted the reaction mixture with $CH_2Cl_2$ (15 mL). and washed with aq. sat. $Na_2S_2O_3$ (5 mL). Separated organic layer was dried over $Na_2SO_4$ filtered and concentrated. The crude
was purified by flash chromatography using hexanes and ethyl acetate as eluent (4:1 to 3:1) to give the hemiacetal as

pale yellowish liquid (0.164 g).

DBU (5 μL, 0.034 mmol) and $Cl_3CCN$ (0.34 mL, 3.42 mmol) were added to a cooled solution of hemiacetal (0.164 g, 0.342 mmol) in $CH_2Cl_2$ (2 mL) 0 °C. After stirring at 0 °C for 1 h, the reaction mixture was evaporated on rotor and the crude was purified by flash chromatography using hexanes and ethyl acetate as eluent (5:1 to 4:1) to obtain the product **19** as colorless oil (0.183 g, 86%, α/β= 2.7/1).

$^1$H NMR (400 MHz, $CDCl_3$) δ 8.60 (s, 1 H), 7.33-7.08 (m, 15H), 6.45 (d, *J* = 3.5 Hz, 1 H), 5.00-4.50 (m, 5H), 4.36 (d, *J* = 10.1 Hz, 1 H), 4.11-3.95 (m, 1 H), 3.82-3.66 (m, 3H), 3.65 (s, 3H);

$^{13}$C NMR (101 MHz, $CDCl_3$) δ 169.3, 161.1, 138.4, 138.0, 137.8, 137.7, 129.2, 128.6, 128.5 (3C), 128.4, 128.3, 128.2, 128.1, 128.0 (2C), 127.9, 127.8 (2C), 125.4, 94.0, 91.1, 83.7, 80.8, 78.9, 78.8, 75.9, 75.7, 75.5, 75.2, 73.2, 72.6, 52.7;

HRMS (ESI): Calcd for $C_{30}H_{30}O_7NCl_3$ [M+Na]$^+$ 644.0986, found: 644.1014.

**Synthesis of disaccharide 18**

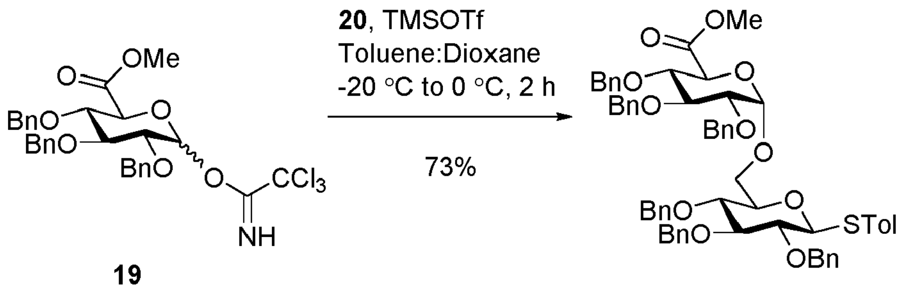


**19** **18**

TMSOTf (4 μL, 0.02 μmol) was added to a solution of donor **19** (0.14 g, 0.22 mmol), and acceptor **20** (90 mg, 0.16 mmol) in a mixture of solvents toluene (2 mL) and dioxane (6 mL) at -20 °C. The reaction mixture was gradually warmed to 0 °C over 2 h. A drop of $Et_3N$ was added and the solvents were removed under vacuum. The crude was purified by flash chromatography using hexanes and ethyl acetate as eluent (7:1 to 5:1) to afford the desired product **18** as pale yellowish oil (0.12 g, 73%, α/β=3.5:1).

$^1$H NMR (400 MHz, $CDCl_3$, α-anomer) δ 7.43 (d, *J* = 7.8 Hz, 2H), 7.40-7.18 (m, 30H), 7.06 (d, *J* = 7.8 Hz, 2H), 5.08 (d, *J* = 3.5 Hz, 1 H), 4.94 (d, *J* = 10.9 Hz, 1 H), 4.88-4.60 (m, 9H), 4.58-4.47 (m, 3H), 4.29 (d, *J* = 10.0 Hz, 1 H), 3.95 (t, *J* = 9.3 Hz, 1 H), 3.86 (dd, *J* = 12.1, 4.3 Hz, 1 H), 3.82-3.69 (m, 3H), 3.67 (d, *J* = 4.9 Hz, 3H), 3.64-3.54 (m, 2H), 3.39 (dd, *J* = 9.7, 3.9 Hz, 1 H), 3.17 (t, *J* = 9.3 Hz, 1 H), 2.21 (s, 3H);

$^{13}$C NMR (101 MHz, $CDCl_3$) δ 170.4, 138.6, 138.3, 138.2, 138.1 (2C), 138.0, 133.2, 129.9, 128.6 (2C), 128.5 (3C), 128.4 (3C), 128.3, 128.1 (2C), 128.0, 127.9, 127.8, 127.7, 127.6 (2C), 98.0, 88.6, 86.7, 81.1, 81.0, 79.8, 79.6, 78.9, 77.3, 75.9, 75.7, 75.6, 75.2, 75.1, 72.6, 70.4, 66.5, 52.5, 21.2;

HRMS (ESI): Calcd for $C_{62}H_{64}O_{11}S$ [M+Na]$^+$ 1039.4067, found: 1039.4091.

**Example A.5: Synthesis of *S. pneumoniae* serotype 2 hexasaccharide 22**

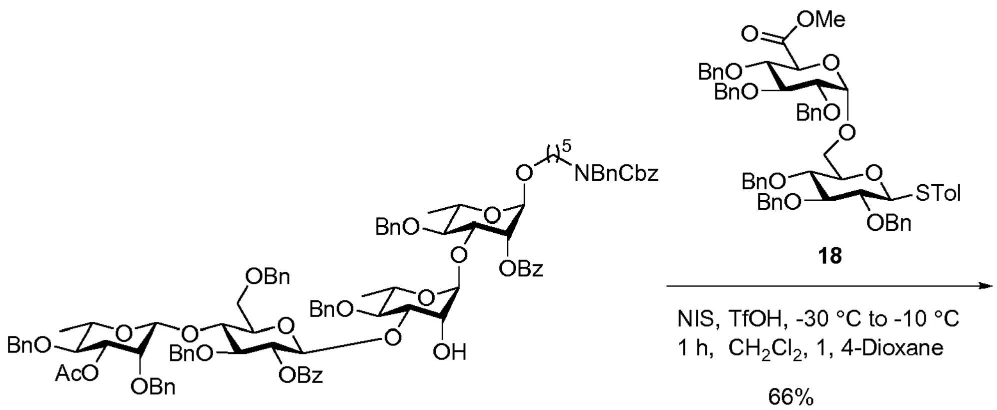


16

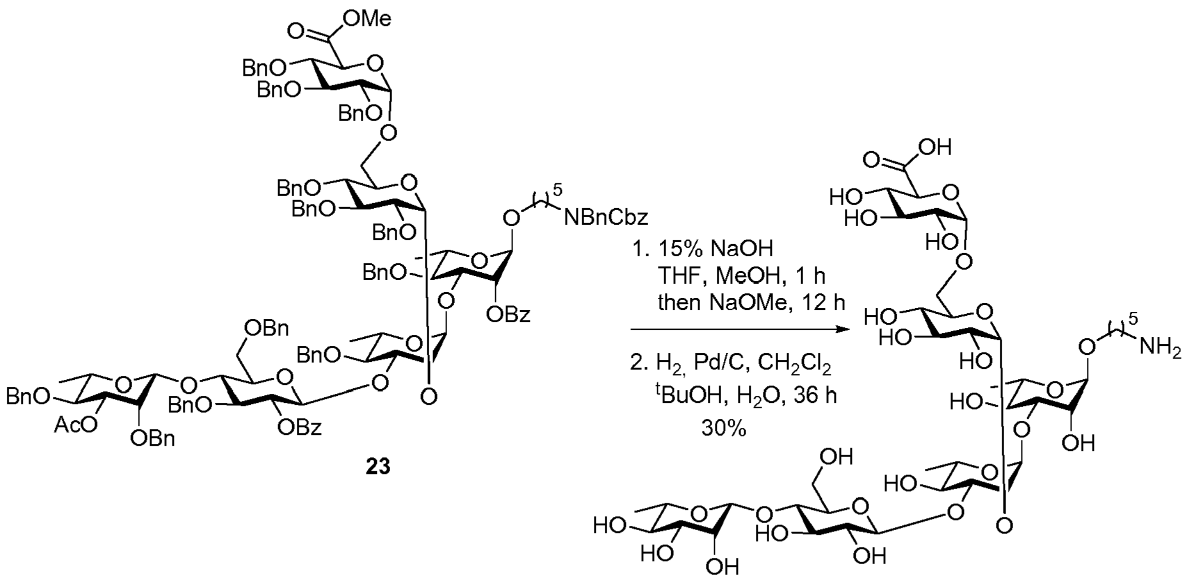


23

22

**Synthesis of hexasaccharide 23**

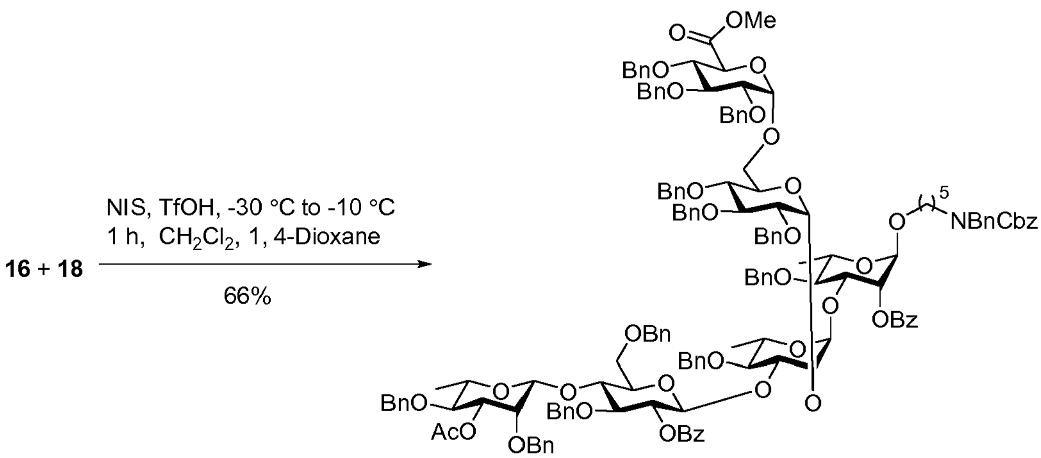


23

NIS (12 mg, 0.05 mmol) and TfOH (1 µL) were added at -30 °C to a cooled solution of donor **18** (52 mg, 0.05

mmol), acceptor **16** (45 mg, 0.026 mmol) and 4 Å acid washed molecular sieves (AWMS) (0.2 g) in mixture of $CH_2Cl_2$ (1 mL) and dioxane (1 mL). Reaction mixture was gradually warmed to -10 °C over 1 h, diluted with $CH_2Cl_2$ (10 mL) and washed with aq. sat. $Na_2S_2O_3$ (5 mL). Separated organic layer was dried over $Na_2SO_4$, filtered and concentrated. The crude was purified by flash chromatography using hexanes and ethyl acetate as eluent (4:1 to 3:1) to obtain the desired product **23** as colorless oil (45 mg, 66%).

$^1$H NMR (400 MHz, $CDCl_3$) δ 8.05 (d, *J* = 7.7 Hz, 2H), 7.78 (d, *J* = 7.8 Hz, 2H), 7.50-7.32 (m, 8H), 7.30-7.01 (m, 63H), 6.98-6.89 (m, 3H), 6.86-6.83 (m, 2H), 5.25 (s, 1 H), 5.19-5.12 (m, 2H), 5.07 (d, *J* = 4.5 Hz, 2H), 4.90-4.45 (m, 20H), 4.42-4.08 (m, 12H), 4.06-3.52 (m, 16H), 3.50 (d, *J* = 4.1 Hz, 3H), 3.48-2.95 (m, 13H), 1.87 (s, 3H), 1.58-1.36 (m, 6H), 1.14 (d, *J* = 6.2 Hz, 3H), 1.07 (d, *J* = 6.1 Hz, 3H), 0.88 (d, *J* = 6.2 Hz, 3H);

$^{13}$C NMR (101 MHz, $CDCl_3$) δ 170.6, 170.2, 166.2, 164.6, 139.4, 139.3, 138.9 (2C), 138.8, 138.7, 138.5, 138.3, 138.2, 137.0, 133.3, 133.0, 130.2, 130.0, 129.9, 128.8, 128.6 (2C), 128.5 (3C), 128.4 (3C), 128.3, 128.2 (3C), 128.1, 128.0, 127.9 (3C), 127.8, 127.7, 127.6, 127.5, 127.4, 127.3, 127.2, 127.1, 126.6, 101.4, 100.7, 99.3, 98.1, 97.0, 95.9, 83.2, 81.7, 81.0, 80.7, 80.4, 80.2, 79.8, 79.6, 78.7, 77.5, 77.4, 77.3, 77.2, 76.8, 76.1, 75.7, 75.2, 75.1, 75.0, 74.7, 73.8, 73.7, 73.3, 73.1, 72.7, 71.8, 71.5, 71.3, 70.4, 67.6, 67.3, 66.9, 58.6, 53.6, 52.3, 31.1, 29.8, 21.1, 18.6, 18.1, 17.8, 17.6; HRMS (ESI): Calcd for $C_{157}H_{167}O_{34}N$ [M+Na]$^+$ 2634.1301, found: 2634.0912.

**Synthesis of 5-amino-pentyl-β-L-rhamnopyranosyl-(1→4)-β-D-glucopyranosyl-(1→3)-α-L-{α-D-glucopyranosyluronate-(1→6)-α-D-glucopyranosyl -(1→2)} rhamnopyranosyl-(1→3)-α- L-rhamnopyranoside (22)**

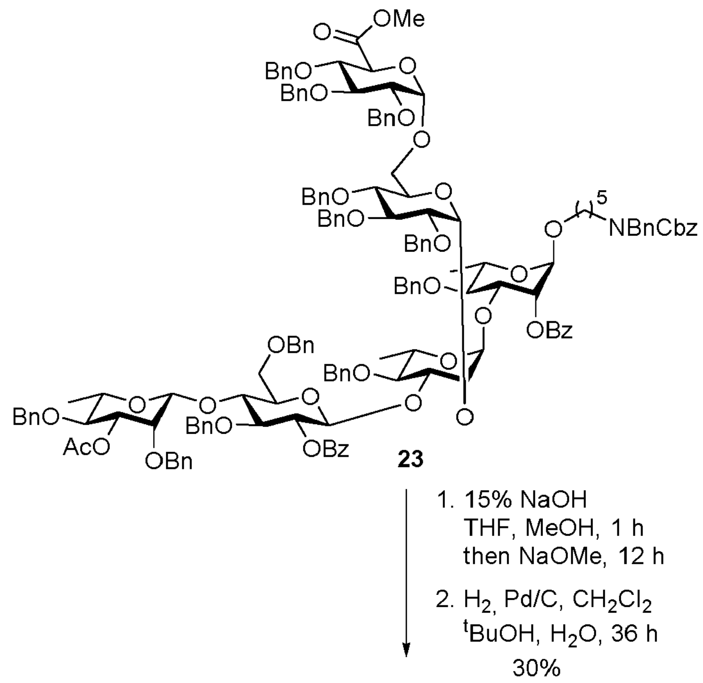


**23**

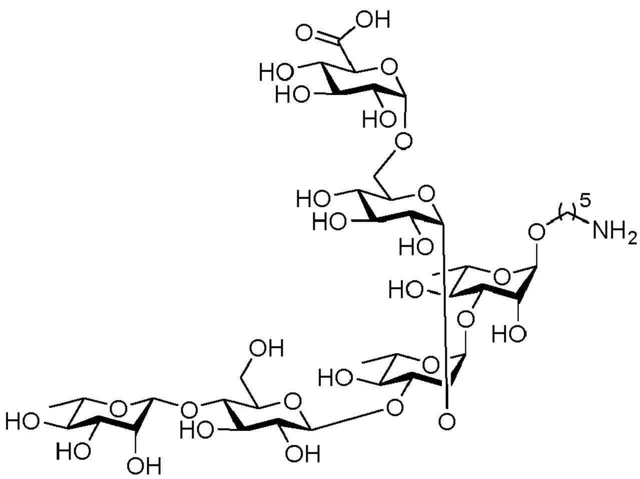

**22**

To a stirred solution of hexasaccharide **23** (6 mg, 2.3 μmol) in THF (0.5 mL) and MeOH (0.5 mL), was added aq. NaOH (15%, 100 μL). After stirring at room temperature for 1 h, NaOMe (6 mg) was added and allowed to stir for 12 h. After complete consumption of starting material, the reaction mixture was neutralized with Amberlite 120 $H^+$ resin, filtered, and concentrated. The crude material was purified by flash column chromatography using hexanes and ethyl acetate as eluent (1:1 to 1:2) to afford the desired deacylated product as white solid. The obtained deacylated product was dissolved in $CH_2Cl_2$ (0.5 mL), $^t$BuOH (1 mL) and water (0.5 mL). To this solution a suspension of Pd/C (50 mg) in a mixture of $^t$BuOH (1 mL) and water (0.5 mL) was added and stirred under hydrogen atmosphere for 36 h. Reaction mixture was then filtered, concentrated and purified by C18 column to afford the desired product **22** (0.7 mg, 30%) as a white solid.

$^1$H NMR (400 MHz, $D_2O$) δ 5.07 (s, 1 H), 5.02-4.94 (m, 2H), 4.91 (d, *J* = 3.7 Hz, 1 H), 4.73 (d, *J* = 1.5 Hz, 1 H), 4.63 (d, *J* = 7.9 Hz, 1 H), 4.35-4.18 (m, 3H), 4.05 (m, 4H), 3.96-3.81 (m, 3H), 3.80-3.61 (m, 8H), 3.61-3.40 (m, 8H), 3.38-3.25 (m, 3H), 3.24-3.13 (m, 1 H), 2.96 (t, *J* = 7.6 Hz, 2H), 1.66 (dt, *J* = 15.9, 8.0 Hz, 4H), 1.43 (p, *J* = 7.8, 7.3 Hz, 2H), 1.33-1.19 (m, 9H);

HRMS (ESI): Calcd for $C_{41}H_{71}O_{29}N$ $[M+Na]^+$ 1064.4009, found: 1064.4067.

**Example A.6: Synthesis and characterization of *S. pneumoniae* serotype 2 conjugate 1**

**General procedure synthesis**

**Formation of the *p*-nitro phenyl (PNP) amide**

To the saccharide **22** (1 equivalent) and diphenyl adipate (7 equivalents) in a glass vial were added a mixture of pyridine and DMSO (1:1) and the mixture let stir for 5 minutes for complete solubilization. Then, triethylamine (0.83 μL, 6 μmol, 10 equivalents) was added and let stir for 20 minutes. TLC indicated complete consumption of the starting material. The solvent was removed in *vacuum.* The residue was washed with dichloromethane (3 x 1 mL) to remove PNP ester excess and the white solid obtained was dried in *vacuum.*

**Conjugation of PNP ester derivatized saccharide to $CRM_{197}$**

40 equivalents of lyophilized $CRM_{197}$ was dissolved in 0.4 mL of sterile 0.1M sodium phosphate, pH 8.0 and transfer into upper chamber of 10,000 Da Millipore centrifugal filter (0.5 mL). Rinse glass vial with 3x 0.4 mL of sterile 0.1 M sodium phosphate, pH 8.0, transfer to the same centrifugal filter. Centrifuge at 10,000 rpm for 6-8 min. If needed, prolong final centrifugation step such that volume in upper chamber is 80 - 100 μL. The $CRM_{197}$ solution was then transfer into 1.5 mL tube containing lyophilized PNP ester derivatized saccharide and shake slowly (around 180 - 200 rpm) for 18 -24 hrs at room temperature. The conjugate was washed once with 0.1 M Sodium phosphate, pH 8.0 and 2-3 times with deionized, autoclaved water using 10,000 Da Millipore centrifugal filters. Take out small sample for MALDI analysis and transfer the conjugate into PBS. If needed, prolong final centrifugation step such that volume in upper chamber is about 250 μL. Transfer content of upper chamber to new 1.5 mL Eppendorf tube, store at 4°C.

**Characterization of glycoconjugate**

**A. MALDI analysis:** The average molecular size of conjugates were determined by *Matrix-assisted laser desorption/ionization* (*MALDI*) analysis using $CRM_{197}$ as standard and calculate the average oligosaccharides attachments with per $CRM_{197}$ molecule.

**B. SDS-PAGE:** The conjugates were resolved by SDS-PAGE (10%) in denaturing condition. The samples were prepared in 6X SDS-PAGE sample loading dye. The electrophoresis was carried out at 120 V and 25 mA for 1 hr 30 min in electrode buffer and gel was stained with Coomassie brilliant blue R250.

**Protein estimation**

The protein concentration was estimated using Micro BCA Protein Assay Kit (Thermo-scientific, USA) following the manufacturer's instructions. The sample was prepared in PBS and mixed with equal volume of reagent mixture (B:C:A : 24:1:25). The plate was incubated at 37°C and the absorbance was measured at 560 nm. The standard curve was plotted with known concentration of BSA provided with the kit.

**Synthesis of *S. pneumoniae* serotype 2 conjugate 1 ($CRM_{197}$ - *S. pneumoniae* serotype 2 hexasaccharide 22)**

Following the above-described procedure, conjugate **$CRM_{197}$ - *S. pneumoniae* serotype 2 hexasaccharide 22** was synthesized. The conjugate **1** was estimated using known amount of BSA as slandered and confirmed by 10% SDS-PAGE, showing a shift toward a higher mass of the glycoconjugates compared with unconjugated $CRM_{197}$ (see **Figures 3A** and **3B).** MALDI-TOF mass spectrometry analysis was used to determine the oligosaccharide-to-$CRM_{197}$ molar ratio (see **Figure 3C).** Mass analysis of the conjugate **$CRM_{197}$- *S. pneumoniae* serotype 2 hexasaccharide 22** revealed that an average of 7 - 8 molecules of hexasaccharide **22** was loaded onto one molecule of $CRM_{197}$.

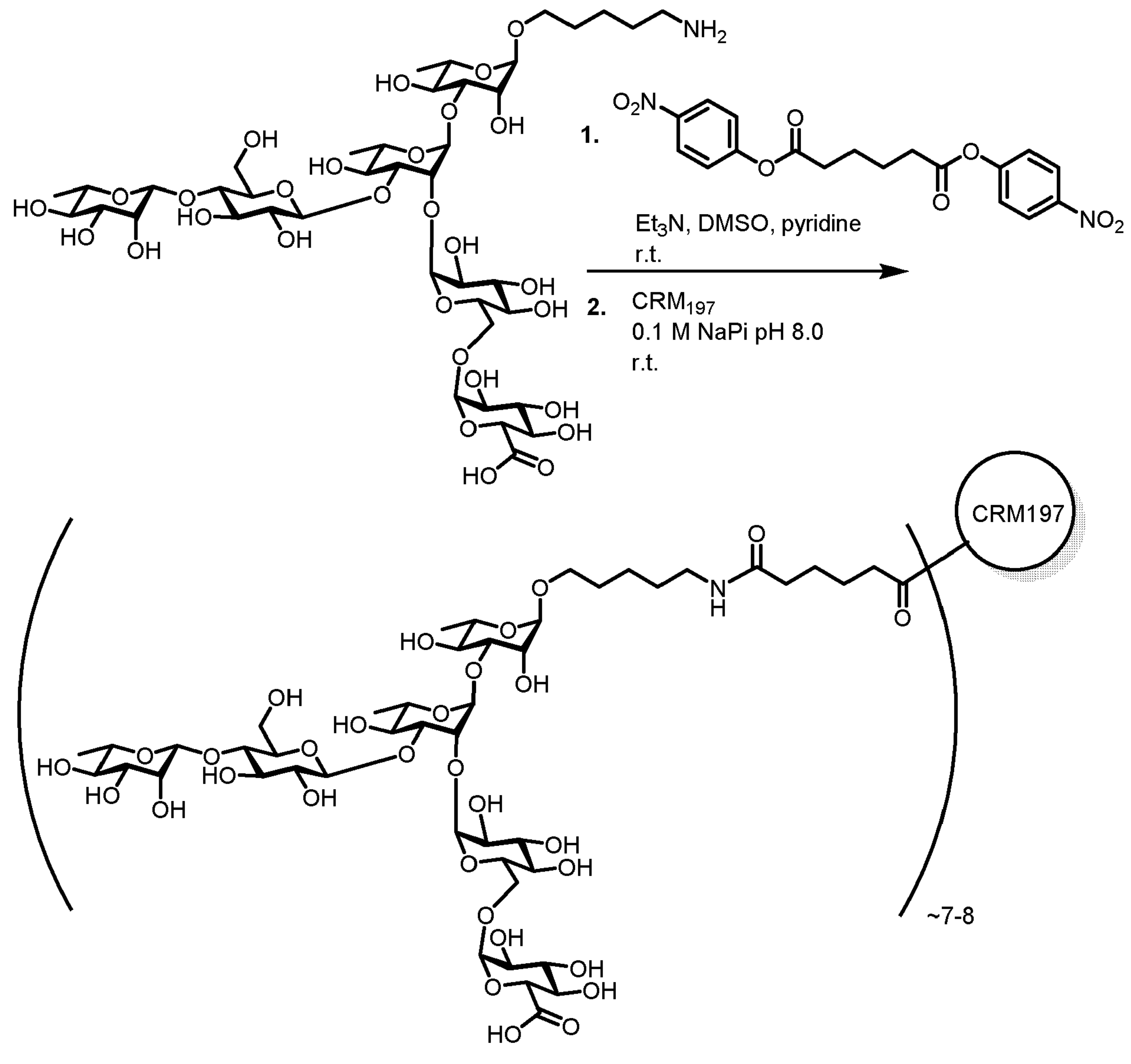


conjugate 1

**Example B. Synthesis of *S. pneumoniae* serotype 3 conjugate 24 ($CRM_{197}$- *S. pneumoniae* serotype 3 tetrasaccharide 25)**

**Example B.1. Synthesis of *S. pneumoniae* serotype 3 tetrasaccharide 25**

The synthesis of the *S. pneumoniae* serotype 3 tetrasaccharide **25** is outlined in the figure below.

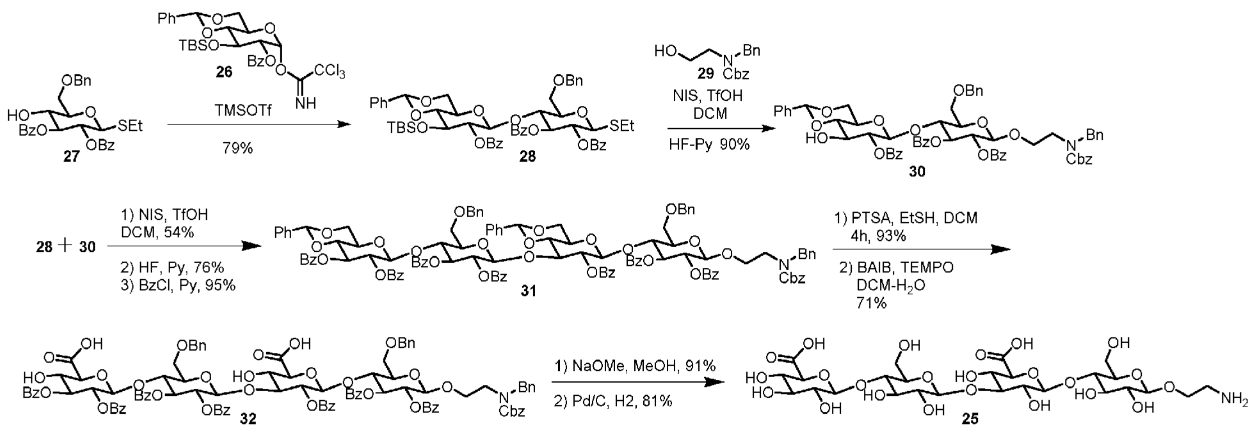


**Example B.1.1. Synthesis of disaccharide 28**

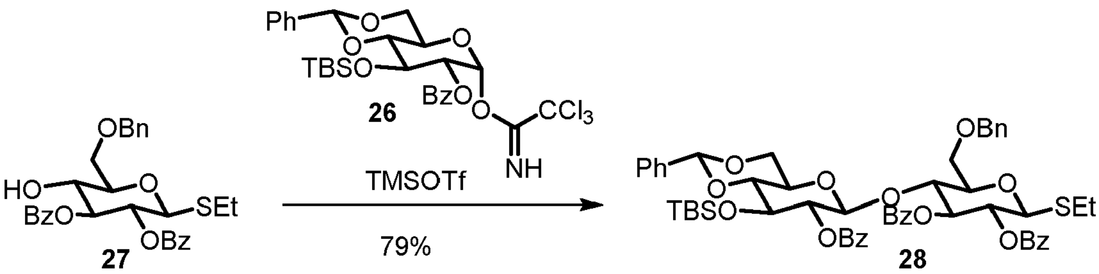


Compounds **26** (Yu, H., and Ensley, H.E. (2003) Tetrahedron Lett. 44, 9363-9366; Mo, K.-F., Li, H., Mague, J.T., and Ensley, H.E. (2009) Carbohydr. Res. 344, 439-447) (4.0 g, 7.65 mmol) and **27** (6.28 g, 9.95 mmol) were taken in DCM (140 mL) and stirred for 30 min. The reaction mixture was cooled to -20 °C and TMSOTf (0.16 mL, 0.88 mmol) was added. After stirring for 45 min at -20 °C, the reaction mixture was quenched by the addition of $Et_3N$ (1.0 mL), and concentrated under vacuum. Purification by flash chromatography using 25% ethyl acetate in hexanes yielded disaccharide **28** (6 g, 79%) as a colorless solid. $^1H$ NMR (400 MHz, $CDCl_3$) $\delta$ 7.88 - 8.03 (m, 6H), 7.29 - 7.72 (m, 19H), 5.63 (t, $J$ = 9.3 Hz, 1 H), 5.41 (t, $J$ = 9.8 Hz, 1H), 5.21 (s, 1 H), 5.12 (dd, $J$ = 8.9, 8.2 Hz, 1 H), 4.67 (d, $J$ = 12.2 Hz, 1 H), 4.59 (dd, $J$ = 15.1, 9.0 Hz, 2H), 4.57 (d, $J$ = 10.0 Hz, 1H), 4.37 (d, $J$ = 12.2 Hz, 1H), 4.19 (t, $J$ = 9.5 Hz, 1H), 3.79 (t, $J$ = 9.0 Hz, 1H), 3.74 - 3.55 (m, 1H), 3.53 - 3.37 (m, 1 H), 3.28 (t, $J$ = 9.2 Hz, 1 H), 3.15 (td, $J$ = 9.7 Hz, 4.9 Hz, 2H), 2.67 (m, 2H), 1.20 (t, $J$ = 7.4 Hz, 3H), 0.63 (s, 9H), -0.11 (s, 3H), -0.19 (s, 3H); $^{13}C$ NMR (101 MHz, $CDCl_3$) $\delta$ 165.3, 165.1, 164.5, 138.2, 137.0, 133.1, 133.1, 133.0, 130.2, 129.8 (3C), 129.3, 129.0, 128.5, 128.4, 128.3, 128.2, 128.1, 128.0 (2C), 126.2, 101.6, 101.1, 83.4, 81.1, 78.7, 77.4, 77.1, 76.8, 75.4, 74.9, 74.5, 73.5, 72.9, 70.6, 67.9, 67.5, 66.0, 25.4, 24.1, 17.8, 14.9, -4.2, -5.0.

**Example B.1.2. Synthesis of glucose building block 33**

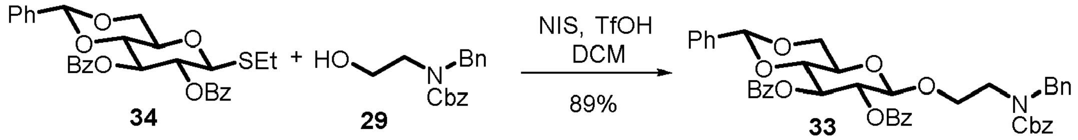


Thioglycoside donor **34** (Yu, H., and Ensley, H.E. (2003) Tetrahedron Lett. 44, 9363-9366) (6.0 g, 11.53 mmol) and amino alcohol acceptor **29** (Beshore, D.C., and Dinsmore, C.J. (2002). Org. Lett. 4, 1201-1204) (3.95 g, 13.83 mmol) were taken in DCM (100 mL). 5 g of microwave (MW) dried 4 Å molecular sieves (MS) were added and the RM stirred at room temperature for 15 min and then cooled to -10 °C. NIS (3.89 g, 17.29 mmol) and TfOH (0.15 mL, 1.73 mmol) were added to the reaction mixture and stirred between -10 °C to -5 °C for 1 h. The reaction was then quenched with

10% aq. $Na_2S_2O_3$ solution (50 mL) and the aqueous was extracted with ethyl acetate (25 mL X 3). Combined organic layer was washed with brine (10 mL), dried over anhyd. $Na_2SO_4$, filtered and concentrated in vacuum to obtain the crude as a pale yellow oil. Purification by flash column chromatography using 20-30% ethyl acetate in hexanes yielded the desired product **33** as pale yellow colored gummy liquid which under vacuum drying became a fluffy solid (7.60 g, 89%). $[\alpha]_D^{20}$ = -0.59° (c = 1.0, $CH_2Cl_2$); IR (thin film, $cm^{-1}$): $v_{max}$ : 1728, 1699; $^1H$ NMR (400 MHz, $CDCl_3$) δ 7.95 (d, *J* = 8.1 Hz, 4H), 7.61 - 6.79 (m, 21 H), 5.90 - 5.63 (m, 1 H), 5.59 - 5.35 (m, 2H), 5.14 (s, 1 H), 5.03 (dd, *J* = 33.7, 12.6 Hz, 1 H), 4.78 (d, *J* = 7.7 Hz, 0.5H), 4.65 (d, *J* = 7.6 Hz, 0.5H), 4.48 - 4.23 (m, 3H), 4.13 - 3.48 (m, 5H), 3.47 - 3.23 (m, 2H); $^{13}C$ NMR (101 MHz, $CDCl_3$) δ 165.7, 165.4, 156.35, 156.2, 137.9, 136.9, 133.4, 133.2, 129.9, 129.5, 129.3, 129.1, 128.7, 128.5, 128.4, 128.3, 128.1, 127.8, 127.4, 127.2, 126.2, 101.9, 101.6, 78.9, 72.6, 72.1, 69.1, 68.7, 67.4, 67.2, 66.7, 51.7, 46.9, 45.8; HRMS (ESI): calculated for $C_{44}H_{41}NNaO_{10}$ $[M + Na]^+$, 766.2628, found 766.2657.

**Example B.1.3. Synthesis of glucose building block 34**

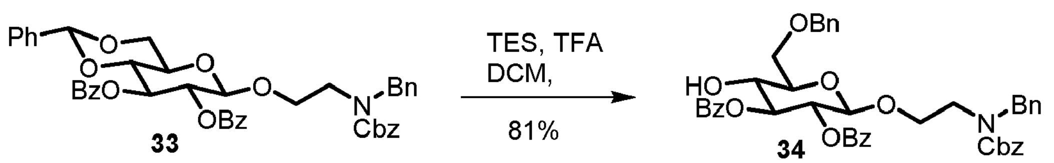


33 34

Compound **33** (7.50 g, 10.08 mmol) was stirred in DCM (75 mL) containing activated 3Å MS for 10 min before cooling to 0 °C. Added triethylsilane (12.88 mL, 81.0 mmol) followed by trifluoroacetic acid (4.66 mL, 60.5 mmol) dropwise and stirred the reaction mixture at room temperature for 16 h before quenching with water (100 mL). Extracted the aqueous with DCM (30 mL X 3), combined organics were washed thoroughly with water (20 mL X 3), brine (20 mL), dried over anhyd. $Na_2SO_4$, filtered, and evaporated under vacuum. Purification by flash column chromatography using 30%-100% ethyl acetate in hexanes yielded **34** as colorless oil (6.1 g, 81%). $[\alpha]_D^{20}$ = 34.17° (c = 1.0, $CH_2Cl_2$); IR (thin film, $cm^{-1}$): $v_{max}$ : 3434, 1727, 1699; $^1H$ NMR (600 MHz, $CDCl_3$) δ 7.97 (dd, *J* = 11.4, 8.2 Hz, 4H), 7.69 - 6.66 (m, 21 H), 5.56 - 5.39 (m, 2H), 5.13 (q, *J* = 12.3 Hz, 1 H), 5.04 (dd, *J* = 38.3, 12.5 Hz, 1 H), 4.79 - 4.54 (m, 3H), 4.40 (ddd, *J* = 30.7, 25.4, 12.9 Hz, 2H), 4.16 - 3.89 (m, 2H), 3.89 - 3.55 (m, 4H), 3.50 - 3.21 (m, 3H); $^{13}C$ NMR (151 MHz, $CDCl_3$) δ 167.1 (2C), 165.4, 156.4, 156.2, 137.9 (2C), 137.7, 137.6, 136.7, 136.5, 133.3, 133.2, 133.1, 129.9, 129.7, 129.3, 129.2, 129.0 (2C), 128.5, 128.4, 128.3, 128.0, 127.9, 127.8 (2C), 127.7, 127.6, 127.2, 127.0, 101.1, 101.0, 76.4, 74.6 (2C), 73.7, 71.5, 71.4, 70.8 (2C), 69.8, 69.7, 68.8 (2C), 67.2, 67.0, 51.5, 51.4, 46.7, 45.6; HRMS (ESI): calculated for $C_{44}H_{43}NNaO_{10}$ $[M + Na]^+$, 768.2785, found 768.2815.

**Example B.1.4. Synthesis of disaccharide 35**

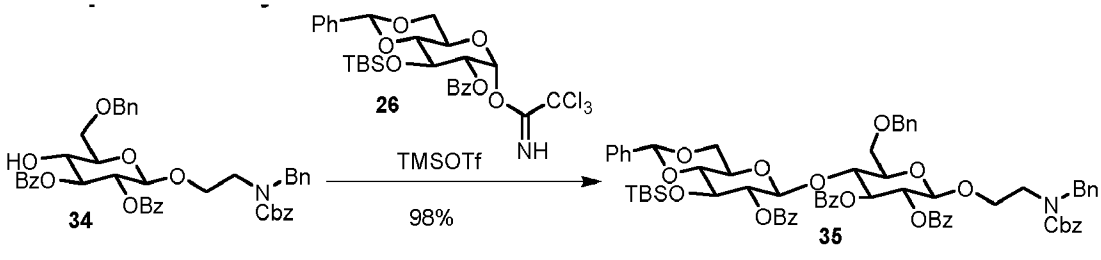


26 34 35

Acceptor **34** (2.0 g, 2.68 mmol) was taken in DCM (30 mL) with activated 4A AW MS and stirred at room temperature for 30 min before cooling to 0 °C. TMSOTf (0.49 μL, 0.27 mmol) was then added followed by the imidate donor **26** (2.20 g, 3.49 mmol) in DCM (5 mL) over 5 min and the reaction mixture was stirred for 30 min at 0 °C. Quenched the reaction mixture with $Et_3N$ (1 mL), filtered and the solvents removed under vacuum. Crude product was purified by flash chromatography using 30-50% ethyl acetate in hexanes yielded **35** as a white fluffy solid (3.2 g, 98%). $[\alpha]_D^{20}$ = 6.20° (c = 1.0, $CH_2Cl_2$); IR (thin film, $cm^{-1}$): $v_{max}$ : 1731; $^1H$ NMR (400 MHz, $CDCl_3$) δ 8.06 - 7.84 (m, 6H), 7.68 - 6.91 (m, 29H), 5.58 (dt, *J* = 22.8, 9.5 Hz, 1 H), 5.47 - 5.31 (m, 1 H), 5.21 (s, 1 H), 5.16 - 4.95 (m, 3H), 4.69 (t, *J* = 12.1 Hz, 1 H), 4.59 - 4.08 (m, 6H), 4.03 - 3.72 (m, 2H), 3.71 - 3.19 (m, 8H), 3.14 (td, *J* = 9.7, 4.9 Hz, 1 H), 2.64 (t, *J* = 10.1 Hz, 1 H), 0.63 (s, 9H), - 0.12 (s, 3H), -0.19 (s, 3H); $^{13}C$ NMR (101 MHz, $CDCl_3$) δ 165.2, 165.1, 164.4, 156.2, 156.0, 138.0,

137.8 (2C), 136.9, 136.5, 133.2, 133.0, 130.1, 129.8, 129.7, 129.3, 129.0, 128.6, 128.5, 128.4, 128.3, 128.2, 128.1(2C), 128.0, 127.9, 127.7, 127.2, 127.0, 126.2, 101.5, 101.1, 101.0, 81.0, 75.3, 74.8, 74.4, 73.6, 73.2, 72.8, 71.8, 71.7, 68.7, 67.8, 67.2, 67.0, 65.9, 51.5, 46.7, 45.7, 25.4, 17.8, -4.2, -5.0; HRMS (ESI): calculated for $C_{70}H_{75}NNaO_{16}Si$ $[M + Na]^+$, 1236.4753, found 1236.4796.

**Example B.1.5. Synthesis of disaccharide 35**

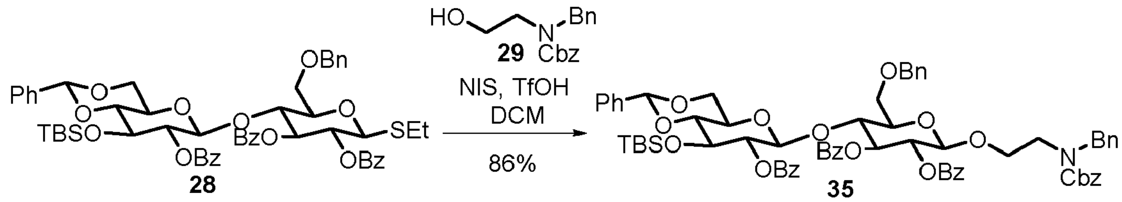


Thioglycoside disaccharide donor **28** (1.0 g, 1.01mmol) and amino alcohol acceptor **29** (0.37 g, 1.31 mmol) were dried azeotropically using toluene in rotary evaporator. DCM (15 mL) was then added followed by activated 4Å AW MS and the solution stirred at room temperature for 30 min before cooling to -10 °C. NIS (0.238 g, 1.06 mmol) and TfOH (0.02 mL, 0.20 mmol) were added and the reaction mixture stirred between -10 °C to 0 °C for 1 h. The reaction mixture was then quenched with 10% aq. $Na_2S_2O_3$ solution (50 mL) and extracted with DCM (25 mL X 3). Combined organic layer was washed with brine (10 mL), dried over anhyd. $Na_2SO_4$, filtered and concentrated under vacuum. Purification was by flash chromatography using 30-50% ethyl acetate in hexanes yielded **35** as a white fluffy solid (1.05 g, 86%).

**Example B.1.6. Synthesis of disaccharide 30**

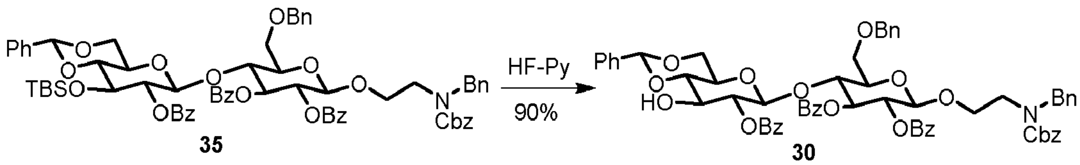


Substrate **35** (1.60 g, 1.32 mmol) was taken in pyridine (10 mL) and cooled to 0 °C. Added HF-pyridine (3.56 mL, 39.5 mmol) and stirred the reaction mixture at room temperature for 24 h. The reaction mixture was then washed with water and extracted with DCM (20 mL X 3). Combined organics were washed with cold dil. HCl (50 mL X 2), sat. $NaHCO_3$ solution (50 mL), brine (10 mL), dried over $Na_2SO_4$, filtered and concentrated under vacuum. Purification using flash column chromatography using 35-50% ethyl acetate in hexanes yielded compound **30** as a white foam (1.30 g, 90%). $[\alpha]_D^{20}$ = 12.18° (c = 1.0, $CH_2Cl_2$); IR (thin film, $cm^{-1}$): $\nu_{max}$ : 3446, 1724, 1691; $^1H$ NMR (600 MHz, $CDCl_3$) δ 8.07 - 7.85 (m, 6H), 7.68-6.81 (m, 29H), 5.59 (dt, *J* = 31.0, 9.5 Hz, 1 H), 5.39 (dt, *J* = 36.6, 8.7 Hz, 1 H), 5.23 (s, 1 H), 5.15 - 4.93 (m, 3H), 4.73 - 4.57 (m, 2H), 4.53 (d, *J* = 7.8 Hz, 0.5H), 4.47 - 4.25 (m, 3.5H), 4.17 (dt, *J* = 27.2, 9.3 Hz, 1 H), 4.00 - 3.75 (m, 2H), 3.72 - 3.22 (m, 8H), 3.15 (td, *J* = 9.5, 5.0 Hz, 1 H), 2.67 (t, *J* = 10.3 Hz, 1 H), 2.51 (s, 1 H); $^{13}C$ NMR (101 MHz, $CDCl_3$) δ 165.4, 165.3, 156.4, 156.2, 138.2, 136.9, 133.6, 133.2, 130.3, 130.0, 129.9, 129.4, 128.7 (2C), 128.5 (2C), 128.4, 128.1 (2C), 127.8, 127.4, 126.4, 101.8, 101.2, 101.1, 80.6, 75.9, 74.9, 74.7, 73.7, 73.5, 72.6, 72.0, 71.9, 68.9, 67.9, 67.4, 67.2, 66.0, 51.7, 46.9, 45.9; HRMS (ESI): calculated for $C_{64}H_{61}NNaO_{16}$ $[M + Na]^+$, 1122.3888, found 1122.3931.

**Example B.1.7. Synthesis of tetrasaccharide 36**

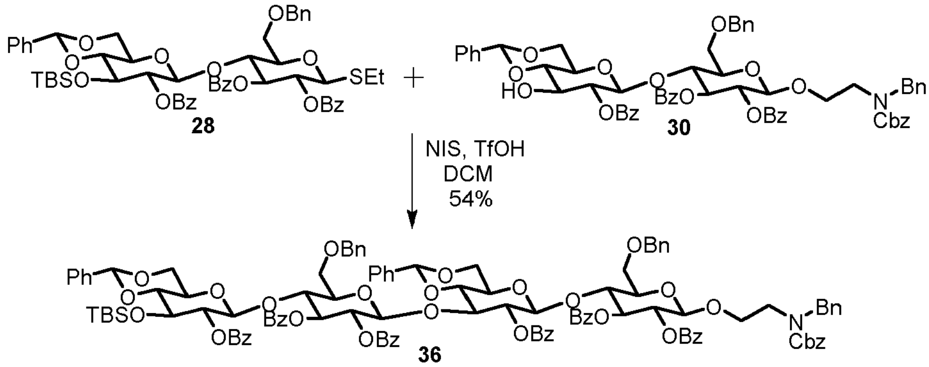


Acceptor disaccharide **30** (1.0 g, 0.91 mmol) and donor substrate **28** (1.08 g, 1.09 mmol) were taken in DCM (30 mL) to which was added 20 g of 4A MS and stirred at room temperature for 15 min and then cooled to -10 °C. NIS (0.245 g, 1.09 mmol) and TfOH (0.016 mL, 0.18 mmol) were then added to reaction mixture and stirred at -5 °C for 1 h. Donor substrate **28** (0.45 g, 0.45 mmol) and NIS (0.102 mg, 0.45 mmol) were added again to the reaction mixture and stirred at -5 °C for 1 h, and then slowly warmed to 5 °C. The reaction mixture was then quenched with 10% $Na_2S_2O_3$ solution (25 mL) and filtered through a Celite® bed and the aqueous extracted with DCM (15 mL X 3). Combined organic layer was washed with sat. $NaHCO_3$ solution (15 mL), brine (10 mL), dried over anhyd. $Na_2SO_4$, filtered and concentrated in vacuum. Purification by silica gel flash column chromatography using 30-35% ethyl acetate in hexanes yielded tetrasaccharide **36** as a fluffy white solid (1.0 g, 54%). $[\alpha]_D^{20}$ = 11.68° (c = 1.0, $CH_2Cl_2$); IR (thin film, $cm^{-1}$): $v_{max}$ 1732, 1701; $^1H$ NMR (600 MHz, $CDCl_3$) δ 8.07 - 6.91 (m, 60H), 5.49 (dt, *J* = 31.2, 9.5 Hz, 1 H), 5.32 (dt, *J* = 17.9, 9.5 Hz, 2H), 5.25 - 4.90 (m, 7H), 4.61 (d, *J* = 7.9 Hz, 1 H), 4.58 - 4.19 (m, 8H), 4.16 (d, *J* = 12.2 Hz, 1 H), 4.06 (dt, *J* = 24.7, 9.4 Hz, 1 H), 4.00 - 3.89 (m, 1.5H), 3.85 - 3.70 (m, 2.5H), 3.69 - 3.56 (m, 2H), 3.53 (dd, *J* = 10.7, 4.8 Hz, 1 H), 3.49 - 2.99 (m, 12H), 2.64 (t, *J* = 9.8 Hz, 1 H), 2.55 (t, *J* = 10.4 Hz, 1H), 0.60 (s, 9H), -0.16 (s, 3H), -0.24 (s, 3H); $^{13}C$ NMR (151 MHz, $CDCl_3$) δ 165.4, 165.1, 164.8, 164.5, 164.0, 156.3, 156.2, 138.4, 138.0, 137.1, 137.0, 136.7, 133.2, 133.1 (2C), 133.0, 132.6, 130.2, 130.1, 130.0, 129.9 (2C), 129.8, 129.7, 129.5, 129.4, 129.2, 129.1, 128.6, 128.5 (2C), 128.4, 128.3 (2C), 128.2, 128.1 (3C), 128.0, 127.8, 127.3, 127.1, 126.4, 126.1, 101.7, 101.4, 101.2 (2C), 101.0, 100.2, 81.1, 79.4, 78.4, 75.8, 75.6, 75.0, 74.5, 74.4, 73.6, 73.5, 73.4, 73.1, 72.3, 72.0, 68.8, 68.0, 67.9, 67.4, 67.2, 67.0, 66.2, 66.0, 51.7, 46.9, 45.8, 25.6, 17.9, - 4.1, -4.9; HRMS (ESI): calculated for $C_{n7}H_{n7}NNaO_{29}Si$ $[M + Na]^+$, 2050.7378, found 2050.7372.

**Example B.1.8. Synthesis of tetrasaccharide 37**

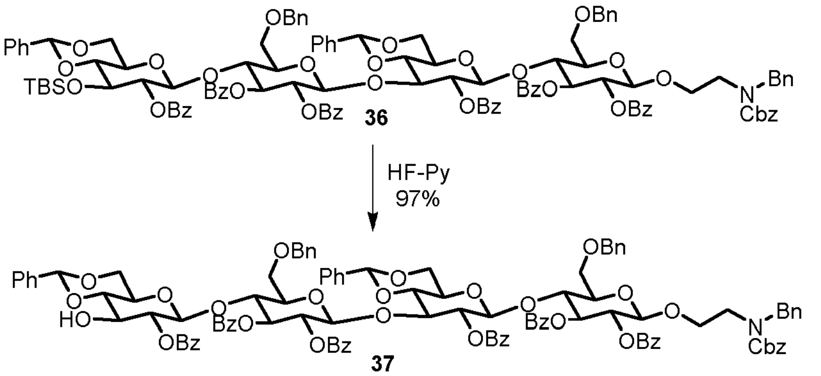


Tetrasaccharide substrate **36** (0.60 g, 0.30 mmol) was taken in pyridine (10 mL) at 0 °C and to it was added HF-pyridine (0.80 mL, 8.87 mmol) and stirred at room temperature for 24 to 36 h. The reaction mixture was diluted with water and extracted with DCM (20 mL X 3). Combined organics were then washed with cold dil. HCl (50 mL X 2), sat. $NaHCO_3$ solution (50 mL), brine (10 mL), dried over $Na_2SO_4$, filtered and concentrated in vacuum. Purification by silica gel column chromatography using 50% ethyl acetate in hexanes yielded **37** as a white colored foam (0.55 g, 97%). $[\alpha]D^{20}$ = 17.88° (c = 1.0, $CH_2Cl_2$); IR (thin film, $cm^{-1}$): $v_{max}$ : 3455, 1732; $^1H$ NMR (600 MHz, $CDCl_3$) δ 8.06 - 6.84 (m, 60H), 5.49 (dt, *J* = 31.0, 9.5 Hz, 1 H), 5.32 (dt, J = 17.7, 9.5 Hz, 2H), 5.25 - 4.90 (m, 7H), 4.61 (dd, J = 7.9, 1.9 Hz, 2H), 4.54 (dd, J = 16.2, 12.5 Hz, 1H), 4.49 - 4.18 (m, 7H), 4.06 (dt, J = 24.6, 9.4 Hz, 1 H), 3.98 (t, J = 9.4 Hz, 1H), 3.95 - 3.89 (m, 0.5H), 3.86 - 3.73 (m, 2.5H), 3.66 - 3.56 (m, 2H), 3.50 (dd, J = 10.7, 4.9 Hz, 1H), 3.48 - 3.33 (m, 4H), 3.33 - 2.98 (m,

8H), 2.65 (t, J = 10.2 Hz, 1 H), 2.56 (t, J = 10.4 Hz, 1H), 2.42 (d, J = 3.5 Hz, 1H); $^{13}C$ NMR (151 MHz, $CDCl_3$) δ 165.4, 165.37, 165.1, 165.08, 164.8, 164.0, 156.3, 156.2, 138.3, 138.1, 138.0, 137.0, 136.9, 136.7, 133.6, 133.2, 133.1, 132.7, 130.3, 130.2, 130.1, 130.0, 129.9, 129.7, 129.6, 129.4, 129.3, 129.2, 128.6, 128.5 (2C), 128.4 (2C), 128.2 (2C), 128.1 (2C), 128.0, 127.8, 127.3, 127.1, 126.3, 126.1, 101.7, 101.5, 101.2, 101.0 (2C), 100.2, 80.5, 79.5, 78.4, 76.1, 75.6, 74.9, 74.5, 74.4, 73.7, 73.5, 73.4, 73.1, 72.7, 72.3, 72.0, 71.9, 68.9, 68.0, 67.8, 67.4, 67.3, 67.2, 67.0, 66.2, 65.8, 51.7, 46.9, 45.8; HRMS (ESI): calculated for $C_{111}H_{103}NNaO_{29}$ $[M + Na]^+$, 1936.6513, found 1936.6508.

**Example B.1.9. Synthesis of tetrasaccharide 31**

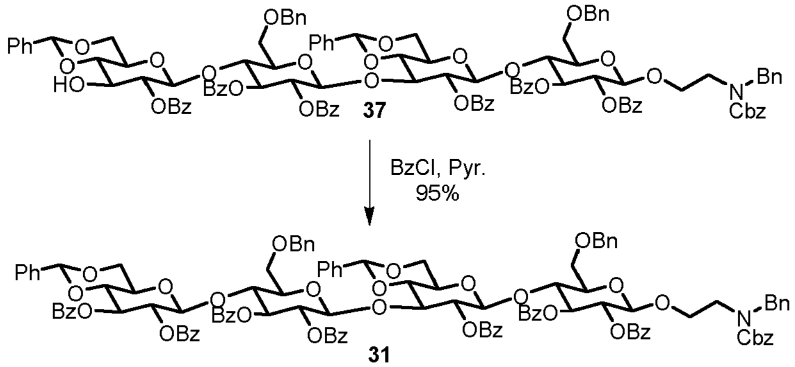


37

31

Intermediate **37** (0.65 g, 0.34 mmol) was taken in pyridine (5 mL) and benzoyl chloride (0.08 mL, 0.68 mmol) was added and the reaction mixture stirred at room temperature for 16 h. Diluted the reaction mixture with water and extracted with DCM (20 mL X 3). Combined organics were washed with cold dil. HCl (10 mL X 2), sat. $NaHCO_3$ solution (10 mL X 2), water (10 mL), brine (20 mL), dried over $Na_2SO_4$, filtered, and concentrated. Trituration with cold methanol (5 mL X 3) yielded **31** as a white solid (0.65 g, 95%). $[\alpha]_D^{20}$ = 11.05° (c = 1.0, $CH_2Cl_2$); IR (thin film, $cm^{-1}$): $v_{max}$ : 1732; $^1H$ NMR (400 MHz, $CDCl_3$) δ 8.18 - 6.86 (m, 65H), 5.54 - 5.40 (m, 2H), 5.38 - 5.14 (m, 4H), 5.12 - 4.94 (m, 4H), 4.91 (s, 1 H), 4.65 (d, *J* = 7.9 Hz, 1 H), 4.59 (d, *J* = 7.9 Hz, 1 H), 4.55 - 4.47 (m, 1 H), 4.43 (d, *J* = 7.9 Hz, 1H), 4.41 - 4.13 (m, 6H), 4.09 - 3.85 (m, 3H), 3.8 - 3.70 (m, 1 H), 3.64 - 3.3 (m, 6H), 3.33 - 3.15 (m, 6H), 3.31 - 2.98 (m, 3H), 2.61 (dd, *J* = 20.1, 9.9 Hz, 2H); $^{13}C$ NMR (101 MHz, $CDCl_3$) δ 165.6, 165.4, 165.2, 165.1, 164.8, 164.0, 156.3, 156.2, 138.1 (2C), 138.0, 137.0, 136.8, 133.4, 133.1, 132.7, 130.2, 129.9, 129.9, 129.8, 129.8, 129.7, 129.5, 129.3, 129.3, 129.2, 129.1, 128.6 (3C), 128.5, 128.4 (2C), 128.3, 128.2 (2C), 128.1, 127.8, 127.3, 126.2, 126.1, 125.4, 101.5, 101.3, 101.2, 101.0, 100.1, 79.5, 78.4, 77.4, 76.3, 75.6, 74.3, 73.7, 73.5, 73.4, 73.1, 72.3, 72.3 (2C), 68.8, 68.0, 67.8, 67.1, 66.2 (2C), 51.7, 46.9, 45.8; HRMS (ESI): calculated for $C_{118}H_{107}NNaO_{30}$ $[M + Na]^+$, 2040.6776, found 2040.6770.

**Example B.1.10. Synthesis of tetrasaccharide 38**

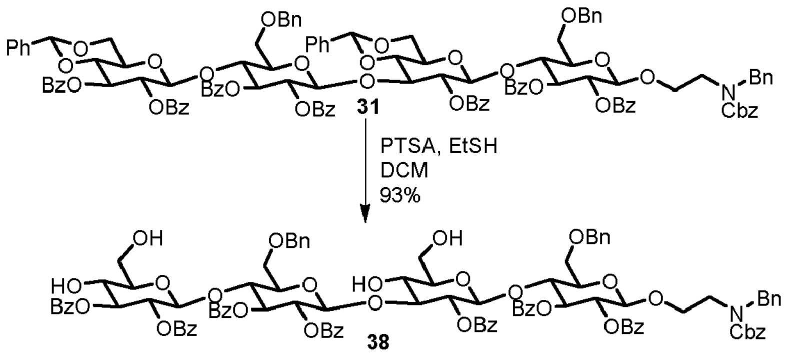


31

38

Compound **31** (0.54 g, 0.267 mmol) was taken in DCM (5 mL) at room temperature, added PTSA (10 mg, 0.053 mmol) and EtSH (0.30 mL, 4.01 mmol) and stirred the reaction mixture for 4 h. Reaction mixture was then quenched with $Et_3N$ (1 mL) and evaporated under vacuum. Purification by flash chromatography using 60% ethyl acetate in hexanes

yielded the tetraol **38** as white solid (0.46 g, 93%). $[\alpha]_D^{20}$ = 3.83° (c = 1.0, $CH_2Cl_2$); IR (thin film, $cm^{-1}$): $v_{max}$ : 3479, 1732; $^1H$ NMR (400 MHz, $CDCl_3$) δ 8.02 - 7.81 (m, 10H), 7.59 - 6.91 (m, 45H), 5.52 (t, *J* = 9.2 Hz, 1 H), 5.47 - 5.15 (m, 5H), 5.12 - 4.90 (m, 3H), 4.62 (d, *J* = 7.6 Hz, 1 H), 4.61 - 4.57 (m, 1 H), 4.52 (d, *J* = 7.7 Hz, 1 H), 4.47 - 4.36 (m, 3H), 4.34 - 4.18 (m, 4H), 4.13 - 3.99 (m, 2H), 3.95 - 3.73 (m, 2H), 3.69 (td, *J* = 9.1, 4.3 Hz, 1 H), 3.61 - 3.50 (m, 3H), 3.45 - 3.13 (m, 10H), 3.11 - 2.95 (m, 3H), 2.89 (d, *J* = 4.3 Hz, 1 H); $^{13}C$ NMR (101 MHz, $CDCl_3$) δ 167.4 (2C), 165.2 (2C), 164.9, 163.8, 156.3, 156.2, 138.1, 137.3, 133.7, 133.6, 133.3, 133.0, 132.7, 130.1, 130.0, 129.9, 129.8, 129.7, 129.6, 129.5 (2C), 129.2, 129.0, 128.9, 128.8, 128.7 (2C), 128.6, 128.5, 128.4, 128.3, 128.1 (2C), 127.8, 127.3, 127.2, 101.3, 101.1, 100.8, 100.3, 85.1, 77.4, 77.0, 76.0, 75.8, 74.8, 74.7, 74.4, 73.8, 73.6, 73.3, 72.2, 71.7 (2C), 69.4, 69.3, 68.9, 67.6, 67.1, 62.5, 61.6, 51.7, 46.9, 45.9; HRMS (ESI): calculated for $C_{104}H_{99}NNaO_{30}$ [M + Na]+, 1864.6150, found 1864.6144.

**Example B.1.11. Synthesis of tetrasaccharide 32**

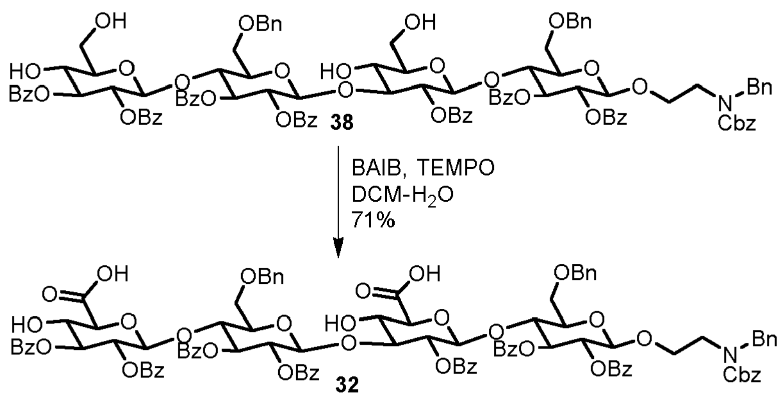


Tetraol **38** (125 mg, 0.07 mmol) was taken in a mixture of DCM (5 mL) - water (2 mL) and cooled to 0 °C. Added TEMPO (2.1 mg, 0.014 mmol) and BAIB (0.11 g, 0.34 mmol) and stirred at 0 °C for 20 min and slowly warmed to room temperature and stirred at rt for 2-3 h. Reaction mixture was then diluted with DCM (5 mL) and water (5 mL) and the aqueous layer was extracted with DCM (5 mL X 4). Combined organics were dried over $Na_2SO_4$, filtered, and concentrated. Purification by flash chromatography using 10-15% acetone in DCM + 1-2% AcOH yielded the diacid **32** as a pale yellowish solid (0.09 g, 71%). $^1H$ NMR (400 MHz, $CD_3OD$) δ 8.00 - 6.52 (m, 55H), 5.61 - 5.29 (m, 3H), 5.27 - 5.05 (m, 3H), 4.99 (d, *J* = 9.8 Hz, 1 H), 4.94 (d, *J* = 8.0 Hz, 1 H), 4.88- 4.83 (m, 2H), 4.76 (d, *J* = 7.9 Hz, 1 H), 4.53 (d, *J* = 8.0 Hz, 1.5H), 4.43 (dd, J = 12.1, 4.8 Hz, 1 H), 4.37 (d, *J* = 7.8 Hz, 0.5H), 4.33 - 4.02 (m, 7H), 3.87 - 3.39 (m, 11 H), 3.26 - 3.03 (m, 4H); $^{13}C$ NMR (101 MHz, $CD_3OD$) δ 170.4 (2C), 167.2 (2C), 166.9, 166.7, 166.5, 166.4, 165.7, 158.0, 157.7, 139.2, 138.8, 138.6, 134.8, 134.5, 134.4, 134.1, 134.0, 131.4, 130.8 (2C), 130.7, 130.6, 130.5, 130.3, 130.1, 129.8, 129.7, 129.5 (2C), 129.4, 129.3, 129.2, 129.1, 128.8, 128.6, 128.2, 102.2, 101.9, 101.8, 101.7, 84.2, 77.6, 76.7, 76.4, 75.4, 74.6, 74.4, 73.7, 73.5, 73.3, 71.5, 71.1, 69.5, 69.4, 68.5, 68.3 (2C), 52.6, 52.5, 47.1; HRMS (ESI): calculated for $C_{104}H_{95}NNaO_{32}$ [M + Na]+, 1892.5735, found 1892.5703.

**Example B.1.12. Synthesis of tetrasaccharide 39**

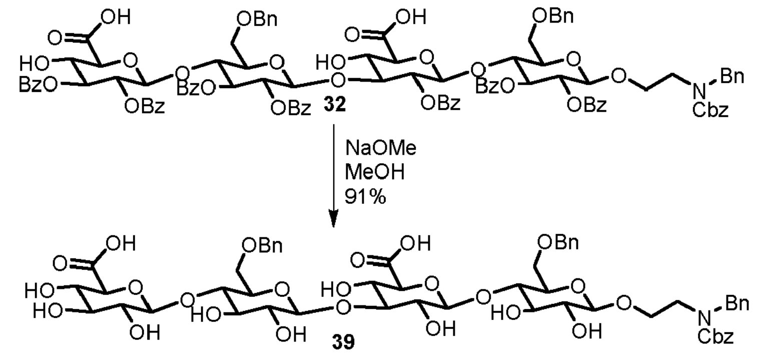


Diacid **32** (0.09 g, 0.05 mmol) was taken in methanol (5 mL) and to it was added 0.5 M solution of NaOMe in methanol (4.81 mL, 2.41 mmol) and stirred at room temperature for 24 to 36 h. The reaction mixture was neutralized with Amberlite® 120 $H^+$ resin and stirred for 10 min. The clear solution was filtered through a cotton plug and washed thoroughly with methanol and evaporated under vacuum. Diethyl ether (5 mL) was then added and triturated to get a pale yellowish solid which was then decanted. DCM (5 mL) was then added to the crude solid and triturated to get a off-white solid on decanting, which was then dried under vacuum to obtain **39** (0.05 g, 91%) as a white powder. $^1H$ NMR (400 MHz, $CD_3OD$) δ 7.48 - 7.11 (m, 20H), 5.14 (d, *J* = 9.1 Hz, 2H), 4.69 - 4.31 (m, 9H), 4.28 - 4.18 (m, 1 H), 3.99 - 3.40 (m, 20H), 3.41 - 3.33 (m, 2H), 3.30 - 3.17 (m, 2H); $^{13}C$ NMR (101 MHz, $CD_3OD$) δ 172.2, 171.9, 158.2, 139.6, 139.4, 138.0, 129.6, 129.5, 129.3, 129.2, 129.2, 129.04, 129.0, 128.8, 128.4, 104.8, 104.5, 104.1, 88.2, 86.9, 80.8, 77.7, 77.3, 76.9, 76.0, 75.97, 75.5, 75.0, 74.7, 74.5, 74.4, 74.1, 73.8, 73.0, 71.7, 71.5, 69.8, 69.6, 69.2, 69.0, 68.5, 62.4, 52.4, 47.5. LCMS (ESI): calculated for $C_{55}H_{66}NO_{25}$ [M - H]$^+$, 1140.39, found 1140.2.

**Example B.1.13. Synthesis of tetrasaccharide 25**

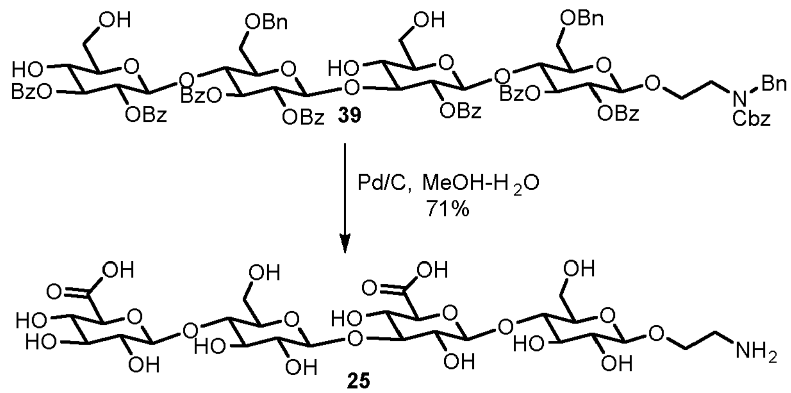


Substrate **39** (50 mg) was taken in methanol (2 mL) and 10% Pd/C (100 mg) was added. Hydrogenated the reaction mixture at room temperature using balloon for 18 h. Reaction mixture was then filtered through a PTFE hydrophobic filter and the filter washed thoroughly with methanol (3 mL X 5), water-methanol (3 mL X 5), and finally with $NH_4OH$ in methanol (3 mL in 15 mL). After evaporation of the filtrate and drying under vacuum, target molecule **25** (23 mg, 71%) was obtained as an off-white glassy material. $^1H$ NMR (400 MHz, $D_2O$) δ 4.84 (d, *J* = 8.0 Hz, 1 H), 4.56 (d, *J* = 8.0 Hz, 2H), 4.53 (d, *J* = 7.9 Hz, 1 H), 4.14 (dt, *J* = 11.5, 4.9 Hz, 1 H), 4.04 - 3.92 (m, 3H), 3.89 - 3.75 (m, 5H), 3.72 - 3.49 (m, 10H), 3.43 - 3.34 (m, 3H), 3.29 (t, *J* = 5.1 Hz, 2H); $^{13}C$ NMR (101 MHz, $d_2o$) δ 175.4, 175.2, 102.3, 102.2, 102.0, 101.8, 82.7, 78.9, 78.6, 75.7, 75.2, 74.8, 74.7, 74.1, 73.1, 72.9, 72.7, 71.6, 70.1, 65.7, 60.0 39.3; HRMS (ESI): calculated for $C_{26}H_{43}NNaO_{23}$ [M + Na]$^+$, 760.2124, found 760.2134.

**Example B.2. Preparation of *S. pneumoniae* serotype 3 conjugate 2 ($CRM_{197}$-*S. pneumoniae* serotype 3 tetrasaccharide 25)**

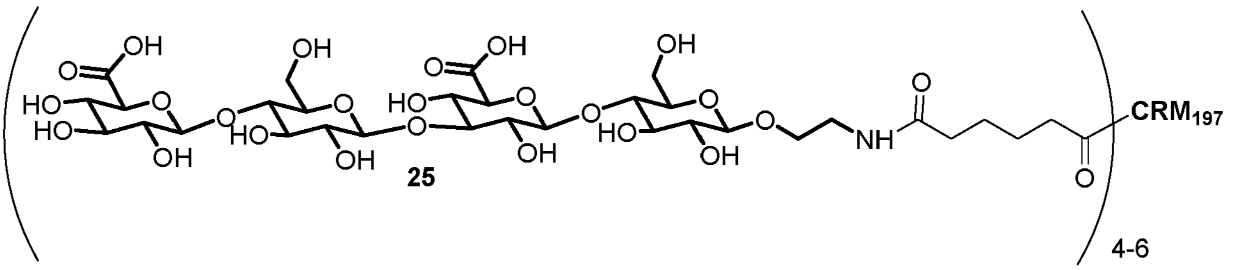

25

The $CRM_{197}$-tetrasaccharide **25** conjugate was prepared as described previously (Anish et al., Angew. Chem. Int. Ed. 2013, 52, 9524 - 9528; Anish et al. ACS Chem. Biol. 2013, 8, 2412 - 2422). Tetrasaccharide **25** was reacted with di-(*N*-succinimidyl) adipate in DMSO catalyzed by triethylamine for 2 h at RT. Addition of coupling buffer (100 mM sodium phosphate buffer, pH 7.5) and extraction of unreacted linker with chloroform was followed by reaction of linker appended tetrasaccharide with recombinant $CRM_{197}$ (Pfenex Inc,) overnight in coupling buffer. After desalting with 10 kDa Amicon ultrafiltration devices (Millipore), The $CRM_{197}$-tetrasaccharide **25** conjugate was characterized by SDS-PAGE and MALDI-TOF MS. Coupling was confirmed by SDS-PAGE. Matrix-assisted laser desorption/ionization time-of-flight mass spectrometry (MALDI-TOF MS) allowed the calculation that on average four to six oligosaccharide haptens were loaded on each CRM197 molecule (see **Figure 4**).

**Example C. Synthesis of *S. pneumoniae* serotype 8 conjugates 40 ($CRM_{197}$-*S. pneumoniae* serotype 8 tetrasaccharide 44), 41 ($CRM_{197}$- *S. pneumoniae* serotype 8 tetrasaccharide 45), 42 ($CRM_{197}$- *S. pneumoniae* serotype 8 tetrasaccharide 46) and 43 ($CRM_{197}$- *S. pneumoniae* serotype 8 hexasaccharide 66)**

**Example C.1. Synthesis of *S. pneumoniae* serotype 8 tetrasaccharides 44, 45 and 46**

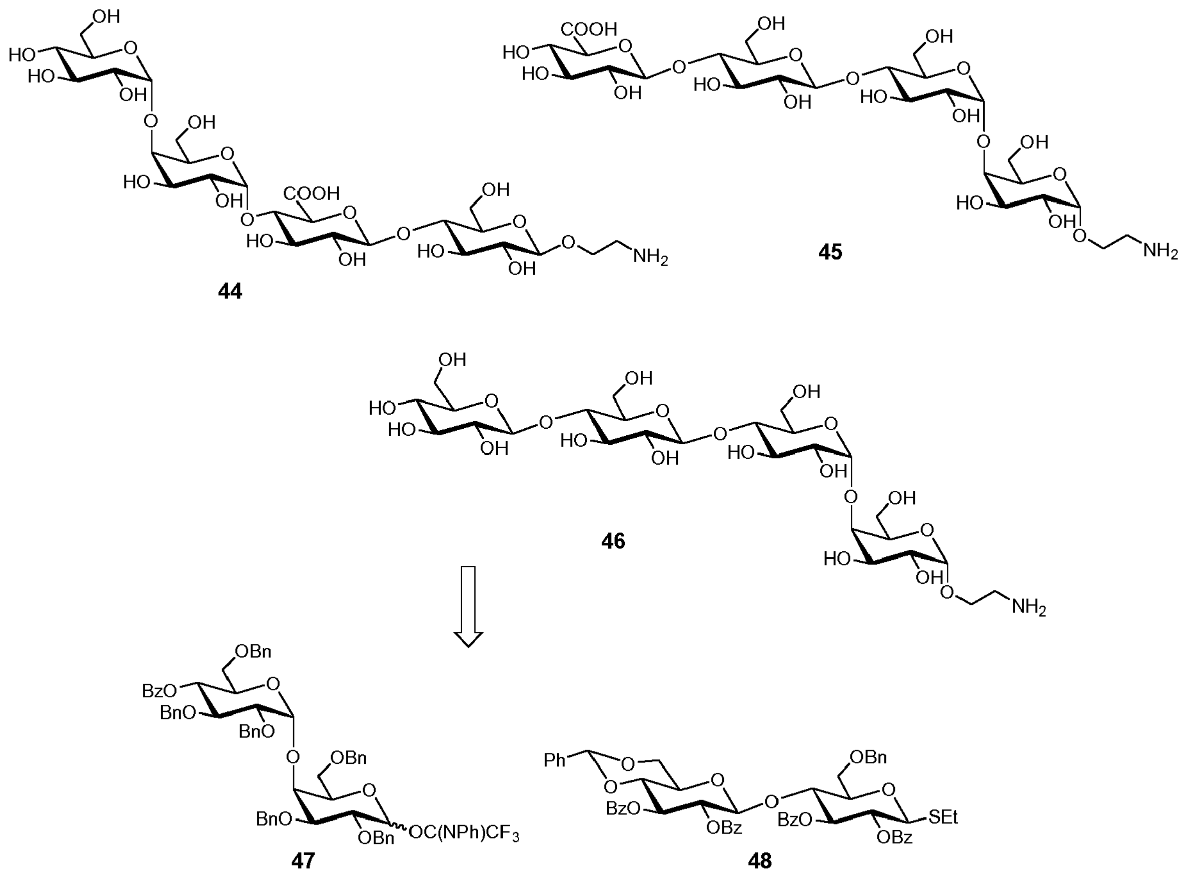

44

45

46

47

48

**Example C.1.1. Synthesis of compound 48**

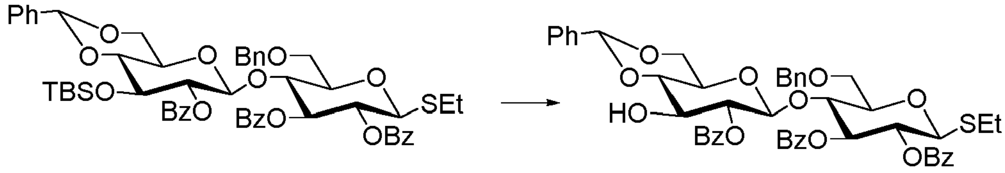

28 49

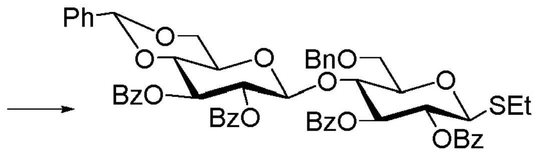

48

TBS substrate **28** (2.0g, 2.018 mmol, 1 equiv.) obtained from Example B.1. was taken in pyridine (10 mL) at 0 °C and added 70% HF-pyridine (5.45 mL, 60.5 mmol, 30 equiv.) to it and stirred at rt for 36 h.
RM was washed with water (50 mL) and extracted with DCM (50 mL X 3). Combined organics were then washed with sat. $NaHCO_3$ solution (50 mL), brine (20 mL), dried over $Na_2SO_4$, filtered and concentrated in vacuum to get crude product which on purification using silica column chromatography using 35-40% EtOAc/Hexanes to yield white colored foam **49** (1.7g, 96%).
$^1$H NMR (400 MHz, $CDCl_3$) δ = 8.08 - 7.85 (m, 6H), 7.69 - 7.28 (m, 19H), 5.62 (t, *J* = 9.3 Hz, 1 H), 5.41 (t, *J* = 9.8 Hz, 1H), 5.22 (s, 1H), 5.08 (dd, *J* = 9.2, 7.9 Hz, 1 H), 4.70 *(d, J* = 7.8 Hz, 1H), 4.63 (d, *J* = 12.1 Hz, 1H), 4.58 (d, *J* = 10.0 Hz, 1H), 4.40 (d, *J* = 12.1 Hz, 1H), 4.19 (t, *J* = 9.5 Hz, 1H), 3.82 (td, *J* = 9.2, 3.6 Hz, 1H), 3.70 (dd, *J* = 11.2, 3.4 Hz, 1 H), 3.63 (dd, *J* = 10.6, 5.0 Hz, 1 H), 3.59 - 3.55 (m, 1 H), 3.54 - 3.48 (m, 1H), 3.32 (t, *J* = 9.3 Hz, 1H), 3.15 (td, *J* = 9.7, 5.0 Hz, 1H), 2.78 - 2.60 (m, 3H), 2.50 (d, *J* = 3.6 Hz, 1H), 1.22 (t, *J* = 7.5 Hz, 3H). $^{13}$C NMR (101 MHz, $CDCl_3$) δ = 165.38, 165.29, 165.13, 138.02, 136.70, 133.43, 133.14, 133.09, 130.08, 129.87, 129.81, 129.71, 129.30, 129.28, 129.23, 128.50, 128.48, 128.29, 127.95, 127.86, 126.19, 101.59, 100.88, 83.42, 80.42, 78.71, 75.71, 74.67, 74.51, 73.46, 72.42, 70.48, 67.70, 67.51, 65.76, 24.11, 14.85.

Substrate **49** (1.6g, 1.824 mmol, 1 equiv.) was taken in anhydrous DCM (10 mL) at 0°C and added pyridine (10 mL) and BzCl (0.635 mL, 5.47 mmol, 3 equiv.) to it dropwise and RM was stirred for 16 h.
RM was then evaporated in vacuum to remove solvents and then taken again in DCM (25 mL) and washed with aq.$NaHCO_3$ solution (5mL X 2). Organic layer was then dried on $Na_2SO_4$, filtered and evaporated in vacuum which was then triturated using methanol to get off-white solid **(48),** filtered, dried in vacuum (1.5g, 84%).

$^1$H NMR (400 MHz, $CDCl_{33}$) δ 7.95 (ddd, *J* = 16.9, 12.1, 7.3 Hz, 8H), 7.60 - 7.12 (m, 22H), 5.66 (t, *J* = 9.3 Hz, 1 H), 5.58 (t, *J* = 9.6 Hz, 1 H), 5.43 (t, *J* = 9.8 Hz, 1 H), 5.36 (dd, *J* = 9.5, 7.9 Hz, 1 H), 5.20 (s, 1 H), 4.77 (d, *J* = 7.9 Hz, 1 H), 4.60 (t, *J* = 10.3 Hz, 2H), 4.39 (d, *J* = 12.1 Hz, 1 H), 4.23 (t, *J* = 9.5 Hz, 1 H), 3.74 - 3.43 (m, 5H), 3.29 (td, *J* = 9.7, 4.9 Hz, 1 H), 2.83 - 2.55 (m, 3H), 1.22 (t, *J* = 7.4 Hz, 3H). $^{13}$C NMR (101 MHz, $CDCl_3$) δ 165.46, 165.25, 165.16, 164.72, 137.82, 136.62, 133.31, 133.12, 133.02, 130.05, 129.80, 129.73, 129.70, 129.27, 129.24, 128.99, 128.97, 128.63, 128.43, 128.29, 128.22, 128.17, 128.10, 127.98, 126.03, 101.12, 83.39, 78.65, 78.29, 75.85, 74.44, 73.44, 72.43, 71.96, 70.47, 67.71, 67.31, 66.16, 24.04, 14.84.

**Example C.1.2. Synthesis of 2,3-di-*O*-benzoyl-β-D-glucopyranosyl-(1→4)-2,3-di-*O*-benzoyl-6-*O*-benzyl-β-D-glucoyranosyl-(1→1)-(2-N-benzyl-N-benzyloxycarbonylamino)ethanol (50)**

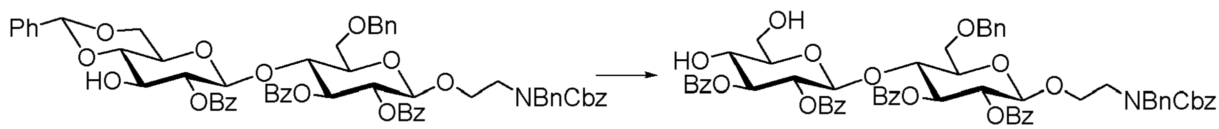

49 50

To a stirred solution of alcohol **49** (400 mg, 0.36 mmol) in pyridine (5.0 mL) was added at 0 °C benzoyl chloride (63 μL, 0.55 mmol). The reaction was slowly warmed to room temperature and stirred for 16 h at that temperature. An additional 0.5 equiv. BzCl were added to drive the reaction to completion. The mixture was stirred for 2 h at room temperature, quenched with water (30 ml) and diluted with EtOAc (50 mL). After separation, the organic fraction was washed with 0.1 M HCl (20 mL) and the aqueous fraction was re-extracted with EtOAc (30 mL). The combined organic fractions were washed with sat. aq. $NaHCO_3$ (20 mL) and brine (10 mL), dried over $Na_2SO_4$ and concentrated to give the intermediate tetrabenzoate as a yellow oil.

To a stirred solution of the intermediate tetrabenzoate in $CH_2Cl_2$ (6.5 mL) were added at room temperature ethanethiol (0.36 mL, 4.9 mmol) and *p*-toluenesulfonic acid (12 mg, 0.06 mmol). The mixture was stirred for 2 h at that temperature, quenched with $Et_3N$ (50 μL) and concentrated. The residue was purified by flash chromatography (EtOAc/hexanes 0:1 to 1:10 to 1:8) to give diol **50** (389 mg, 0.349 mmol) as a white foam. HRMS (ESI) calcd. for $C_{64}H_{61}NO_{17}$ $(M+Na)^+$ 1138.3837 found 1138.3850 *m/z.*

**Example C.1.3. Synthesis of methyl(2,3-di-*O*-benzoyl-β-D-glucopyranosyl)uronate-(1→4)-2,3-di-*O*-benzoyl-6-*O*-benzyl-β-D-glucoyranosyl-(1→1)-(2-*N*-benzyl-*N*-benzyloxycarbonylamino)ethanol (51)**

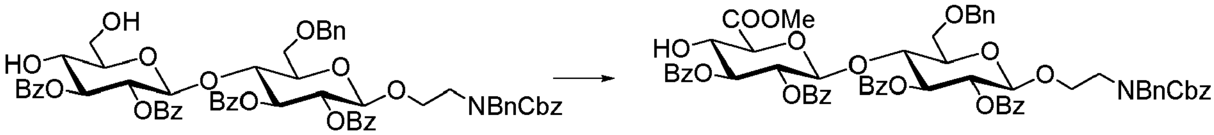

**50** **51**

To a stirred solution of alcohol **50** (90 mg, 0.081 mmol) in $CH_2Cl_2$ (2.0 mL) and water (0.8 mL) were added at 0 °C TEMPO (2.5 mg, 0.016 mmol) and BAIB (55 mg, 0.170 mmol). The reaction was stirred for 20 min at that temperature and warmed to room temperature. The mixture was stirred for 2 h at that temperature and diluted with EtOAc (20 mL) and water (10 mL). After separation, the aqueous fraction was extracted with EtOAc (2x 10 mL), the combined organic fractions were dried over $Na_2SO_4$ and concentrated. The residue was purified by flash chromatography (EtOAc/hexanes 1:2 to 1:1, then 1:1 + 5% AcOH) to give the intermediate carboxylic acid as a white foam.

To a stirred solution of the intermediate carboxylic acid in toluene (1.6 mL) and MeOH (0.8 mL) was added at room temperature TMS-diazomethane (0.04 mL, 0.081 mmol). The reaction was stirred for 2 h at that temperature. An additional 0.25 equiv. TMS-diazomethane was added to drive the reaction to completion. The mixture was stirred for 1 h at that temperature, quenched with AcOH (0.1 mL) and concentrated. The residue was purified by flash chromatography (EtOAc/hexanes 0:1 to 1:1) to give methyl ester **51** (73 mg, 0.064 mmol, 79% over two steps) as a clear oil. HRMS (ESI) calcd. for $C_{65}H_{61}NO_{18}$ $(M+Na)^+$ 1166.3786 found 1166.3762 m/z.

**Example C.1.4. Synthesis of *t*-hexyl 2,3,6-tri-*O*-benzyl-β-D-galactopyranoside (52)**

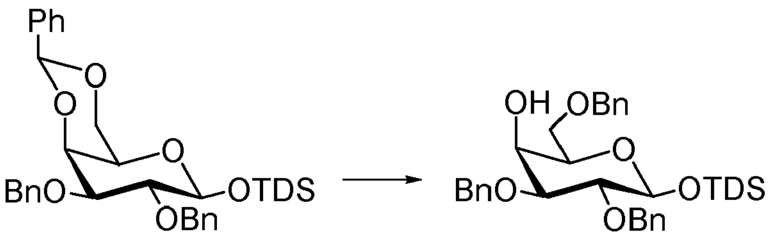

**53** **52**

To a stirred solution of benzylidene acetal **53** (J Carbohyd Chem 1996, 15 (2), 241) (1.68 g, 2.84 mmol) in $CH_2Cl_2$ (60 mL) over activated MS (3 A-AW) were added at 0 °C triethyl silane (2.72 mL, 17.06 mmol) and trifluoroacetic acid (1.81 mL, 17.06 mmol). The reaction was slowly warmed to room temperature and stirred for 16 h at that temperature. The reaction was quenched with $Et_3N$ (2 mL), filtered through Celite and concentrated. The residue was purified by flash chromatography (EtOAc/hexanes 1:20 to 1:7) to give alcohol **52** (1.46 g, 2.46 mmol, 87%) as a clear oil. HRMS (ESI)

calcd. for $C_{35}H_{48}O_6Si$ $(M+Na)^+$ 615.3117 found 615.3104 *m/z*.

**Example C.1.5. Synthesis of *t*Hexyl 4-*O*-benzoyl-2,3,6-tri-*O*-benzyl-α-D-glucopyranosyl-(1→4)-2,3,6-tri-*O*-benzyl-β-D-galactopyranoside (54)**

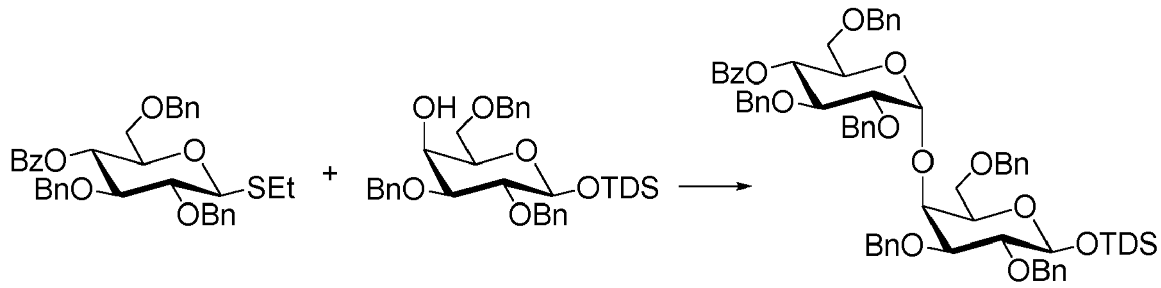

**55** **52** **54**

Thioglycoside **55** (J. Org. Chem. 2012, 77 (1), 291). (667 mg, 1.11 mmol) and alcohol **52** (550 mg, 0.93 mmol) were co-evaproated with dry toluene (3x10 mL) and kept under high vacuum for 30 min. The mixture was dissolved in $Et_2O$ (14 mL) and $CH_2Cl_2$ (2.8 mL) and stirred over activated molecular sieves (3 A-AW) for 30 min at room temperature. The solution was cooled to -20 °C and treated with NIS (250 mg, 1.11 mmol) and triflic acid (16 μL, 0.19 mmol). The mixture was stirred for 1 h and slowly warmed to -10 °C. The reaction was quenched with $Et_3N$ (0.05 mL), diluted with $CH_2Cl_2$ (20 mL), filtered through Celite and concentrated. The residue was purified by flash chromatography (EtOAc/hexanes 0:1 to 1:8 to 1:6) to give disaccharide **54** (553 mg, 0,490 mmol, 53%) along with the corresponding β-anomer (231 mg, 0.205 mmol, 22%). Analytical data for **54**: Clear oil. HRMS (ESI) calcd. for $C_{69}H_{80}O_{12}Si$ $(M+Na)^+$ 1151.5316 found 1151.5293 *m/z.*

**Example C.1.6. Synthesis of 4-*O*-benzoyl-2,3,6-tri-*O*-benzyl-α-D-glucopyranosyl-(1→4)-2,3,6-tri-*O*-benzyl-αβ-D-galactopyranosyl trifluoro-(*N*-phenyl)acetimidate (47)**

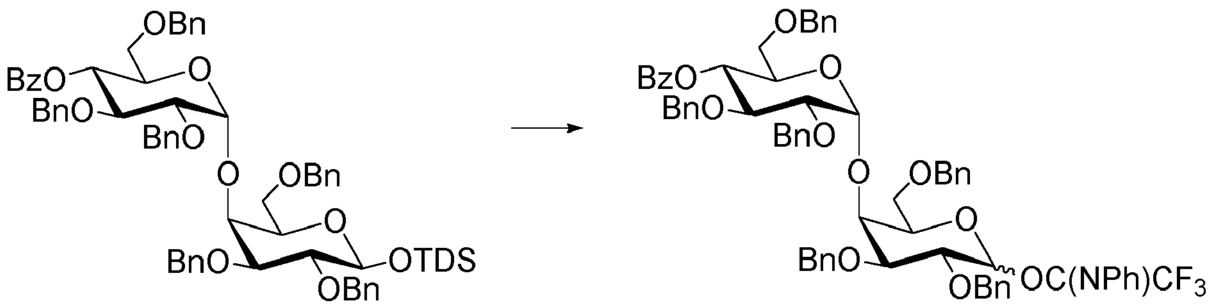

**54** **47**

To a stirred solution of silyl ether **54** (470 mg, 0.416 mmol) in THF (8.3 mL) was added at 0 °C acetic acid (0.24 mL, 4.19 mmol) and TBAF (1.0 M solution in THF, 4.2 mL, 4.20 mmol). The reaction was slowly warmed to room temperature and stirred for 2 h at that temperature. Acetic acid (0.24 mL, 4.19 mmol) and TBAF (1.0 M solution in THF, 4.2 mL, 4.20 mmol) were added and the reaction was stirred for 16 h at room temperature. The mixture was diluted with $Et_2O$ (50 mL), washed with water (3x30 mL) and the aqueous phase was re-extracted with $Et_2O$ (2x20 mL). The combined organic fractions were dried over $Na_2SO_4$ and concentrated. The residue was filtered through a short plug of silica gel (EtOAc/hexanes 1:3 to 1:1) to give the intermediate lactol mixture as a clear oil.
To a stirred solution of the lactol mixture in $CH_2Cl_2$ (7.8 mL) were added at room temperature cesium carbonate (318 mg, 0.975 mmol) and $F_3CC(NPh)Cl$ (202 mg, 0.975 mmol). The mixture was stirred for 2.5 h at that temperature, diluted with hexanes (0.5% (v/v) $Et_3N$, (10 mL) and filtered through Celite. The residue was purified by flash chromatography (EtOAc/hexanes 0:1 + 0.5%$Et_3N$ to 1:3 + 0.5% $Et_3N$) to give imidate mixture **47** (404 mg, 0.349 mmol, 84% over two

steps) as a clear oil. HRMS (ESI) calcd. for $C_{69}H_{66}F_3NO_{12}$ $(M+Na)^+$ 1180.4434 found 1180.4458 *m/z.*

**Example C.1.7: Synthesis of 4-*O*-benzoyl-2,3,6-tri-*O*-benzyl-α-D-glucopyranosyl-(1→4)-2,3,6-tri-*O*-benzyl-α-D-galactopyranosyl-(1→4)-methyl [2,3-Di-*O*-benzoyl-β-D-glucopyranosyl]uronate-(1→4)-2,3-di-*O*-benzoyl-6-*O*-benzyl-β-D-glucoyranosyl-(1→1)-(2-*N*-benzyl-*N*-benzyloxycarbonylamino)ethanol (56)**

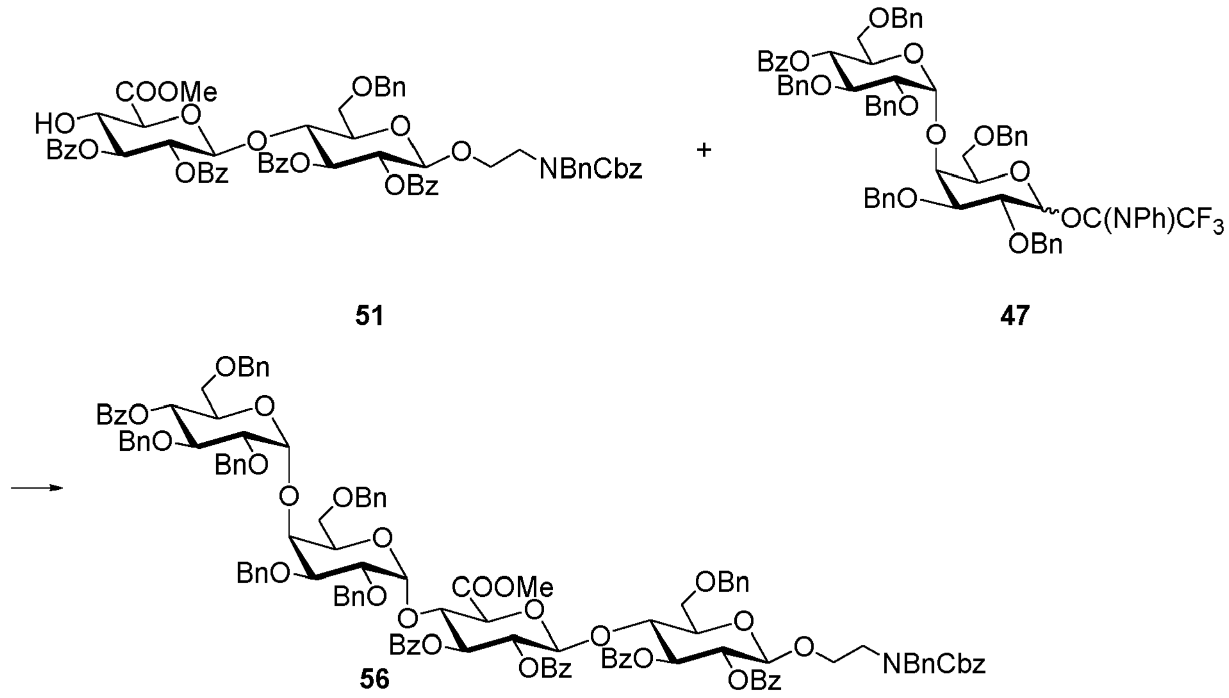

51

47

56

Alcohol **51** (100 mg, 87 μmol) and imidate **47** (121 mg, 105 μmol) were co-evaproated with dry toluene (3x10 mL) and kept under high vacuum for 30 min.

The mixture was dissolved in $Et_2O$ (3.3 mL) and $CH_2Cl_2$ (1.1 mL) and stirred over activated molecular sieves (3 A-AW) for 30 min at room temperature. The solution was cooled to -20 °C and treated with TMSOTf (3.2 μL, 17 μmol). The mixture was stirred for 1 h and slowly warmed to 0 °C. The reaction was quenched with sat. aq. $NaHCO_3$ (10 mL), extracted with $CH_2Cl_2$ (3x20 mL) and the combined organic fractions were dried over $Na_2SO_4$ and concentrated. The residue was purified by flash chromatography (EtOAc/hexanes/toluene 1:3:3 to 1:2:2) to give tetrsaccharide **56** (130 mg, 62 μmol, 71%) as a clear oil. HRMS (ESI) calcd. for $C_{126}H_{121}NO_{29}$ $(M+Na)^+$ 2134.7921 found 2134.7879 *m/z.*

**Example C.1.8 Synthesis of α-D-glucopyranosyl-(1→4)-α-D-galactopyranosyl-(1→4)-β-D-glucopyranosyluronic acid-(1→4)-β-D-glucoyranosyl-(1→1)-(2-amino)ethanol (44)**

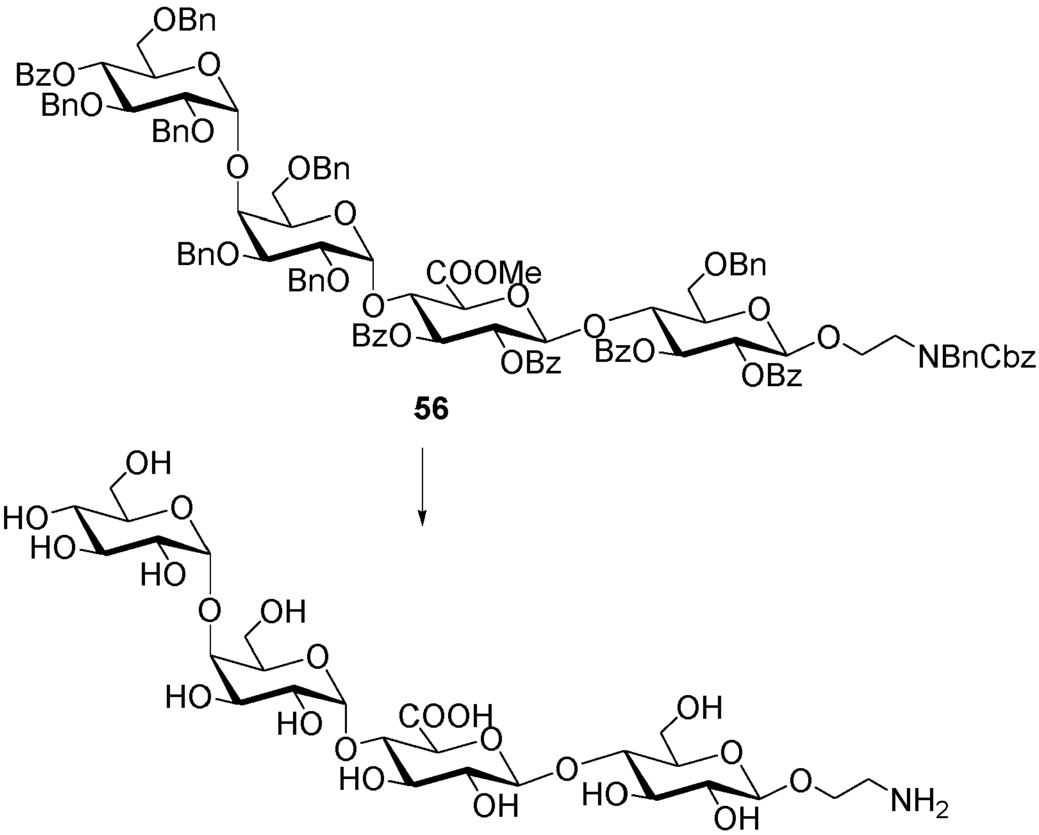

56

44

To a stirred solution of ester **56** (56 mg, 26 μmol) in THF (5 mL) and MeOH (1 mL) were added at 0 °C hydrogen peroxide (6% aq. solution, 265 μL, 530 μmol) and LiOH (1 M aq. solution, 265 μL, 132 mol). The reaction was stirred for 1 h and warmed to room temperature. The reaction was kept at that temperature and treated after 2 h with hydrogen peroxide (6% aq. solution, 265 μL, 530 μmol) and LiOH (1 M aq. solution, 265 μL, 132 mol). After 2 h, NaOH (15% aq. solution, 1 mL) was added and the mixture was stirred for 72 h at room temperature. The solvents were evaporated under reduced pressure, the residue was co-evaporated with toluene (2x5 mL) and dissolved in MeOH (5 mL). The solution was treated at room temperature with sodium methoxide (143 mg, 2.65 mmol) and stirred for 96 h at that temperature. The solvent was evaporated and the residue was dissolved in water (5 mL). The solution was neutralized at 0 °C with 0.5 M aq. $NaHSO_4$ and extracted with EtOAc (5x5 mL). The combined organic fractions were dried over $Na_2SO_4$ and concentrated to give the intermediate acid as a white foam.
The intermediate acid in MeOH (2 mL) was added at room temperature to a suspension of Pd/C (50 mg) in MeOH (1 mL), water (0.1 mL) and AcOH (5 drops). The reaction was stirred under an atmosphere of $H_2$ for 48 h, filtered and concentrated. The residue was purified by solid phase extraction (Chromabond C18, Macherey-Nagel) and lyophilized to give tetrasaccharide 44 (acetate salt, 13.6 mg, 18 μmol, 69% over 3 steps) as a white solid. HRMS (MALDI) calcd. for $C_{26}H_{45}NO_{22}$ $(M+Na)^+$ 746.2330 found 746.2323 *m/z.*

**Example C.1.9. Synthesis of 4-*O*-Benzoyl-2,3,6-tri-*O*-benzyl-α-D-glucopyranosyl-(1→4)-2,3,6-tri-*O*-benzyl-α-D-galactopyranosyl-(1→1)-(2-*N*-benzyl-*N*-benzyloxycarbonylamino)ethanol** (57)

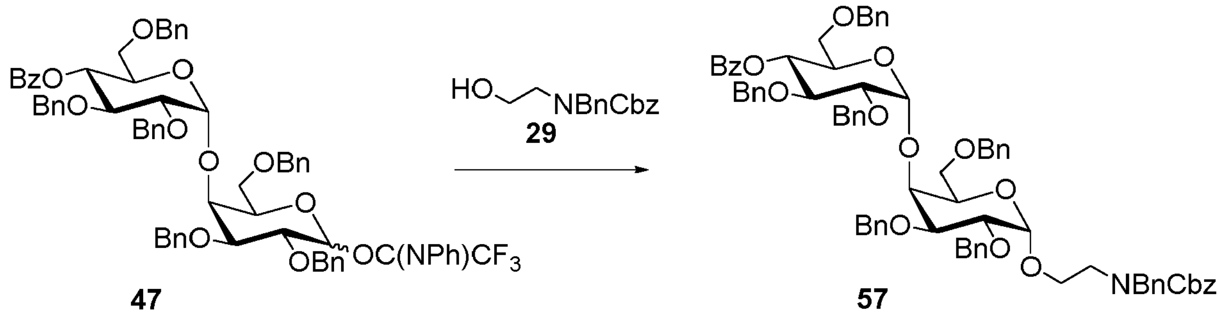

47 29 57

Imidate **47** (200 mg, 0.173 mmol) and alcohol **29** (74 mg, 0.259 mmol) were co-evaproated with dry toluene (2x5 mL) and kept under high vacuum for 30 min. The mixture was dissolved in $Et_2O$ (2.8 mL) and $CH_2Cl_2$ (0.7 mL) and stirred over activated molecular sieves (3 A-AW) for 30 min at room temperature. The solution was cooled to -40 °C and treated with TMSOTf (6.2 μL, 35 μmol). The mixture was stirred for 10 min at that temperature and then slowly warmed to -10 °C. The reaction was quenched with sat. aq. $NaHCO_3$ (5 mL), extracted with $CH_2Cl_2$ (3x20 mL) and the combined organic fractions were dried over $Na_2SO_4$ and concentrated. The residue was purified by flash chromatography (EtOAc/hexanes 0:1 to 1:6 to 1:4) to give carbamate **57** (160 mg, 0.128 mmol, 74%) along with the corresponding β-anomer (32 mg, 0.026 mmol, 15%). Analytical data for **57**: Clear oil. HRMS (ESI) calcd. for $C_{78}H_{79}NO_{14}$ $(M+Na)^+$ 1276.5398 found 1276.5405 *m/z.*

**Example C.1.10. Synthesis of 2,3,6-tri-O-benzyl-α-D-glucopyranosyl-(1→4)-2,3,6-tri-O-benzyl-α-D-galactopyranosyl-(1→1)-(2-N-benzyl-N-benzyloxycarbonylamino)ethanol (58)**

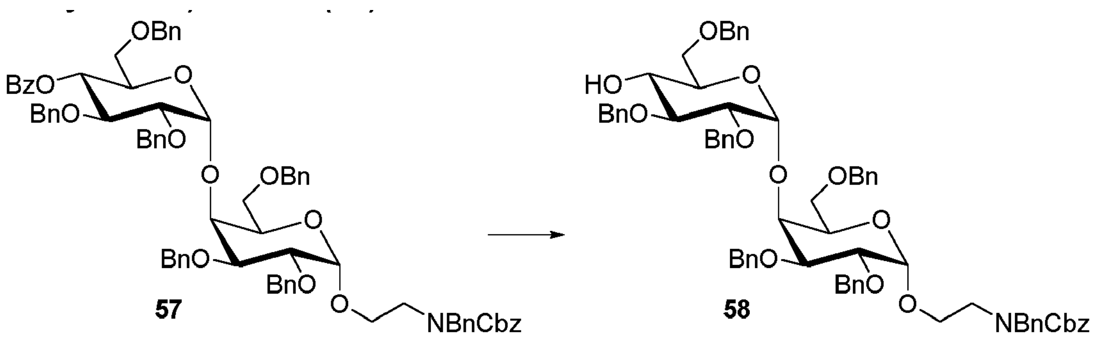

57 58

To a stirred solution of ester **57** (126 mg, 0.100 mmol) in THF (5 mL) and MeOH (5 mL) was added at 0 °C sodium methoxide (0.5 M in MeOH, 1 mL, 0.500 mmol). The reaction was slowly warmed to room temperature and kept at that temperature for 24 h. Sodium methoxide (0.5 M in MeOH, 1 mL, 0.500 mmol) was added and the reaction was warmed to 37 °C. The mixture was stirred for 7 h at that temperature, neutralized with Amberlite IR120 ($H^+$ form), filtered and concentrated. The residue was purified by flash chromatography (EtOAc/hexanes 0:1 to 1:6 to 1:4) to give alcohol 58 (98 mg, 85 μmol, 85%) a s a clear oil. HRMS (ESI) calcd. for $C_{71}H_{75}NO_{13}$ $(M+Na)^+$ 1172.5136 found 1172.5103 *m/z.*

**Example C.1.11. Synthesis of α-D-glucopyranosyl-(1→4)-β-D-galactopyranosyl-(1→1)-(2-amino)ethanol (59)**

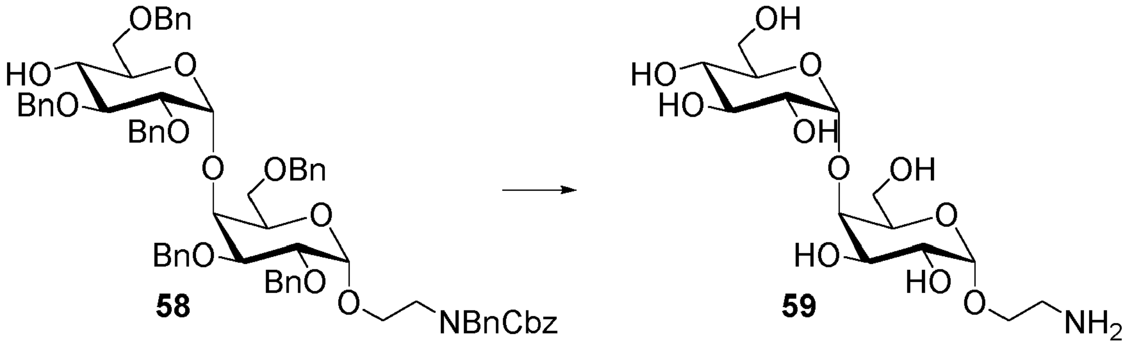

58 59

Benzyl ether 58 in EtOAc (1 mL) was added at room temperature to a suspension of Pd/C (30 mg) in MeOH (3 mL), water (0.5 mL) and AcOH (3 drops). The reaction was stirred under an atmosphere of $H_2$ for 24 h, filtered and concentrated to give disaccharide **59** (2.9 mg, 18 μmol, 87%) as a white solid. HRMS (ESI) calcd. for $C_{14}H_{27}NO_{11}$ $(M+Na)^+$ 408.1481 found 408.1499 *m/z.*

**Example C.1.12. Synthesis of 2,3-di-*O*-benzoyl-β-D-glucopyranosyl-(1→4)-2,3-di-*O*-benzoyl-6-*O*-benzyl-β-D-glucoyranosyl-(1→4)-2,3,6-tri-O-benzyl-α-D-glucopyranosyl-(1→4)-2,3,6-tri-*O*-benzyl-α-D-galactopyranosyl-(1→1)-(2-N-benzyl-*N*-benzyloxycarbonylamino)ethanol (60)**

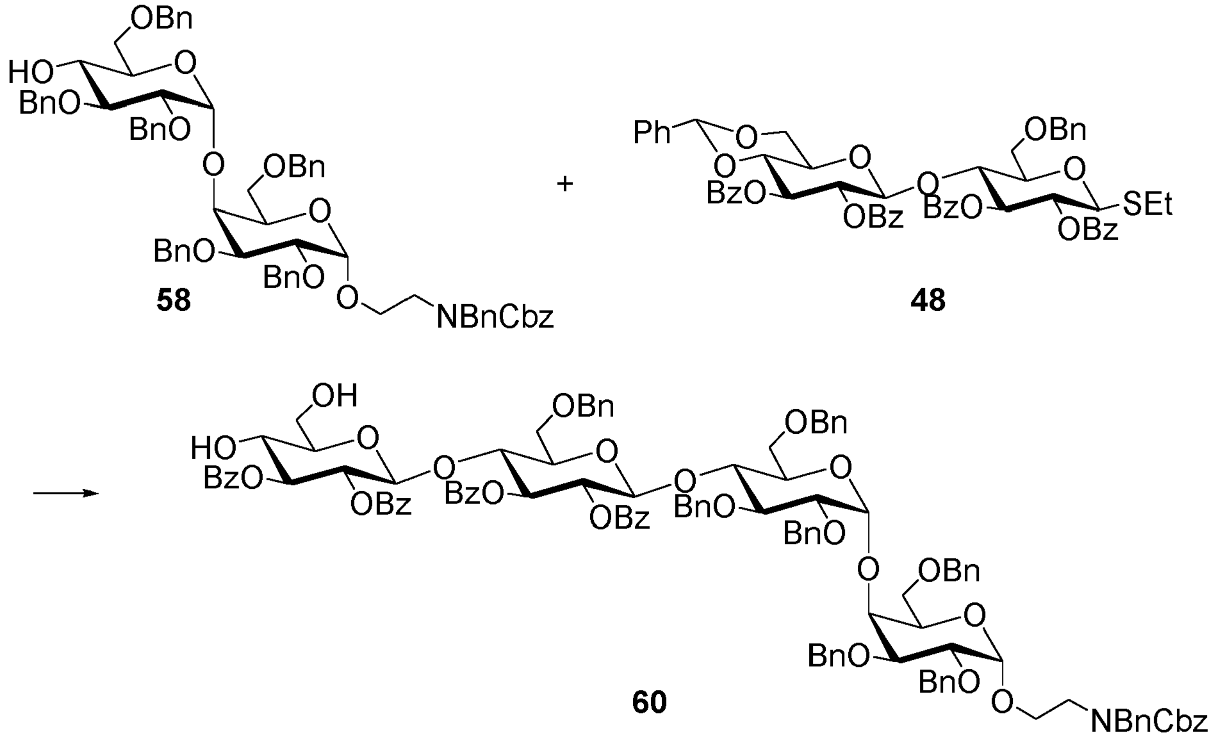

58 + 48

60

Alcohol **58** (47 mg, 41 μmol) and thioglycoside **48** (60 mg, 61 μmol) were co-evaporated with dry toluene (2x5 mL) and kept under high vacuum for 30 min. The mixture was dissolved in $CH_2Cl_2$ (2 mL) and stirred over activated molecular sieves (3 A-AW) for 30 min at room temperature. The solution was cooled to -10 °C and treated with NIS (13.8 mg, 61 μmol) and triflic acid (1 μL, 11 μmol). The mixture was kept for 1 h at that temperature and slowly warmed to 0 °C. The reaction was quenched with $Et_3N$ (50 μL), filtered and concentrated to give the intermediate benzylidene acetal as a yellow oil.

To a stirred solution of the intermediate benzylidene acetal in $CH_2Cl_2$ (2 mL) were added at room temperature ethanethiol (0.3 mL, 4.06 mmol) and *p*-toluenesulfonic acid (10 mg, 0.053 mmol). The mixture was stirred for 1 h at that temperature, quenched with $Et_3N$ (20 μL) and concentrated. The residue was purified by flash chromatography (EtOAc/hexanes 0:1

to 1:3 to 1:2) to give diol **60** (78 mg, 39 μmol, 96%) as a clear oil. HRMS (ESI) calcd. for $C_{n8}H_{117}NO_{27}$ $(M+Na)^+$ 2002.7710 found 2002.7731 *m/z.*

**Example C.1.13. Synthesis of 2,3-di-*O*-benzoyl-β-D-glucopyranosyluronate-(1→4)-2,3-di-*O*-benzoyl-6-*O*-benzyl-β-D-glucoyranosyl-(1→4)-2,3,6-tri-*O*-benzyl-α-D-glucopyranosyl-(1→4)-2,3,6-tri-*O*-benzyl-α-D-galactopyranosyl-(1β1)-(2-*N*-benzyl-*N*-benzyloxycarbonylamino)ethanol (61)**

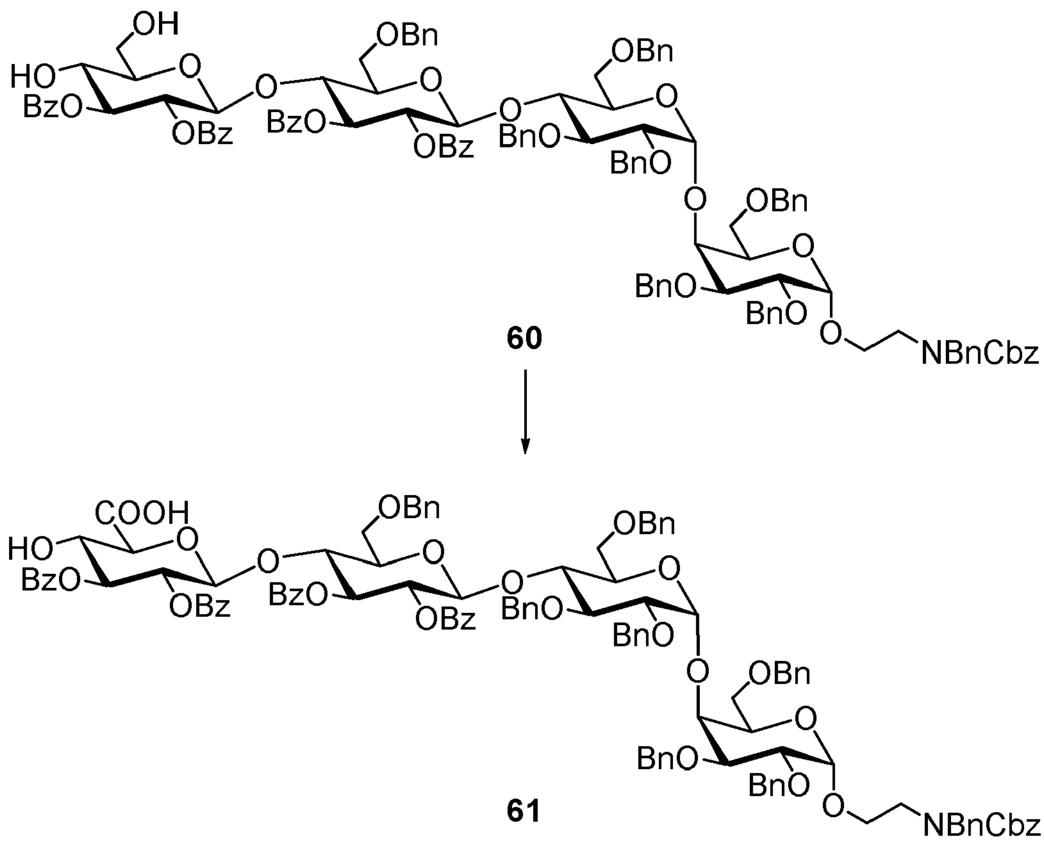

60

61

To a vigorously stirred solution of alcohol **60** (45 mg, 23 μmol, 73%) in $CH_2Cl_2$ (2 mL) and water (0.8 mL) were added at 0 °C TEMPO (3 crystals) and BAIB (15.4 mg, 48 μmol). The reaction was stirred for 20 min at that temperature and slowly warmed to room temperature. After 1 h, TEMPO (2 crystals) and BAIB (10 mg, 31 μmol) were added and the mixture was stirred for 2 h at room temperature. The reaction was diluted with $CH_2Cl_2$ (5 mL) and quenched with 10% aq. $Na_2S_2O_3$ (5 mL). Following separation, the aqueous phase was extracted with EtOAc (2x10 mL), the combined organic fractions were dried over $Na_2SO_4$ and concentrated. The residue was purified by flash chromatography twice (EtOAc/hexanes 0:1 to 1:2 to 8:1, then EtOAc/hexanes 1:1+1% AcOH) and co-evaporated with heptane repeatedly to give acid **61** (33 mg, 17 μmol, 74%) as a clear oil. HRMS (ESI) calcd. for $C_{118}H_{115}NO_{28}$ $(M+Na)^+$ 2016.7503 found 2016.7558 *m/z.*

**Example C.1.14. Synthesis of β-D-Glucopyranosyluronic acid-(1→4)-β-D-glucoyranosyl-(1→4)-a-D-glucopyranosyl-(1→4)-α-D-galactopyranosyl-(1→1)-(2-amino)ethanol (45)**

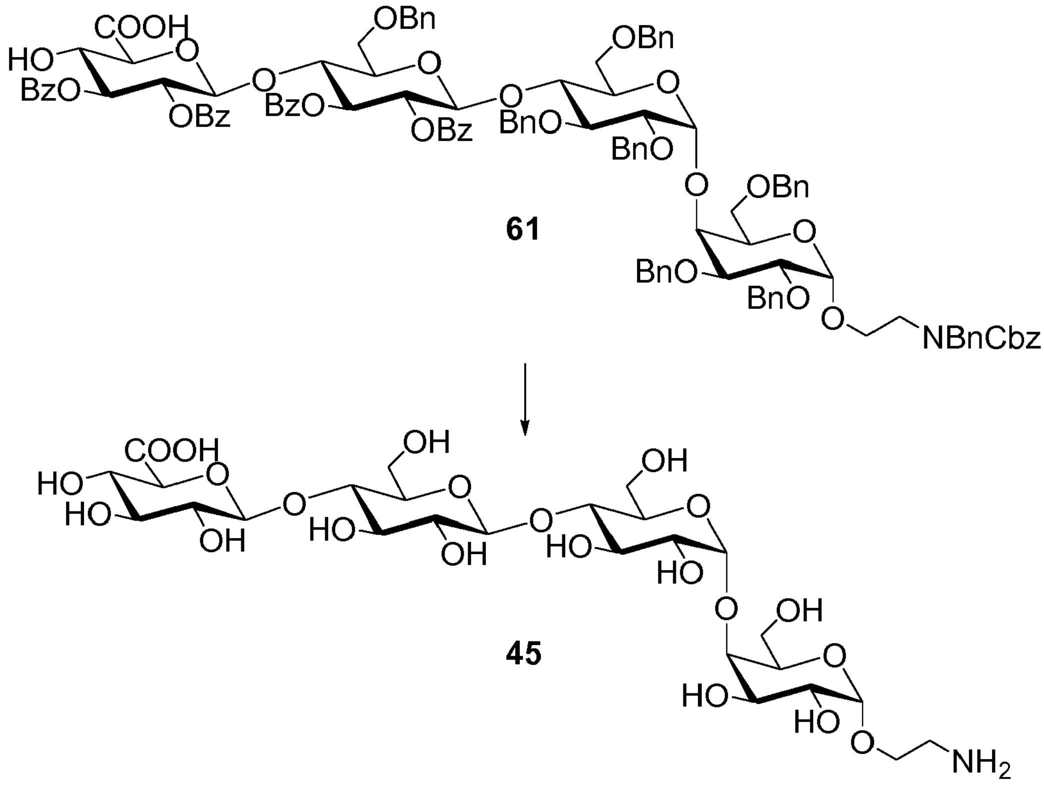

61

45

To a stirred solution of ester **61** (45 mg, 23 μmol) in THF (4 mL) and MeOH (0.5 mL) were added at 0 °C NaOH (1 M aq. solution, 1 mL). The reaction was slowly warmed to room temperature and stirred for 16 h at that temperature. The solution was neutralized at 0 °C with 0.5 M aq. $NaHSO_4$ and extracted with EtOAc (5x5 mL). The combined organic fractions were dried over $Na_2SO_4$ and concentrated to give the intermediate alcohol as a white foam.
The intermediate alcohol in MeOH (3 mL) was added at room temperature to a suspension of Pd/C (20 mg) in MeOH (6 mL), water (6 drops) and AcOH (3 drops). The reaction was stirred under an atmosphere of $H_2$ for 96 h, filtered and concentrated. Since the reaction had not proceeded to completion, the residue was subjected to the same conditions again and stirred for 72 h at room temperature. The reaction was filtered and concentrated, the residue was purified by solid phase extraction (Chromabond C18, Macherey-Nagel) and lyophilized to give tetrasaccharide **45** (11.3 mg, 16 μmol, 68% over 2 steps) as a white solid. HRMS (MALDI) calcd. for $C_{26}H_{45}NO_{22}$ $(M+Na)^+$ 746.2330 found 746.2416 *m/z.*

**Example C.1.15. 2-Amino-ethyl β-D-glucopyranosyl-(1→4)-β-D-glucopyranosyl-(1→4)-α-D-glucopyranosyl-(1→4)-α-D-galactopyranoside (46)**

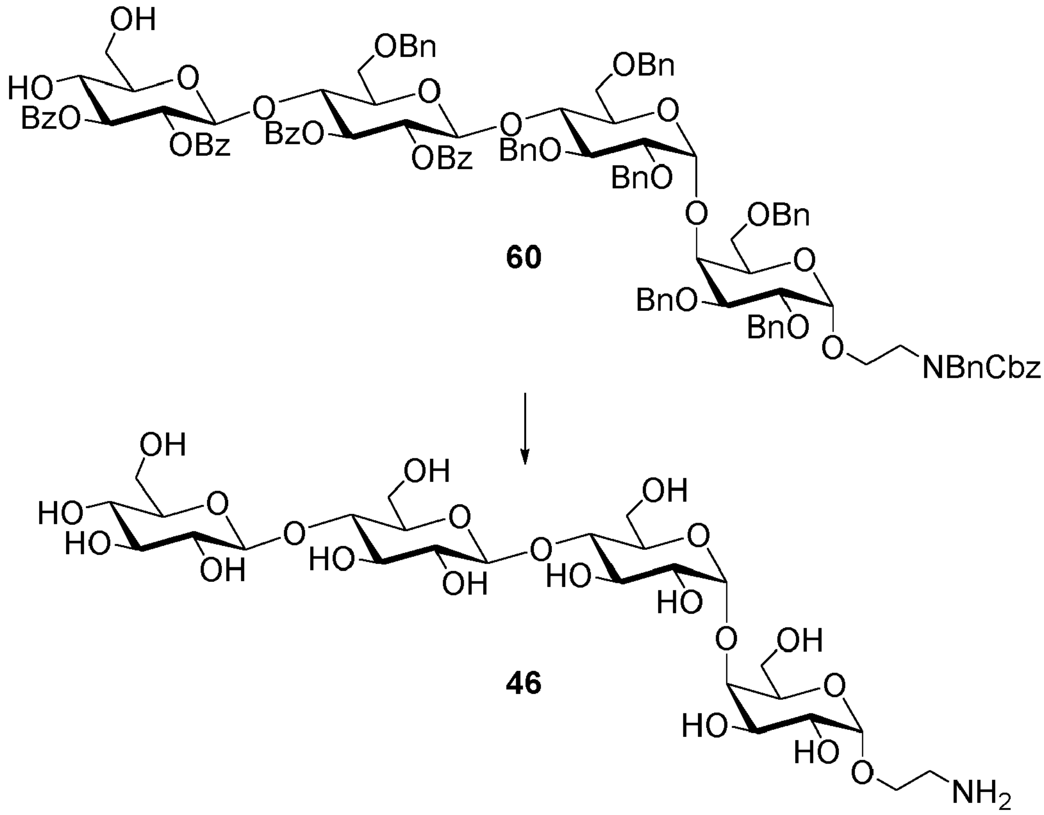

60

46

To a stirred solution of ester **60** (20 mg, 10.1 μmol) in THF (1 mL) and MeOH (0.33 mL) was added at room temperature NaOMe (0.5 M solution in MeOH, 0.5 mL). The reaction was warmed to 40 °C and stirred for 5 h at that temperature. The mixture was cooled to room temperature and stirred for 16 h at that temperature. The reaction was neutralized with Amberlite IR-120 (H+ form), filtered and concentrated. The residue was purified by size exclusion chromatography (Sephadex LH-20, $CH_2Cl_2$/MeOH 2:1) to give the intermediate hexanol as a white foam.
The intermediate hexaol in $CH_2Cl_2$/*t*BuOH/water (1:16:8, 1 mL) was purged with argon and treated at 0 °C with a suspension of $Pd(OH)_2$ on carbon (20% (w/w) loading, 20 mg) in the same solvent mixture (1 mL). The suspension was purged with hydrogen, stirred under a hydrogen atmosphere for 18 h, filtered and concentrated. The residue was purified by solid phase extraction (Chromabond C18, Macherey-Nagel) and lyophilized to give tetrasaccharide **46** (6.8 mg, 9.0 μmol, 89% over two steps) as a white solid. 1 H NMR (600 MHz, $D_2O$) δ 5.15 (d, *J* = 3.5 Hz, 1 H, H-1 b), 5.02 (d, *J* = 3.7 Hz, 1 H, H-1 a), 4.66 (d, *J* = 7.9 Hz, 1 H, H-1 c or H-1 d), 4.61 (d, *J* = 7.9 Hz, 1 H, H-1 c or H-1 d), 4.33 (d, *J* = 10.1 Hz, 1 H), 4.18 (d, *J* = 2.2 Hz, 1 H), 4.14 - 4.06 (m, 4H), 4.05 - 3.97 (m, 4H), 3.96 - 3.89 (m, 4H), 3.87 - 3.67 (m, 7H), 3.64 - 3.56 (m, 2H), 3.55 - 3.49 (m, 1 H), 3.46 (t, *J* = 8.4 Hz, 1 H), 3.45 - 3.34 (m, 3H); 13C NMR (150 MHz, $D_2O$) δ 105.2 (C-1 c or C-1 d), 104.9 (C-1c or C-1d), 102.5 (C-1a), 101.2 (C-1b), 81.2, 81.0, 80.9, 78.6, 78.1, 77.4, 76.7, 75.7, 75.6, 74.2, 74.1, 73.8, 73.3, 72.1, 71.4, 70.9, 66.6, 63.2, 63.1, 62.5, 62.1, 41.9; HRMS (ESI) calcd. for C26H47NO21 (M+Na)+ 732.2538 found 732.2504 *m/z.*

**Example C.2. Synthesis of *S. pneumoniae* serotype 8 pentasaccharide 62**

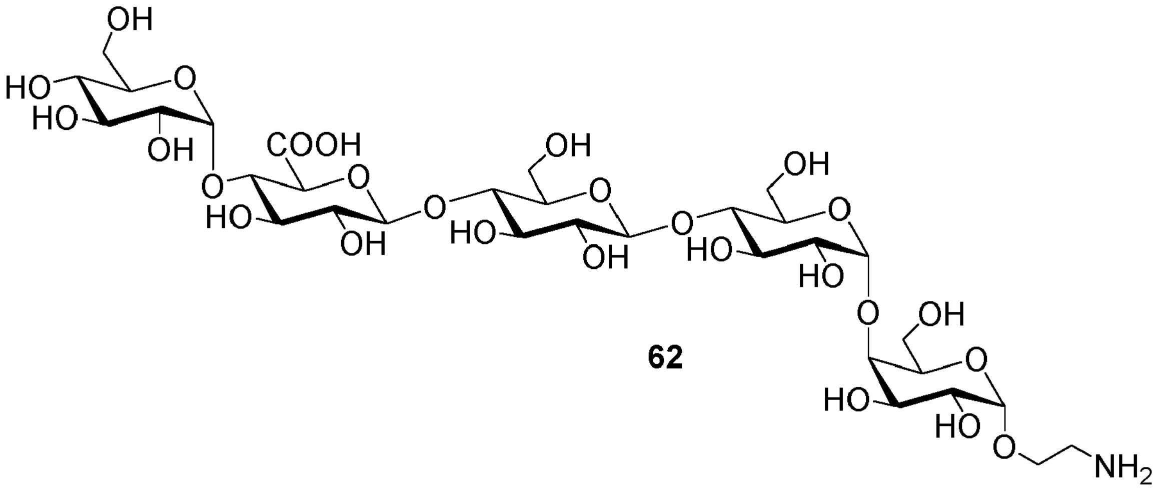

62

**Example C.2.1. *N*-Benzyl-*N*-benzyloxycarbonyl-2-amino-ethyl methyl[2,3-di-*O*-benzoyl-β-D-glucopyranosyl]uronate-(1→4)-2,3-di-O-benzoyl-6-O-benzyl-β-D-glucopyranosyl-(1→4)-2,3,6-tri-*O*-benzyl-α-D-glucopyranosyl-(1→4)-2,3,6-tri-*O*-benzyl-α-D-galactopyranoside (63)**

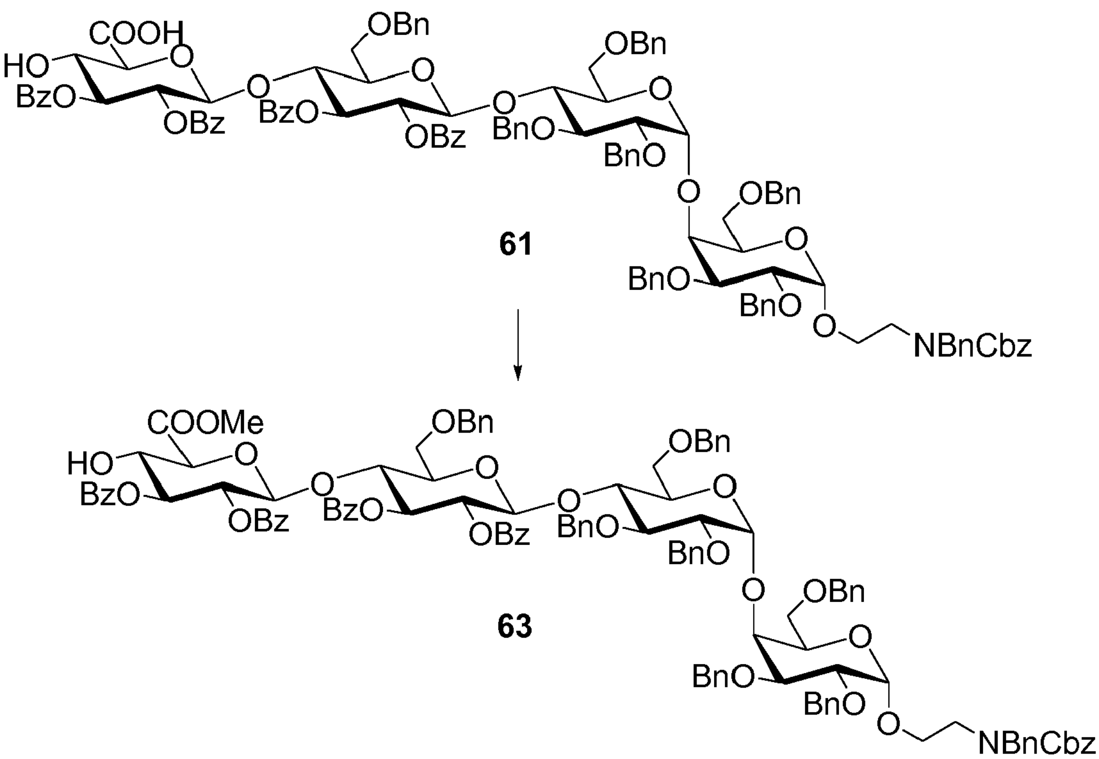

61

63

To a stirred solution of carboxylic acid **61** (100 mg, 50 μmol) in DMF (2.5 mL) were added at room temperature $Cs_2CO_3$ (24.5 mg, 75 μmol) and methyl iodide (10.7 mg, 75 μmol) and the reaction was stirred at that temperature. After 2 h, methyl iodide (10.7 mg, 75 μmol) was added and the mixture was stirred for another 2 h at room temperature. The reaction was quenched with sat. aq. $NH_4Cl$ (5 mL), extracted with EtOAc (4x10 mL), the combined organic extracts were dried over $Na_2SO_4$ and concentrated. The residue was purified by flash chromatography (EtOAc/hexanes 0:1 to 2:3) to give methyl ester **63** (81 mg, 40 μmol, 80%) as a white foam. $R_f$ (EtOAc/hexanes 1:1) = 0.83; $[\alpha]_D^{20}$ = +33.1° (c = 0.25, $CH_2Cl_2$); 1 H NMR (600 MHz, $CDCl_3$) δ 8.07 - 7.91 (m, 6H), 7.76 (d, *J* = 7.4 Hz, 2H), 7.66 (t, *J* = 7.4 Hz, 1 H), 7.62 - 7.03 (m, 56H), 5.39 (m, 3H), 5.30 (t, *J* = 9.5 Hz, 1 H), 5.25 (t, *J* = 9.5 Hz, 1 H), 5.21 - 5.14 (m, 2H), 5.00 (s, 1 H), 4.81 (d, *J* = 11.9 Hz, 1 H), 4.77 (d, *J* = 11.4 Hz, 1 H), 4.71 (d, *J* = 8.0 Hz, 1 H), 4.68 - 4.62 (m, 3H), 4.60 - 4.47 (m, 5H), 4.32 (m, 4H), 4.22 (d, *J* = 9.2 Hz, 2H), 4.12 (d, *J* = 12.1 Hz, 2H), 4.08 - 3.87 (m, 6H), 3.83 - 3.64 (m, 5H), 3.62 - 3.47 (m, 6H),

3.45 (s, 3H), 3.42 - 3.29 (m, 2H), 3.17 (d, *J* = 2.6 Hz, 1 H), 3.05 (d, *J* = 9.4 Hz, 2H); 13C NMR (150 MHz, $CDCl_3$) δ 168.4, 166.7, 165.6, 164.83, 164.79, 156.6, 156.3, 140.1, 138.80, 138.76, 138.6, 138.4, 138.34, 138.29, 138.1, 138.0, 137.9, 137.6, 136.83, 136.76, 133.6, 133.5, 133.1, 132.6, 130.4, 130.0, 129.84, 129.80, 129.7, 129.3, 129.18, 129.15, 129.1, 129.0, 128.9, 128.8, 128.7, 128.6, 128.51, 128.46, 128.44, 128.35, 128.3, 128.2, 128.1, 128.0, 127.94, 127.87, 127.7, 127.6, 127.5, 127.43, 127.40, 127.0, 100.3 (C-1 c), 100.2 (C-1 d), 99.8 (C-1 a), 98.5 (C-1 b), 80.7, 79.2, 76.7, 76.6, 75.2, 75.12, 75.07, 75.0, 74.8, 74.6, 74.40, 74.36, 74.1, 73.7, 73.62, 73.59, 73.4, 73.1, 72.5, 72.1, 71.4, 70.5, 69.6, 69.5, 67.8, 67.3, 67.0, 66.8, 52.6, 51.4, 46.5, 45.5; IR (thin film) 2928, 1734, 1602, 1453, 1364, 1272, 1094, 1070, 740, 710 $cm^{-1}$; HRMS (MALDI) calcd. for $C_{119}H_{117}NO_{28}$ (M+Na)+ 2030.7659 found 2030.7660 *m/z.*

**Example C.2.2. *N*-Benzyl-*N*-benzyloxycarbonyl-2-amino-ethyl 2,3,4,6-tetra-O-benzyl-α-D-galactopyranosyl-(1→4)-methyl[2,3-di-*O*-benzoyl-β-D-glucopyranosyl]uronate-(1→4)-2,3-di-*O*-benzoyl-6-*O*-benzyl-β-D-glucoyranosyl-(1→4)-2,3,6-tri-*O*-benzyl-α-D-glucopyranosyl-(1→4)-2,3,6-tri-*O*-benzyl-α-D-galactopyranoside (64)**

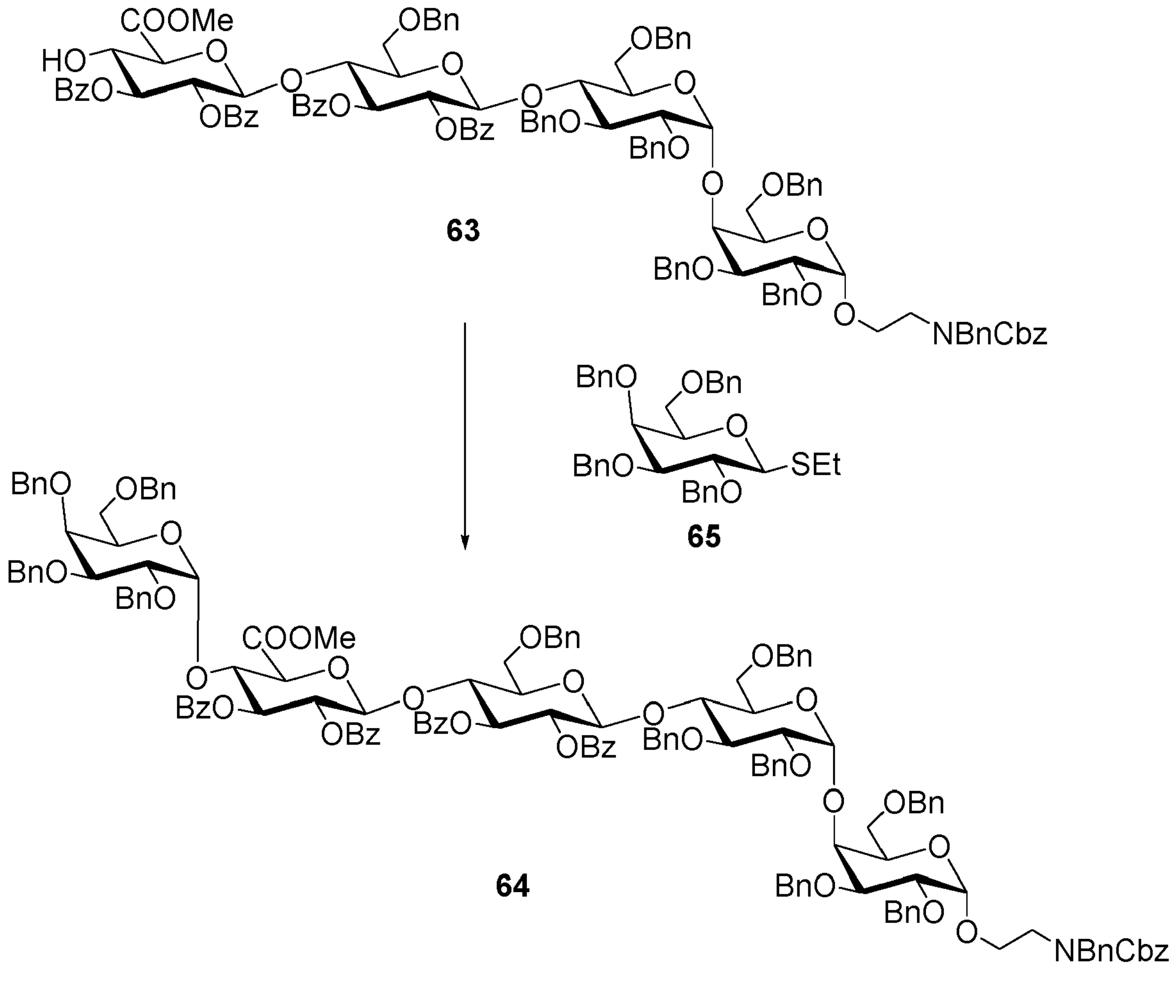

**63**

**65**

**64**

Alcohol **63** (14 mg, 7 μmol) and thioglycoside **65** (16 mg, 28 μmol) were co-evaporated with anhydrous toluene (3x10 mL) and kept under high vacuum for 30 min. The mixture was dissolved in $Et_2O$ (1.05 mL) and $CH_2Cl_2$ (0.35 mL) and stirred over activated molecular sieves (3 A-AW) for 30 min at room temperature. The solution was cooled to -20 °C and treated with NIS (6.3 mg, 28 μmol) and TMSOTf (1 μL, 5.5 μmol). The mixture was stirred for 1 h at that temperature and slowly warmed to 0 °C. The reaction was quenched with a 1:1 (v/v) mixture of sat. aq. $NaHCO_3$ (10 mL) and 10% (w/v) $Na_2SO_3$ (5 mL) and extracted with $CH_2Cl_2$ (4x10 mL). The combined organic fractions were dried over $Na_2SO_4$ and concentrated. The residue was purified by flash chromatography (EtOAc/hexanes 0:1 to 1:4 to 1:3) to give pentasaccharide **64** (12.5 mg, 4.9 μmol, 70%) along with the corresponding β-anomer (3.4 mg, 1.3 μmol, 19%). Analytical data for **64:** Clear oil. $R_f$(EtOAc/hexanes 2:3) = 0.63; $[\alpha]_D^{20}$ = +20.4° (c = 0.33, $CH_2Cl_2$); $^1H$ NMR (600 MHz, $CDCl_3$) δ 7.97 - 7.82 (m, 5H), 7.67 (d, *J* = 7.3 Hz, 2H), 7.57 (t, *J* = 7.4 Hz, 1 H), 7.48 - 6.98 (m, 77H), 5.52 (t, *J* = 9.6 Hz, 1H), 5.37 (dd, *J* = 9.9, 8.2 Hz, 1H), 5.27 (t, *J* = 9.1 Hz, 2H), 5.17 (t, *J* = 9.5 Hz, 1H), 5.11 (t, *J* = 9.9 Hz, 2H), 4.92 (s, 1

H), 4.82 (d, *J* = 11.3 Hz, 1H), 4.77 - 4.53 (m, 9H), 4.52 - 4.37 (m, 7H), 4.32 - 4.07 (m, 9H), 4.03 (d, *J* = 6.9 Hz, 1 H), 3.96 (d, *J* = 12.0 Hz, 2H), 3.91 - 3.78 (m, 6H), 3.76 - 3.56 (m, 7H), 3.54 - 3.39 (m, 7H), 3.38 - 3.21 (m, 4H), 3.07 (s, 3H), 3.05 - 2.91 (m, 2H); $^{13}$C NMR (150 MHz, $CDCl_3$) δ 166.8, 165.49, 165.46, 164.84, 164.79, 156.6, 156.3, 140.0, 139.1, 138.9, 138.8, 138.6, 138.5, 138.4, 138.3, 138.2, 138.1, 138.0, 137.5, 136.8, 133.5, 133.0, 132.5, 130.2, 129.9, 129.8, 129.7, 129.4, 129.2, 129.1, 129.03, 128.95, 128.8, 128.7, 128.6, 128.5, 128.43, 128.41, 128.35, 128.28, 128.26, 128.22, 128.15, 128.03, 127.98, 127.93, 127.91, 127.8, 127.6, 127.52, 127.49, 127.45, 127.2, 127.0, 100.5 (C-1c), 100.2 (C-1d), 99.8 (C-1 b), 99.47 (C-1 a), 98.46 (C-1 b), 80.7, 79.2, 78.4, 76.8, 76.61, 76.55, 75.34, 75.30, 75.26, 75.1, 75.0, 74.9, 74.8,74.5, 74.2, 73.7, 73.6, 73.5, 73.4, 73.3, 73.1, 72.6, 72.1, 71.6, 70.5, 70.0, 69.6, 67.9, 67.5, 67.3, 67.0, 66.9, 52.2, 51.4, 46.5, 45.5; IR (thin film) 2928, 1737, 1498, 1454, 1271, 1094, 1047, 1028, 738, 699 $cm^{-1}$; HRMS (MALDI) calcd. for $C_{153}H_{151}NO_{33}$ (M+Na)+ 2553.0066 found 2553.0066 m/z.

**Example C.2.3. 2-Amino-ethyl α-D-galactopyranosyl-(1→4)-β-D-glucopyranosyluronate-(1→4)-β-D-glucoyranosyl-α-D-glucopyranosyl-(1→4)-α-D-galactopyranoside (62)**

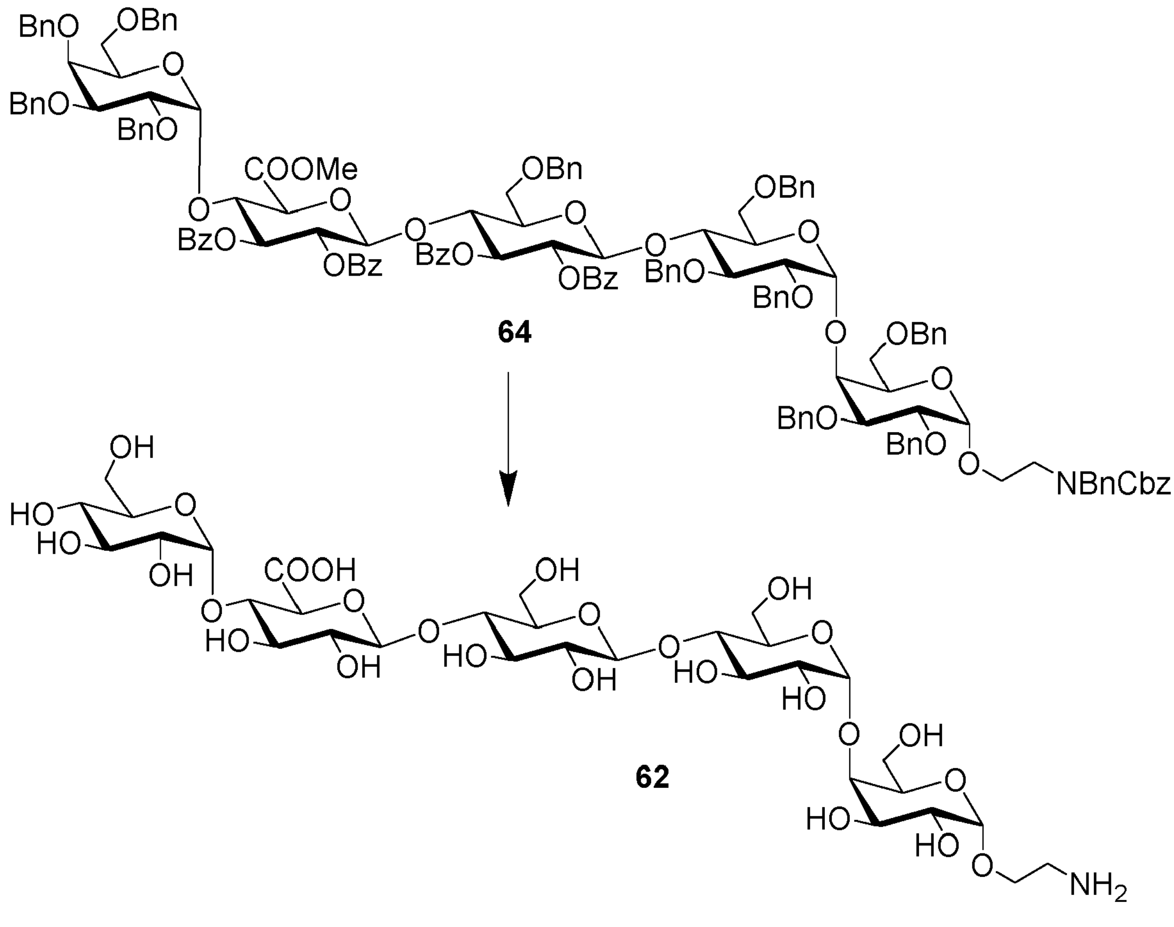

To a stirred solution of ester **64** (26 mg, 10.3 μmol) in THF (1 mL) and MeOH (1 mL) was added at 0 °C a 1:1 (v/v) mixture (450 μL) of hydrogen peroxide (6% (v/v) aq. solution, 397 μmol) and LiOH (0.5 M aq. solution, 113 μmol). The reaction was warmed to room temperature and stirred for 1 h at that temperature. The reaction was treated with NaOH (0.5 M aq. solution, 1 mL) and stirred for 16 h at room temperature. The solvents were evaporated under reduced pressure, the residue was co-evaporated with toluene (2x5 mL) and dissolved in MeOH (1 mL). The solution was treated at room temperature with NaOMe (0.5 M in MeOH, 1 mL) and stirred for 16 h at that temperature. The reaction was diluted with water (0.5 mL) and $CH_2Cl_2$ (0.5 mL), neutralized at 0 °C with Amberlite IR-120 ($H^+$ form), filtered and concentrated. The residue was purified by flash chromatography (EtOAc/hexanes 0:1 to 1:4 to 1:2 to 1:2 + 1 % (v/v) AcOH to 1:1 + 1 % (v/v) AcOH) to give the intermediate carboxylic acid as a clear oil.
The intermediate carboxylic acid in $CH_2Cl_2$/*t*BuOH/water (1:16:8, 1 mL) was purged with argon and treated at 0 °C with a suspension of $Pd(OH)_2$ on carbon (20% (w/w) loading, 20 mg) in the same solvent mixture (0.5 mL). The suspension was purged with hydrogen, stirred under a hydrogen atmosphere for 16 h, filtered and concentrated. Since the reaction

had not proceeded to completion, the residue was subjected to the same conditions again and stirred for 24 h at room temperature. The mixture was filtered and concentrated, the residue was purified by solid phase extraction (Chromabond C18, Macherey-Nagel) and lyophilized to give pentasaccharide **62** (8.1 mg, 9.1 μmol, 88% over three steps) as a white solid. $^1$H NMR (600 MHz, $D_2O$) δ 5.52 (d, *J* = 3.5 Hz, 1H, H-1 b), 5.07 (d, *J* = 3.7 Hz, 1 H, H-1 b), 4.93 (d, *J* = 3.8 Hz, 1H, H-1 a), 4.56 (2xd, *J* = 8.4 and 8.4 Hz, 2H, H-1 c and H-1 d), 4.24 (d, *J* = 10.0 Hz, 1 H), 4.09 (d, *J* = 2.6 Hz, 1 H), 4.07 - 3.78 (m, 19H), 3.77 - 3.58 (m, 8H), 3.39 (dt, *J* = 24.4, 8.5 Hz, 2H), 3.34 - 3.26 (m, 2H); 13C NMR (150 MHz, $D_2O$) δ 104.86 (C-1c and C-1d), 104.85 (C-1a), 102.5 (C-1b), 101.2 (C-1 b), 101.0, 81.2, 81.0, 80.9, 78.7, 78.6, 77.4, 76.6, 75.7, 75.5, 74.2, 74.1, 73.8, 73.31, 73.29, 71.8, 71.6, 71.5, 71.0, 70.9, 66.5, 63.3, 63.1, 62.5, 62.1, 41.9; HRMS (MALDI) calcd. for $C_{32}H_{55}NO_{27}$ (M+Na)+ 884.2883 found 884.2942 *m/z.*

**Example C.3. Synthesis of S. *pneumoniae* serotype 8 hexasaccharide 66**

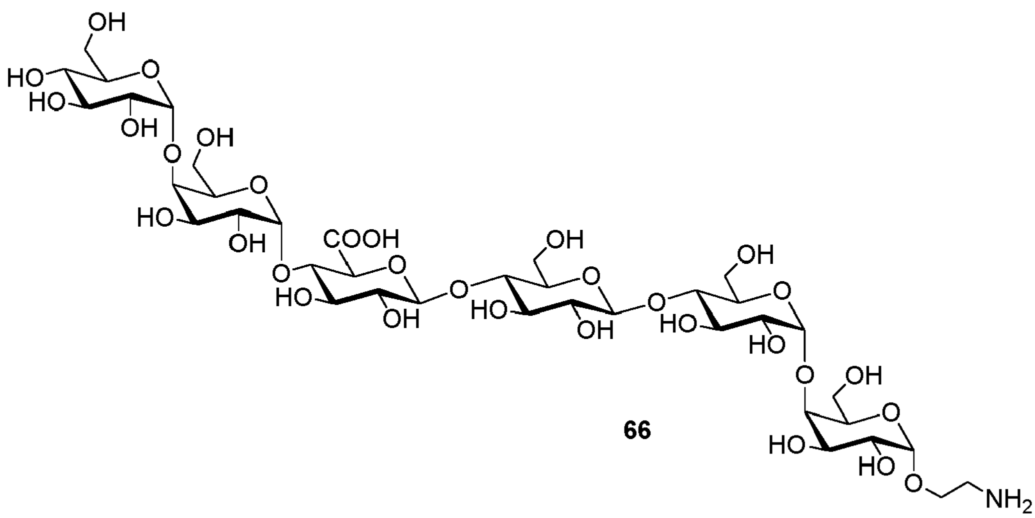

**66**

**Example C.3.1. *N*-Benzyl-*N*-benzyloxycarbonyl-2-amino-ethyl 4-*O*-benzoyl-2,3,6-tri-*O*-benzyl-α-D-glucopyranosyl-(1→4)-2,3,6-tri-*O*-benzyl-α-D-galactopyranosyl-(1→4)-methyl[2,3-di-*O*-benzoyl-β-D-glucopyranosyl]uronate-(1→4)-2,3-di-*O*-benzoyl-6-*O*-benzyl-β-D-glucopyranosyl-(1→4)-2,3,6-tri-*O*-benzyl-α-D-glucopyranosyl-(1→4 )-2,3,6-tri-*O*-benzyl-α-D-galactopyranoside** (67)

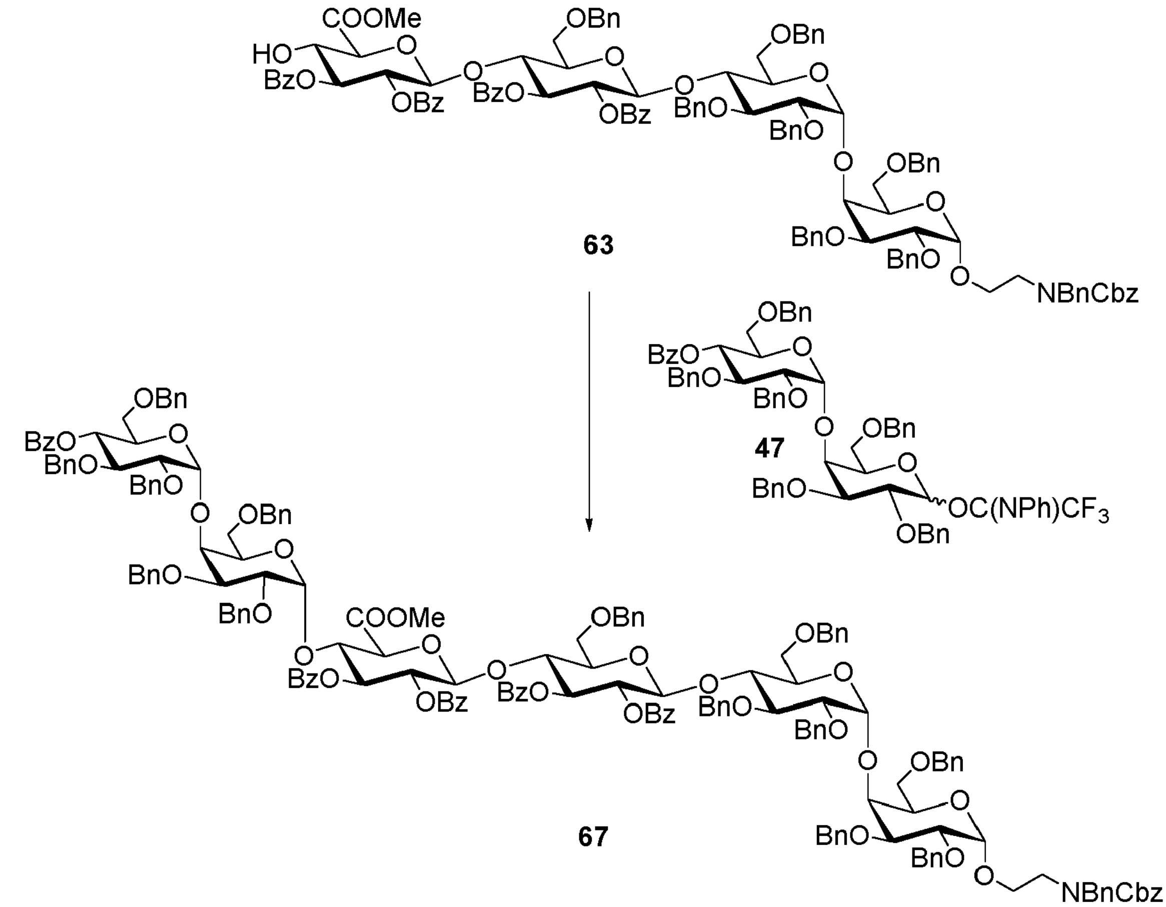
63

47

67

Alcohol **63** (50 mg, 25 μmol) and imidate **47** (72.1 mg, 62 μmol) were co-evaporated with anhydrous toluene (3x10 mL) and kept under high vacuum for 30 min. The mixture was dissolved in $Et_2O$ (2 mL) and $CH_2Cl_2$ (0.67 mL) and stirred over activated molecular sieves (3 A-AW) for 30 min at room temperature. The solution was cooled to -20 °C and treated with TMSOTf (2 μL, 11 μmol). The mixture was stirred for 1 h at that temperature and slowly warmed to 0 °C. The reaction was quenched with sat. aq. $NaHCO_3$ (10 mL) and extracted with $CH_2Cl_2$ (4x10 mL). The combined organic fractions were dried over $Na_2SO_4$ and concentrated. The residue was purified by flash chromatography (EtOAc/hexanes 0:1 to 1:3 to 3:7 to 1:2) to give hexasaccharide **67** (51 mg, 17 μmol, 68%) as a clear oil. $R_f$ (EtOAc/hexanes 2:3) = 0.63; $[\alpha]_D^{20}$ = +36.6° (c = 0.21, $CH_2Cl_2$); $^1H$ NMR (600 MHz, $CDCl_3$) δ 7.95 (d, *J* = 7.3 Hz, 2H), 7.89 (d, *J* = 7.7 Hz, 4H), 7.84 (d, *J* = 7.3 Hz, 2H), 7.67 (d, *J* = 7.4 Hz, 2H), 7.55 (dt, *J* = 26.6, 7.4 Hz, 2H), 7.48 - 6.98 (m, 88H), 5.49 (dt, *J* = 19.5, 9.8 Hz, 2H), 5.39 (dd, *J* = 9.9, 8.2 Hz, 1 H), 5.28 (m, 2H), 5.16 (t, *J* = 9.5 Hz, 1 H), 5.09 (m, 3H), 4.92 (s, 1 H), 4.74 (dd, *J* = 11.7, 9.1 Hz, 2H), 4.71 - 4.66 (m, 3H), 4.60 (m, 7H), 4.53 - 4.41 (m, 6H), 4.35 - 4.28 (m, 3H), 4.28 - 4.20 (m, 5H), 4.18 - 4.01 (m, 8H), 4.00 - 3.78 (m, 9H), 3.77 - 3.59 (m, 8H), 3.55 (d, *J* = 9.6 Hz, 1 H), 3.53 - 3.20 (m, 8H), 3.17 (dd, *J* = 8.8, 4.9 Hz, 1 H), 3.06 (s, 3H), 2.97 (m, 3H); $^{13}C$ NMR (150 MHz, $CDCl_3$) δ 167.0, 165.54, 165.50, 165.2, 164.8, 156.6, 156.3, 140.1, 138.9, 138.79, 138.76, 138.6, 138.41, 138.36, 138.3, 138.2, 138.1, 138.0, 137.6, 136.84, 136.77, 133.5, 133.1, 133.0, 132.5, 130.4, 130.2, 130.1, 129.9, 129.8, 129.7, 129.3, 129.2, 129.1, 129.04, 128.95, 128.8, 128.7, 128.6, 128.44, 128.41, 128.37, 128.35, 128.33, 128.30, 128.25, 128.18, 128.15, 128.1, 128.03, 127.99, 127.96, 127.94, 127.85, 127.8, 127.7, 127.64, 127.61, 127.55, 127.4, 127.3, 127.2, 127.0, 100.4 ($J_{1,2}$ = 165.6 Hz, C-1 c), 100.2 ($J_{1,2}$ = 165.6 Hz, C-1d), 100.0 ($J_{1,2}$ = 171.3 Hz, C-1a), 99.8 ($J_{1,2}$ = 171.0 Hz, C-1 b), 99.3 ($J_{1,2}$ = 172.8 Hz, C-1 a), 98.5 ($J_{1,2}$ = 174.0 Hz, C-1 b), 80.7, 80.1, 79.7, 79.2, 77.6, 76.64, 76.56, 75.9, 75.3, 75.2, 74.9, 74.43, 74.41, 74.2, 73.9, 73.62, 73.57, 73.4, 73.3, 73.2, 73.1, 73.0, 72.6, 72.4, 72.1, 71.6, 71.1, 70.5, 70.1, 69.6, 69.5, 69.2, 67.9, 67.8, 67.6, 67.3, 67.0, 66.9, 66.8, 66.3, 52.2, 51.40, 51.37, 46.5, 45.5; IR (thin film) 2926, 1736, 1454, 1271, 1095, 1045, 737, 699 cm-1; HRMS (MALDI) calcd. for $C_{180}H_{177}NO_{39}$ $(M+2Na)^{2+}$ 1511.0847 found 1511.0576 *m/z*.

**Example C.3.2. 2-Amino-ethyl α-D-glucopyranosyl-(1→4)-α-D-galactopyranosyl-(1→4)-β-D-glucopyranosyluronate-(1→4)-p-D-glucopyranosyl-(1→4)-α-D-glucopyranosyl-(1→4)-α-D-galactopyranoside (66)**

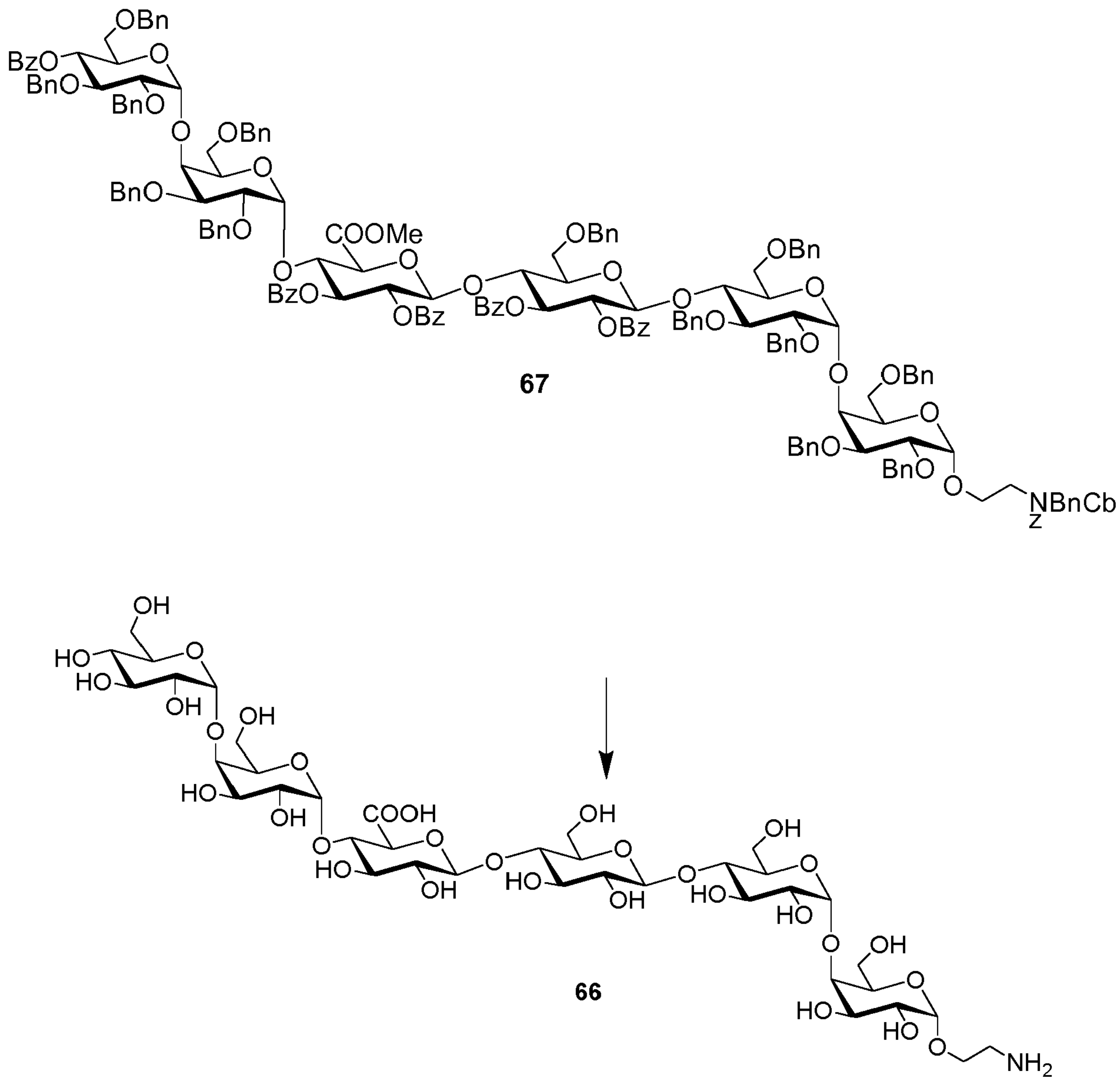

67

66

To a stirred solution of ester **67** (22 mg, 7.4 μmol) in THF (1 mL) and MeOH (1 mL) was added at 0 °C a 1:1 (v/v) mixture (296 μL) of hydrogen peroxide (6% (v/v) aq. solution, 295 μmol) and LiOH (0.5 M aq. solution, 74 μmol). The reaction was warmed to room temperature and treated after 2 h and 4 h with another 294 μL of the same lithium peroxide solution, respectively. The mixture was stirred for 16 h at room temperature and treated with NaOH (1 M aq. solution, 0.5 mL) and MeOH (0.5 mL). The reaction was stirred for 20 h at that temperature, quenched with 10% aq. $Na_2SO_3$ (0.8 mL) and concentrated under reduced pressure. The residue was dissolved in water (4 mL), acidified at 0 °C with 0.5 M aq. $NaHSO_4$ to approx. pH 4 and extracted with EtOAc (4x10 mL). The combined organic fractions were dried over $Na_2SO_4$ and concentrated. The residue was treated with NaOMe (0.5 M solution in MeOH, 1 mL), warmed to 40 °C and stirred for 5 h at that temperature. The reaction was cooled to room temperature, stirred for another 16 h at that temperature and treated with water (0.5 mL). The mixture was neutralized with Amberlite IR-120 (H+ form), filtered and concentrated. The residue was purified by flash chromatography (EtOAc/hexanes 0:1:0 to 1:4 + 2% (v/v) AcOH to 1:1 + 2% (v/v) AcOH) to give the intermediate carboxylic acid as a clear oil. The intermediate carboxylic acid in $CH_2Cl_2$/*t*BuOH/water (1.5:16:8, 3 mL) was purged with argon and treated at 0 °C with a suspension of $Pd(OH)_2$ on carbon (20% (w/w) loading, 30 mg) in the same solvent mixture (1 mL). The suspension was purged with hydrogen, warmed to room temperature, stirred under a hydrogen atmosphere for 18 h, filtered and concentrated. The residue was purified by solid phase extraction (Chromabond C18, Macherey-Nagel) and lyophilized to give hexasaccharide **66** (7 mg, 6.7 μmol, 86% over three steps) as a white solid. $^1H$ NMR (600 MHz, $D_2O$) δ 5.57 (d, *J* = 3.2 Hz, 1 H, H-1 b), 5.07 (d, *J* = 2.9 Hz, 1 H, H-1 b), 4.97 (d, *J* = 3.0 Hz, 1 H, H-1 a), 4.94 (d, *J* = 3.0 Hz, 1 H, H-1 a), 4.56 (2xd, *J* = 8.0 and 7.9 Hz, 2H, H-1 c and H-1 d), 4.24 (d, *J* = 9.8 Hz, 1 H), 4.15 - 4.07 (m, 3H), 4.01 (m, 4H), 3.89 (m, 16H), 3.77 - 3.60 (m,

8H), 3.56 (dd, $J$ = 9.9, 3.1 Hz, 1 H), 3.47 (t, $J$ = 9.6 Hz, 1 H), 3.43 - 3.28 (m, 4H); $^{13}C$ NMR (150 MHz, $D_2O$) δ 104.9 (C-1c or C-1d), 104.82 (C-1c or C-1d), 102.80 (C-1a), 102.5 (C-1a), 101.2 (C-1b), 101.0 (C-1 b), 81.2, 81.01, 80.98, 80.9, 78.68, 78.67, 77.4, 76.6, 75.7, 75.5, 75.4, 74.5, 74.4, 74.2, 74.1, 73.8, 73.6, 73.3, 71.9, 71.5, 71.3, 71.1, 70.9, 66.5, 63.1, 62.7, 62.5, 62.4, 62.1, 41.9; HRMS (ESI) calcd. for $C_{38}H_{65}NO_{32}$ $(M+Na)^+$ 1070.3387 found 1070.3391 *m/z.*

**Example C.4. Conjugation of *S. pneumoniae* serotype 8 saccharides 44, 45, 46 and 66 to $CRM_{197}$**

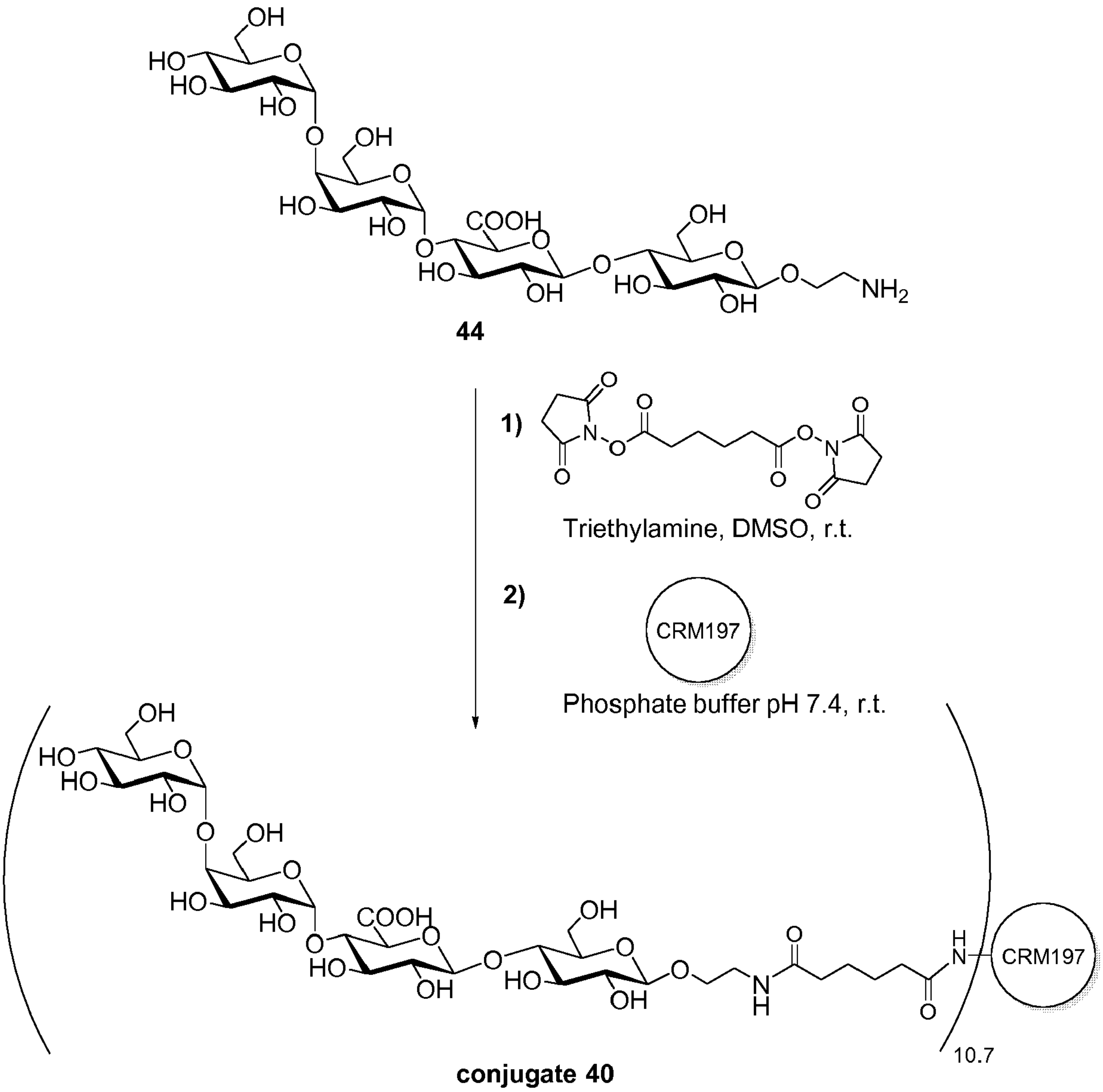


**conjugate 40**

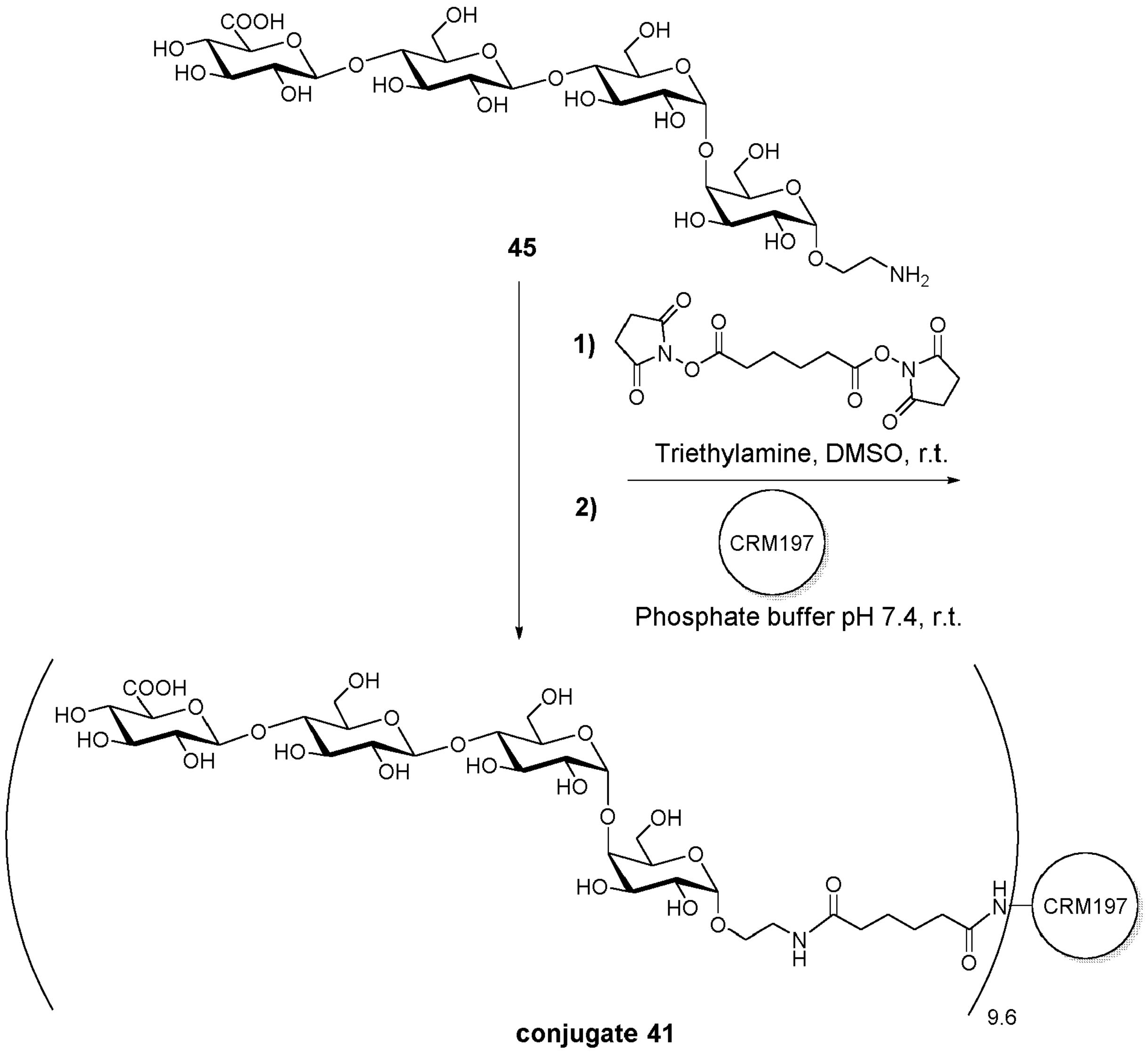


45

**conjugate 41**

Method A:

To a stirred solution of disuccinimidyl adipate (10 mg, 29 μmol) and triethylamine (10 μL, 72 μmol) in anhydrous DMSO (150 μL) was added at room temperature dropwise a suspension of tetrasaccharide **44** or **45** (approx. 2 mg, 2.8 μmol) in anhydrous DMSO (150 μL). The reaction was stirred for 2 h at that temperature under an Argon atmosphere and treated with a 100 mM sodium phosphate buffer pH 7.4 (NaPi, 200 μL). The mixture was extracted with chloroform (10 mL) and the phases separated by centrifugation (2 min, 1800 g, room temperature). The organic phase was discarded and the extraction step was repeated two times. The aqueous layer was clarified by centrifugation in a 1.5 mL reaction tube (1 min, 14500 g, room temperature) and added to a stirring solution of $CRM_{197}$ (1 mg, 17.3 nmol) in NaPi (1 mL). The mixture was stirred for 16 h at room temperature and dialyzed using a centrifugal filter (10 kDa MWCO, Millipore, Darmstadt, Germany). The conjugate was characterized by MALDI-MS:

Conjugate **40 ($CRM_{197}$-44):** ca. 67000 m/z (incorporation of 10.7 tetrasaccharide molecules on average)
Conjugate **41 ($CRM_{197}$-45):** ca. 66000 m/z (incorporation of 9.6 tetrasaccharide molecules on average)

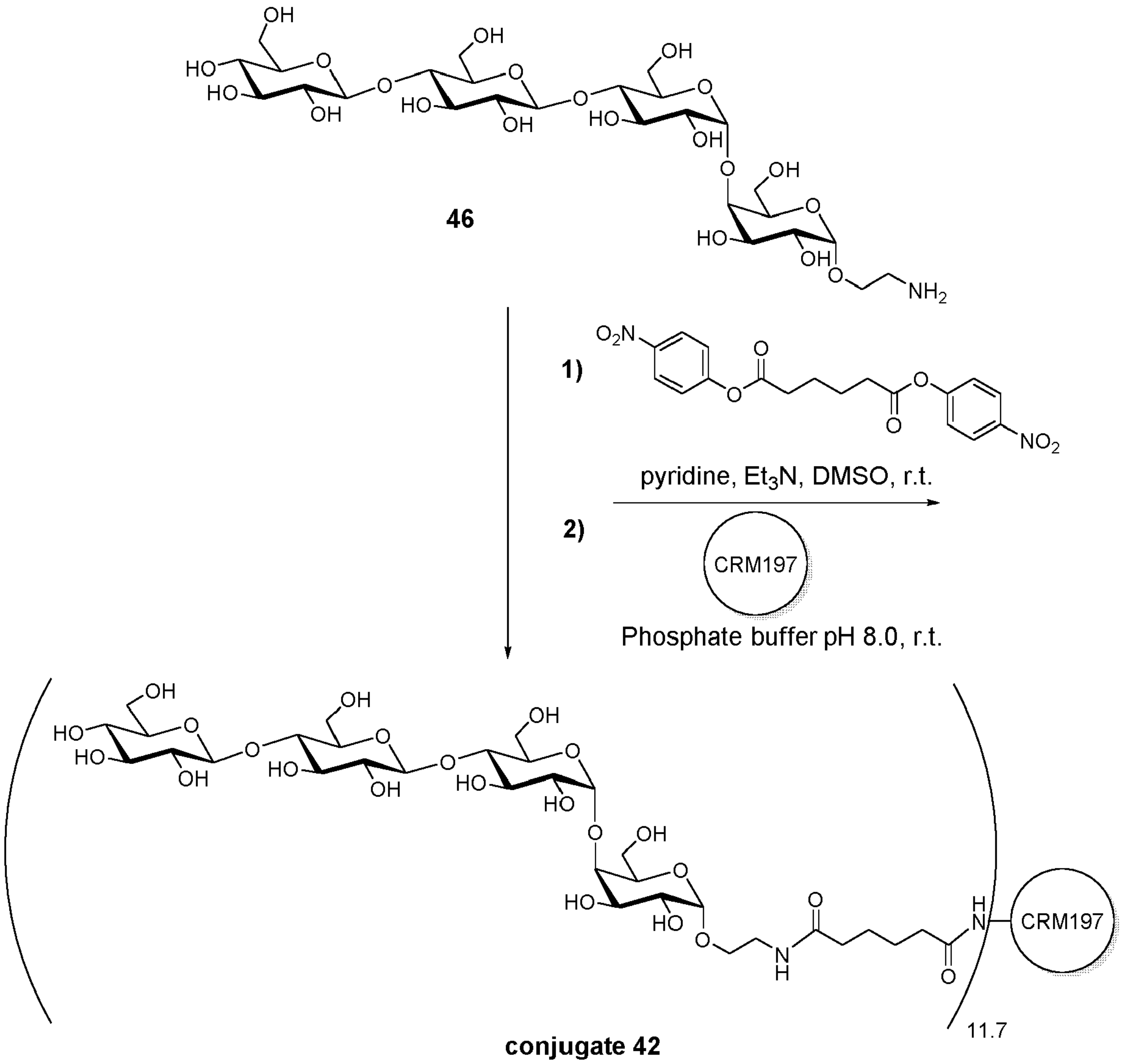


46

conjugate 42

66

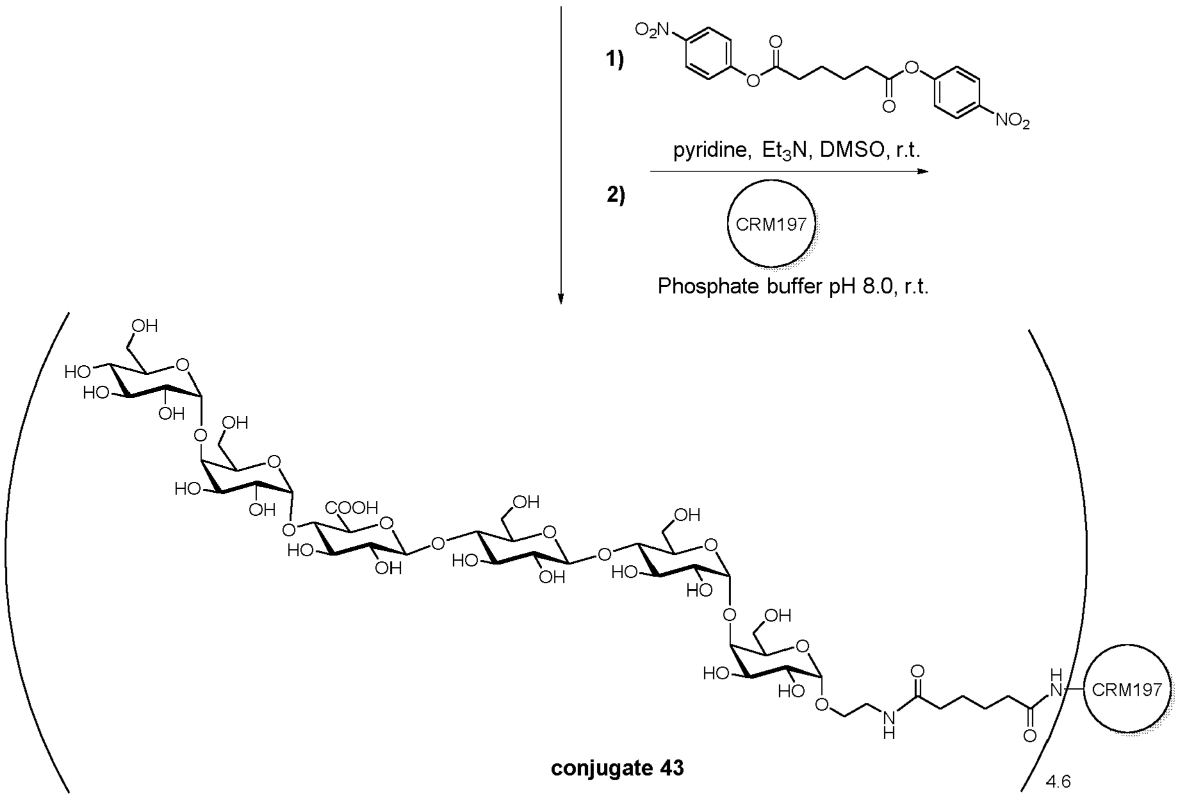


conjugate 43

Method B:

To a stirred solution of the oligosaccharide **46** or **66** (2 μmol) in a 1:3 (v/v) mixture of anhydrous DMSO and anhydrous pyridine (100-200 μL) were added bis(4-nitrophenyl)adipate (DNAP, 9.3 mg, 24 μmol) and triethylamine (10 μL). The reaction was stirred for 2 h at room temperature under argon atmosphere. The mixture was shock-frozen and lyophilized. The residue was triturated with chloroform (4x0.5 mL) and dichloromethane (4x0.5 mL), transferred to a new reaction tube using DMSO as a solvent and lyophilized again. $CRM_{197}$ (3 mg, 52 nmol) was dialyzed twice against 0.1 M sodium phosphate buffer pH 8.0 using a centrifugal filter (10 kDa MWCO), concentrated to approx. 300 μL and added to the activated oligosaccharide. The mixture was stirred at room temperature for 16 h and dialyzed against water four times. An aliquot was taken for characterization and the mixture was dialyzed three times against phosphate-buffered saline. The glycoconjugates were characterized by MALDI-TOF MS, SDS-PAGE and Immunoblot. Conjugates were stable for at least two months at 4 °C (if handled under sterile conditions) or -20 °C.

Conjugate **42 ($CRM_{197}$-46):** ca. 68281 m/z (incorporation of 12.9 tetrasaccharide molecules on average).
Conjugate **43 ($CRM_{197}$-66):** ca. 63535 *m/z* (incorporation of 4.6 hexasaccharide molecules on average)

**Example C.5: Synthesis of building blocks BB2, BB4 and BB5**

**Synthesis of building block BB2**

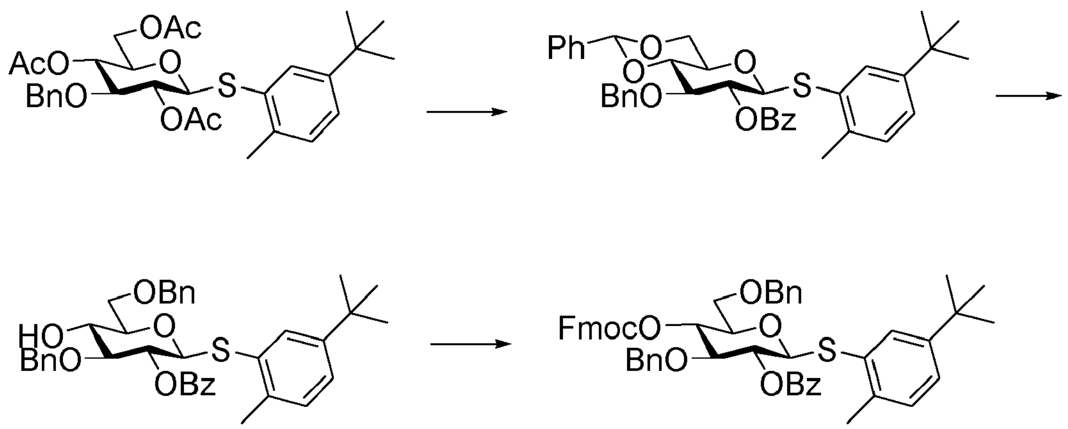

**BB2**

**Step 1: Synthesis of (2-methyl-5-tert-butylphenyl) 2-*O*-benzoyl-3-*O*-benzyl-4,6-*O*-benzylidene-1-thio-β-D-glucopyranose**

(2-Methyl-5-tert-butylphenyl) 2,4,6-tri-O-acetyl-3-O-benzyl-1-thio-β- D glucopyranose (Chem. Eur. J. 2013, 19, 12497-12503) was dissolved in MeOH, NaOMe (1.0 eq) was added and the reaction mixture was stirred overnight. The mixture was neutralized with IR-120-$H^+$ amberlite resin, filtered off, concentrated and co-evaporated with toluene. The crude triol was dissolved in DMF. Benzaldehyde dimethyl acetal (2.0 eq) and a catalytic amount of para-toluene sulfonic acid were added and the mixture was stirred at 80 °C for 2 h. After the mixture was cooled to room temperature, saturated aqueous $NaHCO_3$ was added. After phase separation, the organic phase was extracted three times with ethyl acetate. The combined organic layers were washed with brine, dried over $MgSO_4$, filtered and concentrated. The crude was dissolved in DCM, cooled to 0 °C and $Bz_2O$ (2.0 eq), DMAP (0.5 eq) and triethylamine (4.0 eq) were added. After complete conversion, the reaction was quenched with saturated aqueous $NaHCO_3$ and the combined organic layers were washed with brine, dried over $MgSO_4$, filtered and concentrated. The residue was purified by flash column chromatography on silica gel to provide compound (88% over three steps). Rf: 0.25 (Hexane:EtOAc = 9:1). [α]D = 68.96 (c = 3.18, $CHCl_3$). IR (thin film, chloroform): u = 2962, 1729, 1265, 1093 cm-1; 1H-NMR (400 MHz, $CDCl_3$) δ = 8.01 (ddd, J = 5.8, 5.3, 0.8 Hz, 2H, H Ar), 7.60 (ddd, J = 7.1, 2.6, 1.3 Hz, 1 H, H Ar), 7.51 (ddd, J = 6.0, 5.6, 2.3 Hz, 3H, H Ar), 7.49 - 7.43 (m, 2H, H Ar), 7.43 - 7.35 (m, 3H, H Ar), 7.19 (dt, J = 6.3, 3.1 Hz, 1 H, H Ar), 7.13 (dd, J = 6.7, 4.0 Hz, 3H, H Ar), 7.10 - 7.04 (m, 3H, H Ar), 5.63 (s, 1 H CHO2Ph), 5.42 - 5.32 (m, 1 H, H-2), 4.82 (d, J = 11.9 Hz, 1H, CHHPh), 4.81 (d, J = 10.2 Hz, 1H, H-1), 4.69 (d, J = 12.0 Hz, 1 H, CHHPh), 4.39 (dd, J = 10.5, 5.0 Hz, 1 H, H-6'), 3.94 - 3.86 (m, 3H, H-3, H-4, H-6), 3.56 (dt, J = 14.6, 4.9 Hz, 1 H, H-5), 2.19 (s, 3H), 1.27 (s, 9H). 13C-NMR (100 MHz, $CDCl_3$) δ 165.23 (C=O), 149.66, 137.84, 137.28, 137.03, 133.34, 132.37, 130.07, 130.04, 129.93, 129.90, 129.19, 128.51, 128.43, 128.29, 128.22, 127.71, 126.13, 125.38 (Ar), 101.44 ($CHO_2Ph$), 88.03 (C-1), 81.64 (C-3), 79.43 (C-4), 74.36 (CH2Ph), 72.25 (C-2), 70.66 (C-5), 68.81 (C-6), 34.56 (Cq tBu thio), 31.36 (tBu), 20.36 (CH3 thio); MS ESI+-HRMS m/z [M+Na]+ calcd for $C_{38}H_{40}O_6SNa$ 647.2462, found 647.

**Step 2: Synthesis of (2-methyl-5-tert-butylphenyl) 2-*O*-benzoyl-3,6-di-*O*-benzyl-1-thio-β-D-glucopyranose**

A solution of the compound of step 1 was co-evaporated with toluene, and dissolved in DCM (6.5 mL) under an argon atmosphere. Triethylsilane (0.62 mL, 3.88 mmol) and trifluoroacetic anhydride (0.27 mL, 1.94 mmol) were added and the solution was cooled to 0 °C. Trifluoroacetic acid (0.30 mL, 3.88 mmol) were added dropwise, and the reaction was stirred and allowed to warm to room temperature. After complete conversion of the starting material, the solution was diluted with DCM, and quenched with saturated aqueous $NaHCO_3$. The combined organic layer was dried over $MgSO_4$ and the solvent was removed in vacuo. The residue was purified by column chromatography on silica gel (hexane/ethyl acetate, 9:1 to 7:3) to give a white foam (92%). MS ESI+-HRMS m/z [M+Na]+ calcd for $C_{38}H_{42}O_6SNa$ 649.2600 found 649.2585.

**Step 3: Synthesis of (2-methyl-5-tert-butylphenyl) 2-*O*-benzoyl-3,6-di-*O*-benzyl-4-O-fluorenylmethoxycarbonyl-1-thio-β-D-glucopyranoside**

The compound obtained at step 2 was dissolved in DCM (6.5 mL) under an argon atmosphere. 9-fluorenylmethyl chloroformate (8.3 g, 31.9 mmol) and pyridine (3.44 mL, 42.5 mmol) were added into the solution at 0 °C. After complete conversion of the starting material, the solution was diluted with DCM, extracted with 1 M aqueous HCl and saturated

aqueous $NaHCO_3$. The combined organic phase was dried over $MgSO_4$ and the solvent was removed in vacuo. The crude product was purified by silica gel flash column chromatography affording the title compound. MS ESI+-HRMS m/z [M+Na]+ calcd for $C_{53}H_{52}O_8SNa$ 871.3281 found 871.3311.

**Synthesis of building block BB5**

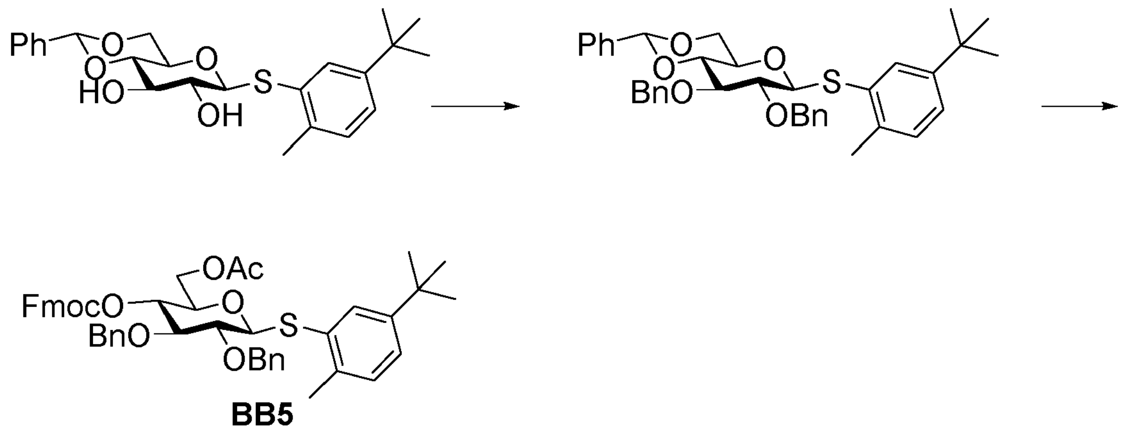

**BB5**

**Step 1: Synthesis of (2-methyl-5-*tert*-butylphenyl) 2,3-di-*O*-benzyl-4,6-*O*-benzylidene-1-thio-β-D-glucopyranoside**

(2-Methyl-5-*tert*-butylphenyl) 4,6-*O*-benzylidene-1-thio-β-D-glucopyranoside (Chem. Eur. J. 2013, 19, 12497-12503) was dissolved in DCM (6.5 mL) under an argon atmosphere. Benzyl bromide, and NaH were added into the solution at 0 °C. After complete conversion of the starting material, the reaction mixture was quenched with methanol, and diluted with ether, extracted with saturated $NH_4Cl$. The combined organic phase was dried over $MgSO_4$ and the solvent was removed *in vacuo.* The crude product was purified by silica gel flash column chromatography affording the title compound.
MS ESI+-HRMS m/z [M+Na]+ calcd for $C_{38}H_{42}O_5SNa$ 633.2651 found 633.2644.

**Step 2: Synthesis of (2-Methyl-5-*tert*-butylphenyl) 6-*O*-acetyl-2,3-di-*O*-benzyl-4-*O*-fluorenylmethoxycarbonyl-1-thio-β-D-galactopyranoside**

To the solution of (2-methyl-5-*tert*-butylphenyl) 2,3-di-*O*-benzyl-4,6-*O*-benzylidene-1-thio-β-D-glucopyranoside in DCM (6.5 mL) were added TFA and water. After completion, the crude was decanted with hexane to remove byproduct. The crude was used to the next reaction. To the solution of the crude was added acetic acid, 2-chloro-1-methylpyridium iodide, and DABCO at -15 °C. After complete conversion of the starting material, the solution was diluted with DCM, extracted with 1 M aqueous HCl and saturated aqueous $NaHCO_3$. The combined organic phase was dried over $MgSO_4$ and the solvent was removed in vacuo. The crude product was purified by silica gel flash column chromatography affording the title compound. The crude was dissolved in DCM (6.5 mL) under an argon atmosphere. 9-fluorenylmethyl chloroformate (8.3 g, 31.9 mmol) and pyridine (3.44 mL, 42.5 mmol) were added into the solution at 0 °C. After complete conversion of the starting material, the solution was diluted with DCM, extracted with 1 M aqueous HCl and saturated aqueous $NaHCO_3$. The combined organic phase was dried over $MgSO_4$ and the solvent was removed *in vacuo.* The crude product was purified by silica gel flash column chromatography affording the title compound. MS ESI+-HRMS m/z [M+Na]+ calcd for $C_{48}H_{50}O_8SNa$ 809.3124 found 809.3137.

**Synthesis of building block BB4**

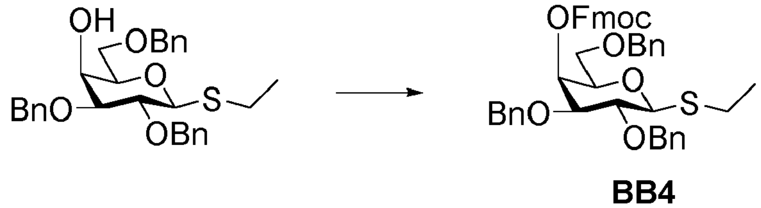

**BB4**

To a solution of 2,3,6-tri-*O*-benzyl-1-thio-β-D-galactopyranoside (Chem. Commun., 2011, 47, 10260-10262) were added 9-fluorenylmethyl chloroformate (8.3 g, 31.9 mmol) and pyridine (3.44 mL, 42.5 mmol) at 0 °C. After complete conversion of the starting material, the solution was diluted with DCM, extracted with 1 M aqueous HCl and saturated aqueous $NaHCO_3$. The combined organic phase was dried over $MgSO_4$ and the solvent was removed in vacuo. The crude product was purified by silica gel flash column chromatography affording the title compound ethyl 2,3,6-tri-*O*-benzyl-4-*O*-fluorenylmethoxycarbonyl-1-thio-β-D-galactopyranoside.
MS ESI+-HRMS m/z [M+Na]+ calcd for $C_{44}H_{44}O_7SNa$ 739.2705 found 739.2673.

**Example D. Coformulation of Prevnar13® and conjugate 41 or 42**

**Example D.1. Coformulation**

Conjugate **41** or conjugate **42** (2.2 μg glycan per dose) in 20 μL PBS was admixed to a full dose Prevnar13® for each immunization dose and incubated overnight at 4 °C.

**Example D.2. Characterization of conjugate adsorption to Prevnar13®**

A flow cytometry-based assay was used to assess adsorption of conjugates **41** and **42** to Prevnar13®. Aliquots of 10% (50 μL) of a dose of Prevnar13® were mixed with different amounts of conjugate **41** or **42,** equivalent to 25%, 100% or 400% of the normal glycan content (2.2 μg glycan/dose) of CPSs of other serotypes in the vaccine. Suspensions were incubated overnight at 4 °C, particles were harvested (3000 g, 5 min, room temperature) and blocked with 10% (w/v) BSA in PBS (50 μL) for 30 min at room temperature. Particles were harvested and incubated with primary antibody samples (1:100 dilutions of ST1 typing serum and 10 μg/mL mAb 1H8 in 1% (w/v) BSA in PBS) for 30 min at room temperature.

Particles were harvested, washed once with PBS (100 μL) and incubated with fluorescently labeled secondary antibodies (1:100 dilutions in 1% (w/v) BSA in PBS, 100 μL) for 20 min at room temperature. Particles were washed, suspended in PBS and analyzed by flow cytometry. Control samples included non-treated or $CRM_{197}$-treated (9.2 μg $CRM_{197}$/aliquot, corresponding to the $CRM_{197}$ dose of the highest conjugate concentration) Prevnar13® particles (see **Figure 5**). Co-adsorbed conjugates **41** and **42** were stable for at least one week after adsorption.

**Example E. Immunogenicity studies**

***Antisera*, *Polysaccharides and Carrier Protein***

Human pooled pneumococcal antiserum (WHO 1 st International Standard for Human Anti-pneumococcal capsule Reference Serum, prod. no. 007sp) was obtained from NIBSC (South Mimms, UK). Rabbit ST8 typing serum (Type 8 Neufeld antiserum), pneumococcal capsular polysaccharides and pneumococcal cell wall polysaccharide (CWPS) were purchased from SSI Diagnostica (Hillerød, Denmark).

***Antibodies***

Fluorescent antibodies were used from commercial sources: Goat anti-Rabbit IgG H+L FITC conjugate (abcam, Cambridge, UK), Goat anti-Human IgG H+L Alexa Fluor® 647 conjugate (life Technologies), Goat anti-Mouse IgG H+L FITC conjugate (Sigma-Aldrich), Goat anti-Mouse IgG H+L Alexa Fluor® 635 conjugate (life Technologies), Goat anti-Mouse IgM H chain Alexa Fluor® 680 conjugate (life Technologies), Donkey anti-Mouse IgM H chain Alexa Fluor® 594 conjugate (dianova, Hamburg, Germany).

Secondary antibodies used for ELISA and immunoblot were horseradish peroxidase (HRP)-labeled: goat anti-mouse IgG HRP conjugate (cat. no. 115-035-062, dianova, Hamburg, Germany), goat anti-mouse IgM H chain HRP conjugate (cat. no. 62-6820, Life Technologies) or goat anti-rabbit IgG (whole molecule)-peroxidase conjugate (cat. no. A6154, Sigma-Aldrich) and used according to the manufacturers' specifications.

**Example E.1 Immunization in Rabbits**

Rabbit immunization experiments were performed by Biogenes GmbH. Rabbits were housed and handled according to international animal regulations (EU Directive 2010/63/EU) and sanctioned by governmental authorities (Landesamt für Landwirtschaft, Lebensmittelsicherheit und Fischerei Mecklenburg-Vorpommern). Rabbits (female Zika rabbits, 10-12 weeks, 2.5-3 kg, *n* = 3 per group) were immunized subcutaneously at four different sites. The rabbits (*n* = 3 per group) were s.c. immunized three times with Prevnar13®, with Prevnar13® coformulated with conjugate **41** or with Prevnar13® coformulated with conjugate **42.** Booster doses were given at days 14 and 28 using the same strategy. Sera were collected at days 0, 14, 21 and 35. Prevnar13® without any additive was used for control immunizations. Sera of Prevnar13®-immunized rabbits were pre-adsorbed to 5 μg pneumococcal CWPS and 5 μg/mL S. *pneumoniae* 22F CPS prior to evaluation by glycan microarray or ELISA. Serum was collected at day 35 and analyzed by ELISA and OPKA.

**Example E.2 Enzyme-linked Immunosorbent Assay (ELISA)**

ELISA was performed using Costar™ high-binding polystyrene 96-well plates (cat. no. 3361, Corning, Corning, US). Plates were coated using native pneumococcal polysaccharides (SSI Diagnostica, Copenhagen) at a concentration of 10 μg/mL in PBS for 20 h at 4 °C. Plates were blocked with 10% (v/v) fetal calf serum in PBS for 2 h at 37 °C and washed once with PBS-T. After applying cell culture supernatants or mAb dilutions (30-50 μL), plates were incubated for 1 h at 37 °C, washed with PBS-T three times and treated with a horseradish peroxidase (HRP)-labeled secondary antibody. Plates were washed with PBS-T three times and HRP activity was measured with TMB substrate (BD Biosciences, San Jose, US) according to the manufacturer's instructions. Endpoint titers were determined as the reciprocal of the highest dilution resulting in an absorbance above a value of 0.1. The anti-polysaccharide IgG endpoint titers of rabbits immunized with Prevnar13® alone or coformulated with conjugate **41** or **42,** as determined by polysaccharide ELISA are shown in the table below.

Table 1: Anti-polysaccharide IgG endpoint titers of rabbits immunized with Prevnar13® alone or coformulated with conjugate **41** or **42,** as determined by polysaccharide ELISA.

| | **Prevnar13®** | | | **Prevnar13® + conjugate 41** | | | **Prevnar13® + conjugate 42** | | |
|---|---|---|---|---|---|---|---|---|---|
| | Rabbit 1 | Rabbit 2 | Rabbit 3 | Rabbit 1 | Rabbit 2 | Rabbit 3 | Rabbit 1 | Rabbit 2 | Rabbit 3 |
| ST8 | 400 | 1600 | 1600 | 12800 | 12800 | 6400 | 6400 | 3200 | 3200 |
| ST1 | 1600 | 1600 | 3200 | 6400 | 3200 | 1600 | 1600 | 1600 | 3200 |
| ST4 | 6400 | 25600 | 12800 | 6400 | 6400 | 3200 | 12800 | 12800 | 12800 |
| ST5 | 6400 | 12800 | 12800 | 6400 | 3200 | 3200 | 6400 | 3200 | 6400 |
| ST7F | 1600 | 6400 | 1600 | 800 | 1600 | 1600 | 1600 | 800 | 1600 |
| ST9V | 1600 | 3200 | 1600 | 3200 | 3200 | 1600 | 1600 | 800 | 3200 |
| ST19A | 1600 | 6400 | 6400 | 1600 | 6400 | 3200 | 12800 | 12800 | 3200 |

**Example E.3 Opsonophagocytic Killing Assay (OPKA)**

HL-60 cells were differentiated for one week with *N,N*-dimethylformamide as reported, washed twice with Hank's balanced sterile saline supplemented with 0.1% (w/v) gelatin (OPKA buffer) and diluted to a density of 107 cells/mL in the same buffer directly before use. Bacteria were grown in growth medium at 37 °C/5% $CO_2$ to log phase (OD600 approx. 0.2-0.3), diluted in freezing medium to a density of 106 cfu/mL and frozen in 0.5 mL aliquots at -80 °C. Bacteria were diluted with OPKA buffer and aliquoted (1000 cfu in 20 μL each) in a 96 well-plate. Bacterial suspensions were treated with mAb solutions or antiserum dilutions and incubated for 15 min at 37 °C. Complement source (10% (v/v) of the total volume, rabbit complement, CedarLane, Ontario, Canada) and differentiated HL-60 cell suspension (40 μL, phagocyte/bacteria ratio 400:1) were added and the suspensions were incubated for 45 min at 37 °C with shaking. Opsonophagocytosis was performed in triplicates. An aliquot of the contents of each well was plated on Columbia Agar plates with 5% (v/v) sheep blood, and cfu were counted after incubation at 37 °C/5% $CO_2$ overnight. Control wells lacked either antibody or complement sources. Killing was calculated relative to wells lacking antiserum. Antibody concentration in graphs relates to the initial opsonization stage (mAbs) or dilution after addition of all assay components (antisera).

The coformulations containing Prevnar13® and conjugate **41** or **42** induced a pronounced immune response

against Streptococcus pneumoniae serotype 8 CPS (see **Figure 1A** and **Figure 2A**) that induced opsonophagocytic killing of Streptococcus pneumoniae serotype 8 pneumococci (**Figure 2B**). Thereby, 50% killing was achieved at serum dilutions between 1:32 and 1:128, well above the criteria of 1:8 for protective immunity against pneumococci and similar to the opsonophagocytic killing efficacy of 007sp, the reference serum established by the World Health Organization to evaluate new pneumococcal conjugate vaccines. Rabbits immunized with Prevnar13® alone developed a significantly lower immune response against Streptococcus pneumoniae serotype 8 with no detectable opsonophagocytic killing of Streptococcus pneumoniae serotype 8 bacteria **(Figure 1A).** Coformulations with conjugate **41** or conjugate **42** did not impair the immunogenicity of other Prevnar13®-specific conjugates, as the binding capacity of rabbit sera towards six Prevnar13®-specific CPSs was unchanged (**Figure 1B-G**). Thus, conjugate **41** and conjugate **42** induce a robust, antibacterial immune response when coformulated with a multivalent, polysaccharide conjugate vaccine.

## Example F. Synthesis of capsular polysaccharides-$CRM_{197}$ conjugates

### Example F.1: Preparation of serotype 1 *S. pneumoniae* capsular polysaccharide - $CRM_{197}$ conjugate

Preparation of Master and Working Cell Banks

*S. pneumoniae* serotype 1 was obtained from the American Type Culture Collection, ATCC, strain 6301. Several generations of seed stocks were created in order to expand the strain and remove components of animal origin (generations F1 , F2, and F3). Two additional generations of seed stocks were produced. The first additional generation was made from an F3 vial, and the subsequent generation was made from a vial of the first additional generation. Seed vials were stored frozen (<-70°C) with synthetic glycerol as a cryopreservative. In addition to frozen vials, lyophilized vials were prepared for the F4 generation. For cell bank preparation, all cultures were grown in a soy-based medium. Prior to freezing, cells were concentrated by centrifugation, spent medium was removed, and cell pellets were re-suspended in fresh medium containing a cryopreservative, such as synthetic glycerol.

Fermentation and Harvesting

Cultures from the working cell bank were used to inoculate seed bottles containing a soy-based medium. The bottles were incubated at 36°C ± 2° C without agitation until growth requirements were met. A seed bottle was used to inoculate a seed fermentor containing soy-based medium. A pH of about 7.0 was maintained with sterile sodium carbonate solution. After the target optical density was reached, the seed fermentor was used to inoculate the production fermentor containing soy-based medium. The pH was maintained with sterile sodium carbonate solution. The fermentation was terminated after cessation of growth or when the working volume of the fermentor was reached. An appropriate amount of sterile 12% deoxycholate sodium was added to the culture to lyse the bacterial cells and release cell- associated polysaccharide. After lysing, the fermentor contents were cooled. The pH of the lysed culture broth was adjusted to approximately pH 6.6 with acetic acid. The lysate was clarified by continuous flow centrifugation followed by depth filtration and 0.45 μm microfiltration.

In an alternate process, the fermentation pH of about 7.0 was maintained with 3N NaOH. After the target optical density was reached, the seed fermentor was used to inoculate the production fermentor containing soy-based medium. The pH was maintained with 3N NaOH. The fermentation was terminated after cessation of growth or when the working volume of the fermentor was reached. An appropriate amount of sterile 12% deoxycholate sodium was added to the culture to obtain a 0.12% concentration in the broth, to lyse the bacterial cells and release cell-associated polysaccharide. After lysing, the fermentor contents were held, with agitation, for a time interval between 8 and 24 hours at a temperature between 7°C and 13°C, to assure that complete cellular lysis and polysaccharide release had occurred. Agitation during this hold period prevented lysate sediment from settling on the fermentor walls and pH probe, thereby allowing the pH probe integrity to be maintained. Next, the pH of the lysed culture broth was adjusted to approximately pH 5.0 with 50% acetic acid. After a hold time without agitation, for a time interval between 12 and 24 hours at a temperature between 15°C and 25°C, a significant portion of the previously soluble proteins dropped out of solution as a solid precipitate with little loss or degradation of the polysaccharide, which remained in solution. The solution with the precipitate was then clarified by continuous flow centrifugation followed by depth filtration and 0.45 μm microfiltration.

Purification

The purification of the pneumococcal polysaccharide consisted of several concentration/diafiltration operations, precipitation/elution, column chromatography, and depth filtration steps. All procedures were performed at room temperature unless otherwise specified.

Clarified broth from the fermentor cultures of S. *pneumoniae* serotype 1 were concentrated and diafiltered using

a 100 kDa MWCO (kilodalton molecular weight cutoff) filter. Diafiltration was accomplished using sodium phosphate buffer at neutral pH. Diafiltration removed the low molecular weight medium components from the higher molecular weight biopolymers such as nucleic acid, protein and polysaccharide.

The polysaccharide was precipitated from the concentrated and diafiltered solution by adding hexadecyltrimethyl ammonium bromide (HB) from a stock solution to give a final concentration of 1% HB (w/v). The polysaccharide/HB precipitate was captured on a depth filter and the filtrate was discarded. The polysaccharide precipitate was resolubilized and eluted by recirculating a sodium chloride solution through the precipitate-containing depth filter. The filters were then rinsed with additional sodium chloride solution.

Sodium iodide (NaI) was added to the polysaccharide solution from a stock NaI solution to achieve a final concentration of 0.5% to precipitate HB. The precipitate was removed by depth filtration. The filtrate contains the target polysaccharide. The precipitation vessel and the filter were rinsed with a NaCl/NaI solution and the rinse was combined with the partially purified polysaccharide solution. The filter was discarded. The polysaccharide was then filtered through a 0.2 μm filter.

The polysaccharide solution was concentrated on a 30 kDa MWCO ultrafilter and diafiltered with a sodium chloride solution.

The partially purified polysaccharide solution was further purified by filtration through a depth filter impregnated with activated carbon. After filtration, the carbon filter was rinsed with a sodium chloride solution. The rinse is combined with the polysaccharide solution, which is then filtered through a 0.2 μm filter.

The polysaccharide solution was concentrated on a 30 kDa MWCO ultrafilter and adjusted with a 1 M sodium phosphate buffer to achieve a final concentration of 0.025 M sodium phosphate. The pH was checked and adjusted to 7.0 ± 0.2. The ceramic hydroxyapatite (HA) column was equilibrated with sodium phosphate buffer containing sodium chloride to obtain the appropriate conductivity (<15 μS). The polysaccharide solution was then loaded onto the column. Under these conditions, impurities bound to the resin and the polysaccharide was recovered in the flow-through from the column. The polysaccharide solution was filtered through 0.2μm inline filters located before and after the column.

The polysaccharide solution was concentrated using a 30 kDa MWCO filter. The concentrate was then diafiltered with Water for Injection (WFI). The diafiltered polysaccharide solution was filtered through a 0.2 μm membrane filter into polypropylene bottles. Samples were removed for release testing and the purified polysaccharide was stored frozen at -25° ± 5°C.

Characterization

The $^1$H-NMR data of the purified polysaccharide can be compared to the $^1$H-NMR data of the polysaccharide molecule. The $^1$H-NMR spectrum showed a series of well-resolved signals (protons from the methyl group) for the quantitation of the O-acetyl functional group in the polysaccharide.

The identity of the monovalent polysaccharide was confirmed by countercurrent immunoelectrophoresis using specific antisera.

High performance gel filtration chromatography coupled with refractive index and multiangle laser light scattering (MALLS) detectors was used in conjunction with the sample concentration to calculate the molecular weight.

Size exclusion chromatography media (CL-4B) was used to profile the relative molecular size distribution of the polysaccharide.

Activation and Conjugation

Containers of purified polysaccharide were thawed and combined in a reaction vessel. To the vessel, 0.2 M sodium carbonate, pH 9.0 was added for partial deacetylation (hydrolysis) for 3 hours at 50°C. The reaction was cooled to 20°C and neutralization was performed by 0.2 M acetic acid. Oxidation in the presence of sodium periodate was performed by incubation at 2-8°C, and the mixture was stirred for 15-21 hours.

The activation reaction mixture was concentrated and diafiltered 10x with 0.9% NaCl using a 30K MWCO membrane. The retentate was 0.2 μm filtered. The activated saccharide was filled into 100 mL glass lyophilization bottles and shell-frozen at -75°C and lyophilized. "Shell-freezing" is a method for preparing samples for lyophilization (freeze-drying). Flasks are automatically rotated by motor driven rollers in a refrigerated bath containing alcohol or any other appropriate fluid. A thin coating of product is evenly frozen around the inside "shell" of a flask, permitting a greater volume of material to be safely processed during each freeze-drying run. These automatic, refrigerated units provide a simple and efficient means of pre-freezing many flasks at a time, producing the desired coatings inside, and providing sufficient surface area for efficient freeze-drying.

Bottles of lyophilized material were brought to room temperature and resuspended in $CRM_{197}$ solution at a saccharide/protein ratio of 2:1. To the saccharide/protein mixture 1 M sodium phosphate buffer was added to a final 0.2M ionic strength and a pH of 7.5, then sodium cyanoborohydride was added. The reaction was incubated at 23°C for

18 hours, followed by a second incubation at 37°C for 72 hours. Following the cyanoborohydride incubations, the reaction mixture was diluted with cold saline followed by the addition of 1 M sodium carbonate to adjust the reaction mixture to pH 9.0. Unreacted aldehydes were quenched by addition of sodium borohydride by incubation at 23°C for 3-6 hours.

The reaction mixture was diluted 2-fold with saline and transferred through a 0.45 - 5 μm prefilter into a retentate vessel. The reaction mixture is diafiltered 30x with 0.15 M phosphate buffer, pH 6, and 20x with saline. The retentate was filtered through a 0.2 μm filter.

The conjugate solution was diluted to a target of 0.5 mg/mL in 0.9% saline, and then sterile filtered into final bulk concentrate (FBC) containers in a Class 100 hood. The conjugate was stored at 2 - 8°C.

Characterization

Size exclusion chromatography media (CL-4B) was used to profile the relative molecular size distribution of the conjugate.

The identity of the conjugate was confirmed by the slot-blot assay using specific antisera.

The saccharide and protein concentrations were determined by the uronic acid and Lowry assays, respectively. The ratio of saccharide to protein in the covalently bonded conjugate complex was obtained by the calculation:

$$\text{Ratio} = (\mu g/mL\ \text{saccharide})/(\mu g/mL\ \text{protein})$$

*O*-acetyl content was measured by the Hestrin method (Hestrin et. al., J. Biol. Chem. 1949, 180, p. 249). The ratio of O-acetyl concentration to total saccharide concentration gave μmoles of O-acetyl per mg of saccharide.

**Example F.2: Preparation of serotype 3 S. *pneumoniae* capsular polysaccharide - $CRM_{197}$ conjugate**

Preparation of Master and Working Cell Banks

S. pneumoniae serotype 3 was obtained from American Type Culture Collection, ATCC, strain 6303. For preparation of the cell bank system, see Example F.1.

Fermentation and Harvesting

Cultures from the working cell bank were used to inoculate seed bottles containing soy-based medium. The bottles were incubated at 36°C ± 2° C without agitation until growth requirements were met. A seed bottle was used to inoculate a seed fermentor containing soy-based medium. A pH of about 7.0 was maintained with sterile sodium carbonate solution. After the target optical density was reached, the seed fermentor was used to inoculate an intermediate seed fermentor. After the target optical density was reached, the intermediate seed fermentor was used to inoculate the production fermentor. The pH was maintained with sterile sodium carbonate solution. The fermentation was terminated after the working volume of the fermentor was reached. An appropriate amount of sterile 12% sodium deoxycholate was added to the culture to lyse the bacterial cells and release cell-associated polysaccharide. After lysing, the fermentor contents were cooled. The pH of the lysed culture broth was adjusted to approximately pH 6.6 with acetic acid. The lysate was clarified by continuous flow centrifugation followed by depth filtration and 0.45 μm microfiltration.

Purification

The purification of the pneumococcal polysaccharide consisted of several concentration/diafiltration operations, precipitation/elution, column chromatography, and depth filtration steps. All procedures were performed at room temperature unless otherwise specified.

Clarified broth from the fermentor cultures of S. *pneumoniae* serotype 3 were concentrated and diafiltered using a 100 kDa MWCO filter. Diafiltration was accomplished using sodium phosphate buffer at neutral pH. Diafiltration removed the low molecular weight medium components from the higher molecular weight biopolymers such as nucleic acid, protein and polysaccharide.

Prior to the addition of hexadecyltrimethyl ammonium bromide (HB), a calculated volume of a NaCl stock solution was added to the concentrated and diafiltered polysaccharide solution to give a final concentration of 0.25 M NaCl. The polysaccharide was then precipitated by adding HB from a stock solution to give a final concentration of 1 % HB (w/v). The polysaccharide/HB precipitate was captured on a depth filter and the filtrate was discarded. The polysaccharide precipitate was resolubilized and eluted by recirculating a sodium chloride solution through the precipitate-containing depth filter. The filters were then rinsed with additional sodium chloride solution.

Sodium iodide (NaI) was added to the polysaccharide solution from a stock NaI solution to achieve a final concentration of 0.5% to precipitate HB. The precipitate was removed by depth filtration. The filtrate contained the target polysaccharide. The precipitation vessel and the filter were rinsed with a NaCl/NaI solution and the rinse was combined with the partially purified polysaccharide solution. The filter was discarded. The polysaccharide was then filtered through a 0.2 μm filter. The polysaccharide solution was concentrated on a 30 kDa MWCO ultrafilter and diafiltered with a sodium chloride solution.

The partially purified polysaccharide solution was further purified by filtration through a depth filter impregnated with activated carbon. After filtration, the carbon filter was rinsed with a sodium chloride solution. The rinse was combined with the polysaccharide solution, which was then filtered through a 0.2 μm filter.

The polysaccharide solution was concentrated on a 30 kDa MWCO ultrafilter and adjusted with a 1 M sodium phosphate buffer to achieve a final concentration of 0.025M sodium phosphate. The pH was checked and adjusted to 7.0 ± 0.2.

The ceramic hydroxyapatite (HA) column was equilibrated with sodium phosphate buffer containing sodium chloride to obtain the appropriate conductivity (15 μS). The polysaccharide solution was then loaded onto the column. Under these conditions, impurities bound to the resin and the polysaccharide was recovered in the flow-through from the column. The polysaccharide was flushed through the column with buffer and was filtered through a 0.2μm filter.

The polysaccharide solution was concentrated using a 30 kDa MWCO filter. The concentrate was then diafiltered with WFI.

The diafiltered polysaccharide solution was filtered through a 0.2 μm membrane filter into stainless steel containers. Samples were removed for release testing and the purified polysaccharide was stored frozen at -25° ± 5°C.

Activation and Conjugation

Containers of purified serotype 3 saccharide were thawed and combined in a reaction vessel. To the vessel, WFI and 2M acetic acid were added to a final concentration of 0.2M and 2mg/mL saccharide. The temperature of the solution was raised to 85°C for one hour to hydrolyze the polysaccharide. The reaction was cooled to <25°C and 1 M magnesium chloride was added to a final concentration of 0.1 M. Oxidation in the presence of sodium periodate was performed by incubation for 16-24 hours at 23°C.

The activation reaction mixture was concentrated and diafiltered 10x with WFI using a 100K MWCO membrane. The retentate was filtered through a 0.2-μm filter.

For compounding, 0.2M sodium phosphate, pH 7.0, was added to the activated saccharide to a final concentration of 10mM and a pH of 6.0-6.5. $CRM_{197}$ carrier protein was mixed with the saccharide solution to a ratio of 2g of saccharide per 1 g of $CRM_{197}$. The combined saccharide/protein solution was filled into 100 mL glass lyophilization bottles with a 50mL target fill, shell-frozen at -75°C, and lyophilized.

Bottles of co-lyophilized saccharide/protein material were brought to room temperature and resuspended in 0.1 M sodium phosphate buffer, pH 7.0, to a final saccharide concentration of 20 mg/mL The pH was adjusted to 6.5 and then a 0.5 molar equivalent of sodium cyanoborohydride was added. The reaction was incubated at 37°C for 48 hours. Following the cyanoborohydride incubation, the reaction mixture was diluted with cold 5mM succinate/0.9% saline buffer. Unreacted aldehydes were quenched by the addition of sodium borohydride and incubation at 23°C for 3-6 hours. The reaction mixture was transferred through a 0.45-5 μm prefilter into a retentate vessel.

The reaction mixture was diafiltered 30x with 0.1 M phosphate buffer (pH 9), 20x with 0.15M phosphate butter (pH 6), and 20x with 5mM succinate/0.9% saline. The retentate was filtered through a 0.2-μm filter.

The conjugate solution was diluted to a saccharide target of 0.5 mg/mL, and then sterile filtered into FBC containers in a Class 100 hood. The conjugate was stored at 2 - 8°C.

Characterization

Size exclusion chromatography media (CL-4B) was used to profile the relative molecular size distribution of the conjugate.

The identity of the conjugate was confirmed by the slot-blot assay using specific antisera.

The saccharide and protein concentrations were determined by the Anthrone and Lowry assays, respectively. The ratio of saccharide to protein in the covalently bonded conjugate complex was obtained by the calculation:

$$\text{Ratio} = (\mu g/mL\ \text{saccharide})/(\mu g/mL\ \text{protein})$$

**Example F.3: Preparation of serotype 5 *S. pneumoniae* capsular**

**polysaccharide - $CRM_{197}$ conjugate**

S. *pneumoniae* serotype 5 was obtained from American Type Culture Collection, ATCC, strain 6305. For preparation of the cell bank system, see Example F.1. For fermentation, harvesting, purification and characterization of the polysaccharide, see **Example F.1.**

Alternate Fermentation Process

Cultures from the working cell bank were used to inoculate seed bottles containing a soy-based medium and a 10mM sterile $NaHCO_3$ solution. The bottles were incubated at 36°C ± 2° C without agitation until growth requirements were met. A seed bottle was used to inoculate a seed fermentor containing soy-based medium and a 10mM sterile $NaHCO_3$ solution. A pH of about 7.0 was maintained with 3N NaOH. After the target optical density was reached, the seed fermentor was used to inoculate the production fermentor containing soy-based medium with a 10mM $NaHCO_3$ concentration. The pH was maintained with 3N NaOH. The fermentation was terminated after cessation of growth or when the working volume of the fermentor was reached. An appropriate amount of sterile 12% sodium deoxycholate was added to the culture to obtain a 0.12% concentration in the broth, to lyse the bacterial cells and release cell-associated polysaccharide. After lysing, the fermentor contents were held, with agitation, for a time interval between 8 and 24 hours at a temperature between 7°C and 13°C to assure that complete cellular lysis and polysaccharide release had occurred. Agitation during this hold period prevented lysate sediment from settling on the fermentor walls and pH probe, thereby allowing the pH probe integrity to be maintained. Next, the pH of the lysed culture broth was adjusted to approximately pH 4.5 with 50% acetic acid. After a hold time without agitation, for a time interval between 12 and 24 hours at a temperature between 15°C and 25°C, a significant portion of the previously soluble proteins dropped out of solution as a solid precipitate with little loss or degradation of the polysaccharide, which remained in solution. The solution with the precipitate was then clarified by continuous flow centrifugation followed by depth filtration and 0.45μm microfiltration.

Activation and Conjugation

Containers of serotype 5 saccharide were thawed and combined in a reaction vessel. To the vessel, 0.1 M sodium acetate, pH 4.7, was added followed by oxidation in the presence of sodium periodate by incubation for 16-22 hours at 23°C. The activation reaction mixture was concentrated and diafiltered 10x with WFI using a 100K MWCO membrane. The retentate was filtered through a 0.2 μm filter.

The serotype 5 activated saccharide was combined with $CRMi_{97}$ at a ratio of 0.8:1. The combined saccharide/protein solution was filled into 100 mL glass lyophilization bottles (50 mL target fill), shell-frozen at -75°C, and co-lyophilized. Bottles of co-lyophilized material were brought to room temperature and resuspended in 0.1 M sodium phosphate, pH 7.5, and sodium cyanoborohydride was added. The reaction was incubated at 30°C for 72 hours, followed by a second addition of cyanoborohydride and incubated at 30°C for 20-28 hours. Following the cyanoborohydride incubations, the reaction mixture was diluted 2-fold with saline and transferred through a 0.45-5μm prefilter into a retentate vessel.

The reaction mixture was diafiltered 30x with 0.01 M phosphate buffer, pH 8, 20x with 0.15M phosphate buffer, pH 6, and 20x with saline. The retentate was filtered through a 0.2 μm filter.

The conjugate solution was diluted to a saccharide target of 0.5 mg/mL, and then sterile filtered into FBC containers in a Class 100 hood. The conjugate was stored at 2 - 8°C.

For the characterization of the conjugate, see **Example F.1.**

**Example F.4: Preparation of serotype 6A *S. pneumoniae* capsular polysaccharide - $CRM_{197}$ conjugate**

S. *pneumoniae* serotype 6A was obtained from American Type Culture Collection, ATCC, strain 6306. For preparation of the cell bank system, see **Example F.1.** For fermentation, harvesting and purification of the polysaccharide, see **Example F.1,** except that during purification, the 30 kDa MWCO concentration step, prior to the chromatography step, is omitted.

Activation and Conjugation

Serotype 6A polysaccharide is a high molecular weight polymer that had to be reduced in size prior to oxidation. Containers of serotype 6A saccharide were thawed and combined in a reaction vessel. To the vessel, 2M acetic acid was added to a final concentration of 0.1 M for hydrolysis for 1.5 hours at 60°C. The reaction was cooled to 23°C and

neutralization was performed by adjusting the reaction mixture with 1M NaOH to pH 6. Oxidation in the presence of sodium periodate was performed by incubation at 23°C for 14-22 hours. The activation reaction mixture was concentrated and diafiltered 10x with WFI using a 100K MWCO membrane. The retentate was filtered through a 0.2 μm filter.

Serotype 6A was compounded with sucrose and filled into 100 mL glass lyophilization bottles (50mL target fill) and shell-frozen at -75°C and lyophilized.

Bottles of lyophilized material were brought to room temperature and resuspended in dimethylsulfoxide (DMSO) at a saccharide/protein ratio of 1 :1. After addition of sodium cyanoborohydride, the reaction mixture was incubated at 23°C for 18 hours. Following the cyanoborohydride incubation, the reaction mixture was diluted with cold saline. Unreacted aldehydes were quenched by addition of sodium borohydride by incubation at 23°C for 3-20 hours.

The diluted reaction mixture was transferred through a 5 μm prefilter into a retentate vessel. The reaction mixture was diafiltered 10x with 0.9% NaCl and 30x with succinate-buffered NaCl. The retentate was filtered through a 0.2μm filter. The conjugate solution was diluted to a saccharide target of 0.5 mg/mL, and then sterile filtered into FBC containers in a Class 100 hood. The conjugate was stored at 2 - 8°C.

For the characterization of the conjugate, see **Example F.1.**

### Example F.5: Preparation of serotype 7F *S. pneumoniae* capsular polysaccharide - $CRM_{197}$ conjugate

*S. pneumoniae* serotype 7F was obtained from American Type Culture Collection, ATCC, strain 10351. For preparation of the cell bank system, and for fermentation and harvesting of the polysaccharide, see **Example F.2.** For an alternate fermentation and harvesting process, see the alternate process described in **Example F.1.**

Purification

The purification of the pneumococcal polysaccharide consisted of several concentration/diafiltration operations, precipitation/elution, column chromatography, and depth filtration steps. All procedures were performed at room temperature unless otherwise specified.

Clarified broth from fermentor cultures of *S. pneumoniae* serotype 7F were concentrated and diafiltered using a 100 kDa MWCO filter. Diafiltration was accomplished using sodium phosphate buffer at neutral pH. Diafiltration removed the low molecular weight medium components from the higher molecular weight biopolymers such as nucleic acid, protein and polysaccharide.

Serotype 7F does not form a precipitate with HB. Instead, impurities were precipitated from the concentrated and diafiltered solution by adding the HB from a stock solution to a final concentration of 1% HB. The precipitate was captured on a depth filter and the filter was discarded. The polysaccharide was contained in the filtrate.

Sodium iodide (NaI) was added to the polysaccharide solution from a stock NaI solution to achieve a final concentration of 0.5% to precipitate HB. The precipitate was removed by depth filtration. The filtrate contained the target polysaccharide. The precipitation vessel and the filter were rinsed with a NaCl/NaI solution and the rinses were combined with the partially purified polysaccharide solution. The filter was discarded. The polysaccharide was then filtered through a 0.2μm filter.

The polysaccharide solution was concentrated on a 30 kDa MWCO ultrafilter and diafiltered with a sodium chloride solution. The partially purified polysaccharide solution was further purified by filtration through a depth filter impregnated with activated carbon. After filtration, the carbon filter was rinsed with a sodium chloride solution. The rinse was combined with the polysaccharide solution, which was then filtered through a 0.2 μm filter.

The polysaccharide solution was concentrated on a 30 kDa MWCO ultrafilter and adjusted with a 1M sodium phosphate buffer to achieve a final concentration of 0.025M sodium phosphate. The pH was checked and adjusted to 7.0 ± 0.2.

The ceramic hydroxyapatite (HA) column was equilibrated with sodium phosphate buffer containing sodium chloride to obtain the appropriate conductivity (15 μS). The polysaccharide solution was then loaded onto the column. Under these conditions, impurities bound to the resin and the polysaccharide was recovered in the flow-through from the column. The polysaccharide was flushed through the column with buffer and was filtered through a 0.2 μm filter.

The polysaccharide solution was concentrated using a 30 kDa MWCO filter. The concentrate was then diafiltered with WFI. The diafiltered polysaccharide solution was filtered through a 0.2μm membrane filter into stainless steel containers. Samples were removed for release testing and the purified polysaccharide was stored at 2° - 8°C.

Activation and Conjugation

Oxidation in the presence of sodium periodate was performed by incubation for 16-24 hrs at 23°C.

The activation reaction mixture was concentrated and diafiltered 10x with 10mM NaOAc, pH 4.5, using a 100K MWCO membrane. The retentate was filtered through a 0.2 μm filter. Serotype 7F was filled into 100 mL glass lyophilization bottles (50 ml. target fill) and shell-frozen at -75°C and lyophilized.

Bottles of lyophilized serotype 7F and $CRM_{197}$ were brought to room temperature and resuspended in DMSO at a saccharide/protein ratio of 1.5:1. After the addition of sodium cyanoborohydride, the reaction was incubated at 23°C for 8-10 hours. Unreacted aldehydes were quenched by the addition of sodium borohydride by incubation at 23°C for 16 hours.

The reaction mixture was diluted 10-fold with cold saline and transferred through a 5 μm prefilter into a retentate vessel. The reaction mixture was diafiltered 10x with 0.9% saline and 30x with succinate-buffered saline. The retentate was filtered through a 0.2μm filter.

The conjugate solution was diluted to a saccharide target of 0.5 mg/mL 0.9% saline, and then sterile filtered into FBC containers in a Class 100 hood. The conjugate was stored at 2 - 8°C.

**Example F.6: Preparation of serotype 19A S. *pneumoniae* capsular polysaccharide - $CRM_{197}$ conjugate**

S. *pneumoniae* serotype 19A was obtained from American Type Culture Collection, ATCC, strain 10357. For preparation of the cell bank system, see **Example F.1.** For fermentation, harvesting and purification of the polysaccharide, see **Example F.4.**

Activation and Conjugation

Containers of serotype 19A saccharide were thawed and combined in a reaction vessel. Sodium acetate was added to 10mM (pH 5.0) and oxidation was carried out in the presence of sodium periodate by incubation for 16-24 hrs at 23°C.

The activation reaction mixture was concentrated and diafiltered 10x with 10mM acetate, pH 5.0, using a 100K MWCO membrane. The retentate was filtered through a 0.2 μm filter.

The activated saccharide was compounded with sucrose followed by the addition of $CRM_{197}$. The serotype 19A activated saccharide and $CRM_{197}$ mixture (0.8:1 ratio) was filled into 100 mL glass lyophilization bottles (50 mL target fill) and shell-frozen at -75°C and lyophilized.

Bottles of lyophilized material were brought to room temperature and resuspended in DMSO. To the saccharide/protein mixture, sodium cyanoborohydride (100 mg/ml) was added. The reaction was incubated at 23°C for 15 hours. Following the cyanoborohydride incubation, unreacted aldehydes were quenched by the addition of sodium borohydride by incubation at 23°C for 3-20 hours.

The reaction mixture was diluted 10-fold with cold saline and transferred through a 5 μm prefilter into a retentate vessel. The reaction mixture was diafiltered 10x with 0.9% NaCl, 0.45-μm filtered, and 30x with diafiltration using 5mM succinate/ 0.9% NaCl buffer, pH 6. The retentate was filtered through a 0.2 μm filter.

The conjugate solution was diluted to a target of 0.5 mg/mL using 5mM succinate/0.9% saline, and then sterile filtered into FBC containers in a Class 100 hood. The conjugate was stored at 2 - 8°C.

For characterization of the conjugate, see **Example F.2.**

**Example F.7: Preparation of serotype 4, 6B, 9V, 14, 18C, 19F and 23F S. *pneumoniae* capsular polysaccharide - $CRM_{197}$ conjugates**

Preparation of the *S. pneumoniae* Seed Culture

S. *pneumoniae* serotypes 4, 6B, 9V, 14, 18C, 19F and 23F were obtained from American Type Culture Collection, ATCC, strain 6304, strain 6326, strain 10368, strain 6314, strain 10356, strain 6319 and strain 6323.

Separately, one vial of each of the desired serotypes of *Streptococcus pneumoniae* was used to start a fermentation batch. Two bottles containing a soy-based medium and phenol red were adjusted to a pH range of 7.4 ± 0.2 using sodium carbonate, and the required volume of 50% dextrose/1% magnesium sulfate solution was then added to the bottles. The two bottles were inoculated with different amounts of seed. The bottles were incubated at 36° ± 2°C until the medium turned yellow. Following incubation, samples were removed from each bottle and tested for optical density (OD) (0.3 to 0.9) and pH (4.6 to 5.5). One of the two bottles was selected for inoculation of the seed fermentor.

Soy-based medium was transferred to the seed fermentor and sterilized. Then a volume of 50% dextrose/1 % magnesium sulfate solution was added to the fermentor. The pH and agitation of the seed fermentor were monitored and controlled (pH 6.7 to 7.4). The temperature was maintained at 36° ± 2°C. The seed inoculum (bottle) was aseptically connected to the seed fermentor and the inoculum was transferred. The fermentor was maintained in pH control and samples were periodically removed and tested for OD and pH. When the desired OD of 0.5 at 600 nm was reached, the intermediate fermentor was inoculated with the fermentation broth from the seed fermentor.

Soy-based medium was transferred to the intermediate fermentor and sterilized. Then a volume of 50% dextrose/1% magnesium sulfate solution was added to the fermentor. The pH and agitation of the intermediate fermentor were monitored and controlled (pH 6.7 to 7.4). The temperature was maintained at 36° ± 2°C. The contents of the seed fermentor were transferred to the intermediate fermentor. The fermentor was maintained in pH control and samples were periodically removed and tested for OD and pH. When the desired OD of 0.5 at 600 nm was reached, the production fermentor was inoculated with the fermentation broth from the intermediate fermentor.

Soy-based medium was transferred to the production fermentor and sterilized. Then a volume of 50% dextrose/1% magnesium sulfate solution was added to the fermentor. The pH and agitation of the production fermentor were monitored and controlled (pH 6.7 to 7.4). The temperature was maintained at 36° ± 2°C. The fermentor was maintained in pH control and samples were periodically removed and tested for OD and pH, until the fermentation was complete.

Deoxycholate sodium was added to the fermentor to a final concentration of approximately 0.12% w/v. The culture was mixed for a minimum of thirty minutes and the temperature set point was reduced to 10°C. The culture was incubated overnight and following confirmation of inactivation, the pH of the culture was adjusted to between 6.4 and 6.8, as necessary, with 50% acetic acid. The temperature of the fermentor was increased to 20° ± 5°C and the contents were transferred to the clarification hold tank.

The contents of the clarification hold tank (including the cellular debris) were processed through a centrifuge at a flow rate between 25 and 600 liters per hour (except serotype 4, wherein the cell debris was discarded and the flow rate tightened to between 25 and 250 liters per hour). Samples of the supernatant were removed and tested for OD. The desired OD during the centrifugation was < 0.15. Initially, the supernatant was recirculated through a depth filter assembly until an OD of 0.05 ± 0.03 was achieved. Then the supernatant was passed through the depth filter assembly and through a 0.45 μm membrane filter to the filtrate hold tank.

Subsequently, the product was transferred through closed pipes to the purification area for processing.

All of the above operations (centrifugation, filtration and transfer) were performed between 10°C to 30°C.

For an alternate fermentation and harvesting process for serotypes 4 and 6B, see the alternate process described in **Example F.1.**

<u>Purification</u>

The purification of each pneumococcal polysaccharide consisted of several concentration/diafiltration operations, precipitation/elution, column chromatography, and depth filtration steps. All procedures were performed at room temperature unless otherwise specified.

Clarified broth from the fermentor cultures of the desired S. *pneumoniae* serotype was concentrated and diafiltered using a 100 kDa MWCO filter. Diafiltration was accomplished using sodium phosphate buffer at pH < 9.0. Diafiltration removed the low molecular weight medium components from the higher molecular weight biopolymers such as nucleic acid, protein and polysaccharide.

The polysaccharide was precipitated from the concentrated and diafiltered solution by adding HB from a stock solution to give a final concentration of 1 % HB (w/v) (except serotype 23F, which had a final concentration of 2.5%). The polysaccharide/HB precipitate was captured on a depth filter and the filtrate was discarded. (Note: serotype 14 does not precipitate; therefore the filtrate was retained.) The polysaccharide precipitate was resolubilized and eluted by recirculating a sodium chloride solution through the precipitate-containing depth filter. The filters were then rinsed with additional sodium chloride solution.

Sodium iodide (NaI) was added to the polysaccharide solution from a stock NaI solution to achieve a final concentration of 0.5% to precipitate HB (except for Serotype 6B, which had a final concentration of 0.25%). The precipitate was removed by depth filtration. The filtrate contained the target polysaccharide. The filter was discarded. The polysaccharide was then filtered through a 0.2μm filter.

The polysaccharide solution was concentrated on a 30 kDa MWCO ultrafilter and diafiltered with a sodium chloride solution.

The partially purified polysaccharide solution was further purified by filtration through a depth filter impregnated with activated carbon. After filtration, the carbon filter was rinsed with a sodium chloride solution. The rinse was combined with the polysaccharide solution, which was then filtered through a 0.2μm filter.

The polysaccharide solution was concentrated on a 30 kDa MWCO ultrafilter and the filter was rinsed with a sodium chloride solution. The pH was checked and adjusted to 7.0 ± 0.3.

The ceramic hydroxyapatite (HA) column was equilibrated with sodium phosphate buffer containing sodium chloride until the pH is 7.0 ± 0.3 and the conductivity was 26 + 4μS. The polysaccharide solution was then loaded onto the column. Under these conditions, impurities bound to the resin and the polysaccharide was recovered in the flow through from the column. The polysaccharide solution was filtered through a 0.2μm filter. The polysaccharide solution was concentrated using a 30 kDa MWCO filter.

The concentrate was then diafiltered with WFI until the conductivity was < 15μS. The diafiltered polysaccharide

solution was filtered through a 0.2μm membrane filter into bulk containers and stored at 2-8°C.

Activation Process

The different serotype saccharides follow different pathways for activation (hydrolysis or no hydrolysis prior to activation) and conjugation (aqueous or DMSO reactions) as described in this example.
Polysaccharide was transferred from the bulk containers to the reactor vessel. The polysaccharide was then diluted in WFI and sodium phosphate to a final concentration range of 1.6 - 2.4 mg/mL

*Step 1*

For serotypes 6B, 9V, 14, 19F and 23F, pH was adjusted to pH 6.0 ± 0.3.
For serotype 4, hydrochloric acid (0.01 M final acid concentration) was added and the solution was incubated for 25 - 35 minutes at 45° ± 2°C. Hydrolysis was stopped by cooling to 21 - 25°C and adding 1M sodium phosphate to a target of pH 6.7 ± 0.2. An in-process test was done to confirm an appropriate level of depyruvylation.
For serotype 18C, glacial acetic acid (0.2 M final acid concentration) was added and the solution was incubated for 205 - 215 minutes at 94° ± 2°C. Temperature was then decreased to 21 - 25°C and 1 - 2 M sodium phosphate was added to a target of pH 6.8 ± 0.2.

*Step 2: Periodate Reaction*

The required sodium periodate molar equivalents for pneumococcal saccharide activation was determined using total saccharide content (except for serotype 4). For serotype 4, a ratio of 0.8-1.2 moles of sodium periodate per mole of saccharide was used. With thorough mixing, the oxidation reaction was allowed to proceed between 16 to 20 hours at 21 - 25°C for all serotypes except 19F for which the temperature was ≤ 15°C.

*Step 3: Ultrafiltration*

The oxidized saccharide was concentrated and diafiltered with WFI (0.01 M sodium phosphate buffer pH 6.0 for serotype 19F) on a 100 kDa MWCO ultrafilter (5 kDa ultrafilter for 18C). The permeate was discarded and the retentate was filtered through a 0.22 μm filter.

*Step 4: Lyophilization*

For serotypes 4, 9V, and 14 the concentrated saccharide was mixed with $CRM_{197}$ carrier protein, filled into glass bottles, shell-frozen and stored at < -65°C. The frozen concentrated saccharide-$CRM_{197}$ was lyophilized and then stored at -25° ± 5°C. For serotypes 6B, 19F, and 23F a specified amount of sucrose was added which was calculated to achieve a 5% ± 3% sucrose concentration in the conjugation reaction mixture. Serotype 18C did not require sucrose addition. The concentrated saccharide was then filled into glass bottles, shell-frozen and stored at < -65°C. The frozen concentrated saccharide was lyophilized and then stored at -25° ± 5°C.

Conjugation Process

Two conjugation processes were used: aqueous conjugation for serotypes 4, 9V, 14 and 18C, and DMSO conjugation for serotypes 6B, 19F and 23F.

Aqueous Conjugation

*Step 1: Dissolution*

For serotypes 4, 9V and 14, the lyophilized activated saccharide-$CRM_{197}$ mixture was thawed and equilibrated at room temperature. The lyophilized activated saccharide-$CRM_{197}$ was then reconstituted in 0.1 M sodium phosphate buffer at a typical ratio of:

1 L of buffer per 16 - 24 g of saccharide for serotype 4 and 9V;
1 L of buffer per 6 - 10 g of saccharide for serotype 14;

The reaction mixture was incubated at 37° ± 2°C until total dissolution for the serotype 9V and at 23° ± 2°C

for serotypes 4 and 14.

For serotype 18C, the lyophilized saccharide was reconstituted in a solution of $CRM_{197}$ in 1M dibasic sodium phosphate at a typical ratio of 0.11 L of sodium phosphate per 1L of $CRM_{197}$ solution. The reaction mixture (8-12 g/L saccharide concentration) was incubated at 23° + 2°C until total dissolution.

The pH was tested as an in-process control at this stage.

*Step 2: Conjugation Reaction*

For serotypes 4 and 9V, the conjugation reaction was initiated by adding the sodium cyanoborohydride solution (100 mg/mL) to achieve 1.0 - 1.4 moles sodium cyanoborohydride per mole of saccharide. The reaction mixture was incubated for 44 - 52 hours at 37° ± 2°C. The temperature was then reduced to 23° ± 2°C and sodium chloride 0.9% was added to the reactor. Sodium borohydride solution (100mg/mL) was added to achieve 1.8 - 2.2 molar equivalents of sodium borohydride per mole saccharide. The mixture was incubated for 3 - 6 hours at 23° ± 2°C. The mixture was diluted with sodium chloride 0.9% and the reactor was rinsed. The diluted conjugation mixture was filtered using a 1.2 μm pre-filter into a holding vessel.

For serotypes 14 and 18C, the conjugation reaction was initiated by adding the cyanoborohydride solution (100 mg/mL) to achieve 1.0 - 1.4 moles of sodium cyanoborohydride per mole of saccharide. The reaction mixture was incubated for 12 - 24 hours at 23° ± 2°C. The temperature was increased to 37° ± 2°C and the reaction was incubated for 72 - 96 hours. The temperature was then reduced to 23° ± 2°C and 0.9% sodium chloride was added to the reactor. Sodium borohydride solution (100mg/mL) was added to achieve 1.8 - 2.2 molar equivalents of sodium borohydride per mole of saccharide. The mixture was incubated for 3 - 6 hours at 23° ± 2°C. The mixture was diluted with 0.9% sodium chloride and the reactor was rinsed. The diluted conjugation mixture was then filtered using a 1.2 μm pre-filter into a holding vessel.

*Step 3: Ultrafiltration 100 kDa*

The diluted conjugation mixture was concentrated and diafiltrated on a 100 kDa MWCO ultrafilter with either a minimum of 15 volumes (serotype 4) or 40 volumes (serotypes 9V, 14, and 18C) of 0.9% sodium chloride. The permeate was discarded.

For serotype 4, the retentate was filtered through a 0.45μm filter. An in-process control (saccharide content) was performed at this step.

*Step 4: HA Column Purification*

This step was only performed for the serotype 4 conjugate. The HA column was first neutralized using 0.5M sodium phosphate buffer (pH 7.0 + 0.3) and then equilibrated with 0.9% sodium chloride. The filtered retentate (serotype 4) was loaded onto the column at a flow rate of 1.0 L/min. The column was washed with 0.9% sodium chloride at a flow rate of < 2.0 L/min. The product was then eluted with 0.5M sodium phosphate buffer at a flow rate of < 2.0 L/min.

The HA fraction was then concentrated and diafiltered on a 100 kDa MWCO membrane with a minimum of 20 volumes of 0.9% sodium chloride. The permeate was discarded.

*Step 5: Sterile Filtration*

The retentate after the 100 kDa MWCO diafiltration was filtered through a 0.22μm filter. In-process controls (saccharide content, free protein, free saccharide and cyanide) were performed on the filtered product. In-process controls on filtered retentate were performed to determine whether additional concentration, diafiltration, and/or dilution were needed to meet FBC targets. These and additional tests were repeated in FBC samples.

As necessary, the filtered conjugate was diluted with 0.9% sodium chloride in order to achieve a final concentration of less than 0.55 g/L. Release tests for saccharide content, protein content and saccharide: protein ratio were performed at this stage. Finally, the conjugate was filtered (0.22 μm) and filled into 10 L stainless steel canisters at a typical quantity of 2.64 g/canister. At this stage, yield, saccharide content, protein content, pH, saccharide:protein ratio and lysine content were performed as in-process controls. Release testing (appearance, free protein, free saccharide, endotoxin, molecular size determination, residual cyanide, saccharide identity, $CRM_{197}$ identity) was performed at this stage.

DMSO Conjugation

*Step I: Dissolution*

The lyophilized activated saccharide serotypes 6B, 19F, 23F and the lyophilized $CRM_{197}$ carrier protein were equilibrated at room temperature and reconstituted in DMSO. The dissolution concentration typically ranged from 2-3 grams of saccharide (2-2.5 g protein) per liter of DMSO.

*Step II: Conjugation Reaction*

The activated saccharide and $CRM_{197}$ carrier protein were mixed for 60 - 75 minutes at 23° ± 2°C at a ratio range of 0.6 g - 1.0 g saccharide/g $CRM_{197}$ for serotypes 6B and 19F or 1.2 to 1.8 g saccharide/g $CRM_{197}$ for serotype 23F.

The conjugation reaction was initiated by adding the sodium cyanoborohydride solution (100mg/mL) at a ratio of 0.8 - 1.2 molar equivalents of sodium cyanoborohydride to one mole activated saccharide. WFI was added to the reaction mixture to a target of 1% (v/v) and the mixture was incubated for over 40 hours at 23°± 2°C.

Sodium borohydride solution, 100 mg/mL (typical 1.8 - 2.2 molar equivalents sodium borohydride per mole activated saccharide) and WFI (target 5% v/v) were added to the reaction and the mixture was incubated for 3 - 6 hours at 23° ± 2°C. This procedure reduced any unreacted aldehydes present on the saccharides. Then the reaction mixture was transferred to a dilution tank containing 0.9% sodium chloride at < 15°C.

*Step III: 100 kDa Ultrafiltration*

The diluted conjugate mixture was filtered through a 1.2 μm filter and concentrated and diafiltered on a 100 kDa MWCO membrane with a minimum of 15 volumes of 0.9% sodium chloride (0.01 M sodium phosphate/0.05M NaCl buffer was used for serotype 23F). The permeate was discarded. The retentate was filtered through a 0.45 μm filter. An in-process saccharide content sample was taken at this stage.

*Step IV: DEAE Column Purification*

This step was only performed for serotype 23F.

The DEAE column was equilibrated with 0.01 M sodium phosphate/0.05M sodium chloride buffer. The filtered retentate (serotype 23F) was loaded onto the column and washed with 0.01 M sodium phosphate/0.05M sodium chloride buffer. The column was then washed with 0.01 M sodium phosphate/0.9% NaCl buffer. The product was then eluted with 0.01 M sodium phosphate/0.5M sodium chloride buffer.

*Step V: 100 kDa Ultrafiltration*

The retentate from 6B and 19F was concentrated and diafiltered with at least 30 volumes of 0.9% sodium chloride. The permeate was discarded. The eluate from serotype 23F was concentrated and diafiltered with a minimum of 20 volumes of 0.9% sodium chloride. The permeate was discarded.

*Step VI: Sterile Filtration*

The retentate after the 100 kDa MWCO dialfiltration was filtered through 0.22 μm filter. In-process controls (saccharide content, free protein, free saccharide, residual DMSO and residual cyanide) were performed on the filtered product. In-process controls on filtered retentate were performed to determine whether additional concentration, diafiltration, and/or dilution were needed to meet FBC targets. These and additional tests were repeated in FBC samples. As necessary, the filtered conjugate was diluted with 0.9% sodium chloride to achieve a final concentration of less than 0.55 g/L. Release tests for saccharide content, protein content and saccharide:protein ratio were performed at this stage.

Finally, the conjugate was filtered (0.22 μm) and filled into 10 L stainless steel canisters at a quantity of 2.64 g/canister. At this stage, yield, saccharide content, protein content, pH, saccharide: protein ratio and lysine content were performed as in- process controls. Release testing (appearance, free protein, free saccharide, endotoxin, molecular size determination, residual cyanide, residual DMSO, saccharide identity and $CRM_{197}$ identity) was performed at this stage.

**Example G. Preparation of *Streptococcus pneumoniae* capsular polysaccharides conjugated to protein D/tetanus toxoid/diphtheria toxoid**

Capsular polysaccharides of different S. *pneumoniae* serotypes were isolated from the corresponding S. *pneu-*

*moniae* strain producing said polysaccharides by extraction from fermented cultures according to EP0072513 or similar methods.

**Example G.1. *S. pneumoniae* serotype 1 polysaccharide-protein D-conjugate**

Fermentation

A sample of a strain of *Streptococcus pneumoniae* producing capsular polysaccharide of serotype 1 stored at -70 °C in Tryptic Soy Broth supplemented with 10% glycerol is rapidly thawed. The organisms are collected using a platinum loop and transferred to a Petri dish containing 15 ml of culture medium consisting of 40 g of blood agar base per liter of water, sterilized at 121 °C for 20 minutes and then cooled to 50 °C and supplemented with 5% of fresh defibrinated sheep blood and the microorganism is then incubated at 37 °C for 16 hours in atmosphere enriched with $CO_2$ (5% w/v).

The produced bacteria are collected and a second run is performed in a Roux culture bottle containing 100 ml of synthetic medium according Hoeprich (Paul D. Hoeprich J. Bact. 1955, 69, p. 682-688, and J. Bact. 1957, 74, p. 587-590), solidified by adding agar (20 g/l). After an incubation period of 6 to 9 hours in anaerobic atmosphere, the produced organism is harvested and suspended in 5 ml of aqueous sodium chloride solution (8.5 g per liter). The harvest is used to inoculate a culture vessel containing 8 liters of liquid semi-synthetic medium according to Hoeprich. The medium is maintained without agitation or aeration for 16 hours at 37 °C to form the inoculum for the production culture.

For the production culture, a 100 liter fermenter is used containing 72 liters of semi-synthetic medium following Hoeprich. The medium is inoculated with the above-referenced culture and the incubation is maintained without aeration but gentle stirring at 35.5 °C (±1), the pH being continuously adjusted to 7.2 using a 25% sodium hydroxide solution. The development of the culture is regularly monitored by sampling and measuring the optical density of the medium at 620 nm. When the optical density reaches 0.15 a solution of one kilogram of dextrose in 2 liters of autoclaved deionized water is added over 20 minutes at 121 °C. The culture is stopped at the beginning of stationary phase, which corresponds to an optical density of 0.8.

Extraction of polysaccharide

The fermentation medium (80 l) is transferred into a storage tank and inactivated by adding one liter of 90% phenol; the pH is adjusted to 7.2 with 25% sodium hydroxide solution. After standing overnight at 5 - 10 °C, 1168 g of sodium chloride are dissolved, which corresponds to an addition of 0.25 mol of sodium chloride per liter of medium (so-called concentration A).

While stirring, 4 kg of inert porous support (Celite 545) are then added and to the suspension, 800 ml (said volume B) of an aqueous solution of cetrimide (5% w/v) which is previously stored at a temperature of 25 °C is added and the stirring is continued for 10 minutes after the addition of cetrimide.

The medium is then filtered and the cake is washed with 4 L of an aqueous solution of sodium chloride of concentration A, with additional 40 ml of an aqueous solution of cetrimide (5% w/v).

The filtrate is concentrated to a volume of 5 liters by ultrafiltration in an AMICON DC unit 30 provided with cartridges with a cut-off of molecular weight of 100 kDa. The retentate is diafiltrated first with 40 L of sodium chloride solution of concentration A and then with 40 L of water keeping the volume between 10 and 5 liters. Finally, the volume of the solution is reduced to about 3 liters by ultrafiltration and the apparatus is rinsed with water to obtain approximately 5 liters of crude polysaccharide solution still containing substance C.

This polysaccharide can be isolated by dissolving 40 g of sodium acetate trihydrate in the solution and then adding with stirring 3.5 volumes of ethanol, causing the precipitation of the polysaccharide of serotype 1 which can be separated by filtration.

Purification of polysaccharide

Alternatively and preferably, a purification of the polysaccharide solution is carried out. To this end, 34 g of sodium acetate trihydrate is dissolved to obtain a saline solution (called solution C) whose concentration (said concentration C) in sodium acetate is 0.05 N, the pH is adjusted to 5.2 (±0.1) with 1 N hydrochloric acid and the medium is clarified by successive filtrations on 3 membranes whose porosity is 3, 1 and 0.45 micron, respectively.

The polysaccharide is precipitated onto 250 g of filter medium (Celite 512) whith stirring and by adding 500 ml (said volume D) of cetrimide aqueous solution of 5% (w/v), stirring being maintained for 15 minutes after addition of cetrimide.

The mixture is then filtered over a Celite 512 precoat.

The precipitate is purified chromatographically with 10 L of an aqueous solution of sodium acetate trihydrate

(concentration C) with additionally 100 ml (said volume E) of aqueous cetrimide solution of 5% (w/v).

The cake is resuspended in 2 liters of an 0.3 N aqueous solution (said eluent F) of sodium acetate. After 15 minutes stirring, the suspension is filtered and the cake is purified chromatographically with 2 liters of eluent F.

Both eluates are filtered through a glass fiber prefilter followed by two membrane filters respectively having a porosity of 0.8 and 0.45 micron. sodium acetate trihydrate is added to obtain a concentration of 8% and the solution is refiltered through a membrane filter with a porosity of 0.2 micron.

The purified serotype 1 polysaccharide is precipitated from the solution by adding 3 volumes of ethanol.

After settling, the supernatant solution is separated and the precipitate is triturated in ethanol until a homogeneous powder is obtained which is filtered and dried under reduced pressure at room temperature.

The resulting product is purified pneumococcal serotype 1 polysaccharide.

Sizing

Purified polysaccharide of *S. pneumoniae* serotype 1 was dissolved in water to a concentration of 2 - 3 mg/ml. The dissolved polysaccharide was passed through a mechanical homogenizer with pressure preset from 0-1000 bar. Subsequently, the saccharide was concentrated and diafiltered with sterile water on a 10 kDa MWCO ultrafilter. The permeate was discarded and the retentate was transferred to dialysis tubing (12,000 MW cutoff; 45 mm) and dialyzed vs. distilled $H_2O$ for 27 hours with 2 additional changes of distilled $H_2O$. Then the dialyzed sample was transferred to lyophilization flasks, shell-frozen in a dry ice-methanol bath and lyophilized for 2-1/2 days

Conjugation with Protein D

A 2.5 mg/ml solution of sized polysaccharide of S. *pneumoniae* serotype 1 in water for injection was prepared. To that solution a 100 mg/ml solution of CDAP in acetonitrile/water (1:1 v/v) was added until a ratio of CDAP to polysaccharide of 0.5 (w/w) was reached. 1 minute later the pH was adjusted to 9.0 by adding 0.25 M sodium hydroxide solution under stirring at 25 °C. After 3 minutes a 10 mg/ml solution of protein D in 0.2 M aqueous sodium chloride was added until a ratio of protein D/polysaccharide of 1.5 : 1 was reached. The reaction mixture was stirred for two hours while the pH was maintained at 9.0. Excess amount of 2 M glycine solution in water for injection was added to quench unreacted cyanate ester groups. The pH was readjusted to 9.0 and the solution was stirred for 30 minutes at 25 °C and afterwards overnight at 2-8 °C.

The S. *pneumoniae* serotype 1/PD-conjugate was purified by gel filtration using a Sehacryl S400HR gel filtration column equilibrated with 0.15 sodium chloride (S500HR for 18C) to remove small molecules, salts and unconjugated polysaccharide and protein D. The conjugate eluted first, followed by the polysaccharide and free protein D. Fractions containing the conjugate were detected by UV spectroscopy at 280 mm, combined, sterile filtered (22 μm) and stored between 2-8 °C.

Characterization of *S. pneumoniae* serotype 1/PD-conjugate

The polysaccharide content was measured by the Resorcinol test and the protein content of the conjugate by the Lowry test. Consequently the polysaccharide-protein-ratio was determined from the ratio of the protein content and the polysaccharide content. The molecular weight distribution was measured on a HPLC-SEC gel filtration.

**Example G.2. S. *pneumoniae* serotype 4 polysaccharide-protein D-conjugate**

The purified pneumococcal serotype 4 polysaccharide is obtained by the same method as the pneumococcal serotype 1 polysaccharide using a strain of *Streptococcus pneumoniae* producing capsular polysaccharide of serotype 4.

Fermentation

A sample of a strain of *Streptococcus pneumoniae* producing capsular polysaccharide of serotype 4 stored at -70 °C in Tryptic Soy Broth supplemented with 10% glycerol is rapidly thawed. The organisms are collected using a platinum loop and transferred to a Petri dish containing 15 ml of culture medium consisting of 40 g of Blood agar base per liter of water, sterilized at 121 °C for 20 minutes and then cooled to 50 °C and supplemented with 5% of fresh defibrinated sheep blood and the microorganism is then incubated at 37 °C for 16 hours in atmosphere enriched with $CO_2$ (5% w/v).

The produced bacteria are collected and a second run is performed in a Roux culture bottle containing 100 ml of synthetic medium according Hoeprich (Paul D. Hoeprich J. Bact. 1955, 69, p. 682-688, and J. Bact. 1957, 74, p. 587-590), solidified by adding agar (20 g/l). After an incubation period of 6 to 9 hours in anaerobic atmosphere, the

produced organism is harvested and suspended in 5 ml of aqueous sodium chloride solution (8.5 g per liter). The harvest is used to inoculate a culture vessel containing 8 liters of liquid semi-synthetic medium according to Hoeprich. The medium is maintained without agitation or aeration for 16 hours at 37 °C to form the inoculum for the production culture.

For the production culture, a 100 liter fermenter is used containing 72 liters of semi-synthetic medium following Hoeprich. The medium is inoculated with the above-referenced culture and the incubation is maintained without aeration but gentle stirring at 35.5 °C (±1), the pH being continuously adjusted to 7.2 using a 25% sodium hydroxide solution. The development of the culture is regularly monitored by sampling and measuring the optical density of the medium at 620 nm. When the optical density reaches 0.15 a solution of one kilogram of dextrose in 2 liters of autoclaved deionized water is added over 20 minutes at 121 °C. The culture is stopped at the beginning of stationary phase, which corresponds to an optical density of 0.8.

Extraction of polysaccharide

The fermentation medium (80 l) is transferred into a storage tank and inactivated by adding one liter of 90% phenol; the pH is adjusted to 7.2 with 25% sodium hydroxide solution. After standing overnight at 5 - 10 °C, 702 g of sodium chloride are dissolved, which corresponds to an addition of 0.15 mol of sodium chloride per liter of medium (so-called concentration A).

While stirring, 4 kg of inert porous support (Celite 545) are then added and to the suspension, 800 ml (said volume B) of an aqueous solution of cetrimide (5% w/v) which is previously stored at a temperature of 25 °C is added and the stirring is continued for 10 minutes after the addition of cetrimide.

The medium is then filtered and the cake is washed with 4 L of an aqueous solution of sodium chloride of concentration A, with additional 40 ml of an aqueous solution of cetrimide (5% w/v).

The filtrate is concentrated to a volume of 5 liters by ultrafiltration in an AMICON DC unit 30 provided with cartridges with a cut-off of molecular weight of 100 kDa. The retentate is diafiltrated first with 40 L of sodium chloride solution of concentration A and then with 40 L of water keeping the volume between 10 and 5 liters. Finally, the volume of the solution is reduced to about 3 liters by ultrafiltration and the apparatus is rinsed with water to obtain approximately 5 liters of crude polysaccharide solution still containing substance C.

This polysaccharide can be isolated by dissolving 40 g of sodium acetate trihydrate in the solution and then adding with stirring 3.5 volumes of ethanol, causing the precipitation of the polysaccharide of serotype 4 which can be separated by filtration.

Purification of polysaccharide

Alternatively and preferably, a purification of the polysaccharide solution is carried out. To this end, 17 g of sodium acetate trihydrate is dissolved to obtain a saline solution (called solution C) whose concentration (said concentration C) in sodium acetate is 0.025 N, the pH is adjusted to 5.2 (±0.1) with 1 N hydrochloric acid and the medium is clarified by successive filtrations on 3 membranes whose porosity is 3, 1 and 0.45 micron, respectively.

The polysaccharide is precipitated onto 250 g of filter medium (Celite 512) whith stirring and by adding 500 ml (said volume D) of cetrimide aqueous solution of 5% (w/v), stirring being maintained for 15 minutes after addition of cetrimide.

The mixture is then filtered over a Celite 512 precoat.

The precipitate is purified chromatographically with 10 L of an aqueous solution of sodium acetate trihydrate (concentration C) with additionally 100 ml (said volume E) of aqueous cetrimide solution of 5% (w/v).

The cake is resuspended in 2 liters of a 0.15 N aqueous solution (said eluent F) of sodium acetate. After 15 minutes stirring, the suspension is filtered and the cake is purified chromatographically with 2 liters of eluent F.

Both eluates are filtered through a glass fiber prefilter followed by two membrane filters respectively having a porosity of 0.8 and 0.45 micron. sodium acetate trihydrate is added to obtain a concentration of 8% and the solution is refiltered through a membrane filter with a porosity of 0.2 micron.

The purified serotype 4 polysaccharide is precipitated from the solution by adding 4 volumes of ethanol.

After settling, the supernatant solution is separated and the precipitate is triturated in ethanol until a homogeneous powder is obtained which is filtered and dried under reduced pressure at room temperature.

The resulting product is purified pneumococcal serotype 4 polysaccharide.

Sizing

Purified polysaccharide of S. *pneumoniae* serotype 4 was dissolved in water to a concentration of 2 - 3 mg/ml. The dissolved polysaccharide was passed through a mechanical homogenizer with pressure preset from 0-1000 bar. Subsequently, the saccharide was concentrated and diafiltered with sterile water on a 10 kDa MWCO ultrafilter. The

permeate was discarded and the retentate was adjusted to a pH of 5.0 with a sodium acetate buffer with a final concentration of 100 mM. The solution was incubated at 50 ° ± 2 °C. Hydrolysis was stopped by cooling to 20 - 24 °C. The solution was transferred to dialysis tubing (12,000 MW cutoff; 45 mm) and dialyzed vs. distilled $H_2O$ for 27 hours with 2 additional changes of distilled $H_2O$. Then the dialyzed sample was transferred to lyophilization flasks, shell-frozen in a dry ice-methanol bath and lyophilized for 2-1/2 days

Conjugation with Protein D

A 2.5 mg/ml solution of sized polysaccharide of S. *pneumoniae* serotype 4 in water for injection was prepared. To that solution a 100 mg/ml solution of CDAP in acetonitrile/water (1:1 v/v) was added until a ratio of CDAP to polysaccharide of 0.5 (w/w) was reached. 1 minute later the pH was adjusted to 9.5 by adding 0.25 M sodium hydroxide solution under stirring at 25 °C. After 3 minutes a 10 mg/ml solution of protein D in 0.2 M aqueous sodium chloride was added until a ratio of protein D/polysaccharide of 1.5 : 1 was reached. The reaction mixture was stirred for two hours while the pH was maintained at 9.5. Excess amount of 2 M glycine solution in water for injection was added to quench unreacted cyanate ester groups. The pH was readjusted to 9.0 and the solution was stirred for 30 minutes at 25 °C and afterwards overnight at 2-8 °C.

The S. *pneumoniae* serotype 4/PD-conjugate was purified by gel filtration using a Sehacryl S400HR gel filtration column equilibrated with 0.15 sodium chloride (S500HR for 18C) to remove small molecules, salts and unconjugated polysaccharide and protein D. The conjugate eluted first, followed by the polysaccharide and free protein D. Fractions containing the conjugate were detected by UV spectroscopy at 280 mm, combined, sterile filtered (22 μm) and stored between 2-8 °C.

Characterization of *S. pneumoniae* serotype 4/PD-conjugate

The polysaccharide content was measured by the Resorcinol test and the protein content of the conjugate by the Lowry test. Consequently the polysaccharide-protein-ratio was determined from the ratio of the protein content and the polysaccharide content. The molecular weight distribution was measured on a HPLC-SEC gel filtration.

**Example G.3. *S. pneumoniae* serotype 5 polysaccharide-protein D-conjugate**

The purified pneumococcal serotype 5 polysaccharide is obtained by the same method as the pneumococcal serotype 4 polysaccharide using a strain of *Streptococcus pneumoniae* producing capsular polysaccharide of serotype 5.

Fermentation

A sample of a strain of *Streptococcus pneumoniae* producing capsular polysaccharide of serotype 5 stored at -70 °C in Tryptic Soy Broth supplemented with 10% glycerol is rapidly thawed. The organisms are collected using a platinum loop and transferred to a Petri dish containing 15 ml of culture medium consisting of 40 g of Blood agar base per liter of water, sterilized at 121 °C for 20 minutes and then cooled to 50 °C and supplemented with 5% of fresh defibrinated sheep blood and the microorganism is then incubated at 37 °C for 16 hours in atmosphere enriched with $CO_2$ (5% w/v).

The produced bacteria are collected and a second run is performed in a Roux culture bottle containing 100 ml of synthetic medium according Hoeprich (Paul D. Hoeprich J. Bact. 1955, 69, p. 682-688, and J. Bact. 1957, 74, p. 587-590), solidified by adding agar (20 g/l). After an incubation period of 6 to 9 hours in anaerobic atmosphere, the produced organism is harvested and suspended in 5 ml of aqueous sodium chloride solution (8.5 g per liter). The harvest is used to inoculate a culture vessel containing 8 liters of liquid semi-synthetic medium according to Hoeprich. The medium is maintained without agitation or aeration for 16 hours at 37 °C to form the inoculum for the production culture.

For the production culture, a 100 liter fermenter is used containing 72 liters of semi-synthetic medium following Hoeprich. The medium is inoculated with the above-referenced culture and the incubation is maintained without aeration but gentle stirring at 35.5 °C (±1), the pH being continuously adjusted to 7.2 using a 25% sodium hydroxide solution. The development of the culture is regularly monitored by sampling and measuring the optical density of the medium at 620 nm. When the optical density reaches 0.15 a solution of one kilogram of dextrose in 2 liters of autoclaved deionized water is added over 20 minutes at 121 °C. The culture is stopped at the beginning of stationary phase, which corresponds to an optical density of 0.8.

Extraction of polysaccharide

The fermentation medium (80 l) is transferred into a storage tank and inactivated by adding one liter of 90%

phenol; the pH is adjusted to 7.2 with 25% sodium hydroxide solution. After standing overnight at 5 - 10 °C, 702 g of sodium chloride are dissolved, which corresponds to an addition of 0.15 mol of sodium chloride per liter of medium (so-called concentration A).

While stirring, 4 kg of inert porous support (Celite 545) are then added and to the suspension, 800 ml (said volume B) of an aqueous solution of cetrimide (5% w/v) which is previously stored at a temperature of 25 °C is added and the stirring is continued for 10 minutes after the addition of cetrimide.

The medium is then filtered and the cake is washed with 4 L of an aqueous solution of sodium chloride of concentration A, with additional 40 ml of an aqueous solution of cetrimide (5% w/v).

The filtrate is concentrated to a volume of 5 liters by ultrafiltration in an AMICON DC unit 30 provided with cartridges with a cut-off of molecular weight of 100 kDa. The retentate is diafiltrated first with 40 L of sodium chloride solution of concentration A and then with 40 L of water keeping the volume between 10 and 5 liters. Finally, the volume of the solution is reduced to about 3 liters by ultrafiltration and the apparatus is rinsed with water to obtain approximately 5 liters of crude polysaccharide solution still containing substance C.

This polysaccharide can be isolated by dissolving 40 g of sodium acetate trihydrate in the solution and then adding with stirring 3.5 volumes of ethanol, causing the precipitation of the polysaccharide of serotype 5 which can be separated by filtration.

Purification of polysaccharide

Alternatively and preferably, a purification of the polysaccharide solution is carried out. To this end, 17 g of sodium acetate trihydrate is dissolved to obtain a saline solution (called solution C) whose concentration (said concentration C) in sodium acetate is 0.025 N, the pH is adjusted to 5.0 (±0.1) with 1 N hydrochloric acid and the medium is clarified by successive filtrations on 3 membranes whose porosity is 3, 1 and 0.45 micron, respectively.

The polysaccharide is precipitated onto 250 g of filter medium (Celite 512) whith stirring and by adding 500 ml (said volume D) of cetrimide aqueous solution of 5% (w/v), stirring being maintained for 15 minutes after addition of cetrimide.

The mixture is then filtered over a Celite 512 precoat.

The precipitate is purified chromatographically with 10 L of an aqueous solution of sodium acetate trihydrate (concentration C) with additionally 100 ml (said volume E) of aqueous cetrimide solution of 5% (w/v).

The cake is resuspended in 2 liters of a 0.15 N aqueous solution (said eluent F) of sodium chloride. After 15 minutes stirring, the suspension is filtered and the cake is purified chromatographically with 2 liters of eluent F.

Both eluates are filtered through a glass fiber prefilter followed by two membrane filters respectively having a porosity of 0.8 and 0.45 micron. sodium acetate trihydrate is added to obtain a concentration of 8% and the solution is refiltered through a membrane filter with a porosity of 0.2 micron.

The purified serotype 5 polysaccharide is precipitated from the solution by adding 3 volumes of n-propanol.

After settling, the supernatant solution is separated and the precipitate is triturated in ethanol until a homogeneous powder is obtained which is filtered and dried under reduced pressure at room temperature.

The resulting product is purified pneumococcal serotype 5 polysaccharide.

Conjugation with Protein D

A 7.1 mg/ml solution of polysaccharide of S. *pneumoniae* serotype 5 in water for injection was prepared. To that solution a 100 mg/ml solution of CDAP in acetonitrile/water (1:1 v/v) was added until a ratio of CDAP to polysaccharide of 0.79 (w/w) was reached. 1 minute later the pH was adjusted to 9.0 by adding 0.25 M sodium hydroxide solution under stirring at 25 °C. After 3 minutes a 5.0 mg/ml solution of protein D in 0.2 M aqueous sodium chloride was added until a ratio of protein D/polysaccharide of 1 : 1 was reached. The reaction mixture was stirred for two hours while the pH was maintained at 9.0. Excess amount of 2 M glycine solution in water for injection was added to quench unreacted cyanate ester groups. The pH was readjusted to 9.0 and the solution was stirred for 30 minutes at 25 °C and afterwards overnight at 2-8 °C.

The *S. pneumoniae* serotype 5/PD-conjugate was purified by gel filtration using a Sehacryl S400HR gel filtration column equilibrated with 0.15 sodium chloride (S500HR for 18C) to remove small molecules, salts and unconjugated polysaccharide and protein D. The conjugate eluted first, followed by the polysaccharide and free protein D. Fractions containing the conjugate were detected by UV spectroscopy at 280 mm, combined, sterile filtered (22 μm) and stored between 2-8 °C.

Characterization of *S. pneumoniae* serotype 5/PD-conjugate

The polysaccharide content was measured by the Resorcinol test and the protein content of the conjugate by

the Lowry test. Consequently the polysaccharide-protein-ratio was determined from the ratio of the protein content and the polysaccharide content. The molecular weight distribution was measured on a HPLC-SEC gel filtration.

**Example G.4. *S. pneumoniae* serotype 6B polysaccharide-protein D-conjugate**

The purified pneumococcal serotype 6B polysaccharide is obtained by the same method as the pneumococcal serotype 4 polysaccharide using a strain of *Streptococcus pneumoniae* producing capsular polysaccharide of serotype 6B.

Fermentation

A sample of a strain of *Streptococcus pneumoniae* producing capsular polysaccharide of serotype 6B stored at -70 °C in Tryptic Soy Broth supplemented with 10% glycerol is rapidly thawed. The organisms are collected using a platinum loop and transferred to a Petri dish containing 15 ml of culture medium consisting of 40 g of Blood agar base per liter of water, sterilized at 121 °C for 20 minutes and then cooled to 50 °C and supplemented with 5% of fresh defibrinated sheep blood and the microorganism is then incubated at 37 °C for 16 hours in atmosphere enriched with $CO_2$ (5% w/v).

The produced bacteria are collected and a second run is performed in a Roux culture bottle containing 100 ml of synthetic medium according Hoeprich (Paul D. Hoeprich J. Bact. 1955, 69, p. 682-688, and J. Bact. 1957, 74, p. 587-590), solidified by adding agar (20 g/l). After an incubation period of 6 to 9 hours in anaerobic atmosphere, the produced organism is harvested and suspended in 5 ml of aqueous sodium chloride solution (8.5 g per liter). The harvest is used to inoculate a culture vessel containing 8 liters of liquid semi-synthetic medium according to Hoeprich. The medium is maintained without agitation or aeration for 16 hours at 37 °C to form the inoculum for the production culture.

For the production culture, a 100 liter fermenter is used containing 72 liters of semi-synthetic medium following Hoeprich. The medium is inoculated with the above-referenced culture and the incubation is maintained without aeration but gentle stirring at 35.5 °C (±1), the pH being continuously adjusted to 7.2 using a 25% sodium hydroxide solution. The development of the culture is regularly monitored by sampling and measuring the optical density of the medium at 620 nm. When the optical density reaches 0.15 a solution of one kilogram of dextrose in 2 liters of autoclaved deionized water is added over 20 minutes at 121 °C. The culture is stopped at the beginning of stationary phase, which corresponds to an optical density of 0.8.

Extraction of polysaccharide

The fermentation medium (80 l) is transferred into a storage tank and inactivated by adding one liter of 90% phenol; the pH is adjusted to 7.2 with 25% sodium hydroxide solution. After standing overnight at 5 - 10 °C, 702 g of sodium chloride are dissolved, which corresponds to an addition of 0.15 mol of sodium chloride per liter of medium (so-called concentration A).

While stirring, 4 kg of inert porous support (Celite 545) are then added and to the suspension, 800 ml (said volume B) of an aqueous solution of cetrimide (5% w/v) which is previously stored at a temperature of 25 °C is added and the stirring is continued for 10 minutes after the addition of cetrimide.

The medium is then filtered and the cake is washed with 4 L of an aqueous solution of sodium chloride of concentration A, with additional 40 ml of an aqueous solution of cetrimide (5% w/v).

The filtrate is concentrated to a volume of 5 liters by ultrafiltration in an AMICON DC unit 30 provided with cartridges with a cut-off of molecular weight of 100 kDa. The retentate is diafiltrated first with 40 L of sodium chloride solution of concentration A and then with 40 L of water keeping the volume between 10 and 5 liters. Finally, the volume of the solution is reduced to about 3 liters by ultrafiltration and the apparatus is rinsed with water to obtain approximately 5 liters of crude polysaccharide solution still containing substance C.

This polysaccharide can be isolated by dissolving 40 g of sodium acetate trihydrate in the solution and then adding with stirring 3.5 volumes of ethanol, causing the precipitation of the polysaccharide of serotype 6B which can be separated by filtration.

Purification of polysaccharide

Alternatively and preferably, a purification of the polysaccharide solution is carried out. To this end, 34 g of sodium acetate trihydrate is dissolved to obtain a saline solution (called solution C) whose concentration (said concentration C) in sodium acetate is 0.05 N, the pH is adjusted to 5.2 (±0.1) with 1 N hydrochloric acid and the medium is clarified by successive filtrations on 3 membranes whose porosity is 3, 1 and 0.45 micron, respectively.

The polysaccharide is precipitated onto 250 g of filter medium (Celite 512) whith stirring and by adding 1000

ml (said volume D) of cetrimide aqueous solution of 5% (w/v), stirring being maintained for 15 minutes after addition of cetrimide.

The mixture is then filtered over a Celite 512 precoat.

The precipitate is purified chromatographically with 10 L of an aqueous solution of sodium acetate trihydrate (concentration C) with additionally 1000 ml (said volume E) of aqueous cetrimide solution of 5% (w/v).

The cake is resuspended in 2 liters of a 0.25 N aqueous solution (said eluent F) of sodium acetate. After 15 minutes stirring, the suspension is filtered and the cake is purified chromatographically with 2 liters of eluent F.

Both eluates are filtered through a glass fiber prefilter followed by two membrane filters respectively having a porosity of 0.8 and 0.45 micron. sodium acetate trihydrate is added to obtain a concentration of 8% and the solution is refiltered through a membrane filter with a porosity of 0.2 micron.

The purified serotype 6B polysaccharide is precipitated from the solution by adding 3 volumes of ethanol.

After settling, the supernatant solution is separated and the precipitate is triturated in ethanol until a homogeneous powder is obtained which is filtered and dried under reduced pressure at room temperature.

The resulting product is purified pneumococcal serotype 6B polysaccharide.

Conjugation with Protein D

A 5.0 mg/ml solution of polysaccharide of S. *pneumoniae* serotype 6B and 2 M aqueous sodium chloride solution was prepared. To that solution a 100 mg/ml solution of CDAP in acetonitrile/water (1:1 v/v) was added until a ratio of CDAP to polysaccharide of 0.83 (w/w) was reached. 1 minute later the pH was adjusted to 9.5 by adding 0.25 M sodium hydroxide solution under stirring at 25 °C. After 3 minutes a 5 mg/ml solution of protein D in 0.2 M aqueous sodium chloride was added until a ratio of protein D/polysaccharide of 1.1 : 1 was reached. The reaction mixture was stirred for two hours while the pH was maintained at 9.5. Excess amount of 2 M glycine solution in water for injection was added to quench unreacted cyanate ester groups. The pH was readjusted to 9.0 and the solution was stirred for 30 minutes at 25 °C and afterwards overnight at 2-8 °C.

The *S. pneumoniae* serotype 6B/PD-conjugate was purified by gel filtration using a Sehacryl S400HR gel filtration column equilibrated with 0.15 sodium chloride (S500HR for 18C) to remove small molecules, salts and unconjugated polysaccharide and protein D. The conjugate eluted first, followed by the polysaccharide and free protein D. Fractions containing the conjugate were detected by UV spectroscopy at 280 mm, combined, sterile filtered (22 μm) and stored between 2-8 °C.

Characterization of *S. pneumoniae* serotype 6B/PD-conjugate

The polysaccharide content was measured by the Resorcinol test and the protein content of the conjugate by the Lowry test. Consequently the polysaccharide-protein-ratio was determined from the ratio of the protein content and the polysaccharide content. The molecular weight distribution was measured on a HPLC-SEC gel filtration.

### Example G.5. *S. pneumoniae* serotype 7F polysaccharide-protein D-conjugate

The crude pneumococcal serotype 7F polysaccharide is obtained by the same method as the pneumococcal serotype 4 polysaccharide using a strain of *Streptococcus pneumoniae* producing capsular polysaccharide of serotype 7F.

Fermentation

A sample of a strain of *Streptococcus pneumoniae* producing capsular polysaccharide of serotype 7F stored at -70 °C in Tryptic Soy Broth supplemented with 10% glycerol is rapidly thawed. The organisms are collected using a platinum loop and transferred to a Petri dish containing 15 ml of culture medium consisting of 40 g of Blood agar base per liter of water, sterilized at 121 °C for 20 minutes and then cooled to 50 °C and supplemented with 5% of fresh defibrinated sheep blood and the microorganism is then incubated at 37 °C for 16 hours in atmosphere enriched with $CO_2$ (5% w/v).

The produced bacteria are collected and a second run is performed in a Roux culture bottle containing 100 ml of synthetic medium according Hoeprich (Paul D. Hoeprich J. Bact. 1955, 69, p. 682-688, and J. Bact. 1957, 74, p. 587-590), solidified by adding agar (20 g/l). After an incubation period of 6 to 9 hours in anaerobic atmosphere, the produced organism is harvested and suspended in 5 ml of aqueous sodium chloride solution (8.5 g per liter). The harvest is used to inoculate a culture vessel containing 8 liters of liquid semi-synthetic medium according to Hoeprich. The medium is maintained without agitation or aeration for 16 hours at 37 °C to form the inoculum for the production culture.

For the production culture, a 100 liter fermenter is used containing 72 liters of semi-synthetic medium following Hoeprich. The medium is inoculated with the above-referenced culture and the incubation is maintained without aeration

but gentle stirring at 35.5 °C (±1), the pH being continuously adjusted to 7.2 using a 25% sodium hydroxide solution. The development of the culture is regularly monitored by sampling and measuring the optical density of the medium at 620 nm. When the optical density reaches 0.15 a solution of one kilogram of dextrose in 2 liters of autoclaved deionized water is added over 20 minutes at 121 °C. The culture is stopped at the beginning of stationary phase, which corresponds to an optical density of 0.8.

Extraction of polysaccharide

The fermentation medium (80 l) is transferred into a storage tank and inactivated by adding one liter of 90% phenol; the pH is adjusted to 7.2 with 25% sodium hydroxide solution. After standing overnight at 5 - 10 °C, 702 g of sodium chloride are dissolved, which corresponds to an addition of 0.15 mol of sodium chloride per liter of medium (so-called concentration A).

While stirring, 4 kg of inert porous support (Celite 545) are then added and to the suspension, 800 ml (said volume B) of an aqueous solution of cetrimide (5% w/v) which is previously stored at a temperature of 25 °C is added and the stirring is continued for 10 minutes after the addition of cetrimide.

The medium is then filtered and the cake is washed with 4 L of an aqueous solution of sodium chloride of concentration A, with additional 40 ml of an aqueous solution of cetrimide (5% w/v).

The filtrate is concentrated to a volume of 5 liters by ultrafiltration in an AMICON DC unit 30 provided with cartridges with a cut-off of molecular weight of 100 kDa. The retentate is diafiltrated first with 40 L of sodium chloride solution of concentration A and then with 40 L of water keeping the volume between 10 and 5 liters. Finally, the volume of the solution is reduced to about 3 liters by ultrafiltration and the apparatus is rinsed with water to obtain approximately 5 liters of crude polysaccharide solution still containing substance C.

This polysaccharide can be isolated by dissolving 40 g of sodium acetate trihydrate in the solution and then adding with stirring 3.5 volumes of ethanol, causing the precipitation of the polysaccharide of serotype 7F which can be separated by filtration.

Purification of polysaccharide

This precipitate is redissolved in an amount of 4 g/L in a 0.005 N trometamol/hydrochloric acid buffer solution at pH 7.1. The solution was clarified by centrifugation for 10 minutes at 3000 tours par minute.

Under stirring, for each liter of the solution 20 g of diethylaminoethyl cellulose (DEAE cellulose) treated with the same buffer solution are added. The pH is adjusted to 7.1 with 1 N hydrochloric acid and stirring is continued for one hour after the addition of DEAE cellulose which is then separated by filtration and washed with 240 ml of the same buffer solution per liter of filtrate.

The Treatment of the filtrate with DEAE cellulose is repeated.

The combined filtrates are passed through two membrane filters having a porosity of 0.8 micron and 0.45 micron, respectively. Sodium acetate trihydrate is added (so that the final concentration is 8% w/v) and the solution is passed through a membrane filter having a porosity of 0.2 micron.

The purified polysaccharide is precipitated by adding 3 volumes of ethanol under stirring.

Precipitates of the purified pneumococcal serotype 7F polysaccharide are isolated by decantation followed by filtration and further washed with ethanol and finally dried at room temperature under reduced pressure.

Sizing

The purified pneumococcal serotype 7F polysaccharide powder was solubilized in distilled $H_2O$ with stirring at room temperature for about 4 hours and then stored at 4 °C overnight. The solution was added to DE52 (Whatman, diethylaminoethyl cellulose) which had been pre-swollen for ca. 15 hours in distilled $H_2O$ at pH of ca. 5-6. The slurry was gently shaken on a platform shaker at room temperature for ca. 15 hrs, after which it was centrifuged at 5,000 rpm for 15 min. at 20 °C. The supernatant fluid was further clarified through a sinter glass funnel (150 ml, medium porosity) and collected into a 2L side arm flask.

The DE52-treated pneumococcal serotype 7F polysaccharide was sonicated in a plastic beaker on an ice bath with a Branson Sonifier (one-half inch probe, setting 8) for 2 min. The sample was allowed to cool for ca. 15 min. while the viscosity was determined and then was sonicated for additional 1 min. intervals. A viscosity end point of 1.096 centistokes was reached after the last sonic treatment. The hydrolyzed sample was brought to room temperature and sodium acetate reagent was added to a final concentration of 1 % (w/v).

The hydrolyzed sample was brought to 39.3% isopropanol by the addition of isopropanol (added dropwise with stirring at room temperature). The sample was allowed to stir for 15-30 minutes and then centrifuged at 11,000×g for 30 minutes (Beckman JA-10 rotor; 8,000 rpm; 20 °C) and the supernatant fluid decanted. The waste pellet was triturated

with absolute EtOH in a 250-mL Omnimix jar, then collected on a 60-mL sinter glass funnel. The precipitate was washed directly on the funnel with absolute EtOH, then acetone, and dried in vacuo over $CaCl_2$ at room temperature in preparation for analysis.

The supernatant fluid was brought to 41.8% isopropanol by adding further isopropanol dropwise while stirring at room temperature. The sample was aged and centrifuged as described above. The pellet was triturated, collected, washed and dried as described above. The pellet was solubilized in distilled $H_2O$ at room temperature for 2-3 hours. The solution (2.5 mg/mL) was transferred to dialysis tubing (12,000 MW cutoff; 45 mm) and dialyzed vs. distilled $H_2O$ for 27 hours with 2 additional changes of distilled $H_2O$. Then the dialyzed sample was transferred to lyophilization flasks, shell-frozen in a dry ice-methanol bath and lyophilized for 2-1/2 days.

Conjugation with Protein D

A 5.0 mg/ml solution of sized polysaccharide of S. *pneumoniae* serotype 7F and 2 M aqueous sodium chloride solution was prepared. To that solution a 100 mg/ml solution of CDAP in acetonitrile/water (1:1 v/v) was added until a ratio of CDAP to polysaccharide of 0.75 (w/w) was reached. 1 minute later the pH was adjusted to 9.5 by adding 0.25 M sodium hydroxide solution under stirring at 25 °C. After 3 minutes a 10 mg/ml solution of protein D in 0.2 M aqueous sodium chloride was added until a ratio of protein D/polysaccharide of 1.2 : 1 was reached. The reaction mixture was stirred for two hours while the pH was maintained at 9.5. Excess amount of 2 M glycine solution in water for injection was added to quench unreacted cyanate ester groups. The pH was readjusted to 9.0 and the solution was stirred for 30 minutes at 25 °C and afterwards overnight at 2-8 °C.

The *S. pneumoniae* serotype 7F/PD-conjugate was purified by gel filtration using a Sehacryl S400HR gel filtration column equilibrated with 0.15 sodium chloride (S500HR for 18C) to remove small molecules, salts and unconjugated polysaccharide and protein D. The conjugate eluted first, followed by the polysaccharide and free protein D. Fractions containing the conjugate were detected by UV spectroscopy at 280 mm, combined, sterile filtered (22 μm) and stored between 2-8 °C.

Characterization of *S. pneumoniae* serotype 7F/PD-conjugate

The polysaccharide content was measured by the Resorcinol test and the protein content of the conjugate by the Lowry test. Consequently the polysaccharide-protein-ratio was determined from the ratio of the protein content and the polysaccharide content. The molecular weight distribution was measured on a HPLC-SEC gel.

**Example G.6. *S. pneumoniae* serotype 9V polysaccharide-protein D-conjugate**

The purified pneumococcal serotype 9V polysaccharide is obtained by the same method as the pneumococcal serotype 4 polysaccharide using a strain of *Streptococcus pneumoniae* producing capsular polysaccharide of serotype 9V.

Fermentation

A sample of a strain of *Streptococcus pneumoniae* producing capsular polysaccharide of serotype 4 stored at -70 °C in Tryptic Soy Broth supplemented with 10% glycerol is rapidly thawed. The organisms are collected using a platinum loop and transferred to a Petri dish containing 15 ml of culture medium consisting of 40 g of Blood agar base per liter of water, sterilized at 121 °C for 20 minutes and then cooled to 50 °C and supplemented with 5% of fresh defibrinated sheep blood and the microorganism is then incubated at 37 °C for 16 hours in atmosphere enriched with $CO_2$ (5% w/v).

The produced bacteria are collected and a second run is performed in a Roux culture bottle containing 100 ml of synthetic medium according Hoeprich (Paul D. Hoeprich J. Bact. 1955, 69, p. 682-688, and J. Bact. 1957, 74, p. 587-590), solidified by adding agar (20 g/l). After an incubation period of 6 to 9 hours in anaerobic atmosphere, the produced organism is harvested and suspended in 5 ml of aqueous sodium chloride solution (8.5 g per liter). The harvest is used to inoculate a culture vessel containing 8 liters of liquid semi-synthetic medium according to Hoeprich. The medium is maintained without agitation or aeration for 16 hours at 37 °C to form the inoculum for the production culture.

For the production culture, a 100 liter fermenter is used containing 72 liters of semi-synthetic medium following Hoeprich. The medium is inoculated with the above-referenced culture and the incubation is maintained without aeration but gentle stirring at 35.5 °C (±1), the pH being continuously adjusted to 7.2 using a 25% sodium hydroxide solution. The development of the culture is regularly monitored by sampling and measuring the optical density of the medium at 620 nm. When the optical density reaches 0.15 a solution of one kilogram of dextrose in 2 liters of autoclaved deionized water is added over 20 minutes at 121 °C. The culture is stopped at the beginning of stationary phase, which corresponds

to an optical density of 0.8.

Extraction of polysaccharide

The fermentation medium (80 l) is transferred into a storage tank and inactivated by adding one liter of 90% phenol; the pH is adjusted to 7.2 with 25% sodium hydroxide solution. After standing overnight at 5 - 10 °C, 702 g of sodium chloride are dissolved, which corresponds to an addition of 0.15 mol of sodium chloride per liter of medium (so-called concentration A).

While stirring, 4 kg of inert porous support (Celite 545) are then added and to the suspension, 800 ml (said volume B) of an aqueous solution of cetrimide (5% w/v) which is previously stored at a temperature of 25 °C is added and the stirring is continued for 10 minutes after the addition of cetrimide.

The medium is then filtered and the cake is washed with 4 L of an aqueous solution of sodium chloride of concentration A, with additional 40 ml of an aqueous solution of cetrimide (5% w/v).

The filtrate is concentrated to a volume of 5 liters by ultrafiltration in an AMICON DC unit 30 provided with cartridges with a cut-off of molecular weight of 100 kDa. The retentate is diafiltrated first with 40 L of sodium chloride solution of concentration A and then with 40 L of water keeping the volume between 10 and 5 liters. Finally, the volume of the solution is reduced to about 3 liters by ultrafiltration and the apparatus is rinsed with water to obtain approximately 5 liters of crude polysaccharide solution still containing substance C.

This polysaccharide can be isolated by dissolving 40 g of sodium acetate trihydrate in the solution and then adding with stirring 3.5 volumes of ethanol, causing the precipitation of the polysaccharide of serotype 9V which can be separated by filtration.

Purification of polysaccharide

Alternatively and preferably, a purification of the polysaccharide solution is carried out. To this end, 17 g of sodium acetate trihydrate is dissolved to obtain a saline solution (called solution C) whose concentration (said concentration C) in sodium acetate is 0.025 N, the pH is adjusted to 5.2 (±0.1) with 1 N hydrochloric acid and the medium is clarified by successive filtrations on 3 membranes whose porosity is 3, 1 and 0.45 micron, respectively.

The polysaccharide is precipitated onto 250 g of filter medium (Celite 512) whith stirring and by adding 500 ml (said volume D) of cetrimide aqueous solution of 5% (w/v), stirring being maintained for 15 minutes after addition of cetrimide.

The mixture is then filtered over a Celite 512 precoat.

The precipitate is purified chromatographically with 10 L of an aqueous solution of sodium acetate trihydrate (concentration C) with additionally 100 ml (said volume E) of aqueous cetrimide solution of 5% (w/v).

The cake is resuspended in 2 liters of a 0.15 N aqueous solution (said eluent F) of sodium acetate. After 15 minutes stirring, the suspension is filtered and the cake is purified chromatographically with 2 liters of eluent F.

Both eluates are filtered through a glass fiber prefilter followed by two membrane filters respectively having a porosity of 0.8 and 0.45 micron. sodium acetate trihydrate is added to obtain a concentration of 8% and the solution is refiltered through a membrane filter with a porosity of 0.2 micron.

The purified serotype 9V polysaccharide is precipitated from the solution by adding 3 volumes of ethanol.

After settling, the supernatant solution is separated and the precipitate is triturated in ethanol until a homogeneous powder is obtained which is filtered and dried under reduced pressure at room temperature.

The resulting product is purified pneumococcal serotype 9V polysaccharide.

Sizing

Purified polysaccharide of S. *pneumoniae* serotype 9V was dissolved in water to a concentration of 2 - 3 mg/ml. The dissolved polysaccharide was passed through a mechanical homogenizer with pressure preset from 0-1000 bar. Subsequently, the saccharide was concentrated and diafiltered with sterile water on a 10 kDa MWCO ultrafilter. The permeate was discarded and the retentate was transferred to dialysis tubing (12,000 MW cutoff; 45 mm) and dialyzed vs. distilled $H_2O$ for 27 hours with 2 additional changes of distilled $H_2O$. Then the dialyzed sample was transferred to lyophilization flasks, shell-frozen in a dry ice-methanol bath and lyophilized for 2-1/2 days.

Conjugation with Protein D

A 5.0 mg/ml solution of sized polysaccharide of *S. pneumoniae* serotype 9V and 2 M aqueous sodium chloride solution was prepared. To that solution a 100 mg/ml solution of CDAP in acetonitrile/water (1:1 v/v) was added until a ratio of CDAP to polysaccharide of 0.50 (w/w) was reached. 1 minute later the pH was adjusted to 9.5 by adding 0.25

M sodium hydroxide solution under stirring at 25 °C. After 3 minutes a 10 mg/ml solution of protein D in 0.2 M aqueous sodium chloride was added until a ratio of protein D/polysaccharide of 1.2 : 1 was reached. The reaction mixture was stirred for two hours while the pH was maintained at 9.5.

Excess amount of 2 M glycine solution in water for injection was added to quench unreacted cyanate ester groups. The pH was readjusted to 9.0 and the solution was stirred for 30 minutes at 25 °C and afterwards overnight at 2-8 °C.

The *S. pneumoniae* serotype 9V/PD-conjugate was purified by gel filtration using a Sehacryl S400HR gel filtration column equilibrated with 0.15 sodium chloride (S500HR for 18C) to remove small molecules, salts and unconjugated polysaccharide and protein D. The conjugate eluted first, followed by the polysaccharide and free protein D. Fractions containing the conjugate were detected by UV spectroscopy at 280 mm, combined, sterile filtered (22 μm) and stored between 2-8 °C.

Characterization of *S. pneumoniae* serotype 9V/PD-conjugate

The polysaccharide content was measured by the Resorcinol test and the protein content of the conjugate by the Lowry test. Consequently the polysaccharide-protein-ratio was determined from the ratio of the protein content and the polysaccharide content. The molecular weight distribution was measured on a HPLC-SEC gel filtration.

**Example G.7. *S. pneumoniae* serotype 14 polysaccharide-protein D-conjugate**

The crude pneumococcal serotype 14 polysaccharide is obtained by the same method as the pneumococcal serotype 7F polysaccharide using a strain of *Streptococcus pneumoniae* producing capsular polysaccharide of serotype 14.

Fermentation

A sample of a strain of *Streptococcus pneumoniae* producing capsular polysaccharide of serotype 14 stored at -70 °C in Tryptic Soy Broth supplemented with 10% glycerol is rapidly thawed. The organisms are collected using a platinum loop and transferred to a Petri dish containing 15 ml of culture medium consisting of 40 g of Blood agar base per liter of water, sterilized at 121 °C for 20 minutes and then cooled to 50 °C and supplemented with 5% of fresh defibrinated sheep blood and the microorganism is then incubated at 37 °C for 16 hours in atmosphere enriched with $CO_2$ (5% w/v).

The produced bacteria are collected and a second run is performed in a Roux culture bottle containing 100 ml of synthetic medium according Hoeprich (Paul D. Hoeprich J. Bact. 1955, 69, p. 682-688, and J. Bact. 1957, 74, p. 587-590), solidified by adding agar (20 g/l). After an incubation period of 6 to 9 hours in anaerobic atmosphere, the produced organism is harvested and suspended in 5 ml of aqueous sodium chloride solution (8.5 g per liter). The harvest is used to inoculate a culture vessel containing 8 liters of liquid semi-synthetic medium according to Hoeprich. The medium is maintained without agitation or aeration for 16 hours at 37 °C to form the inoculum for the production culture.

For the production culture, a 100 liter fermenter is used containing 72 liters of semi-synthetic medium following Hoeprich. The medium is inoculated with the above-referenced culture and the incubation is maintained without aeration but gentle stirring at 35.5 °C (±1), the pH being continuously adjusted to 7.2 using a 25% sodium hydroxide solution. The development of the culture is regularly monitored by sampling and measuring the optical density of the medium at 620 nm. When the optical density reaches 0.15 a solution of one kilogram of dextrose in 2 liters of autoclaved deionized water is added over 20 minutes at 121 °C. The culture is stopped at the beginning of stationary phase, which corresponds to an optical density of 0.8.

Extraction of polysaccharide

The fermentation medium (80 l) is transferred into a storage tank and inactivated by adding one liter of 90% phenol; the pH is adjusted to 7.2 with 25% sodium hydroxide solution. After standing overnight at 5 - 10 °C, 702 g of sodium chloride are dissolved, which corresponds to an addition of 0.15 mol of sodium chloride per liter of medium (so-called concentration A).

While stirring, 4 kg of inert porous support (Celite 545) are then added and to the suspension, 800 ml (said volume B) of an aqueous solution of cetrimide (5% w/v) which is previously stored at a temperature of 25 °C is added and the stirring is continued for 10 minutes after the addition of cetrimide.

The medium is then filtered and the cake is washed with 4 L of an aqueous solution of sodium chloride of concentration A, with additional 40 ml of an aqueous solution of cetrimide (5% w/v).

The filtrate is concentrated to a volume of 5 liters by ultrafiltration in an AMICON DC unit 30 provided with

cartridges with a cut-off of molecular weight of 100 kDa. The retentate is diafiltrated first with 40 L of sodium chloride solution of concentration A and then with 40 L of water keeping the volume between 10 and 5 liters. Finally, the volume of the solution is reduced to about 3 liters by ultrafiltration and the apparatus is rinsed with water to obtain approximately 5 liters of crude polysaccharide solution still containing substance C.

This polysaccharide can be isolated by dissolving 40 g of sodium acetate trihydrate in the solution and then adding with stirring 3.5 volumes of ethanol, causing the precipitation of the polysaccharide of serotype 14 which can be separated by filtration.

Purification of polysaccharide

This precipitate is redissolved in an amount of 4 g/L in a 0.005 N trometamol/hydrochloric acid buffer solution at pH 7.1. The solution was clarified by centrifugation for 10 minutes at 3000 tours par minute.

Under stirring, for each liter of the solution 20 g of diethylaminoethyl cellulose (DEAE cellulose) treated with the same buffer solution are added. The pH is adjusted to 7.1 with 1 N hydrochloric acid and stirring is continued for one hour after the addition of DEAE cellulose which is then separated by filtration and washed with 240 ml of the same buffer solution per liter of filtrate.

The Treatment of the filtrate with DEAE cellulose is repeated.

The combined filtrates are passed through two membrane filters having a porosity of 0.8 micron and 0.45 micron, respectively. Sodium acetate trihydrate is added (so that the final concentration is 8% w/v) and the solution is passed through a membrane filter having a porosity of 0.2 micron.

The purified polysaccharide is precipitated by adding 3 volumes of ethanol under stirring.

Precipitates of the purified pneumococcal serotype 14 polysaccharide are isolated by decantation followed by filtration and further washed with ethanol and finally dried at room temperature under reduced pressure.

Sizing

The purified pneumococcal serotype 14 polysaccharide powder was solubilized in distilled $H_2O$ with stirring at room temperature for about 4 hours and then stored at 4 °C overnight. The solution was added to DE52 (Whatman, diethylamino-ethyl cellulose) which had been pre-swollen for ca. 15 hours in distilled $H_2O$ at pH of ca. 5-6. The slurry was gently shaken on a platform shaker at room temperature for ca. 15 hrs, after which it was centrifuged at 5,000 rpm for 15 min. at 20 °C. The supernatant fluid was further clarified through a sinter glass funnel (150 ml, medium porosity) and collected into a 2L side arm flask.

The DE52-treated pneumococcal serotype 14 polysaccharide was sonicated in a plastic beaker on an ice bath with a Branson Sonifier (one-half inch probe, setting 8) for 2 min. The sample was allowed to cool for ca. 15 min. while the viscosity was determined and then was sonicated for additional 1 min. intervals. A viscosity end point of 1.096 centistokes was reached after the last sonic treatment. The hydrolyzed sample was brought to room temperature and sodium acetate reagent was added to a final concentration of 1 % (w/v).

The hydrolyzed sample was brought to 39.3% isopropanol by the addition of isopropanol (added dropwise with stirring at room temperature). The sample was allowed to stir for 15-30 minutes and then centrifuged at 11,000×g for 30 minutes (Beckman JA-10 rotor; 8,000 rpm; 20 °C) and the supernatant fluid decanted. The waste pellet was triturated with absolute EtOH in a 250-mL Omnimix jar, then collected on a 60-mL sinter glass funnel. The precipitate was washed directly on the funnel with absolute EtOH, then acetone, and dried in vacuo over $CaCl_2$ at room temperature in preparation for analysis.

The supernatant fluid was brought to 41.8% isopropanol by adding further isopropanol dropwise while stirring at room temperature. The sample was aged and centrifuged as described above. The pellet was triturated, collected, washed and dried as described above. The pellet was solubilized in distilled $H_2O$ at room temperature for 2-3 hours. The solution (2.5 mg/mL) was transferred to dialysis tubing (12,000 MW cutoff; 45 mm) and dialyzed vs. distilled $H_2O$ for 27 hours with 2 additional changes of distilled $H_2O$. Then the dialyzed sample was transferred to lyophilization flasks, shell-frozen in a dry ice-methanol bath and lyophilized for 2-1/2 days.

Conjugation with Protein D

A 5.0 mg/ml solution of sized polysaccharide of S. *pneumoniae* serotype 14 and 2 M aqueous sodium chloride solution was prepared. To that solution a 100 mg/ml solution of CDAP in acetonitrile/water (1:1 v/v) was added until a ratio of CDAP to polysaccharide of 0.75 (w/w) was reached. 1 minute later the pH was adjusted to 9.5 by adding 0.25 M sodium hydroxide solution under stirring at 25 °C. After 3 minutes a 10 mg/ml solution of protein D in 0.2 M aqueous sodium chloride was added until a ratio of protein D/polysaccharide of 1.2 : 1 was reached. The reaction mixture was stirred for two hours while the pH was maintained at 9.5. Excess amount of 2 M glycine solution in water for injection

was added to quench unreacted cyanate ester groups. The pH was readjusted to 9.0 and the solution was stirred for 30 minutes at 25 °C and afterwards overnight at 2-8 °C.

The *S. pneumoniae* serotype 14/PD-conjugate was purified by gel filtration using a Sehacryl S400HR gel filtration column equilibrated with 0.15 sodium chloride (S500HR for 18C) to remove small molecules, salts and unconjugated polysaccharide and protein D. The conjugate eluted first, followed by the polysaccharide and free protein D. Fractions containing the conjugate were detected by UV spectroscopy at 280 mm, combined, sterile filtered (22 μm) and stored between 2-8 °C.

Characterization of *S. pneumoniae* serotype 14/PD-conjugate

The polysaccharide content was measured by the Resorcinol test and the protein content of the conjugate by the Lowry test. Consequently the polysaccharide-protein-ratio was determined from the ratio of the protein content and the polysaccharide content. The molecular weight distribution was measured on a HPLC-SEC gel filtration.

**Example G.8. *S. pneumoniae* serotype 18C polysaccharide-TT-conjugate**

The purified pneumococcal serotype 18C polysaccharide is obtained by the same method as the pneumococcal serotype 4 polysaccharide using a strain of *Streptococcus pneumoniae* producing capsular polysaccharide of serotype 18C.

Fermentation

A sample of a strain of *Streptococcus pneumoniae* producing capsular polysaccharide of serotype 18C stored at -70 °C in Tryptic Soy Broth supplemented with 10% glycerol is rapidly thawed. The organisms are collected using a platinum loop and transferred to a Petri dish containing 15 ml of culture medium consisting of 40 g of Blood agar base per liter of water, sterilized at 121 °C for 20 minutes and then cooled to 50 °C and supplemented with 5% of fresh defibrinated sheep blood and the microorganism is then incubated at 37 °C for 16 hours in atmosphere enriched with $CO_2$ (5% w/v).

The produced bacteria are collected and a second run is performed in a Roux culture bottle containing 100 ml of synthetic medium according Hoeprich (Paul D. Hoeprich J. Bact. 1955, 69, p. 682-688, and J. Bact. 1957, 74, p. 587-590), solidified by adding agar (20 g/l). After an incubation period of 6 to 9 hours in anaerobic atmosphere, the produced organism is harvested and suspended in 5 ml of aqueous sodium chloride solution (8.5 g per liter). The harvest is used to inoculate a culture vessel containing 8 liters of liquid semi-synthetic medium according to Hoeprich. The medium is maintained without agitation or aeration for 16 hours at 37 °C to form the inoculum for the production culture.

For the production culture, a 100 liter fermenter is used containing 72 liters of semi-synthetic medium following Hoeprich. The medium is inoculated with the above-referenced culture and the incubation is maintained without aeration but gentle stirring at 35.5 °C (±1), the pH being continuously adjusted to 7.2 using a 25% sodium hydroxide solution. The development of the culture is regularly monitored by sampling and measuring the optical density of the medium at 620 nm. When the optical density reaches 0.15 a solution of one kilogram of dextrose in 2 liters of autoclaved deionized water is added over 20 minutes at 121 °C. The culture is stopped at the beginning of stationary phase, which corresponds to an optical density of 0.8.

Extraction of polysaccharide

The fermentation medium (80 l) is transferred into a storage tank and inactivated by adding one liter of 90% phenol; the pH is adjusted to 7.2 with 25% sodium hydroxide solution. After standing overnight at 5 - 10 °C, 702 g of sodium chloride are dissolved, which corresponds to an addition of 0.15 mol of sodium chloride per liter of medium (so-called concentration A).

While stirring, 4 kg of inert porous support (Celite 545) are then added and to the suspension, 800 ml (said volume B) of an aqueous solution of cetrimide (5% w/v) which is previously stored at a temperature of 25 °C is added and the stirring is continued for 10 minutes after the addition of cetrimide.

The medium is then filtered and the cake is washed with 4 L of an aqueous solution of sodium chloride of concentration A, with additional 40 ml of an aqueous solution of cetrimide (5% w/v).

The filtrate is concentrated to a volume of 5 liters by ultrafiltration in an AMICON DC unit 30 provided with cartridges with a cut-off of molecular weight of 100 kDa. The retentate is diafiltrated first with 40 L of sodium chloride solution of concentration A and then with 40 L of water keeping the volume between 10 and 5 liters. Finally, the volume of the solution is reduced to about 3 liters by ultrafiltration and the apparatus is rinsed with water to obtain approximately 5 liters of crude polysaccharide solution still containing substance C.

This polysaccharide can be isolated by dissolving 40 g of sodium acetate trihydrate in the solution and then adding with stirring 3.5 volumes of ethanol, causing the precipitation of the polysaccharide of serotype 18C which can be separated by filtration.

Purification of polysaccharide

Alternatively and preferably, a purification of the polysaccharide solution is carried out. To this end, 20.4 g of sodium acetate trihydrate is dissolved to obtain a saline solution (called solution C) whose concentration (said concentration C) in sodium acetate is 0.03 N, the pH is adjusted to 5.2 (±0.1) with 1 N hydrochloric acid and the medium is clarified by successive filtrations on 3 membranes whose porosity is 3, 1 and 0.45 micron, respectively.
The polysaccharide is precipitated onto 250 g of filter medium (Celite 512) whith stirring and by adding 500 ml (said volume D) of cetrimide aqueous solution of 5% (w/v), stirring being maintained for 15 minutes after addition of cetrimide.
The mixture is then filtered over a Celite 512 precoat.
The precipitate is purified chromatographically with 10 L of an aqueous solution of sodium acetate trihydrate (concentration C) with additionally 100 ml (said volume E) of aqueous cetrimide solution of 5% (w/v).
The cake is resuspended in 2 liters of a 0.15 N aqueous solution (said eluent F) of sodium acetate. After 15 minutes stirring, the suspension is filtered and the cake is purified chromatographically with 2 liters of eluent F.
Both eluates are filtered through a glass fiber prefilter followed by two membrane filters respectively having a porosity of 0.8 and 0.45 micron. Sodium acetate trihydrate is added to obtain a concentration of 8% and the solution is refiltered through a membrane filter with a porosity of 0.2 micron.
The purified serotype 18C polysaccharide is precipitated from the solution by adding 3 volumes of ethanol.
After settling, the supernatant solution is separated and the precipitate is triturated in ethanol until a homogeneous powder is obtained which is filtered and dried under reduced pressure at room temperature.
The resulting product is purified pneumococcal serotype 18C polysaccharide.

Sizing

Purified polysaccharide of *S. pneumoniae* serotype 18C was dissolved in water to a concentration of 2 - 3 mg/ml. The dissolved polysaccharide was passed through a mechanical homogenizer with pressure preset from 0-1000 bar. Subsequently, the saccharide was concentrated and diafiltered with sterile water on a 10 kDa MWCO ultrafilter. The permeate was discarded and the retentate was transferred to dialysis tubing (12,000 MW cutoff; 45 mm) and dialyzed vs. distilled $H_2O$ for 27 hours with 2 additional changes of distilled $H_2O$. Then the dialyzed sample was transferred to lyophilization flasks, shell-frozen in a dry ice-methanol bath and lyophilized for 2-1/2 days

Conjugation with TT

To a 25mg/mL solution of purified tetanus toxoid in 2 M aqueous sodium chloride was added adipic acid dihydrazide (ADH) until an ADH concentration of 0.2 M was reached. The pH was adjusted to 6.2. EDAC was added to reach a concentration of 0.02 M and the mixture was stirred for 1 hour while maintaining the pH at 6.2. The reaction was quenched by adjusting the pH to 9.0 for at least 30 minutes at 25 °C. The modified tetanus toxoid was diafiltrated (10 kDa CO membrane), sterile filtered and stored at -70 °C.
A 4.5 mg/ml solution of sized polysaccharide of *S. pneumoniae* serotype 18C and 2 M aqueous sodium chloride solution was prepared. To that solution a 100 mg/ml solution of CDAP in acetonitrile/water (1:1 v/v) was added until a ratio of CDAP to polysaccharide of 0.75 (w/w) was reached. 1 minute later the pH was adjusted to 9.0 by adding 0.30 M sodium hydroxide solution under stirring at 25 °C. After 3 minutes a 20 mg/ml solution of modified tetanus toxoid (see above) in 0.2 M aqueous sodium chloride was added until a ratio of modified TT/polysaccharide of 2 : 1 was reached. The reaction mixture was stirred for two hours while the pH was maintained at 9.0. Excess amount of 2 M glycine solution in water for injection was added to quench unreacted cyanate ester groups. The pH was readjusted to 9.0 and the solution was stirred for 30 minutes at 25 °C and afterwards overnight at 2-8 °C.
The *S. pneumoniae* serotype 18C/TT-conjugate was purified by gel filtration using a Sehacryl S400HR gel filtration column equilibrated with 0.15 sodium chloride (S500HR for 18C) to remove small molecules, salts and unconjugated polysaccharide and TT. The conjugate eluted first, followed by the polysaccharide and free TT. Fractions containing the conjugate were detected by UV spectroscopy at 280 mm, combined, sterile filtered (22 μm) and stored between 2-8 °C.

Characterization of *S. pneumoniae* serotype 18C/TT-conjugate

The polysaccharide content was measured by the Resorcinol test and the protein content of the conjugate by the Lowry test. Consequently the polysaccharide-protein-ratio was determined from the ratio of the protein content and the polysaccharide content. The molecular weight distribution was measured on a HPLC-SEC gel filtration.

**Example G.9. *S. pneumoniae* serotype 19F polysaccharide-DT-conjugate**

The purified pneumococcal serotype 19F polysaccharide is obtained by the same method as the pneumococcal serotype 4 polysaccharide using a strain of *Streptococcus pneumoniae* producing capsular polysaccharide of serotype 19F.

Fermentation

A sample of a strain of *Streptococcus pneumoniae* producing capsular polysaccharide of serotype 19F stored at -70 °C in Tryptic Soy Broth supplemented with 10% glycerol is rapidly thawed. The organisms are collected using a platinum loop and transferred to a Petri dish containing 15 ml of culture medium consisting of 40 g of Blood agar base per liter of water, sterilized at 121 °C for 20 minutes and then cooled to 50 °C and supplemented with 5% of fresh defibrinated sheep blood and the microorganism is then incubated at 37 °C for 16 hours in atmosphere enriched with $CO_2$ (5% w/v).

The produced bacteria are collected and a second run is performed in a Roux culture bottle containing 100 ml of synthetic medium according Hoeprich (Paul D. Hoeprich J. Bact. 1955, 69, p. 682-688, and J. Bact. 1957, 74, p. 587-590), solidified by adding agar (20 g/l). After an incubation period of 6 to 9 hours in anaerobic atmosphere, the produced organism is harvested and suspended in 5 ml of aqueous sodium chloride solution (8.5 g per liter). The harvest is used to inoculate a culture vessel containing 8 liters of liquid semi-synthetic medium according to Hoeprich. The medium is maintained without agitation or aeration for 16 hours at 37 °C to form the inoculum for the production culture.

For the production culture, a 100 liter fermenter is used containing 72 liters of semi-synthetic medium following Hoeprich. The medium is inoculated with the above-referenced culture and the incubation is maintained without aeration but gentle stirring at 35.5 °C ($\pm$1), the pH being continuously adjusted to 7.2 using a 25% sodium hydroxide solution. The development of the culture is regularly monitored by sampling and measuring the optical density of the medium at 620 nm. When the optical density reaches 0.15 a solution of one kilogram of dextrose in 2 liters of autoclaved deionized water is added over 20 minutes at 121 °C. The culture is stopped at the beginning of stationary phase, which corresponds to an optical density of 0.8.

Extraction of polysaccharide

The fermentation medium (80 l) is transferred into a storage tank and inactivated by adding one liter of 90% phenol; the pH is adjusted to 7.2 with 25% sodium hydroxide solution. After standing overnight at 5 - 10 °C, 702 g of sodium chloride are dissolved, which corresponds to an addition of 0.15 mol of sodium chloride per liter of medium (so-called concentration A).

While stirring, 4 kg of inert porous support (Celite 545) are then added and to the suspension, 800 ml (said volume B) of an aqueous solution of cetrimide (5% w/v) which is previously stored at a temperature of 25 °C is added and the stirring is continued for 10 minutes after the addition of cetrimide.

The medium is then filtered and the cake is washed with 4 L of an aqueous solution of sodium chloride of concentration A, with additional 40 ml of an aqueous solution of cetrimide (5% w/v).

The filtrate is concentrated to a volume of 5 liters by ultrafiltration in an AMICON DC unit 30 provided with cartridges with a cut-off of molecular weight of 100 kDa. The retentate is diafiltrated first with 40 L of sodium chloride solution of concentration A and then with 40 L of water keeping the volume between 10 and 5 liters. Finally, the volume of the solution is reduced to about 3 liters by ultrafiltration and the apparatus is rinsed with water to obtain approximately 5 liters of crude polysaccharide solution still containing substance C.

This polysaccharide can be isolated by dissolving 40 g of sodium acetate trihydrate in the solution and then adding with stirring 3.5 volumes of ethanol, causing the precipitation of the polysaccharide of serotype 19F which can be separated by filtration.

Purification of polysaccharide

Alternatively and preferably, a purification of the polysaccharide solution is carried out. To this end, 17 g of sodium acetate trihydrate is dissolved to obtain a saline solution (called solution C) whose concentration (said concentration C) in sodium acetate is 0.025 N, the pH is adjusted to 5.2 (±0.1) with 1 N hydrochloric acid and the medium is clarified by successive filtrations on 3 membranes whose porosity is 3, 1 and 0.45 micron, respectively.

The polysaccharide is precipitated onto 250 g of filter medium (Celite 512) whith stirring and by adding 500 ml (said volume D) of cetrimide aqueous solution of 5% (w/v), stirring being maintained for 15 minutes after addition of cetrimide.

The mixture is then filtered over a Celite 512 precoat.

The precipitate is purified chromatographically with 10 L of an aqueous solution of sodium acetate trihydrate (concentration C) with additionally 100 ml (said volume E) of aqueous cetrimide solution of 5% (w/v).

The cake is resuspended in 2 liters of a 0.3 N aqueous solution (said eluent F) of sodium acetate. After 15 minutes stirring, the suspension is filtered and the cake is purified chromatographically with 2 liters of eluent F.

Both eluates are filtered through a glass fiber prefilter followed by two membrane filters respectively having a porosity of 0.8 and 0.45 micron. Sodium acetate trihydrate is added to obtain a concentration of 8% and the solution is refiltered through a membrane filter with a porosity of 0.2 micron.

The purified serotype 19F polysaccharide is precipitated from the solution by adding 3 volumes of ethanol.

After settling, the supernatant solution is separated and the precipitate is triturated in ethanol until a homogeneous powder is obtained which is filtered and dried under reduced pressure at room temperature.

The resulting product is purified pneumococcal serotype 19F polysaccharide.

Sizing

Purified polysaccharide of S. *pneumoniae* serotype 1 was dissolved in water to a concentration of 2 - 3 mg/ml. The dissolved polysaccharide was passed through a mechanical homogenizer with pressure preset from 0-1000 bar. Subsequently, the saccharide was concentrated and diafiltered with sterile water on a 10 kDa MWCO ultrafilter. The permeate was discarded and the retentate was transferred to dialysis tubing (12,000 MW cutoff; 45 mm) and dialyzed vs. distilled $H_2O$ for 27 hours with 2 additional changes of distilled $H_2O$. Then the dialyzed sample was transferred to lyophilization flasks, shell-frozen in a dry ice-methanol bath and lyophilized for 2-1/2 days

Conjugation with DT

A 9.0 mg/ml solution of sized polysaccharide of *S. pneumoniae* serotype 19F and 2 M aqueous sodium chloride solution was prepared. To that solution a 100 mg/ml solution of CDAP in acetonitrile/water (1:1 v/v) was added until a ratio of CDAP to polysaccharide of 1.5 (w/w) was reached. 1 minute later the pH was adjusted to 9.0 by adding 0.25 M sodium hydroxide solution under stirring at 25 °C. After 3 minutes a 20 mg/ml solution of diphtheria toxoid (DT) in 0.2 M aqueous sodium chloride was added until a ratio of DT/polysaccharide of 1.5 : 1 was reached. The reaction mixture was stirred for two hours while the pH was maintained at 9.0. Excess amount of 2 M glycine solution in water for injection was added to quench unreacted cyanate ester groups. The pH was readjusted to 9.0 and the solution was stirred for 30 minutes at 25 °C and afterwards overnight at 2-8 °C.

The *S. pneumoniae* serotype 19F/DT-conjugate was purified by gel filtration using a Sehacryl S400HR gel filtration column equilibrated with 0.15 sodium chloride (S500HR for 18C) to remove small molecules, salts and unconjugated polysaccharide and DT. The conjugate eluted first, followed by the polysaccharide and free DT. Fractions containing the conjugate were detected by UV spectroscopy at 280 mm, combined, sterile filtered (22 μm) and stored between 2-8 °C.

Characterization of *S. pneumoniae* serotype 19F/DT-conjugate

The polysaccharide content was measured by the Resorcinol test and the protein content of the conjugate by the Lowry test. Consequently the polysaccharide-protein-ratio was determined from the ratio of the protein content and the polysaccharide content. The molecular weight distribution was measured on a HPLC-SEC gel filtration.

**Example G.10. *S. pneumoniae* serotype 23F polysaccharide-protein D-conjugate**

The purified pneumococcal serotype 23F polysaccharide is obtained by the same method as the pneumococcal serotype 4 polysaccharide using a strain of *Streptococcus pneumoniae* producing capsular polysaccharide of serotype 23F.

Fermentation

A sample of a strain of *Streptococcus pneumoniae* producing capsular polysaccharide of serotype 23F stored

at -70 °C in Tryptic Soy Broth supplemented with 10% glycerol is rapidly thawed. The organisms are collected using a platinum loop and transferred to a Petri dish containing 15 ml of culture medium consisting of 40 g of Blood agar base per liter of water, sterilized at 121 °C for 20 minutes and then cooled to 50 °C and supplemented with 5% of fresh defibrinated sheep blood and the microorganism is then incubated at 37 °C for 16 hours in atmosphere enriched with $CO_2$ (5% w/v).

The produced bacteria are collected and a second run is performed in a Roux culture bottle containing 100 ml of synthetic medium according Hoeprich (Paul D. Hoeprich J. Bact. 1955, 69, p. 682-688, and J. Bact. 1957, 74, p. 587-590), solidified by adding agar (20 g/l). After an incubation period of 6 to 9 hours in anaerobic atmosphere, the produced organism is harvested and suspended in 5 ml of aqueous sodium chloride solution (8.5 g per liter). The harvest is used to inoculate a culture vessel containing 8 liters of liquid semi-synthetic medium according to Hoeprich. The medium is maintained without agitation or aeration for 16 hours at 37 °C to form the inoculum for the production culture.

For the production culture, a 100 liter fermenter is used containing 72 liters of semi-synthetic medium following Hoeprich. The medium is inoculated with the above-referenced culture and the incubation is maintained without aeration but gentle stirring at 35.5 °C (±1), the pH being continuously adjusted to 7.2 using a 25% sodium hydroxide solution. The development of the culture is regularly monitored by sampling and measuring the optical density of the medium at 620 nm. When the optical density reaches 0.15 a solution of one kilogram of dextrose in 2 liters of autoclaved deionized water is added over 20 minutes at 121 °C. The culture is stopped at the beginning of stationary phase, which corresponds to an optical density of 0.8.

Extraction of polysaccharide

The fermentation medium (80 l) is transferred into a storage tank and inactivated by adding one liter of 90% phenol; the pH is adjusted to 7.2 with 25% sodium hydroxide solution. After standing overnight at 5 - 10 °C, 702 g of sodium chloride are dissolved, which corresponds to an addition of 0.15 mol of sodium chloride per liter of medium (so-called concentration A).

While stirring, 4 kg of inert porous support (Celite 545) are then added and to the suspension, 800 ml (said volume B) of an aqueous solution of cetrimide (5% w/v) which is previously stored at a temperature of 25 °C is added and the stirring is continued for 10 minutes after the addition of cetrimide.

The medium is then filtered and the cake is washed with 4 L of an aqueous solution of sodium chloride of concentration A, with additional 40 ml of an aqueous solution of cetrimide (5% w/v).

The filtrate is concentrated to a volume of 5 liters by ultrafiltration in an AMICON DC unit 30 provided with cartridges with a cut-off of molecular weight of 100 kDa. The retentate is diafiltrated first with 40 L of sodium chloride solution of concentration A and then with 40 L of water keeping the volume between 10 and 5 liters. Finally, the volume of the solution is reduced to about 3 liters by ultrafiltration and the apparatus is rinsed with water to obtain approximately 5 liters of crude polysaccharide solution still containing substance C.

This polysaccharide can be isolated by dissolving 40 g of sodium acetate trihydrate in the solution and then adding with stirring 3.5 volumes of ethanol, causing the precipitation of the polysaccharide of serotype 23F which can be separated by filtration.

Purification of polysaccharide

Alternatively and preferably, a purification of the polysaccharide solution is carried out. To this end, ammonium chloride is dissolved to obtain a saline solution (called solution C) whose concentration (said concentration C) in ammonium chloride is 0.08 N, the pH is adjusted to 5.2 (±0.1) with 1 N hydrochloric acid and the medium is clarified by successive filtrations on 3 membranes whose porosity is 3, 1 and 0.45 micron, respectively.

The polysaccharide is precipitated onto 250 g of filter medium (Celite 512) whith stirring and by adding 1500 ml (said volume D) of cetrimide aqueous solution of 5% (w/v), stirring being maintained for 15 minutes after addition of cetrimide.

The mixture is then filtered over a Celite 512 precoat.

The precipitate is purified chromatographically with 10 L of an aqueous solution of ammonium chloride (concentration C) with additionally 3000 ml (said volume E) of aqueous cetrimide solution of 5% (w/v).

The cake is resuspended in 2 liters of a 0.12 N aqueous solution (said eluent F) of ammonium chloride. After 15 minutes stirring, the suspension is filtered and the cake is purified chromatographically with 2 liters of eluent F.

Both eluates are filtered through a glass fiber prefilter followed by two membrane filters respectively having a porosity of 0.8 and 0.45 micron. Sodium acetate trihydrate is added to obtain a concentration of 8% and the solution is refiltered through a membrane filter with a porosity of 0.2 micron.

The purified serotype 23F polysaccharide is precipitated from the solution by adding 3 volumes of ethanol.

After settling, the supernatant solution is separated and the precipitate is triturated in ethanol until a homogeneous powder is obtained which is filtered and dried under reduced pressure at room temperature.

The resulting product is purified pneumococcal serotype 23F polysaccharide.

Conjugation with Protein D

A 2.38 mg/ml solution of polysaccharide of S. *pneumoniae* serotype 23F and 2 M aqueous sodium chloride solution was prepared. To that solution a 100 mg/ml solution of CDAP in acetonitrile/water (1:1 v/v) was added until a ratio of CDAP to polysaccharide of 0.79 (w/w) was reached. 1 minute later the pH was adjusted to 9.5 by adding 0.25 M sodium hydroxide solution under stirring at 25 °C. After 3 minutes a 5 mg/ml solution of protein D in 0.2 M aqueous sodium chloride was added until a ratio of protein D/polysaccharide of 1 : 1 was reached. The reaction mixture was stirred for two hours while the pH was maintained at 9.5. Excess amount of 2 M glycine solution in water for injection was added to quench unreacted cyanate ester groups. The pH was readjusted to 9.0 and the solution was stirred for 30 minutes at 25 °C and afterwards overnight at 2-8 °C.

The S. *pneumoniae* serotype 23F/PD-conjugate was purified by gel filtration using a Sehacryl S400HR gel filtration column equilibrated with 0.15 sodium chloride (S500HR for 18C) to remove small molecules, salts and unconjugated polysaccharide and protein D. The conjugate eluted first, followed by the polysaccharide and free protein D. Fractions containing the conjugate were detected by UV spectroscopy at 280 mm, combined, sterile filtered (22 μm) and stored between 2-8 °C.

Characterization of *S. pneumoniae* serotype 23F/PD-conjugate

The polysaccharide content was measured by the Resorcinol test and the protein content of the conjugate by the Lowry test. Consequently the polysaccharide-protein-ratio was determined from the ratio of the protein content and the polysaccharide content. The molecular weight distribution was measured on a HPLC-SEC gel filtration.

## Claims

1. An immunogenic composition comprising a conjugate of a saccharide from *Streptococcus pneumoniae* serotype 8 and a carrier protein, and
a mixture consisting of capsular polysaccharides from *Streptococcus pneumoniae* serotypes 1, 3, 4, 5, 6A, 6B, 7F, 9V, 14, 18C, 19A, 19F and 23F individually conjugated to $CRM_{197}$ carrier protein; or
a mixture consisting of capsular polysaccharides from *Streptococcus pneumoniae* serotypes 1, 4, 5, 6B, 7F, 9V, 14, 18C, 19F and 23F individually conjugated to a carrier protein, wherein the capsular polysaccharides from *Streptococcus pneumoniae* serotypes 1,4,5, 6B, 7F, 9V, 14 and 23F are individually conjugated to protein D, the capsular polysaccharide from *Streptococcus pneumoniae* serotype 18C is conjugated to tetanus toxoid and the capsular polysaccharide from *Streptococcus pneumoniae* serotype 19F is conjugated to diphtheria toxoid.

2. The immunogenic composition according to claim 1, wherein the conjugate of a saccharide from *Streptococcus pneumoniae* serotype 8 and a carrier protein is of the following general formula (**I**):

$$\mathbf{[V^*\text{-}U_{x+3}\text{-}U_{x+2}\text{-}U_{x+1}\text{-}U_x\text{-}O\text{-}L^1\text{-}NH\text{-}W^1]_{m1}\text{-}carrier\text{-}protein1} \qquad (I)$$

wherein
x is an integer selected from 1, 2, 3 and 4;

$U_1 = U_5 =$ 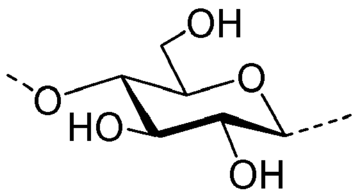 ; $U_2 = U_6 =$ 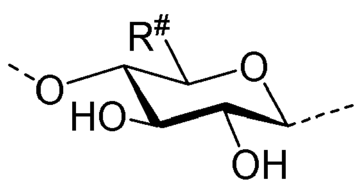

$U_3 = U_7 =$ 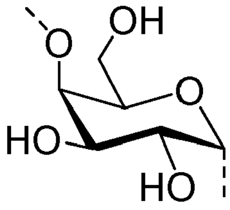 ; $U_4 =$ 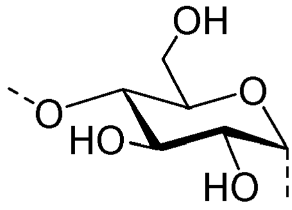 ;

V*- represents H-, H-$U_x$- or H-$U_{x+1}$-$U_x$-;
$R^{\#}$ represents -COOH or -$CH_2OH$;
$L^1$ represents a linker;
m1 is comprised between 2 and 18;
-$W^1$- is selected from:

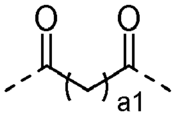 , 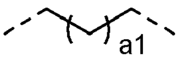 , and 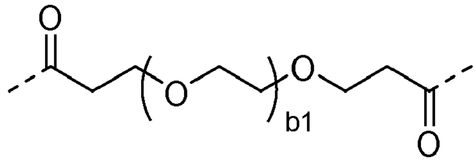 ;

a1 represents an integer from 1 to 10;
b1 represents an integer from 1 to 4; and
carrier-protein1 is selected from $CRM_{197}$, protein D, tetanus toxoid and diphtheria toxoid.

**3.** The immunogenic composition according to claim 2, wherein x represents 3 and/or V*- represents H-.

**4.** The immunogenic composition according to any one of the claims 1 - 3, further comprising a *Streptococcus pneumoniae* serotype 2 conjugate of the following general formula (**II-C**)

$$[B^*\text{-}A_{y+3}\text{-}A_{y+2}\text{-}A_{y+1}\text{-}A_y\text{-O-}L^2\text{-NH-}W^2]_{m2}\text{-carrier-protein2} \qquad (\textbf{II-C})$$

wherein
y is an integer selected from 1,2,3 and 4;

$A_1 = A_5 =$ 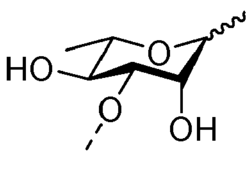 ; $A_2 = A_6 =$ 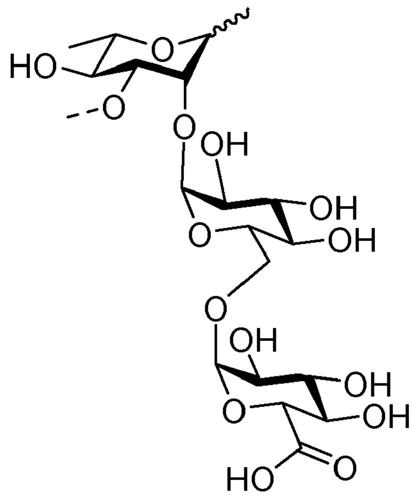 ,

A3 = A7 = 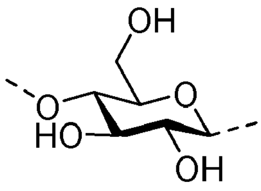 ; A4 = 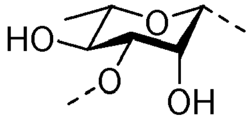 ;

B*- represents H-, H-$A_y$, H-$A_{y+1}$-$A_y$, H-$A_{y+2}$-$A_{y+1}$-$A_y$- or H-$A_{y+3}$-$A_{y+2}$-$A_{y+1}$-$A_y$-;
$L^2$ represents a linker;
m2 is comprised between about 2 and about 18;
-$W^2$- is selected from:

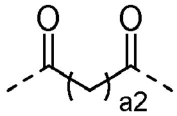 , 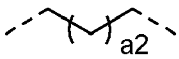 , and 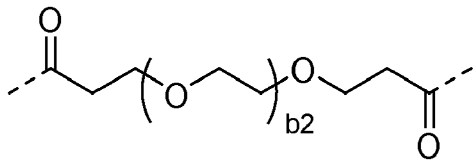 ;

a2 represents an integer from 1 to 10;
b2 represents an integer from 1 to 4; and
carrier-protein2 is selected from $CRM_{197}$, protein D, tetanus toxoid and diphtheria toxoid.

**5.** The immunogenic composition according to claim 4, wherein the *Streptococcus pneumoniae* serotype 2 conjugate has the following general formula (**II**)

**[B*-$A_{y+3}$-$A_{y+2}$-$A_{y+1}$-$A_y$-O-$L^2$-NH-$W^2$]$_{m2}$-carrier-protein2** **(II)**

wherein

A1 = A5 = 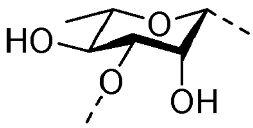 ; A2 = A6 = 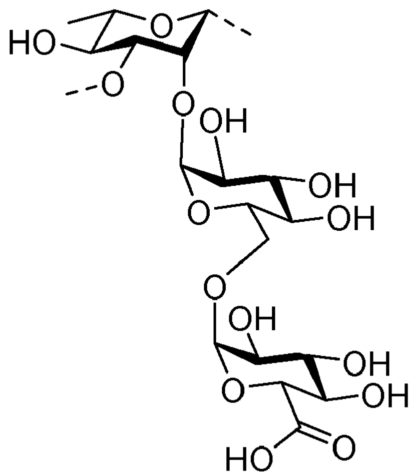 ,

A3 = A7 = 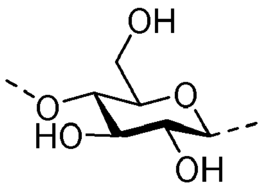 ; A4 = 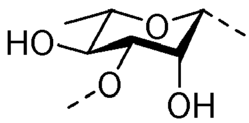 ;

and y, B*-, $L^2$, m2, -$W^2$- and carrier-protein2 have the meanings as defined in claim 4.

**6.** The immunogenic composition according to claim 4, wherein the *Streptococcus pneumoniae* serotype 2 conjugate has the following general formula **(II-D)**

$$\mathbf{[B^{*}\text{-}A_{y+3}\text{-}A_{y+2}\text{-}A_{y+1}\text{-}A_{y}\text{-}O\text{-}L^{2}\text{-}NH\text{-}W^{2}]_{m2}\text{-}carrier\text{-}protein2} \qquad \textbf{(II-D)}$$

wherein

$A_1 = A_5 =$ 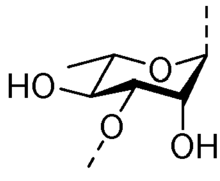 ; $A_2 = A_6 =$ 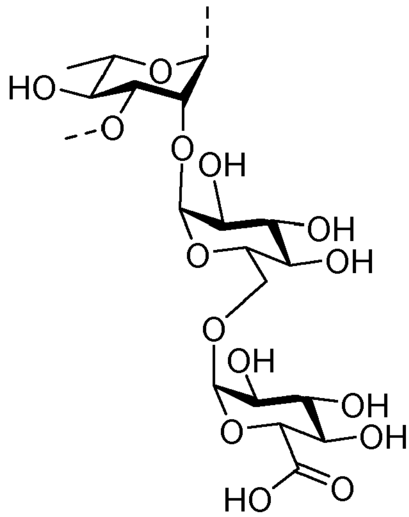 ,

$A_3 = A_7 =$ 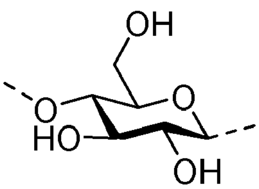 ; $A_4 =$ 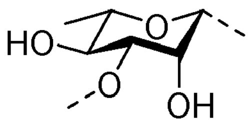 ;

and y, B*-, $L^2$, m2, -$W^2$- and carrier-protein2 have the meanings as defined in claim 4.

**7.** The immunogenic composition according to any one of the claims 4 - 6, wherein y represents 1 and/or B*- represents H-.

**8.** The immunogenic composition according to any one of the claims 1 - 7, wherein the immunogenic composition comprises the mixture consisting of capsular polysaccharides from *Streptococcus pneumoniae* serotypes 1,4,5, 6B, 7F, 9V, 14, 18C, 19F and 23F individually conjugated to a carrier protein, wherein the capsular polysaccharides from *Streptococcus pneumoniae* serotypes 1, 4, 5, 6B, 7F, 9V, 14, and 23F are individually conjugated to protein D, the capsular polysaccharide from *Streptococcus pneumoniae* serotype 18C is conjugated to tetanus toxoid and the capsular polysaccharide from *Streptococcus pneumoniae* serotype 19F is conjugated to diphtheria toxoid.

**9.** The immunogenic composition according to claim 8, further comprising a *Streptococcus pneumoniae* serotype 3 conjugate of general formula (**III**)

$$\mathbf{[H\text{-}(C)_{c3}\text{-}(D\text{-}C)_{c2}\text{-}(D)_{c1}\text{-}O\text{-}L^{3}\text{-}NH\text{-}W^{3}]_{m3}\text{-}carrier\text{-}protein3} \qquad \textbf{(III)}$$

wherein

C represents:

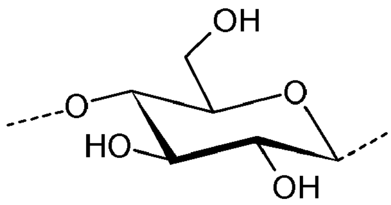

D represents:

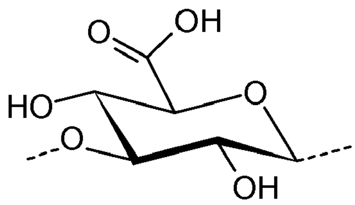

c2 is an integer selected from 1, 2, 3, 4, 5, 6, 7, 8, 9 and 10;
c1 and c3 represent independently of each other an integer selected from 0 and 1;
$L^3$ represents a linker;
m3 is comprised between about 2 and about 18;
$-W^3-$ is selected from:

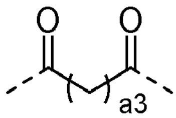
, 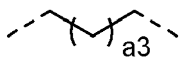
, and 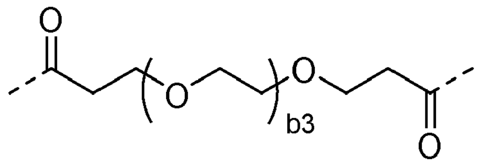
;

a3 represents an integer from 1 to 10;
b3 represents an integer from 1 to 4; and
carrier-protein3 is selected from $CRM_{197}$, protein D, tetanus toxoid and diphtheria toxoid.

**10.** The immunogenic composition according to claim 9, wherein c1 and c3 are 0.

**11.** The immunogenic composition according to claim 9 or 10, wherein carrier-protein3 is $CRM_{197}$ and/or m3 is comprised between 4 and 10.

**12.** The immunogenic composition according to any one of the claims 1 - 11 for use in the prevention and/or treatment of diseases caused by *Streptococcus pneumoniae* serotype 8.

**13.** The immunogenic composition according to any one of the claims 4 - 11 for use in the prevention and/or treatment of diseases caused by *Streptococcus pneumoniae* serotype 8 and serotype 2.

**14.** A vaccine comprising the immunogenic composition according to any one of the claims 1 - 11, a physiologically acceptable vehicle and an adjuvant.

**15.** The vaccine according to claim 14, wherein the adjuvant is aluminium phosphate.

Figure 1

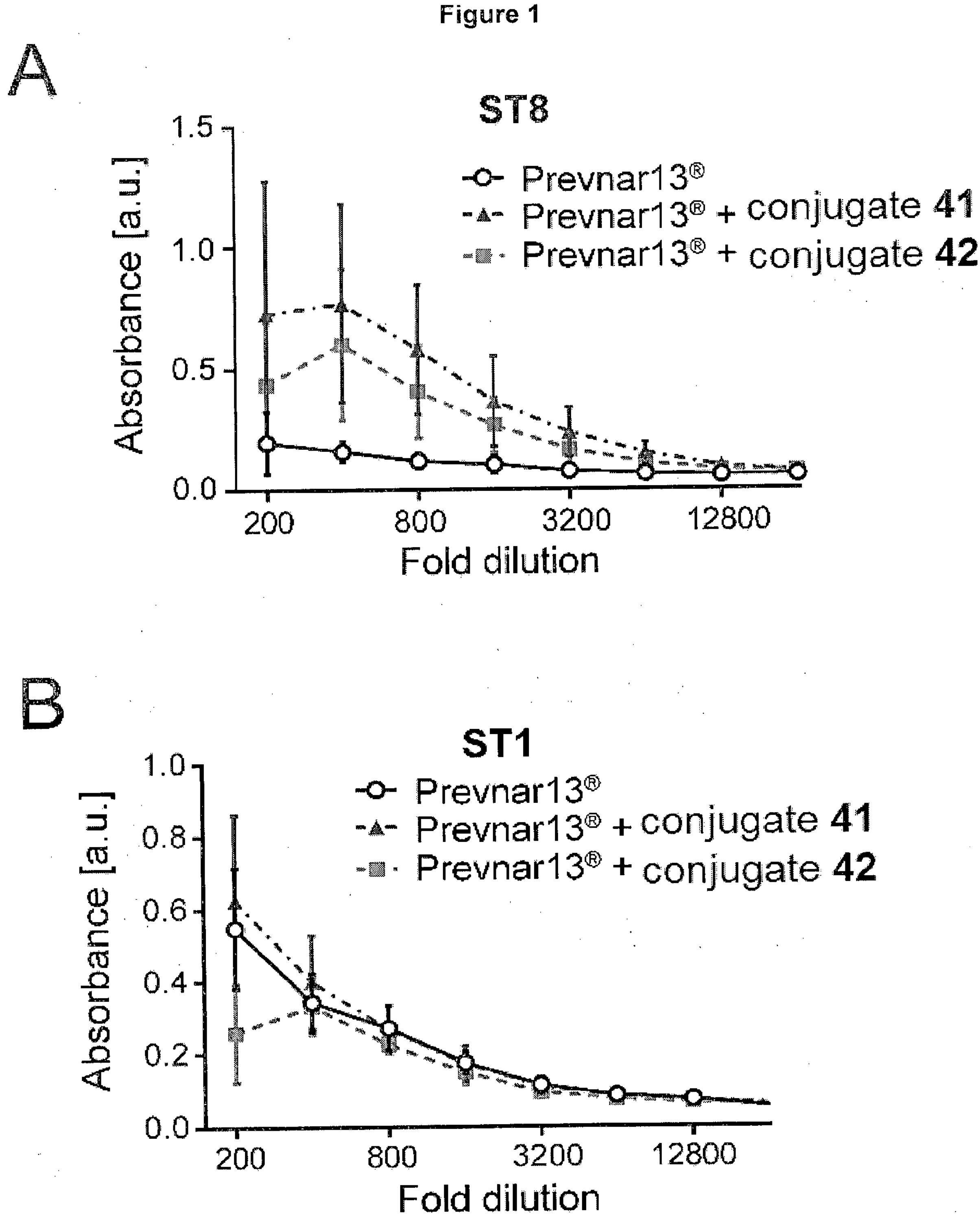

A
ST8
Prevnar13®
Prevnar13® + conjugate 41
Prevnar13® + conjugate 42
Absorbance [a.u.]
1.5
1.0
0.5
0.0
200
800
3200
12800
Fold dilution
B
ST1
Prevnar13®
Prevnar13® + conjugate 41
Prevnar13® + conjugate 42
Absorbance [a.u.]
1.0
0.8
0.6
0.4
0.2
0.0
200
800
3200
12800
Fold dilution

Figure 1 (continued)

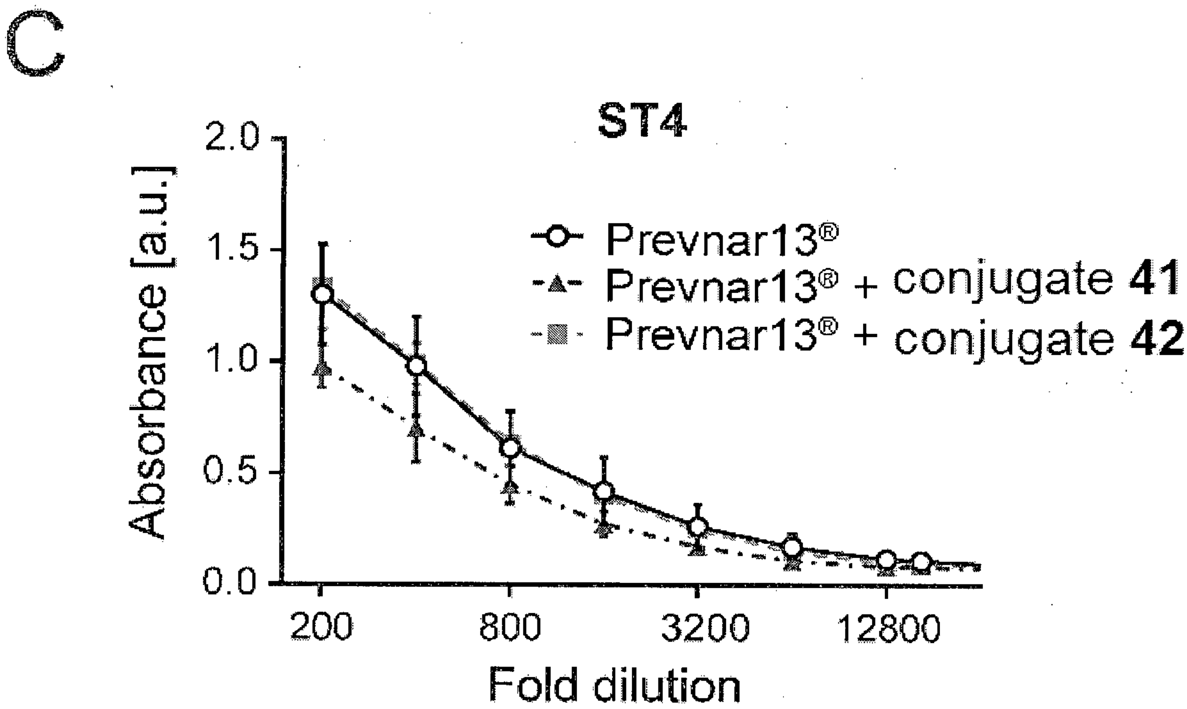


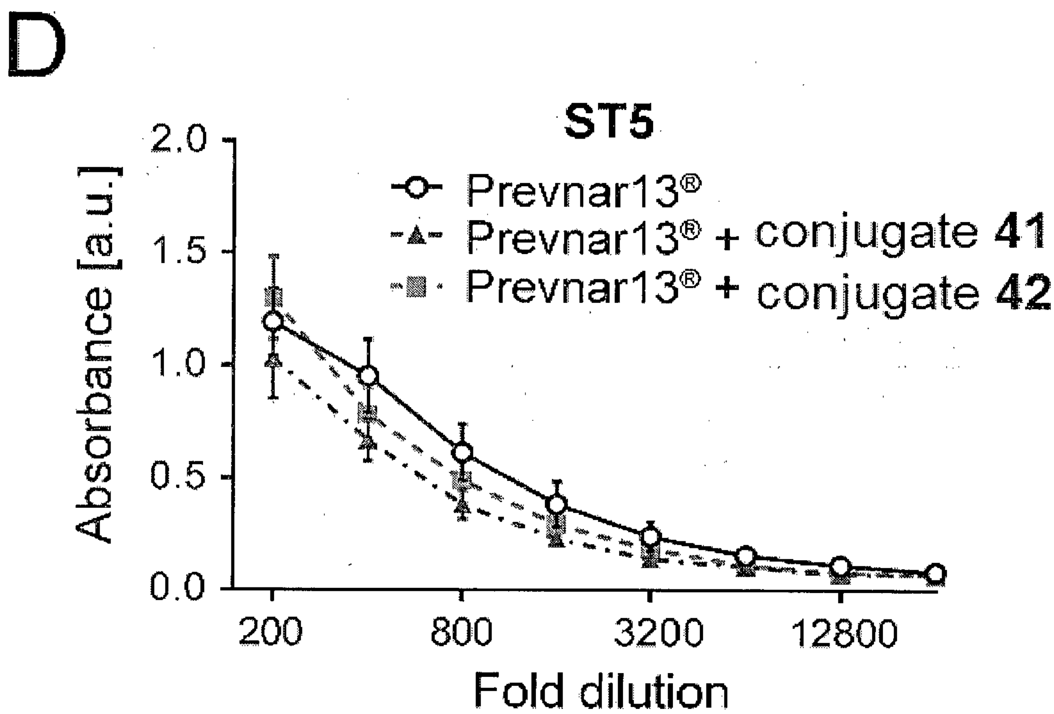

Figure 1 (continued)

E

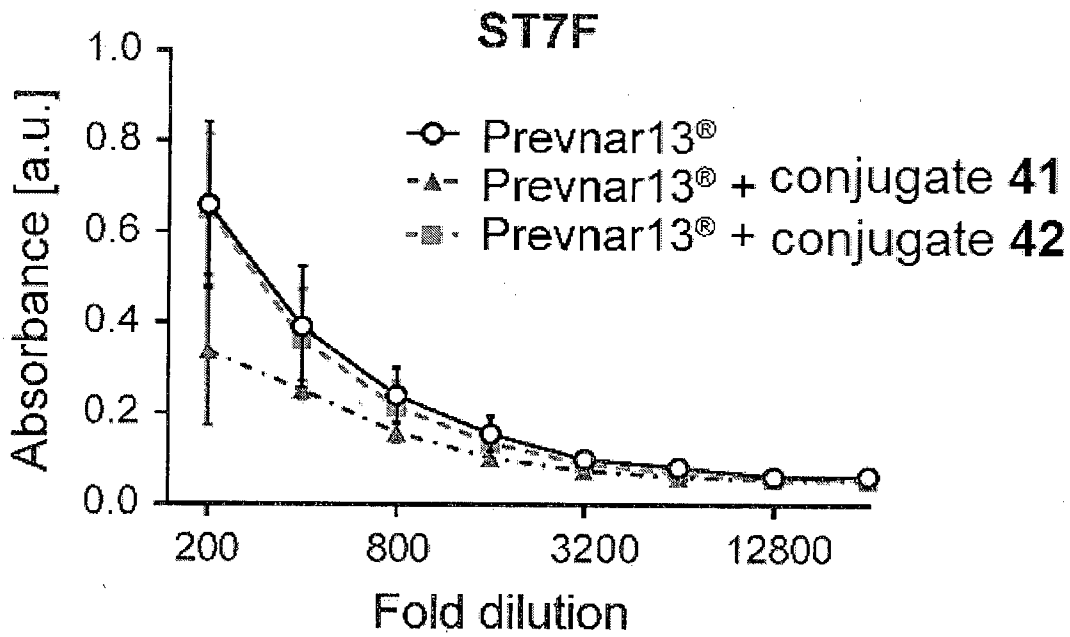


F

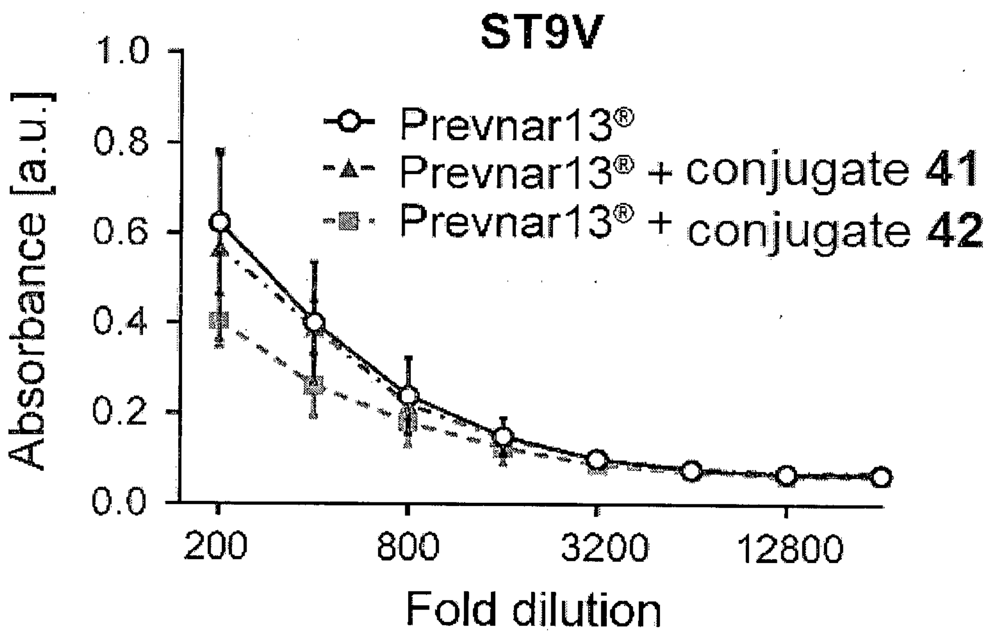

**Figure 1 (continued)**

G

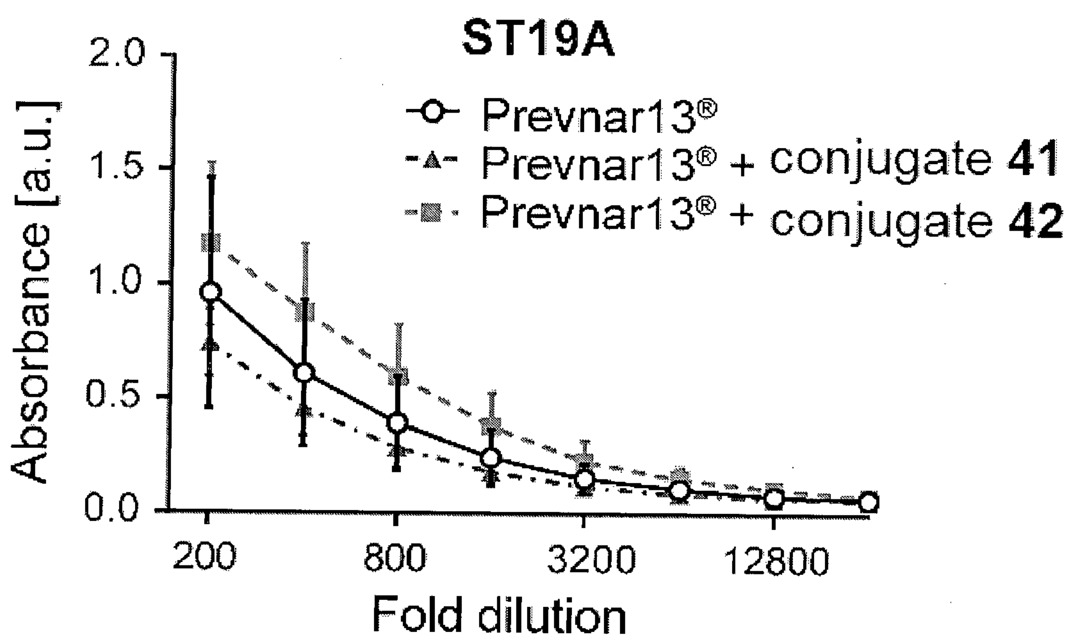

Figure 2

A

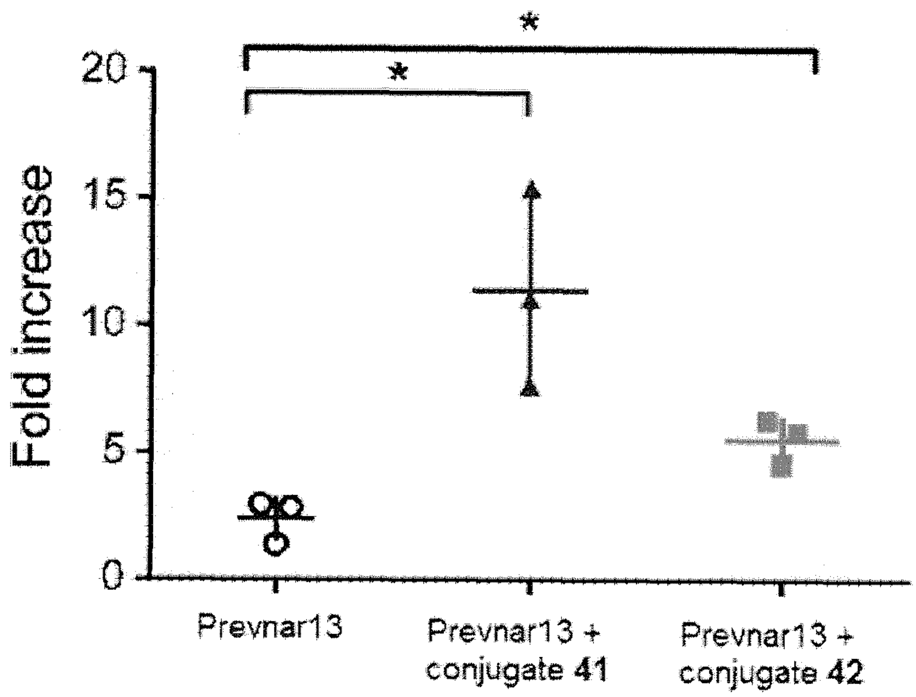
Fold increase
0
5
10
15
20
*
*
Prevnar13
Prevnar13 + conjugate 41
Prevnar13 + conjugate 42

B

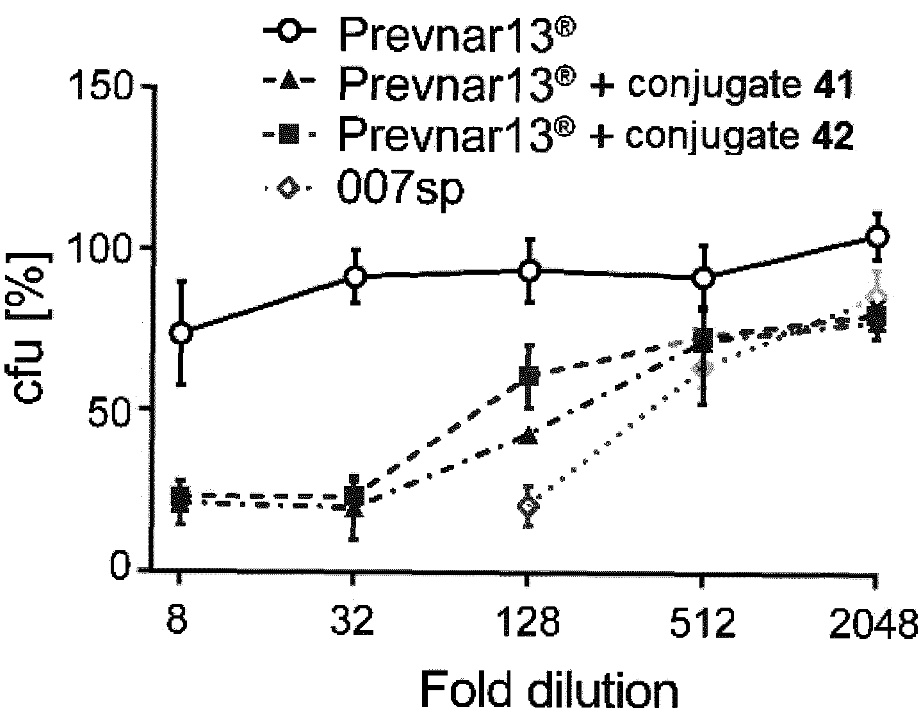
Prevnar13®
Prevnar13® + conjugate 41
Prevnar13® + conjugate 42
007sp
cfu [%]
0
50
100
150
8
32
128
512
2048
Fold dilution

Figure 3

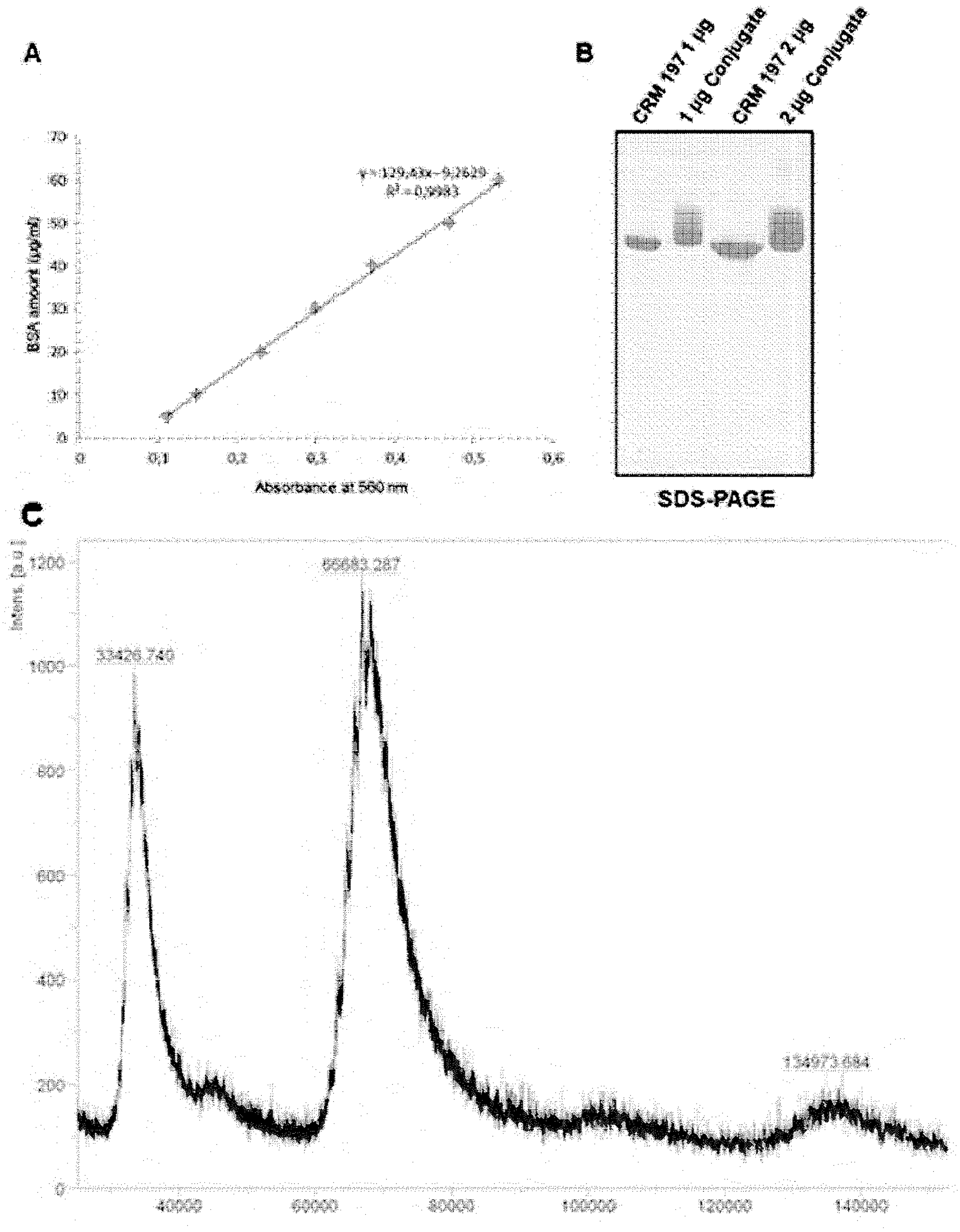
A
y = 129,43x − 9,2629
R² = 0,9983
BSA amount (µg/ml)
0
10
20
30
40
50
60
70
0
0,1
0,2
0,3
0,4
0,5
0,6
Absorbance at 560 nm
B
CRM 197 1 µg
1 µg Conjugate
CRM 197 2 µg
2 µg Conjugate
SDS-PAGE
C
Intens. [a.u.]
33426.749
65883.287
134973.684
0
200
400
600
800
1000
1200
40000
60000
80000
100000
120000
140000

Figure 4

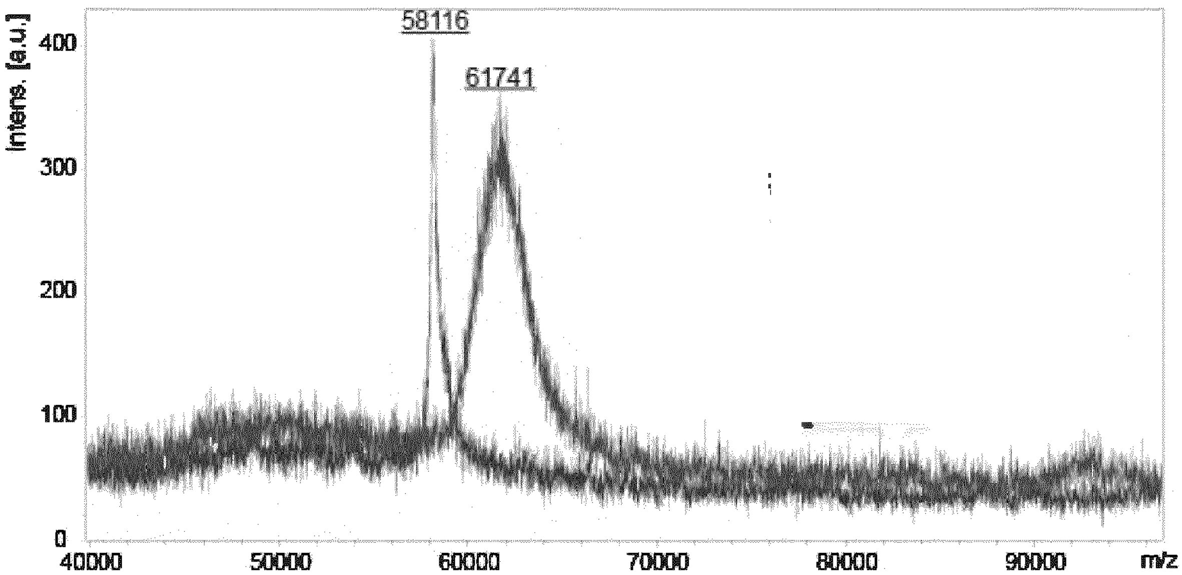

58116
61741
Intens. [a.u.]
400
300
200
100
0
40000
50000
60000
70000
80000
90000
m/z

**Figure 5**

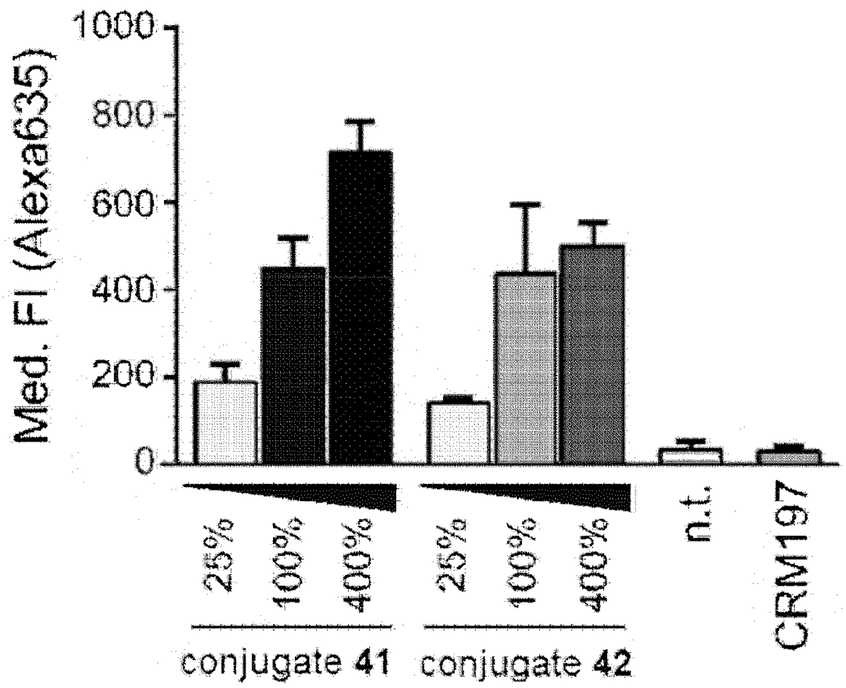
Med. FI (Alexa635)
1000
800
600
400
200
0
25%
100%
400%
25%
100%
400%
n.t.
CRM197
conjugate 41
conjugate 42

Europäisches Patentamt
European Patent Office
Office européen des brevets

# EUROPEAN SEARCH REPORT

Application Number
EP 16 17 9133

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2016/046420 A1 (MAX PLANCK GES ZUR FÖRDERUNG DER WISSENSCHAFTEN E V [DE]) 31 March 2016 (2016-03-31) * pages 2, 3, 41, 42, 53 * claims 1, 8, 9 * | 1-15 | INV. A61K39/09 A61K31/7028 |
| Y | ALONSODEVELASCO E ET AL: "STREPTOCOCCUS PNEUMONIAE: VIRULENCE FACTORS, PATHOGENESIS, AND VACCINES", MICROBIOLOGICAL REVIEWS, AMERICAN SOCIETY FOR MICROBIOLOGY, WASHINGTON, DC, US, vol. 59, no. 4, 1 December 1995 (1995-12-01), pages 591-603, XP000946788, ISSN: 0146-0749 * page 598 - page 599 * | 1-15 | |
| A | J. K. N. JONES ET AL: "The Structure of the Type VIII Pneumococcus Specific Polysaccharide 1a,b", JOURNAL OF THE AMERICAN CHEMICAL SOCIETY, vol. 79, no. 11, 1 June 1957 (1957-06-01), pages 2787-2793, XP055151920, ISSN: 0002-7863, DOI: 10.1021/ja01568a033 | 1-15 | TECHNICAL FIELDS SEARCHED (IPC) A61K |
| Y | WILLIAM C. GRUBER ET AL: "Development and clinical evaluation of Prevnar 13, a 13-valent pneumocococcal CRM197 conjugate vaccine", ANNALS OF THE NEW YORK ACADEMY OF SCIENCES, vol. 1263, no. 1, 25 July 2012 (2012-07-25), pages 15-26, XP055304511, US ISSN: 0077-8923, DOI: 10.1111/j.1749-6632.2012.06673.x * abstract * | 1-15 | |

2

The present search report has been drawn up for all claims

| Place of search | Date of completion of the search | Examiner |
|---|---|---|
| Munich | 21 September 2016 | Heder, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 17 9133

This annex lists the patent family members relating to the patent documents cited in the above-mentioned European search report.
The members are as contained in the European Patent Office EDP file on
The European Patent Office is in no way liable for these particulars which are merely given for the purpose of information.

21-09-2016

| Patent document cited in search report | Publication date | Patent family member(s) | Publication date |
| --- | --- | --- | --- |
| WO 2016046420 A1 | 31-03-2016 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 497524 A
- EP 497525 A
- EP 0594610 B1
- US 5614382 A
- EP 0072513 A2
- WO 9315760 A
- WO 9508348 A
- WO 9629094 A
- WO 9842721 A
- US 4673574 A
- EP 0161188 A
- EP 208375 A
- EP 0477508 A
- WO 9014837 A
- US 4912094 A
- US 5057540 A
- US 6113918 A
- US 6207646 B
- WO 0018434 A
- WO 02098368 A
- WO 02098369 A
- WO 9313302 A
- WO 9219265 A
- WO 2015040140 A1
- EP 0072513 A


### Non-patent literature cited in the description


- Remington's Pharmaceutical Sciences. Mack Publishing Co,
- *Angew. Chem. Int. Ed.,* 2013, vol. 52, 5858
- **BUNDLE D. R. et al.** *ACS Chem. Biol.,* 2012, vol. 7, 1754
- **DHÉNIN S. G. Y. et al.** *Org. Biomol. Chem.,* 2009, vol. 7, 5184
- **RAJPUT V. K.** *J. Org. Chem.,* 2008, vol. 73, 6924
- **BOURKE.** *J. Org. Biomol. Chem.,* 2014, vol. 12, 1114
- **Z. GUAN et al.** *J. Org. Chem.,* 2012, vol. 77, 8888
- **YU, H. ; ENSLEY, H.E.** *Tetrahedron Lett.,* 2003, vol. 44, 9363-9366
- **MO, K.-F. ; LI, H. ; MAGUE, J.T. ; ENSLEY, H.E.** *Carbohydr. Res.,* 2009, vol. 344, 439-447
- **BESHORE, D.C. ; DINSMORE, C.J.** *Org. Lett.,* 2002, vol. 4, 1201-1204
- **ANISH et al.** *Angew. Chem. Int. Ed.,* 2013, vol. 52, 9524-9528
- **ANISH et al.** *ACS Chem. Biol.,* 2013, vol. 8, 2412-2422
- *J Carbohyd Chem,* 1996, vol. 15 (2), 241
- *J. Org. Chem.,* 2012, vol. 77 (1), 291
- *Chem. Eur. J.,* 2013, vol. 19, 12497-12503
- *Chem. Commun.,* 2011, vol. 47, 10260-10262
- **HESTRIN.** *J. Biol. Chem.,* 1949, vol. 180, 249
- **PAUL D. HOEPRICH.** *J. Bact.,* 1955, vol. 69, 682-688
- *J. Bact.,* 1957, vol. 74, 587-590